(19) **Europäisches Patentamt**
**European Patent Office**
**Office européen des brevets**

(11) **EP 2 852 689 B1**

(12) **EUROPEAN PATENT SPECIFICATION**

(45) Date of publication and mention
of the grant of the patent:
**11.12.2019 Bulletin 2019/50**

(21) Application number: **13794236.3**

(22) Date of filing: **22.05.2013**

(51) Int Cl.:
*C12Q 1/68* (2018.01)

(86) International application number:
**PCT/US2013/042157**

(87) International publication number:
**WO 2013/177245 (28.11.2013 Gazette 2013/48)**

(54) **NANO46 GENES AND METHODS TO PREDICT BREAST CANCER OUTCOME**

NANO46-GENE UND VERFAHREN ZUR VORHERSAGE VON BRUSTKREBSERGEBNISSEN

GÈNES NANO-46 ET PROCÉDÉS DE PRÉDICTION DE L'ÉVOLUTION DU CANCER DU SEIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **22.05.2012 US 201261650209 P**
**17.01.2013 US 201361753673 P**

(43) Date of publication of application:
**01.04.2015 Bulletin 2015/14**

(73) Proprietors:
• **Nanostring Technologies, Inc.**
**Seattle, WA 98109 (US)**
• **The University of North Carolina at Chapel Hill**
**Chapel Hill, NC 27517 (US)**
• **University of Utah Research Foundation**
**Salt Lake City, UT 84108 (US)**
• **Washington University**
**Saint Louis, MO 63130 (US)**
• **British Columbia Cancer Agency Branch**
**Vancouver, British Columbia V5Z 1L3 (CA)**

(72) Inventors:
• **FERREE, Sean M.**
**Seattle, WA 98117 (US)**
• **STORHOFF, James Justin**
**Seattle, WA 98117 (US)**
• **PARKER, Joel S.**
**Apex, NC 27502 (US)**
• **PEROU, Charles M.**
**Carrboro, NC 27510 (US)**
• **ELLIS, Matthew J.**
**St. Louis, MO 63119 (US)**

• **BERNARD, Philip S.**
**Salt Lake City, UT 84105 (US)**
• **NIELSEN, Torsten O.**
**North Vancouver**
**, BC V7R 3N6 (CA)**

(74) Representative: **Cooley (UK) LLP**
**Dashwood**
**69 Old Broad Street**
**London EC2M 1QS (GB)**

(56) References cited:
**EP-A2- 1 641 810      WO-A1-2009/108215**
**WO-A1-2009/158143      WO-A1-2011/120984**
**WO-A2-2008/150512      US-A1- 2010 015 607**
**US-A1- 2011 145 176**

• **PARKER JOEL S ET AL: "Supervised risk predictor of breast cancer based on intrinsic subtypes", JOURNAL OF CLINICAL ONCOLOGY, AMERICAN SOCIETY OF CLINICAL ONCOLOGY, US, vol. 27, no. 8, 10 March 2009 (2009-03-10), pages 1160-1167, XP009124878, ISSN: 0732-183X, DOI: 10.1200/JCO.2008.18.1370**
• **MULLINS M ET AL: "Agreement in breast cancer classification between microarray and quantitative reverse transcription PCR from fresh-frozen and formalin-fixed, paraffin-embedded tissues", CLINICAL CHEMISTRY, AMERICAN ASSOCIATION FOR CLINICAL CHEMISTRY, WASHINGTON, DC, vol. 53, no. 7, 1 July 2007 (2007-07-01), pages 1273-1279, XP002553359, ISSN: 0009-9147, DOI: 10.1373/CLINCHEM.2006.083725 [retrieved on 2007-05-24]**

EP 2 852 689 B1

**(Cont. next page)**

- PLUCIENNIK ELZBIETA ET AL: "Breast cancer relapse prediction based on multi-gene RT-PCR algorithm.", MEDICAL SCIENCE MONITOR : INTERNATIONAL MEDICAL JOURNAL OF EXPERIMENTAL AND CLINICAL RESEARCH MAR 2010, vol. 16, no. 3, March 2010 (2010-03), pages CR132-CR136, XP002755989, ISSN: 1643-3750
- SORLIE T ET AL: "Gene expression patterns of breast carcinomas distinguish tumor subclasses with clinical implications", PROCEEDINGS OF THE NATIONAL ACADEMY OF SCIENCES, NATIONAL ACADEMY OF SCIENCES, US, vol. 98, no. 19, 11 September 2001 (2001-09-11), pages 10869-10874, XP002215483, ISSN: 0027-8424, DOI: 10.1073/PNAS.191367098

- "AFFYMETRIX GENECHIP HUMAN GENOME U133 ARRAY SET HG-U133A", GEO, 11 March 2002 (2002-03-11), XP002506341, [retrieved on 2002-03-11]
- VAN DE VIJVER, MJ ET AL.: 'A Gene - Expression Signature As A Predictor Of Survival In Breast Cancer.' THE NEW ENGLAND JOUMAL OF MEDICINE. vol. 347, no. 25, 19 December 2002, pages 1999 - 2009, XP008032093

**Description**

FIELD OF THE INVENTION

This disclosure relates generally to the field of cancer biology, and specifically, to the fields of detection and identification of specific cancer cell phenotypes and correlation with appropriate therapies.

BACKGROUND OF THE INVENTION

Current approaches to treating early breast cancer, including adjuvant therapy, have indeed improved survival and reduced recurrence. However, the risk of recurrence may be underestimated in some patients, but overestimated in others.

While the risk of recurrence does diminish somewhat over time, ongoing risk has been observed in many studies, some of them involving tens of thousands of patients with breast cancer. In fact, some of the patients who experienced recurrence after five years in these studies had previously been considered "low risk" - for example, their cancer had not spread to the lymph nodes at the time of their initial diagnosis, or their estrogen receptor status was positive. In one of these studies, a substantial number of recurrences occurred more than five years post-treatment. Thus, there is a need in the art to determine risk of recurrence and determine therapies which reduce that risk and improve overall survival.

WO2009/158143 and Parker Joel S et al.: "Supervised risk predictor of breast cancer based on intrinsic subtypes", J. of Clinincal Oncology, Americal Soc. of Clinical Oncology, 27: 8, pp. 1160-1167, 10 March 2019 describe methods for classifying and for evaluating the prognosis of a subject having breast cancer. The prediction model is based on a the gene expression profile of a specific set of genes. EP1641810 A2 provides gene sets, the expression of which is important in the diagnosis and/or prognosis of cancer, in particular of breast cancer. "Affymetrix Genechip Human Genome U133 Array Set HG-U133A" GEO provides probes for all the coding genes of the human cell on a microarray.

SUMMARY OF THE INVENTION

The present invention provides a method of determining low or high risk of recurrence (ROR) in a subject having breast cancer comprising: determining the size of the breast cancer tumor from the subject; determining a correlation value of a breast cancer tumor for each of at least four intrinsic subtypes including a Basal-like, Luminal A, Luminal B, or HER-2 enriched, by measuring the RNA expression of at least 40 of the genes in the NANO46 intrinsic gene list of Table 1; determining a proliferation score by measuring the RNA expression of each of the 18-gene subset of the NANO46 intrinsic genes selected from ANLN, CCNE1, CDC20, CDC6, CDCA1, CENPF, CEP55, EXO1, KIF2C, KNTC2, MELK, MKI67, ORC6L, PTTG1, RRM2, TYMS, UBE2C, and UBE2T; calculating a risk of recurrence (ROR) score using the following equation: ROR-PT = -0.0067*Basal + 0.4317*Her2 + -0.3172*LumA + 0.4894*LumB + 0.1981*ProliferationScore + 0.1133*TumorSize; wherein the risk of recurrence score is an integer value on a 0-100 scale, wherein the level of RNA expression for at least 40 of the genes in the NANO46 intrinsic gene list of Table 1 is set forth in Table 3; thereby determining whether the subject has a low or high risk of recurrence based on the risk of recurrence (ROR) score and interpretation of the ROR score using the risk classification by risk of recurrence (ROR) range and nodal status.

The method of the present invention can include determining at least one of, a combination of, or each of, the following: tumor grade, tumor ploidy, estrogen receptor expression, progesterone receptor expression, and HER2/ERBB2 expression.

The risk may be breast cancer specific survival, event-free survival, or response to therapy.

The RNA expression of the members of the NANO46 intrinsic gene list may be determined using the nanoreporter code system (nCounter® Analysis system).

The method may utilise a sampling of cells or tissues. The sample can be a tumor. The tissue can be obtained from a biopsy. The sample can be a sampling of bodily fluids. The bodily fluid can be blood, lymph, urine, saliva or nipple aspirate.

BRIEF DESCRIPTION OF THE DRAWINGS

Figure 1 is a heatmap of the breast cancer intrinsic subtypes and the intrinsic genes of Table 1.
Figure 2 shows a Kaplan Meier survival curves from a cohort of untreated breast cancer patients.
Figure 3 shows a Kaplan Meier survival curves from a cohort of node-negative, ER+ Breast Cancer Patients treated with tamoxifen.

Figure 4 shows a 10 Year event probability as a function of ROR Score in ER+, Node-negative breast cancer patients treated with tamoxifen. The graph shows the subpopulation subtyped as Luminal A or B within this population. RFS = Recurrence-free survival; DSS = disease-specific survival

Figure 5 is a schematic of the breast cancer intrinsic subtyping assay.

Figure 6 is a schematic of the algorithm process.

Figure 7 is an illustration showing the hybridization of the CodeSet to mRNA.

Figure 8 is an illustration showing the removal of excess reporters.

Figure 9 is an illustration showing the binding of the reporters to the surface of a cartridge.

Figure 10 is an illustration showing the immobilization and alignment of a reporter.

Figure 11 is an illustration of data collection.

Figure 12 is an illustration of the nCounter analysis system breast cancer test assay process.

Figure 13 is an illustration of the nCounter Prep Station.

Figure 14 is an illustration of nCounter Digital Analyzer.

## DETAILED DESCRIPTION OF THE INVENTION

The invention is defined by the appended claims. The disclosure presents a method of predicting outcome in a subject having breast cancer comprising: providing a tumor sample from the subject; determining the expression of the genes in the NANO46 intrinsic gene list of Table 1 in the tumor sample; determining the intrinsic subtype of the tumor sample based on the expression of the genes in the NANO46 intrinsic gene list, wherein the intrinsic subtype consists of at least Basal-like, Luminal A, Luminal B or HER2-enriched; determining a proliferation score based on the expression of a subset of proliferation genes in the NANO46 intrinsic gene list; determining the size of the tumor, calculating a risk of recurrence score using a weighted sum of said intrinsic subtype, proliferation score and tumor size; and determining whether the subject has a low or high risk of recurrence based on the recurrence score. In one embodiment a low score indicates a more favorable outcome and high score indicates a less favorable outcome.

Intrinsic genes are statistically selected to have low variation in expression between biological sample replicates from the same individual and high variation in expression across samples from different individuals. Thus, intrinsic genes are used as classifier genes for breast cancer classification. Although clinical information was not used to derive the breast cancer intrinsic subtypes, this classification has proved to have prognostic significance. Intrinsic gene screening can be used to classify breast cancers into five molecular distinct intrinsic subtypes, Luminal A (LumA), Luminal B (LumB), HER2-enriched (Her-2-E), Basal-like, and Normal-like (Perou et al. Nature, 406(6797):747-52 (2000); Sorlie et al. PNAS, 98(19):10869-74 (2001)).

A NANO46 gene expression assay, as described herein, can identify intrinsic subtype from a biological sample, e.g., a standard formalin fixed paraffin embedded tumor tissue. The methods utilize a supervised algorithm to classify subject samples according to breast cancer intrinsic subtype. This algorithm, referred to herein as the NANO46 classification model, is based on the gene expression profile of a defined subset of intrinsic genes that has been identified herein as superior for classifying breast cancer intrinsic subtypes. The subset of genes, along with primers target-specific sequences utilized for their detection, is provided in Table 1. Table 1A provides the sequences of target specific probe sequences for detecting each gene utilized in Table 1. The sequences provided in Table 1A are merely representative. The skilled artisan can utilize any target sequence-specific probe for detecting any of (or each of) the genes in Table 1.

**Table 1**

| GENE | REPRESENTATIVE GENBANK ACCESSION NUMBER | FORWARD PRIMER | SEQ ID NO: | REVERSE PRIMER | SEQ ID NO: |
|---|---|---|---|---|---|
| ACTR3B | NM_020445 NM_001040135 | AAAGATTCCTGGG ACCTGA | 1 | TGGGGCAGTTCTGTA TTACTTC | 47 |
| ANLN | NM_018685 | ACAGCCACTTTCA GAAGCAAG | 2 | CGATGGTTTTGTACA AGATTTCTC | 48 |
| BAG1 | NM_004323 | CTGGAAGAGTTGA ATAAAGAGC | 3 | GCAAATCCTTGGGC AGA | 49 |
| BCL2 | NM_000633 | TACCTGAACCGGC ACCTG | 4 | GCCGTACAGTTCCAC AAAGG | 50 |

(continued)

| GENE | REPRESENTATIVE GENBANK ACCESSION NUMBER | FORWARD PRIMER | SEQ ID NO: | REVERSE PRIMER | SEQ ID NO: |
|---|---|---|---|---|---|
| BLVRA | BX647539 | GCTGGCTGAGCAG AAAG | 5 | TTCCTCCATCAAGAG TTCAACA | 51 |
| CCNE1 | BC035498 | GGCCAAAATCGAC AGGAC | 6 | GGGTCTGCACAGAC TGCAT | 52 |
| CDC20 | BG256659 | CTGTCTGAGTGCC GTGGAT | 7 | TCCTTGTAATGGGGA GACCA | 53 |
| CDC6 | NM_001254 | GTAAATCACCTTC TGAGCCT | 8 | ACTTGGGATATGTGA ATAAGACC | 54 |
| CDCA1 | NM_031423 | GGAGGCGGAAGA AACCAG | 9 | GGGGAAAGACAAAG TTTCCA | 55 |
| CDH3 | BC041846 | GACAAGGAGAAT CAAAAGATCAGC | 10 | ACTGTCTGGGTCCAT GGCTA | 56 |
| CENPF | NM_016343 | GTGGCAGCAGATC ACAA | 11 | GGATTTCGTGGTGGG TTC | 57 |
| CEP55 | AB091343 | CCTCACGAATTGC TGAACTT | 12 | CCACAGTCTGTGATA AACGG | 58 |
| CXXC5 | BC006428 | CATGAAATAGTGC ATAGTTTGCC | 13 | CCATCAACATTCTCT TTATGAACG | 59 |
| EGFR | NM_005228 | ACACAGAATCTAT ACCCACCAGAGT | 14 | ATCAACTCCCAAAC GGTCAC | 60 |
| ERBB2 | NM_001005862 | GCTGGCTCTCACA CTGATAG | 15 | GCCCTTACACATCGG AGAAC | 61 |
| ESR1 | NM_001122742 | GCAGGGAGAGGA GTTTGT | 16 | GACTTCAGGGTGCTG GAC | 62 |
| FXO1 | NM_130398 | CCCATCCATGTGA GGAAGTATAA | 17 | TGTGAAGCCAGCAA TATGTATC | 63 |
| FGFR4 | AB209631 | CTTCTTGGACCTT GGCG | 18 | TATTGGGAGGCAGG AGGTTTA | 64 |
| FOXA1 | NM_004496 | GCTACTACGCAGA CACG | 19 | CTGAGTTCATGTTGC TGACC | 65 |
| FOXC1 | NM_001453 | GATGTTCGAGTCA CAGAGG | 20 | GACAGCTACTATTCC CGTT | 66 |
| GPR160 | AJ249248 | TTCGGCTGGAAGG AACC | 21 | TATGTGAGTAAGCTC GGAGAC | 67 |
| HSPC150 (UBE2T) | NM_014176 | GGAGATCCGTCAA CTCCAAA | 22 | AGTGGACATGCGAG TGGAG | 68 |
| KIF2C | NM_006845 | TGGGTCGTGTCAG GAAAC | 23 | CACCGCTGGAAACT GAAC | 69 |
| KNTC2 | NM_006101 | CGCAGTCATCCAG AGATGTG | 24 | CGTGCACATCCATGA CCTT | 70 |
| KRT14 | BC042437 | ACTCAGTACAAGA AAGAACCG | 25 | GAGGAGATGACCTT GCC | 71 |

(continued)

| GENE | REPRESENTATIVE GENBANK ACCESSION NUMBER | FORWARD PRIMER | SEQ ID NO: | REVERSE PRIMER | SEQ ID NO: |
|---|---|---|---|---|---|
| KRT17 | AK095281 | GTTGGACCAGTCAACATCTCTG | 26 | GCCATAGCCACTGCCACT | 72 |
| KRT5 | M21389 | TGTGGCTCATTAGGCAAC | 27 | CTTCGACTGGACTCTGT | 73 |
| MAPT | NM_001123066 | GACTCCAAGCGCGAAAAC | 28 | CAGACATGTTGGTATTGCACATT | 74 |
| MDM2 | M92424 | CCACAAAATATTCATGGTTCTTG | 29 | AGGCGATCCTGGGAAATTAT | 75 |
| MELK | NM_014791 | CCAGTAGCATTGTCCGAG | 30 | CCCATTTGTCTGTCTTCAC | 76 |
| MIA | BG765502 | GTCTCTGGTAATGCACACT | 31 | CTGATGGTTGAGGCTGTT | 77 |
| MKI67 | NM_002417 | GTGGAATGCCTGCTGACC | 32 | CGCACTCCAGCACCTAGAC | 78 |
| MLPH | NM_024101 | AGGGGTGCCCTCTGAGAT | 33 | TCACAGGGTCAAACTTCCAGT | 79 |
| MMP11 | NM_005940 | CGAGATCGCCAAGATGTT | 34 | GATGGTAGAGTTCCAGTGATT | 80 |
| MYC | NM_002467 | AGCCTCGAACAATTGAAGA | 35 | ACACAGATGATGGAGATGTC | 81 |
| NAT1 | BC013732 | ATCGACTGTGTAAACAACTAGAGAAGA | 36 | AGTAGCTACATCTCCAGGTTCTCTG | 82 |
| ORC6L | NM_014321 | TTTAAGAGGGCAAATGGAAGG | 37 | CGGATTTTATCAACGATGCAG | 83 |
| PGR | NM_000926 | TGCCGCAGAACTCACTTG | 38 | CATTTGCCGTCCTTCATCG | 84 |
| PHGDH | AK093306 | CCTCAGATGATGCCTATCCA | 39 | GCAGGTCAAAACTCTCAAAG | 85 |
| PTTG1 | BE904476 | CAGCAAGCGATGGCATAGT | 40 | AGCGGGCTTCTGTAATCTGA | 86 |
| RRM2 | AK123010 | AATGCCACCGAAGCCTC | 41 | GCCTCAGATTTCAACTCGT | 87 |
| SFRP1 | BC036503 | TCGAACTGAAGGCTATTTACGAG | 42 | CTGCTGAGAATCAAAGTGGGA | 88 |
| SLC39A6 | NM_012319 | GTCGAAGCCGCAATTAGG | 43 | GGAACAAACTGCTCTGCCA | 89 |
| TMEM45B | AK098106 | CAAACGTGTGTTCTGGAGG | 44 | ACAGCTCTTTAGCATTTGTGGA | 90 |
| TYMS | BQ56428 | TGCCCTGTATGATGTCAGGA | 45 | GGGACTATCAATGTTGGGTTCTC | 91 |

(continued)

| GENE | REPRESENTATIVE GENBANK ACCESSION NUMBER | FORWARD PRIMER | SEQ ID NO: | REVERSE PRIMER | SEQ ID NO: |
|------|------------------------------------------|----------------|------------|----------------|------------|
| UBE2C | BC032677 | GTGAGGGGTGTCA GCTCAGT | 46 | CACACAGTTCACTGC TCCACA | 92 |

Table 1a. Probes for detecting NANO46 genes

| Gene Name | RefSeq Accession | Target Sequence | SEQ ID NO: |
|-----------|------------------|-----------------|------------|
| ACTR3B | NM_001040135.1 | CCAGAAGAAGTTTGTTATAGACGTTGGTTACG AAAGATTCCTGGGACCTGAAATATTCTTTCAC CCGGAGTTTGCCAACCCAGACTTTATGGAGTC CATC | 140 |
| ANLN | NM_018685.2 | CGTGCCAGGCGAGAGAATCTTCAGAGAAAAA TGGCTGAGAGGCCCACAGCAGCTCCAAGGTC TATGACTCATGCTAAGCGAGCTAGACAGCCA CTTTCAG | 141 |
| BAG1 | NM_004323.3 | CTTCATGTTACCTCCCAGCAGGGCAGCAGTGA ACCAGTTGTCCAAGACCTGGCCCAGGTTGTTG AAGAGGTCATAGGGGTTCCACAGTCTTTTCAG AAAC | 142 |
| BCL2 | NM_000633.2 | CCAAGCACCGCTTCGTGTGGCTCCACCTGGAT GTTCTGTGCCTGTAAACATAGATTCGCTTTCC ATGTTGTTGGCCGGATCACCATCTGAAGAGCA GACG | 143 |
| BLVRA | NM_000712.3 | TTCCTGAAAAAAGAAGTGGTGGGGAAAGACC TGCTGAAAGGGTCGCTCCTCTTCACAGCTGGC CCGTTGGAAGAAGAGCGGTTTGGCTTCCCTGC ATTCA | 144 |
| CCNE1 | NM_001238.1 | GAGAACTGTGTCAAGTGGATGGTTCCATTTGC CATGGTTATAAGGGAGACGGGGAGCTCAAAA CTGAAGCACTTCAGGGGCGTCGCTGATGAAG ATGCAC | 145 |
| CDC20 | NM_001255.1 | CCCGAGTGGGCTCCCTAAGCTGGAACAGCTA TATCCTGTCCAGTGGTTCACGTTCTGGCCACA TCCACCACCATGATGTTCGGGTAGCAGAACA CCATGT | 146 |
| CDC6 | NM_001254.3 | GGGGAAGTTATATGAAGCCTACAGTAAAGTC TGTCGCAAACAGCAGGTGGCGGCTGTGGACC AGTCAGAGTGTTTGTCACTTTCAGGGCTCTTG GAAGCC | 147 |
| CDCA1 | NM_145697.1 | GCCTGGCGGTGTTTTCGTCGTGCTCAGCGGTG GGAGGAGGCGGAAGAAACCAGAGCCTGGGA GATTAACAGGAAACTTCCAAGATGGAAACTT TGTCTTT | 148 |
| CDH3 | NM_001793.3 | CCCTCGACCGTGAGGATGAGCAGTTTGTGAG GAACAACATCTATGAAGTCATGGTCTTGGCCA TGGACAATGGAAGCCCTCCCACCACTGGCAC GGGAAC | 149 |

7

(continued)

| Gene Name | RefSeq Accession | Target Sequence | SEQ ID NO: |
|---|---|---|---|
| CENPF | NM_016343.3 | AGAAAATCTTGCAGAGTCCTCCAAACCAACA GCTGGTGGCAGCAGATCACAAAAGGTCAAAG TTGCTCAGCGGAGCCCAGTAGATTCAGGCAC CATCCTC | 150 |
| CEP55 | NM_018131.3 | GTACTACCGCATTGCTTGAACAGCTGGAAGA GACAACGAGAGAAGGAGAAAGGAGGGAGCA GGTGTTGAAAGCCTTATCTGAAGAGAAAGAC GTATTGAA | 151 |
| CXXC5 | NM_016463.5 | AGCTGCCCTCTCCGTGCAATGTCACTGCTCGT GTGGTCTCCAGCAAGGGATTCGGGCGAAGAC AAACGGATGCACCCGTCTTTAGAACCAAAAA TATTCT | 152 |
| EGFR | NM_005228.3 | GCAGCCAGGAACGTACTGGTGAAAACACCGC AGCATGTCAAGATCACAGATTTTGGGCTGGCC AAACTGCTGGGTGCGGAAGAGAAAGAATACC ATGCAG | 153 |
| ERBB2 | NM_004448.2 | TGAAGGTGCTTGGATCTGGCGCTTTTGGCACA GTCTACAAGGGCATCTGGATCCCTGATGGGG AGAATGTGAAAATTCCAGTGGCCATCAAAGT GTTGAG | 154 |
| ESR1 | NM_000125.2 | AGGAACCAGGGAAAATGTGTAGAGGGCATGG TGGAGATCTTCGACATGCTGCTGGCTACATCA TCTCGGTTCCGCATGATGAATCTGCAGGGAGA GGAGT | 155 |
| EXO1 | NM_006027.3 | TGGCCCACAAAGTAATTAAAGCTGCCCGGTCT CAGGGGGTAGATTGCCTCGTGGCTCCCTATGA AGCTGATGCGCAGTTGGCCTATCTTAACAAAG CGGG | 156 |
| FGFR4 | NM_002011.3 | CCCACATCCAGTGGCTGAAGCACATCGTCATC AACGGCAGCAGCTTCGGAGCCGACGGTTTCC CCTATGTGCAAGTCCTAAAGACTGCAGACATC AATAG | 157 |
| FOXA1 | NM_004496.2 | TGGATGGTTGTATTGGGCAGGGTGGCTCCAG GATGTTAGGAACTGTGAAGATGGAAGGGCAT GAAACCAGCGACTGGAACAGCTACTACGCAG ACACGCA | 158 |
| FOXC1 | NM_001453.1 | TTCGAGTCACAGAGGATCGGCTTGAACAACT CTCCAGTGAACGGGAATAGTAGCTGTCAAAT GGCCTTCCCTTCCAGCCAGTCTCTGTACCGCA CGTCCG | 159 |
| GPR160 | NM_014373.1 | GGATTTCAGTCCTTGCTTATGTTTTGGGAGAC CCAGCCATCTACCAAAGCCTGAAGGCACAGA ATGCTTATTCTCGTCACTGTCCTTTCTATGTCA GCAT | 160 |
| UBE2T | NM_014176.1 | GTGTCAGCTCAGTGCATCCCAGGCAGCTCTTA GTGTGGAGCAGTGAACTGTGTGTGGTTCCTTC TACTTGGGGATCATGCAGAGAGCTTCACGTCT GAAG | 161 |

(continued)

| Gene Name | RefSeq Accession | Target Sequence | SEQ ID NO: |
|---|---|---|---|
| KIF2C | NM_006845.2 | GTTGTCTACAGGTTCACAGCAAGGCCACTGGT ACAGACAATCTTTGAAGGTGGAAAAGCAACT TGTTTTGCATATGGCCAGACAGGAAGTGGCA AGACAC | 162 |
| KNTC2 | NM_006101.1 | AAAAGGTCATAAGCATGAAGCGCAGTTCAGT TTCCAGCGGTGGTGCTGGCCGCCTCTCCATGC AGGAGTTAAGATCCCAGGATGTAAATAAACA AGGCCT | 163 |
| KRT14 | NM_000526.3 | GCAGTCATCCAGAGATGTGACCTCCTCCAGCC GCCAAATCCGCACCAAGGTCATGGATGTGCA CGATGGCAAGGTGGTGTCCACCCACGAGCAG GTCCTT | 164 |
| KRT17 | NM_000422.1 | CTGACTCAGTACAAGAAAGAACCGGTGACCA CCCGTCAGGTGCGTACCATTGTGGAAGAGGT CCAGGATGGCAAGGTCATCTCCTCCCGCGAG CAGGTCC | 165 |
| KRT5 | NM_000424.2 | CTGGTTCTCTTGCTCCACCAGGAACAAGCCAC CATGTCTCGCCAGTCAAGTGTGTCCTTCCGGA GCGGGGGCAGTCGTAGCTTCAGCACCGCCTCT GCCA | 166 |
| MAPT | NM_016835.3 | GCCGGGTCCCTCAACTCAAAGCTCGCATGGTC AGTAAAAGCAAAGACGGGACTGGAAGCGATG ACAAAAAAGCCAAGACATCCACACGTTCCTC TGCTAA | 167 |
| MDM2 | NM_006878.2 | GGTGAGGAGCAGGCAAATGTGCAATACCAAC ATGTCTGTACCTACTGATGGTGCTGTAACCAC CTCACAGATTCCAGCTTCGGAACAAGAGACC CTGGTT | 168 |
| MELK | NM_014791.2 | AGAGACAGCCAACAAAATATTCATGGTTCTT GAGTACTGCCCTGGAGGAGAGCTGTTTGACT ATATAATTTCCCAGGATCGCCTGTCAGAAGAG GAGACC | 169 |
| MIA | NM_006533.1 | CCGGGGCCAAGTGGTGTATGTCTTCTCCAAGC TGAAGGGCCGTGGGCGGCTCTTCTGGGGAGG CAGCGTTCAGGGAGATTACTATGGAGATCTG GCTGCT | 170 |
| MKI67 | NM_002417.2 | GCTTCCAGCAGCAAATCTCAGACAGAGGTTC CTAAGAGAGGAGGAGAAAGAGTGGCAACCTG CCTTCAAAAGAGAGTGTCTATCAGCCGAAGT CAACATG | 171 |
| MLPH | NM_024101.4 | GAGGAAGTCAAACCTCCCGATATTTCTCCCTC GAGTGGCTGGGAAACTTGGCAAGAGACCAGA GGACCCAAATGCAGACCCTTCAAGTGAGGCC AAGGCA | 172 |
| MMP11 | NM_005940.3 | AGCAGCCAAGGCCCTGATGTCCGCCTTCTACA CCTTTCGCTACCCACTGAGTCTCAGCCCAGAT GACTGCAGGGGCGTTCAACACCTATATGGCC AGCCC | 173 |

(continued)

| Gene Name | RefSeq Accession | Target Sequence | SEQ ID NO: |
|---|---|---|---|
| MYC | NM_002467.3 | CACCGAGGAGAATGTCAAGAGGCGAACACAC AACGTCTTGGAGCGCCAGAGGAGGAACGAGC TAAAACGGAGCTTTTTTGCCCTGCGTGACCAG ATCCCG | 174 |
| NAT1 | NM_000662.4 | AGCACTTCCTCATAGACCTTGGATGTGGGAGG ATTGCATTCAGTCTAGTTCCTGGTTGCCGGCT GAAATAACCTGAATTCAAGCCAGGAAGAAGC AGCAA | 175 |
| ORC6L | NM_014321.2 | GACTGTGTAAACAACTAGAGAAGATTGGACA GCAGGTCGACAGAGAACCTGGAGATGTAGCT ACTCCACCACGGAAGAGAAAGAAGATAGTGG TTGAAGC | 176 |
| PGR | NM_000926.2 | GGGATGAAGCATCAGGCTGTCATTATGGTGTC CTTACCTGTGGGAGCTGTAAGGTCTTCTTTAA GAGGGCAATGGAAGGGCAGCACAACTACTTA TGTGC | 177 |
| PHGDH | NM_006623.2 | GCGACGGCTTCGATGAAGGACGGCAAATGGG AGCGGAAGAAGTTCATGGGAACAGAGCTGAA TGGAAAGACCCTGGGAATTCTTGGCCTGGGC AGGATTG | 178 |
| PTTG1 | NM_004219.2 | CACCAGCCTTACCTAAAGCTACTAGAAAGGC TTTGGGAACTGTCAACAGAGCTACAGAAAAG TCTGTAAAGACCAAGGGACCCCTCAAACAAA AACAGCC | 179 |
| RRM2 | NM_001034.1 | TTCCTTTTGGACCGCCGAGGAGGTTGACCTCT CCAAGGACATTCAGCACTGGGAATCCCTGAA ACCCGAGGAGAGATATTTTATATCCCATGTTC TGGCT | 180 |
| SFRP1 | NM_003012.3 | GTGGGTCACACACACGCACTGCGCCTGTCAGT AGTGGACATTGTAATCCAGTCGGCTTGTTCTT GCAGCATTCCCGCTCCCTTCCCTCCATAGCCA CGCT | 181 |
| SLC39A6 | NM_012319.2 | GATCGAACTGAAGGCTATTTACGAGCAGACT CACAAGAGCCCTCCCACTTTGATTCTCAGCAG CCTGCAGTCTTGGAAGAAGAAGAGGTCATGA TAGCTC | 182 |
| TMEM45B | NM_138788.3 | CTGGCTGCCCTCAGCATTGTGGCCGTCAACTA TTCTCTTGTTTACTGCCTTTTGACTCGGATGAA GAGACACGGAAGGGGAGAAATCATTGGAATT CAGA | 183 |
| TYMS | NM_001071.1 | TGCTAAAGAGCTGTCTTCCAAGGGAGTGAAA ATCTGGGATGCCAATGGATCCCGAGACTTTTT GGACAGCCTGGGATTCTCCACCAGAGAAGAA GGGGAC | 184 |
| UBE2C | NM_007019.2 | GTCTGCCCTGTATGATGTCAGGACCATTCTGC TCTCCATCCAGAGCCTTCTAGGAGAACCCAAC ATTGATAGTCCCTTGAACACACATGCTGCCGA GCTC | 185 |

Table 2 provides select sequences for the NANO46 genes of Table 1.

**Table 2**

| GENBANK ACCESSION NUMBER | SEQUENCE | SEQ ID NO: |
|---|---|---|
| NM_020445 | CAGCGGCGCTGCGGCGGCTCGCGGGAGACGCTGCGCGCGGGGCTAGCGGGCGGCGGAGCGGACGGCGACG GGGCGCTCTCGGGCTGCCGGCGGGGCCGAGCGCCGCGCGTCCCGAGCATGGCAGGCTCCCTGCCTCCCTG CGTGGTGGACTGTGGCACCGGGTATACCAAGCTTGGCTACGCAGGCAACACTGAGCCCCAGTTCATTATT CCTTCATGTATTGCCATCAGAGAGTCAGCAAAGGTAGTTGACCAAGCTCAAAGGAGAGTGTTGAGGGGAG TTGATGACCTTGACTTTTTCATAGGAGATGAAGCCATCGATAAACCTACATATGCTACAAAGTGGCCGAT ACGACATGGAATCATTGAAGACTGGGATCTTATGGAAAGGTTCATGGAGCAAGTGGTTTTTAAATATCTT CGAGCTGAACCTGAGGACCATTATTTTTTAATGACAGAACCTCCACTCAATACACCAGAAAACAGAGAGT ATCTTGCAGAAATTATGTTTGAATCATTTAACGTACCAGGACTCTACATTGCAGTTCAGGCAGTGCTGGC CTTGGCGGCATCTTGGACATCTCGACAAGTGGGTGAACGTACGTTAACGGGGATAGTCATTGACAGCGGA GATGGAGTCACCCATGTTATCCCAGTGGCAGAAGGTTATGTAATTGGAAGCTGCATCAAACACATCCCGA TTGCAGGTAGAGATATTACGTATTTCATTCAACAGCTGCTAAGGGAGAGGGAGGTGGGAATCCCTCCTGA GCAGTCACTGGAGACCGCAAAAGCCATTAAGGAGAAATACTGTTACATTTGCCCCGATATAGTCAAGGAA TTTGCCAAGTATGATGTGGATCCCCGGAAGTGGATCAAACAGTACACGGGTATCAATGCGATCAACCAGA AGAAGTTTGTTATAGACGTTGGTTACGAAAGATTCCTGGGACCTGAAATATTCTTTCACCCGGAGTTTGC CAACCCAGACTTTATGGAGTCCATCTCAGATGTTGTTGATGAAGTAATACAGAACTGCCCCATCGATGTG CGGCGCCCCGCTGTATAAGAATGTCGTACTCTCAGGAGGCTCCACCATGTTCAGGGATTCGGACGCCGAC TGCAGAGGGATTGAAGAGAGTGGTGGATGCTAGGCTGAGGCTCAGCGAGGAGCTCAGCGGCGGGAGGAT CAAGCCGAAGCCTGTGGAGGTCCAGGTGGTCACGCATCACATGCAGCGCTACGCCGTGTGGTTCGGAGGC TCCATGCTGGCCTCGACTCCCGAGTTCTTTCAGGTCTGCCACACCAAGAAGGACTATGAAGAGTACGGGC CCAGCATCTGCCGCCACAACCCCGTCTTTGGAGTCATGTCCTAGTGTCTGCCTGAACGCGTCGTTCGATG GTGTCACGTTGGGGAACAAGTGTCCTTCAGAACCCAGAGAAGGCCGCCGTTCTGTAAATAGCGACGTCGG TGTTGCTGCCCAGCAGCGTGCTTGCATTGCCGGTGCATGAGGCGCGGCGCGGGCCCTTCAGTAAAAGCCA TTTATCCGTGTGCCGACCGCTGTCTGCCAGCCTCCTCCTTCTCCCGCCCTCCTCACCCTCGCTCTCCCTC CTCCTCCTCCTCCGAGCTGCTAGCTGACAAATACAATTCTGAAGGAATCCAAATGTGACTTTGAAAATTG TTAGAGAAAACAACATTAGAAAATGGCGCAAAATCGTTAGGTCCCAGGAGAGAATGTGGGGGCGCAAACC CTTTTCCTCCCAGCCTATTTTTGTAAATAAAATGTTTAAACTTGAAATACAAATCGATGTTTATATTTCC TATCATTTTGTATTTTATGGTATTTGGTACAACTGGCTGATACTAAGCACGAATAGATATTGATGTTATG GAGTGCTGTAATCCAAAGTTTTTAATTGTGAGGCATGTTCTGATATGTTTATAGGCAAACAAATAAAACA GCAAACTTTTTTGCCACATGTTTGCTAGAAAATGATTATACTTTATTGGAGTGACATGAAGTTTGAACAC TAAACAGTAATGTATGAGAATTACTACAGATACATGTATCTTTTAGTTTTTTTTGTTTGAACTTTCTGGA GCTGTTTTATAGAAGATGATGGTTTGTTGTCGGTGAGTGTTGGATGAAATACTTCCTTGCACCATTGTAA TAAAAGCTGTTAGAATATTTGTAAATATC | 93 |
| NM_ 001040135 | CAGCGGCGCTGCGGCGGCTCGCGGGAGACGCTGCGCGCGGGGCTAGCGGGCGGCGGAGCGGACGGCGACG GGGCGCTCTCGGGCTGCCGGCGGGGCCGAGCGCCGCGCGTCCCGAGCATGGCAGGCTCCCTGCCTCCCTG CGTGGTGGACTGTGGCACCGGGTATACCAAGCTTGGCTACGCAGGCAACACTGAGCCCCAGTTCATTATT CCTTCATGTATTGCCATCAGAGAGTCAGCAAAGGTAGTTGACCAAGCTCAAAGGAGAGTGTTGAGGGGAG TTGATGACCTTGACTTTTTCATAGGAGATGAAGCCATCGATAAACCTACATATGCTACAAAGTGGCCGAT ACGACATGGAATCATTGAAGACTGGGATCTTATGGAAAGGTTCATGGAGCAAGTGGTTTTTAAATATCTT CGAGCTGAACCTGAGGACCATTATTTTTTAATGACAGAACCTCCACTCAATACACCAGAAAACAGAGAGT ATCTTGCAGAAATTATGTTTGAATCATTTAACGTACCAGGACTCTACATTGCAGTTCAGGCAGTGCTGGC CTTGGCGGCATCTTGGACATCTCGACAAGTGGGTGAACGTACGTTAACGGGGATAGTCATTGACAGCGGA GATGGAGTCACCCATGTTATCCCAGTGGCAGAAGGTTATGTAATTGGAAGCTGCATCAAACACATCCCGA TTGCAGGTAGAGATATTACGTATTTCATTCAACAGCTGCTAAGGGAGAGGGAGGTGGGAATCCCTCCTGA GCAGTCACTGGAGACCGCAAAAGCCATTAAGGAGAAATACTGTTACATTTGCCCCGATATAGTCAAGGAA TTTGCCAAGTATGATGTGGATCCCCGGAAGTGGATCAAACAGTACACGGGTATCAATGCGATCAACCAGA AGAAGTTTGTTATAGACGTTGGTTACGAAAGATTCCTGGGACCTGAAATATTCTTTCACCCGGAGTTTGC CAACCCAGACTTTATGGAGTCCATCTCAGATGTTGTTGATGAAGTAATACAGAACTGCCCCATCGATGTG CGGCGCCCGCTGTATAAGCCCGAGTTCTTTCAGGTCTGCCACACCAAGAAGGACTATGAAGAGTACGGGC CCAGCATCTGCCGCCACAACCCCGTCTTTGGAGTCATGTCCTAGTGTCTGCCTGAACGCGTCGTTCGATG GTGTCACGTTGGGGAACAAGTGTCCTTCAGAACCCAGAGAAGGCCGCCGTTCTGTAAATAGCGACGTCGG TGTTGCTGCCCAGCAGCGTGCTTGCATTGCCGGTGCATGAGGCGCGGCGCGGGCCCTTCAGTAAAAGCCA TTTATCCGTGTGCCGACCGCTGTCTGCCAGCCTCCTCCTTCTCCCGCCCTCCTCACCCTCGCTCTCCCTC CTCCTCCTCCTCCGAGCTGCTAGCTGACAAATACAATTCTGAAGGAATCCAAATGTGACTTTGAAAATTG TTAGAGAAAACAACATTAGAAAATGGCGCAAAATCGTTAGGTCCCAGGAGAGAATGTGGGGGCGCAAACC CTTTTCCTCCCAGCCTATTTTTGTAAATAAAATGTTTAAACTTGAAATACAAATCGATGTTTATATTTCC TATCATTTTGTATTTTATGGTATTTGGTACAACTGGCTGATACTAAGCACGAATAGATATTGATGTTATG GAGTGCTGTAATCCAAAGTTTTTAATTGTGAGGCATGTTCTGATATGTTTATAGGCAAACAAATAAAACA GCAAACTTTTTTGCCACATGTTTGCTAGAAAATGATTATACTTTATTGGAGTGACATGAAGTTTGAACAC TAAACAGTAATGTATGAGAATTACTACAGATACATGTATCTTTTAGTTTTTTTTGTTTGAACTTTCTGGA GCTGTTTTATAGAAGATGATGGTTTGTTGTCGGTGAGTGTTGGATGAAATACTTCCTTGCACCATTGTAA TAAAAGCTGTTAGAATATTTGTAAATATC | 94 |

(continued)

| GENBANK ACCESSION NUMBER | SEQUENCE | SEQ ID NO: |
|---|---|---|
| NM_018685 | CTCGGCGCTGAAATTCAAATTTGAACGGCTGCAGAGGCCGAGTCCGTCACTGGAAGCCGAGAGGAGAGGA<br>CAGCTGGTTGTGGGAGAGTTCCCCCGCCTCAGACTCCTGGTTTTTTCCAGGAGACACACTGAGCTGAGAC<br>TCACTTTTCTCTTCCTGAATTTGAACCACCGTTTCCATCGTCTCGTAGTCCGACGCCTGGGGCGATGGAT<br>CCGTTTACGGAGAAACTGCTGGAGCGAACCCGTGCCAGGCGAGAGAATCTTCAGAGAAAAATGGCTGAGA<br>GGCCCACAGCAGCTCCAAGGTCTATGACTCATGCTAAGCGAGCTAGACAGCCACTTTCAGAAGCAAGTAA<br>CCAGCAGCCCCTCTCTGGTGGTGAAGAGAAATCTTGTACAAAACCATCGCCATCAAAAAAAACGCTGTTCT<br>GACAACACTGAAGTAGAAGTTTCTAACTTGGAAAATAAACAACCAGTTGAGTCGACATCTGCAAAATCTT<br>GTTCTCCAAGTCCTGTGTCTCCTCAGGTGCAGCCACAAGCAGCAGATACCATCAGTGATTCTGTTGCTGT<br>CCCGGCATCACTGCTGGGCATGAGGAGAGGGCTGAACTCAAGATTGGAAGCAACTGCAGCCTCCTCAGTT<br>AAAACACGTATGCAAAAACTTGCAGAGCAACGGCGCCGTTGGGATAATGATGATATGACAGATGACATTC<br>CTGAAAGCTCACTCTTCTCACCAATGCCATCAGAGGAAAAGGCTGCTTCCCCTCCCAGACCTCTGCTTTC<br>AAATGCCTCGGCAACTCCAGTGGCAGAAGGGGCCGTCTGGCCAATCTTGCTGCAACTATTTGCTCCTGG<br>GAAGATGATGTAAATCACTCATTTGCAAACAAAACAGTGTACAAGAACAGCCTGGTACCGCTTGTTTAT<br>CCAAATTTTCCTCTGCAAGTGGAGCATCTGCTAGGATCAATAGCAGCAGTGTTAAGCAGGAAGCTACATT<br>CTGTTCCCAAAGGGATGGCGATGCCTCTTTGAATAAAGCCCTATCCTCAAGTGCTGATGATGCGTCTTTG<br>GTTAATGCCTCAATTTCCAGCTCTGTGAAAGCTACTTCTCCAGTGAAATCTACTACATCTATCACTGATG<br>CTAAAAGTTGTGAGGGACAAAATCCTGAGCTACTTCCAAAAACTCCTATTAGTCCTCTGAAAACGGGGGT<br>ATCGAAACCAATTGTGAAGTCAACTTTATCCCAGACAGTTCCATCCAAGGGAGAATTAAGTAGAGAAATT<br>TGTCTGCAATCTCAATCTAAAGACAAATCTACGACACCAGGAGGAACAGGAATTAAGCCTTTCCTGGAAC<br>GCTTTGGGAGAGCGTTGTCAAGAACATAGCAAAGAAAGTCCAGCTCGTAGCACACCCCACAGAACCCCCAT<br>TATTACTCCAAATACAAAGGCCATCCAAGAAAGATTATTCAAGCAAGACACATCTTCATCTACTACCCAT<br>TTAGCACAACAGCTCAAGCAGGAACGTCAAAAAGAACTAGCATGTCTTCGTGGCCGATTTGACAAGGGCA<br>ATATATGGAGTGCAGAAAAAGGCGGAAACTCAAAAAGCAAACAACTAGAAACCAAACAGGAAACTCACTG<br>TCAGAGCACTCCCCTCAAAAAAACACCAAGGTGTTTCAAAAACTCAGTCACTTCCAGTAACAGAAAAGGTG<br>ACCGAAAACCAGATACCAGCCAAAAATTCTAGTACAGAACCTAAAGGTTTCACTGAATGCGAAATGACGA<br>AATCTAGCCCTTTGAAAATAACATTGTTTTTAGAAGAGGACAAATCCTTAAAAGTAACATCAGACCCAAA<br>GGTTGAGCAGAAAATTGAAGTGATACGTGAAATTGAGATGAGTGTGGATGATGATGATATCAATAGTTCG<br>AAAGTAATTAATGACCTCTTCAGTGATGTCCTAGAGGAAGGTGAACTAGATATGGAGAAGAGCCAAGAGG<br>AGATGGATCAAGCATTAGCAGAAAGCAGCGAAGAACAGGAAGATGCACTGAATATCTCCTCAATGTCTTT<br>ACTTGCACCATTGGCACAAACAGTTGGTGTGGTAAGTCCAGAGAGTTTAGTGTCCACACCTAGACTGGAA<br>TTGAAAGACACCAGCAGAAGTGATGAAAGTCCAAAACCAGGAAAATTCCAAAGAACTCGTGTCCCTCGAG<br>CTGAATCTGGTGATAGCCTTGGTTCTGAAGATCGTGATCTTCTTTACAGCATTGATGCATATAGATCTCA<br>AAGATTCAAAGAAACAGAACGTCCATCAATAAAGCAGGTGATTGTTCGGAAGGAAGATGTTACTTCAAAA<br>CTGGATGAAAAAAATAATGCCTTTCCTTGTCAAGTTAATATCAAACAGAAAATGCAGGAACTCAATAACG<br>AAATAAATATGCAACAGACAGTGATCTATCAAGCTAGCCAGGCTCTTAACTGCTGTGTTGATGAAGAACA<br>TGGAAAAGGGTCCCTAGAAGAAGCTGAAGCAGAAAGACTTCTTCTAATTGCAACTGGGAAGAGAACACTT<br>TTGATTGATGAATTGAATAAATTGAAGAACGAAGGACCTCAGAGGAAGAATAAGGCTAGTCCCCAAAGTG<br>AATTTATGCCATCCAAAGGATCAGTTACTTTGTCAGAAATCCGCTTGCCTCTAAAAGCAGATTTTGTCTG<br>CAGTACGGTTCAGAAACCAGATGCAGCAAATTACTATTACTTAATTATACTAAAAGCAGGAGCTGAAAAT<br>ATGGTAGCCACACCATTAGCAAGTACTTCAAACTCTCTTAACGGTGATGCTCTGACATTCACTACTACAT<br>TTACTCTGCAAGATGTATCCAATGACTTTGAAATAAATATTGAAGTTTACAGCTTGGTGCAAAAGAAAGA<br>TCCCTCAGGCCTTGATAAGAAGAAAAAAACATCCAAGTCCAAGGCTATTACTCCAAAGCGACTCCTCACA<br>TCTATAACCACAAAAAGCAACATTCATTCTTCAGTCATGGCCAGTCCAGGAGGTCTTAGTGCTGTGCGAA<br>CCAGCAACTTCGCCCTTGTTGGATCTTACACATTATCATTGTCTTCAGTAGGAAATACTAAGTTTGTTCT<br>GGACAAGGTCCCCTTTTTATCTTCTTTGGAAGGTCATATTTATTTAAAAATAAAATGTCAAGTGAATTCC<br>AGTGTTGAAGAAAGAGGTTTTCTAACCATATTTGAAGATGTTAGTGGTTTTGGTGCCTGGCATCGAAGAT<br>GGTGTGTTCTTTCTGGAAACTGTATATCTTATTGGACTTATCCAGATGATGAGAAACGCAAGAATCCCAT<br>AGGAAGGATAAATCTGGCTAATTGTACCAGTCGTCAGATAGAACCAGCCAACAGAGAATTTGTGCAAGA<br>CGCAACACTTTTGAATTAATTACTGTCCGACCACAAAGAGAAGATGACCGAGAGACTCTTGTCAGCCAAT<br>GCAGGGACACACTCTGTGTTACCAAGAACTGGCTGTCTGCAGATACTAAAGAAGAGCGGGATCTCTGGAT<br>GCAAAAACTCAATCAAGTTCTTGTTGATATTCGCCTCTGGCAACCTGATGCTTGCTACAAACCTATTGGA | 95 |

| GENBANK ACCESSION NUMBER | SEQUENCE | SEQ ID NO: |
|---|---|---|
| | AAGCCTTAAACCGGGAAATTTCCATGCTATCTAGAGGTTTTTGATGTCATCTTAAGAAACACACTTAAGAGCATCAGATTTACTGATTGCATTTTATGCTTTAAGTACGAAAGGGTTTGTGCCAATATTCACTACGTATTATGCAGTATTTATATCTTTTGTATGTAAAACTTTAACTGATTTCTGTCATTCATCAATGAGTAGAAGTAAATACATTATAGTTGATTTTGCTAAATCTTAATTTAAAAGCCTCATTTTCCTAGAAATCTAATTATTCAGTTATTCATGACAATATTTTTTTAAAAGTAAGAAATTCTGAGTTGTCTTCTTGGAGCTGTAGGTCTTGAAGCAGCAACGTCTTTCAGGGGTTGGAGACAGAAACCCATTCTCCAATCTCAGTAGTTTTTTCGAAAGGCTGTGATCATTTATTGATCGTGATATGACTTGTTACTAGGGTACTGAAAAAAATGTCTAAGGCCTTTACAGAAACATTTTTAGTAATGAGGATGAGAACTTTTTCAAATAGCAAATATATATTGGCTTAAAGCATGAGGCTGTCTTCAGAAAAGTGATGTGGACATAGGAGGCAATGTGTGAGACTTGGGGGTTCAATATTTTATATAGAAGAGTTAATAAGCACATGGTTTACATTTACTCAGCTACTATATATGCAGTGTGGTGCACATTTTCACAGAATTCTGGCTTCATTAAGATCATTATTTTTGCTGCGTAGCTTACAGACTTAGCATATTAGTTTTTTCTACTCCTACAAGTGTAAATTGAAAAATCTTTATATTAAAAAAAGTAAACTGTTATGAAGCTGCTATGTACTAATAATACTTTGCTTGCCAAAGTGTTTGGGTTTTGTTGTTGTTTGTTTGTTTGTTTTTGGTTCATGAACAACAGTGTCTAGAAACCCATTTTGAAAGTGGAAAATTATTAAGTCACCTATCACCTTTAAACGCCTTTTTTTAAAATTATAAAATATTGTAAAGCAGGGTCTCAACTTTTAAATACACTTTGAACTTCTTCTCTGAATTATTAAAGTTCTTTATGACCTCATTTATAAACACTAAATTCTGTCACCTCCTGTCATTTTATTTTTTATTCATTCAAATGTATTTTTTCTTGTGCATATTATAAAAAATATATTTTATGAGCTCTTACTCAAATAAATACCTGTAAATGTCTAAAGGAAAAAAAAAAAAAAAAAAAA | |
| NM_004323 | AGGCCGGGGCGGGGCTGGGAAGTAGTCGGGCGGGGTTGTGAGACGCCGCGCTCAGCTTCCATCGCTGGGCGGTCAACAAGTGCGGGCCTGGCTCAGCGCGGGGGGCGCGGAGACCGCGAGGCGACCGGGAGCGGCTGGGTTCCCGGCTGCGCGCCCTTCGGCCAGGCGGGAGCCGCGCAGTCGGAGCCCCCGGCCCAGCGTGGTCCGCCTCCCTCTCGGCGTCCACCTGCCCGGAGTACTGCCAGCGGGCATGACCGACCCACCAGGGGCGCCGCCGCCGGCGCTCGCAGGCCGCGGATGAAGAAGAAAACCCGGCGCCGCTCGACCCGGAGCGAGGAGTTGACCCGGAGCGAGGAGTTGACCCTGAGTGAGGAAGCGACCTGGAGTGAAGAGGCGACCCAGAGTGAGGAGGCGACCCAGGGGCGAAGAGATGAATCGGAGCCAGGAGGTGACCCGGGACGAGGAGTCGACCCGGAGCGAGGAGGTGACCAGGGAGGAAATGGCGGCAGCTGGGCTCACCGTGACTGTCACCCACAGCAATGAGAAGCACGACCTTCATGTTACCTCCCAGCAGGGCAGCAGTGAACCAGTTGTCCAAGACCTGGCCCAGGTTGTTGAAGAGGTCATAGGGGTTCCACAGTCTTTTCAGAAACTCATATTTAAGGGAAAATCTCTGAAGGAAATGGAAACACCGTTGTCAGCACTTGGAATACAAGATGGTTGCCGGGTCATGTTAATTGGGAAAAAGAACAGTCCACAGGAAGAGGTTGAACTAAAGAAGTTGAAACATTTGGAGAAGTCTGTGGAGAAGATAGCTGACCAGCTGGAAGAGTTGAATAAAGAGCTTACTGGAATCCAGCAGGGTTTTCTGCCCAAGGATTGCAAGCTGAAGCTCTCTGCAAACTTGATAGGAGAGTAAAAGCCACAATAGAGCAGTTTATGAAGATCTTGGAGGAGATTGACACACTGATCCTGCCAGAAAATTTCAAAGACAGTAGATTGAAAAGGAAAGGCTTGGTAAAAAAGGTTCAGGCATTCCTAGCCGAGTGTGACACAGTGGAGCAGAACATCTGCCAGGAGACTGAGCGGCTGCAGTCTACAAACTTTGCCCTGGCCGAGTGAGGTGTAGCAGAAAAAGGCTGTGCTGCCCTGAAGAATGGCGCCACCAGCTCTGCCGTCTCTGGGAGCGGAATTTACCTGATTTCTTCAGGGCTGCTGGGGGGCAACTGGCCATTTGCCAATTTTCCTACTCTCACACTGGTTCTCAATGAAAAATAGTGTCTTTGTGATTTTGAGTAAAGCTCCTATCTGTTTTCTCCTTCTGTCTCTGTGGTTGTACTGTCCAGCAATCCACCTTTTCTGGAGAGGGCCACCTCTGCCCAAATTTTCCCAGCTGTTTGGACCTCTGGGTGCTTTCTTTGGGCTGGTGAGAGCTCTAATTTGCCTTGGGCCAGTTTCAGGTTTATAGGCCCCCTCAGTCTTCAGATACATGAGGGCTTCTTTGCTCTTGTGATCGTGTAGTCCCATAGCTGTAAAACCAGAATCACCAGGAGGTTGCACCTAGTCAGGAATATTGGGAATGGCCTAGAACAAGGTGTTTGGCACATAAGTAGACCACTTATCCCTCATTGTGACCTAATTCCAGAGCATCTGGCTGGGTTGTTGGGTTCTAGACTTTGTCCTCACCTCCCAGTGACCCTGACTAGCCACAGGCCATGAGATACCAGGGGGCCGTTCCTTGGATGGAGCCTGTGGTTGATGCAAGGCTTCCTTGTCCCCAAGCAAGTCTTCAGAAGGTTAGAACCCAGTGTTGACTGAGTCTGTGCTTGAAACCAGGCCAGAGCCATGGATTAGGAAGGGCAAAGAGAAGGCACCAGAATGAGTAAAGCAGGCAGGTGGTGAAGCCAACCATAAACTTCTCAGGAGTGACATGTGCTTCCTTCAAAGGCATTTTTGTTAACCATATCCTTCTGAGTTCTATGTTTCCTTCACAGCTGTTCTATCCATTTTGTGGACTGTCCCCCACCCCCACCCCATCATTGTTTTTAAAAAAAATTAAGGCCTGGCGCAGCAGCTCATGCCTATAATCCCAGCACTTTGGGAGGCTGAGGCGGGCGGATCACTTGAGGCCAGGAGTTTGAGACCAGCCTGGGCAACATAGCAAAACCCCATTCTGCTTTAAAAAAAAAAAAAAAAAAATTAGCTTGGCGTAGTGGCATGTGCCTATAATCCCAGCTACTGGGGAGGCTGAGGCACAAGAATCATTTGAACCTGGGAGGTAGAGGTTGCTGTGAGCCGAGATTACGCCCCTGCACTCCAGCCTGGGTCACAGAGTGAGACTCCATCTCAGAAAAAAAAAAAAAATTGAGTCAGGTGCAGTAGCTCCTTCCTGTAGTCCCAGCTACTTGGGAGGCTGAGGCTAGAGGATCACTTGAGCCCAGGAGTTTGAGTCTAGTCTGGGCAACATAGCAAGACCCCATCTCTAAAATTTAAGTAAGTAAAAGTAGATAAATAAAAGAAAAAAAACTGTTTATGTGCTCATCATAAAGTAGAAGAGTGGTTTGCTTTTTTTTTTTTTTTGGATTAATGAGGAAATCATTCTGTGGCTCTAGTCATAATTTATGCTTAATAGTAGCCCTTTGCGCTATAACTCTACCTAAAGACTCACATCATTTGGCAGAGAGAGAGTCGTTGAAGTCCCAGGAATTCAGGACTGGGCAGGTTAAGACCTCAGACAAGGTAGTAGAGGTAGACTTGTGGACAAGGCTCGGGTCCCAGCCCACCGCACCCCAACTTTAATCAGAGTGGTTCACTATTGATCTATTTTTGTGTGATAGCTGTGTGGCGTGGGCCACAACATTTAATGAGAAGTTACTGTGCACCAAACTGCCGAACACCATTCTAAACTATTCATATATATTAGTCATTTAATTCTTACATAACTTGAGAGGTAGACAGATATCCTTATTTTAGAGATGAGGAAACCAAGAGAACTTAGGTCATTAGCGCAAGGTTGTAGAGTAAGCGGCAAAGCCAAGCACAAAGCTGGGTGGTTTGGTTTCAGAGCCAGTGCTTTTCCCCTCTACTGTACTGCCTCTCAACCAACACAGGGTTGCACAGCCCATTCTCTGATTTTTTCCTCTTGTCCTCTGCCTCTCCCTCTAGCTCCCACTTCCTCTCTGCTCTAGTTCATTTTCTTTTAGAGCAGCCCGAGTGATCATGAAGTGCAAATCTTGCCATGTCAGTCCCCTGCTTAGAACCCTCCAATGGCTCACTTTCTCTTTAGGCAAAAGTCTTTACCCCATGCCTTCTCCCATCTCATCTCAACCCCCTCCATTTGTTGGCTGTCTGCTGTCAGCCACTCTTCTTTCAGGTCCTCAGATGCACTGCACCCTCTCCTGCCTGGGGGTCTTTGCTCCTGCTACTACCTCTGCTTGAACAGCTCCTCACCTTCCTTCCTCCAACCCTACCCTTGTATAGGTGACTTTT | 96 |

(continued)

| GENBANK ACCESSION NUMBER | SEQUENCE | SEQ ID NO: |
|---|---|---|
| | GTTCATCCTTCAGAATTCAACTCACATGTCTCTTGCATGGAGAACCCTCACCTACTGTGTTGAGACCCTG TCCAGCCCCCAGGTGGGATCCTCTCTCGACTTCCCATACATTTCTTTCACAGCATTTACATAGTCCATGA TAGTTTACTTGTGGGATTATTTGGTTAATCTTTGCCTTTAACACCAGGGTTCCTTGGGTGAAGGAGCTTC TTTATCTTGGTAACAGCATTATTTCAAGCATAACTTGTAATATAGTTATATTACATATATAACATATATA TATATAACATAACATATATAACATATATAACAAGCATAACTTGTTATATAGTCTTGTATATAGTAAGACC TCAATAAATATTTGGAGAACAAAAAAAAAAAAAAA | |
| NM_000633 | TTTCTGTGAAGCAGAAGTCTGGGAATCGATCTGGAAATCCTCCTAATTTTTACTCCCTCTCCCCGCGACT CCTGATTCATTGGGAAGTTTCAAATCAGCTATAACTGGAGAGTGCTGAAGATTGATGGGATCGTTGCCTT ATGCATTTGTTTTGGTTTTACAAAAAGGAAACTTGACAGAGGATCATGCTGTACTTAAAAAATACAACAT CACAGAGGAAGTAGACTGATATTAACAATACTTACTAATAATAACGTGCCTCATGAAATAAAGATCCGAA AGGAATTGGAATAAAAATTTCCTGCATCTCATGCCAAGGGGGAAACACCAGAATCAAGTGTTCCGCGTGA TTGAAGACACCCCTCGTCCAAGAATGCAAAGCACATCCAATAAAATAGCTGGATTATAACTCCTCTTCT TTCTCTGGGGGCCGTGGGGTGGGAGCTGGGGCGAGAGGTGCCGTTGGCCCCCGTTGCTTTTCCTCTGGGA AGGATGGCGCACGCTGGGAGAACAGGGTACGATAACCGGGAGATAGTGATGAAGTACATCCATTATAAGC TGTCGCAGAGGGGCTACGAGTGGGATGCGGGAGATGTGGGCGCCGCGCCCCCGGGGGCCGCCCCCGCACC GGGCATCTTCTCCTCCCAGCCCGGGCACACGCCCCATCCAGCCGCATCCCGGGACCCGGTCGCCAGGACC TCGCCGCTGCAGACCCCGGCTGCCCCCGGCGCCGCCGCGGGGCCTGCGCTCAGCCCGGTGCCCACCTGTGG TCCACCTGACCCTCCGCCAGGCCGGCGACGACTTCTCCCGCCGCTACCGCCGCGACTTCGCCGAGATGTC CAGCCAGCTGCACCTGACGCCCTTCACCGCGCGGGGACGCTTTGCCACGGTGGTGGAGGAGCTCTTCAGG GACGGGGTGAACCTGGGGGAGGATTGTGGCCTTCGTTGAGTTCGGTGGGGTCATGTGTGTGGGAGAGCGTCA ACCGGGAGATGTCGCCCCTGGTGGACAACATCGCCCTGTGTGGATGACTGAGTACCTGAACCGGCACCTGCA CACCTGGATCCAGGATAACGGAGGCTGGGATGCCTTTGTGGAACTGTACGGCCCCAGCATGCGGCCTCTG TTTGATTTCTCCTGGCTGTCTCTGAAGACTCTGCTCAGTTTGGCCCTGGTGGGAGCTTGCATCACCCTGG GTGCCTATCTGGGCCACAAGTGAAGTCAACATGCCTGCCCCAAACAAATATGCAAAAGGTTCACTAAAGC AGTAGAAATAATATGCATTGTCAGTGATGTACCATGAAACAAAGCTGCAGGCTGTTTAAGAAAAAATAAC ACACATATAAACATCACACACACAGACAGACACACACACACAACAATTAACAGTCTTCAGGCAAAACG TCGAATCAGCTATTTACTGCCAAAGGGAAATATCATTTATTTTTTACATTATTATTAAGAAAAAAAGATTTAT TTATTTAAGACAGTCCCATCAAAACTCCTGTCTTTGGAAATCCGACCACTAATTGCCAAGCACCGCTTCG TGTGGCTCCACCTGGATGTTCTGTGCCTGTAAACATAGATTCGCTTTCCATGTTGTTGGCCGGATCACCA TCTGAAGAGCAGACGGATGGAAAAAGGACCTGATCATTGGGGAAGCTGGCTTTCTGGCTGCTGGAGGCTG GGGAGAAGGTGTTCATTCACTTGCATTTCTTTGCCCTGGGGGCTGTGATATTAACAGAGGGAGGGTTCCT GTGGGGGGGAAGTCCATGCCTCCCTGGCCTGAAGAAGAGACTCTTTGCATATGACTCACATGATGCATACC TGGTGGGAGGAAAAGAGTTGGGAACTTCAGATGGACCTAGTACCCACTGAGATTTCCACGCCGAAGGACA GCGATGGGAAAAATGCCCTTAAATCATAGGAAAGTATTTTTTTAAGCTACCAATTGTGCCGAGAAAAGCA TTTTAGCAATTTATACAATATCATCCAGTACCTTAAGCCCTGATTGTGTATATTCATATATTTTGGATAC GCACCCCCCAACTCCCAATACTGGCTCTGTCTGAGTAAGAAACAGAATCCTCTGGAACTTGAGGAAGTGA ACATTTCGGTGACTTCCGCATCAGGAAGGCTAGAGTTACCCAGAGCATCAGGCCGCCACAAGTGCCTGCT TTTAGGAGACCGAAGTCCGCAGAACCTGCCTGTGTCCCAGCTTGGAGGCCTGGTCCTGGAACTGAGCCGG GGCCCTCACTGGCCTCCTCCAGGGATGATCAACAGGGCAGTGTGGTCTCCGAATGTCTGGAAGCTGATGG AGCTCAGAATTCCACTGTCAAGAAAGAGCAGTAGAGGGGTGTGGCTGGGCCTGTCACCCTGGGGCCCTCC AGGTAGGCCCGTTTTCACGTGGAGCATGGGAGCCACGACCCTTCTTAAGACATGTATCACTGTAGAGGGA AGGAACAGAGGCCCTGGGCCCTTCCTATCAGAAGGACATGGTGAAGGCTGGGAACGTGAGGAGGAGGCAAT GGCCACGGCCCATTTGGCTGTAGCACATGGCACGTTGGCTGTGTGGCCTTGGCCCACCTGTGAGTTTAA AGCAAGGCTTTAAATGACTTTGGAGAGGGTCACAAATCCTAAAAGAAGCATTGAAGTGAGGTGTCATGGA TTAATTGACCCCTGTCTATGGAATTACATGTAAAACATTATCTTGTCACTGTAGTTTGGTTTTATTTGAA AACCTGACAAAAAAAAAGTTCCAGGTGTGGAATATGGGGGTTATCTGTACATCCTGGGGCATTAAAAAAA AAATCAATGGTGGGGAACTATAAAGAAGTAACAAAAGAAGTGACATCTTCAGCAAATAAACTAGGAAATT TTTTTTCTTCCAGTTTAGAATCAGCCTTGAAACATTGATGGAATAACTCTGTGGCATTATTGCATTATA TACCATTTATCTGTATTAACTTTGGAATGTACTCTGTTCAATGTTTAATGCTGTGGTTGATATTTCGAAA GCTGCTTTAAAAAAATACATGCATCTCAGCGTTTTTTTTGTTTTTAATTGTATTTAGTTATGGCCTATACA CTATTTGTGAGCAAAGGTGATCGTTTTCTGTTTGAGATTTTTATCTCTTGATTCTTCAAAAGCATTCTGA GAAGGTGAGATAAGCCCTGAGTCTCAGCTACCTAAGAAAAACCTGGATGTCACTGGCCACTGAGGAGCTT TGTTCAACCAAGTCATGTGCATTTCCACGTCAACAGAATTGTTTATTGTGACAGTTATATCTGTTGTCC CTTTGACCTTGTTTCTTGAAGGTTTCCTCGTCCCTGGGCAATTCCGCATTTAATTCATGGTATTCAGGAT TACATGCATGTTTGGTTAAACCCATGAGATTCATTCAGTTAAAAAATCCAGATGGCAAATGACCAGCAGAT TCAAATCTATGGTGGTTTGACCTTTAGAGAGTTGCTTTACGTGGCCTGTTTCAACACAGACCCACCCAGA GCCCTCCTGCCCTCCTTCCGCGGGGGCTTTCTCATGGCTGTCCTTCAGGGTCTTCCTGAAATGCAGTGGT GCTTACGCTCCACCAAGAAAGCAGGAAACCTGTGTGGTATGAAGCCAGACCTCCCCGGCGGGCCTCAGGGAA CAGAATGATCAGACCTTTGAATGATTCTAATTTTTAAGCAAAATATTATTTTATGAAAGGTTTACATTGT CAAAGTGATGAATATGGAATATCCAATCCTGTGCTGCTATCCTGCCAAAATCATTTTAATGGAGTCAGTT TGCAGTATGCTCCACGTGGTAAGATCCTCCAAGCTGCTTTAGAAGTAACAATGAAGAACGTGGACGTTTT TAATATAAAGCCTGTTTTGTCTTTTGTTGTTGTTCAAACGGGATTCACAGAGTATTTGAAAAATGTATAT ATATTAAGAGGTCACGGGGGCTAATTGCTGGCTGGCTGCCTTTTGCTGTGGGGGTTTTGTTACCTGGTTTT AATAACAGTAAATGTGCCCAGCCTCTTGGCCCCAGAACTGTACAGATTGTGGCTGCACTTGCTCTAAGA GTAGTTGATGTTGCATTTTCCTTATTGTTAAAAACATGTTAGAAGCAATGAATGTATATAAAAGCCTCAA CTAGTCATTTTTTTCTCCTCTTCTTTTTTTTCATTATATCTAATTATTTTGCAGTTGGGCAACAGAGAAC CATCCCTATTTTGTATTGAAGAGGGATTCACATCTGCATCTTAACTGCTCTTTATGAATGAAAAAACAGT CCTCTGTATGTACTCCTCTTTACACTGGCCAGGGTCAGAGTTAAATAGAGTATATGCACTTTCCAAATTG | 97 |

(continued)

| GENBANK ACCESSION NUMBER | SEQUENCE | SEQ ID NO: |
|---|---|---|
| | GGGACAAGGGCTCTAAAAAAAGCCCCAAAAGGAGAAGAACATCTGAGAACCTCCTCGGCCCTCCCAGTCC<br>CTCGCTGCACAAATACTCCGCAAGAGAGGCCAGAATGACAGCTGACAGGGTCTATGGCCATCGGGTCGTC<br>TCCGAAGATTTGGCAGGGGCAGAAAACTCTGGCAGGCTTAAGATTTGGAATAAAGTCACAGAATTAAGGA<br>AGCACCTCAATTTAGTTCAAACAAGACGCCAACATTCTCTCCACAGCTCACTTACCTCTCTGTGTTCAGA<br>TGTGGCCTTCCATTTATATGTGATCTTTGTTTTATTAGTAAATGCTTATCATCTAAAGATGTAGCTCTGG<br>CCCAGTGGGAAAAATTAGGAAGTGATTATAAATCGAGAGGAGTTATAATAATCAAGATTAAATGTAAATA<br>ATCAGGGCAATCCCAACACATGTCTAGCTTTCACCTCCAGGATCTATTGAGTGAACAGAATTGCAAATAG<br>TCTCTATTTGTAATTGAACTTATCCTAAAACAAATAGTTTATAAATGTGAACTTAAACTCTAATTAATTC<br>CAACTGTACTTTTAAGGCAGTGGCTGTTTTTAGACTTTCTTATCACTTATAGTTAGTAATGTACACCTAC<br>TCTATCAGAGAAAAACAGGAAAGGCTCGAAATACAAGCCATTCTAAGGAAATTAGGGAGTCAGTTGAAAT<br>TCTATTCTGATCTTATTCTGTGGTGTCTTTTGCAGCCCAGACAAATGTGGTTACACACTTTTTAAGAAAT<br>ACAATTCTACATTGTCAAGCTTATGAAGGTTCCAATCAGATCTTTATTGTTATTCAATTTGGATCTTTCA<br>GGGATTTTTTTTTTAAATTATTATGGGACAAAGGACATTTGTTGGAGGGGTGGGAGGGAGGAAGAATTTT<br>TAAATGTAAAACATTCCCAAGTTTGGATCAGGGAGTTGGAAGTTTTCAGAATAACCAGAACTAAGGGTAT<br>GAAGGACCTGTATTGGGGTCGATGTGATGCCTCTGCGAAGAACCTTGTGTGACAAATGAGAAACATTTTG<br>AAGTTTGTGGTACGACCTTTAGATTCCAGAGACATCAGCCATGGCTCAAAGTGCAGCTCCGTTTGGCAGTG<br>CAATGGTATAAATTTCAAGCTGGATATGTCTAATGGGTATTTAAACAATAAATGTGCAGTTTTAACTAAC<br>AGGATATTTAATGACAACCTTCTGGTTGGTAGGGACATCTGTTTCTAAATGTTTATTATGTACAATACAG<br>AAAAAAATTTTATAAAATTAAGCAATGTGAAACTGAATTGGAGAGTGATAATACAAGTCCTTTAGTCTTA<br>CCCAGTGAATCATTCTGTTCCATGTCTTTGGACAACCATGACCTTGGACAATCATGAAATATGCATCTCA<br>CTGGATGCAAAGAAAATCAGATGGAGCATGAATGGTACTGTACCGGTTCATCTGGACTGCCCCAGAAAAA<br>TAACTTCAAGCAAACATCCTATCAACAACAAGGTTGTTCTGCATACCAAGCTGAGCACAGAAGATGGGAA<br>CACTGGTGGAGGATGGAAAGGCTCGCTCAATCAAGAAAATTCTGAGACTATTAATAAATAAGACTGTAGT<br>GTAGATACTGAGTAAATCCATGCACCTAAACCTTTTGGAAAATCTGCCGTGGGCCCTCCAGATAGCTCAT<br>TTCATTAAGTTTTTCCCTCCAAGGTAGAATTTGCAAGAGTGACAGTGGATTGCATTTCTTTTGGGGAAGC<br>TTTCTTTTGGTGGTTTTGTTTATTATACCTTCTTAAGTTTTCAACCAAGGTTTGCTTTTGTTTTGAGTTA<br>CTGGGGGTTATTTTTGTTTTAAATAAAAATAAGTGTACAATAAGTGTTTTTGTATTGAAAGCTTTTGTTAT<br>CAAGATTTTCATACTTTTACCTTCCATGGCTCTTTTTAAGATTGATACTTTTAAGAGGTGGCTGATATTC<br>TGCAACACTGTACACATAAAAAATACGGTAAGGATACTTTACATGGTTAAGGTAAAGTAAGTCTCCAGTT<br>GGCCACCATTAGCTATAATGGCACTTTGTTTGTGTTGTTGGAAAAAGTCACATTGCCATTAAACTTTCCT<br>TGTCTGTCTAGTTAATATTGTGAAGAAAAATAAAGTACAGTGTGAGATACTG | |
| BX647539 | AATGAGGGTATTTATAAACTACTTAAATTATAAAAAGAATGAGACATCAGACTTACAGTTTTGGATACTA<br>ATTTTTTTCACTTAACGTTCATTATGTGATAGGAGTTTTCCATCCTATTATACCGCTGTGCGATCTGATC<br>TTGGGCACGTTAACCAACCTCTTGTTGCCTCGATTTTCTCACCTGTAAAAGTGGGGGTAATCATAATGCT<br>TACTTAGTAGGATAGCCCTGAAGAATAAGTGACTTAGCGAACATAAATAGCTTACAATAGGGTTTTCAGC<br>ATGGGAAGGATTCAGTAAATGTTAGCTGTCATCATCACCACCTACAAAGGAAGCAATACTGTGCTGAAAG<br>TTTTTCCATCATTAATGTAATTTCTATAGTACGATCCCAAGAAGATATTAAAATTATGGAAATAAAGGT<br>ATTGGTATATTCCTAATTATTTCCTAAAAGATTGTATTGATAAATATGCTCATCCTTCCCTTAACGGGAT<br>GCATTCCAGAAAAACAAGTCAAATGTTAGACAAAGTATCAGAAGGGAAATTCTGTAGCCAGAGAGCTAAA<br>AATTACAATAGGGTCTCTAATTATACTTCAACTTTTTTAGGAATAATTCTCAGTGTGTTTCCCACATTT<br>CATATGTAATTTTTTTTTTTTTTTTTTTGAGACAGAGCCTCGCCCTGTCACCAGGCTGGAGTACAGTG<br>GCGCGATCTCGGCTCACTGCAACTTCCACCTGCTGGGTTCAAGCAATTCTTCTGACCTCAGGTGATCCAC<br>CCGCCTCGGCCTCCCAAAGTGCTGGGATTATAACAGGCGTGGCATGAGTCACCGCGCCCGGCCCGATCTTT<br>ACTTTTTTATTCTTTGTACCCCCTGCCTATCCAGTTAGCATGTGATTAAAGTCAAAGATTTGCCACTTTG<br>GGCCACATCTATTAATTTTCATCTTTGTTATAATTGTATTTAGTTTTTGATCTACACTGCTTATTACTCC<br>CAGTCATTTTTATAGAACTGAAAATCTGGTAAAATACTCAAAATTGCACTGACTTCTATGTAGAGGCGA<br>CACTCCATCAGAACCGTGGGCTGACAGGGAATCCCACTGTGCAGGAGCTGCGCGCATTTTCATTTCTGAT<br>TCTCTTTGGCGTATCCAGGACTCTGATGACATGATCATATATTTATCAGTAGTAACAGGTTGGGCCATTT<br>GTTTTTTGTGGTAAATCATATATTTAAGATTTTAGAAATAAGTTGATAGCCATGTATTTTGGAATTTGAA<br>AAAGACATTGCATTACTCAGCTTCAAATTAAGCTTTAATCAAATAGTGAAACTTTCCATTAATGGACAGT<br>GTATACCTTTTTGTGTATTTAAAAAAAAAAACACTGAATATAGTGCCTTTGTGACAGGGGAGCTTGGTTC<br>CTGACAATGTCCTCTTGAGCCTTTTTTTTTTTTTTGAGATGGAGTCTCACTGTGTCACCCAGGCTGGAGT<br>GCAGTGGCGCCATCTTGGCTCACTGCAACCTCCGCCCCCTGGGTTCAAGTGATTCTCATTCCTCAGCTTC<br>CTAAGTAGCTGGGATTACAGGCACGCACCACCATGACCAGCTAATTTTTATACTTTTAGTAGAGACAGGG<br>TTTTGCCATGTTGGCTAGGTTGGTCTCGAACTCCTGACCTCAAGTAATCCACCCACCATGGCCTCCCCAA<br>AGTGCTGGGATTACAGGCGTGAGCCATTTCACCCGGCCTCTCTTCCGTCTTTGAGCTGTGAGGAAATAGC<br>TACATTACATGAGCTGCTAGATCTGCCTTATGGTCAGAAATGAAGGTTGAACTCTCAGGAACAGTGACAT<br>ATATACACACTGATATTTCCAAAGTACAATGCCCCAAATTGATCCACAAAGGAATTAAGGTCATTTGCAA<br>CAAAATCACAGAATAGTAACAAATAAATAGAAGATAAATATGGCCAGGGATGCTGCAAACTGATATACTG<br>CCAAGTTTATCAGTTGGGAATCCCAACAGTGAAAAGCATAAAAATGAAAGGAATTTTAAGGAGACTTTTT<br>ATAGAAGAGTGGGAAGGATTGGAGGAGCCAACAAGTGATGGTGAGGCACACAGGGAAGAGCTTCAGTGGG<br>CACCATCCCCTCTCTGGTTTGAAGGGGTAGGGAGGGGACCAGAGCTGGGAGGAGGGGGCTGGAATACTGC<br>TGGAGGAGCCACTCCCTTCCAGACCTGCTGTGGCCATCACAGAATGCAGCCACTGCCAGAGCAGCAGCCC<br>GAGGAACCAGGCAGGGGAGCACAAGTACCCTAGCCTCTCTCTTTCTGTTTCTTGGCCTGCCGATCTCCTC<br>CACTGGCTAAACCCAGCTGGATGCTAAGAGTACAGTCAGTCGCTGCCTGCTGAGGAGGGACCACCAGGGACC<br>ACCATCAGCAAGGGATCCAATGTCTTTCTGCCTCTGCAGAATGAAGGTTGGGGCGCGGGGGGCGCTCTAC<br>TTCTTAGGGATATTGTGGGAATAAAAGGAAATAGGCAAAAAATGTTTTTGAAAAACAAAGCACATACTGC<br>GCACCCGTGGGCCACTACTGCTTTTGACCCCTGGCTCTGTTTCATGAAGTAATGTCGTGTCATTCTCTTT | 98 |

(continued)

| GENBANK ACCESSION NUMBER | SEQUENCE | SEQ ID NO: |
|---|---|---|
| | TTAGGTGCTACAGGATTTCTTTAGGTTTGTTTTCTGTCCACCATATTTCAACTCATGTGTGCTGTTTGTT GTGCTAAAACAAATATTTGCTGATGCCTGAGTGAATAGTTGAATATTTTATATAAGTCAAATTTATACGT AATGATTTTTCTTGTAACTTAGCCGTTTCTCTTTTACAAACTCAGAAAACCTCAGACTTTGAAAAGGCCT TGAAGTTCCTCACCTGAAATCTGAGAACTTGGAGCGCCTTAAAAAATCTAAAGGAAAACAAAACAGTGAA AGAACATGATATAGTCAGTGTAGAGAATAAAATTATTTATGTAATTAATATTGAGGATGCAGATAACACA TTGTGAAATCTTGCTTGTAAAAAATCTCGATCTGCTGAAGAAAGATGTTCTCTCTAGAGATCTTTGAAAG CATAATTATTGAGCTTTTAAAATGTTAGAAACAAAAGTTAGACCCACACATATTCTGGCGTGTGGAAGAT TTGCATTCCTTCCCCTGCCCGCCCCGCCCCCACACTTGTGAGTTGTGCCTGTGTACGCAGTTCCTGTAGC ACTCGGCTGGGCAGAAATCATCTTTCAGCACTAAGGGAACATAGTTATGATCTGGACCTTCTGGGAGTGG TCAGTGCCCAAGAACAGGTATGGGACTCCAGAAAGTTCTGCTCTCAACCCTATTTTGAAATAGAGTTACA CATTGTTCTACAATTATTTGAGTTAATAAGCAGCTCTTTTCAAACGTGATTATGCCCTTCCAAGTTTAAA TACACTAGACTTTAGTGAAAGTAATTGACCTCATCTCATTTCTCTCCTGTTATATTAAGATCACTTTCAG TAAAAGGTAGAAGCTTTTGAAGTGGTGAGGAGGAGGTAGAGGAGGGACATAGAGCAGATAGGGGCTGGAA AGTGGGGTGAGGAAGAGAGTGGCTTCTCTTTGGCAGAGTACCAAGGAAAAGCCCTATCTGTACAGAACCT TTGTGCCTGGGAACTTGATGGCTGCAACCTGAGCCTCAACCTAGTTTGCTTGCGGAGCCAGAAGAGAAGC TAAAAACCTTCAGTTAACCAAGCCAGACACCAAGAAAGTTAAACCGAAAGAGAACCCCCCACCCCCCGCA AAAAAAGAAGTAAAGTGGGTTAAAGTGATATCATGTTAGCACAGAAAGAGAACATAAGGGTCATCTAAG TTCATCTGCCCCCTCTTCTATTTCAAGGTGCAGAAACTAAGGCACAAGGGACCCCGTGTCCTGCTCTTGA TCACATAGCTAGTGGGTGCCAAGCCAGGTCTAGAACTCTGTTCTCTGGGGTCACAGGCTGGCTCTTCATC CCTCTAGAGAGATAGCTCATCTGTGTGCACCTGAGCCCGTTGTGTTTCGGAGTCAAAGCAAATAAAGGCT CAAACTCCAAGACTGTTTTGCAGACCGGCTGCAGTAGATATGGGGGGGAGGAGAAACCTGCTTTAAATTGC TTCAAGCAAGTTGTTTCTGCAAAGGTGTTGACTTTTTTCTTTCAACTTTCTAGTGAGTCACTGCAGCCTG AGCTGTTATTTGTCATTATGCAATAATTCAGGAACTAACTCAAGATTCTTCTTTTTAAATTATTTGTTTA TTTAGAGACAGAGTCTTGCTCTGTTGCCCAGGCTGGAGTGCAGTGGTGTGATCTCGGCTCACTGCAGCCT CTGCCTCCTGGGTTCAAGCAATTCTCATGTCTCAGCCTCCCGAATAGCTGGTATTGCAGGCTCGTGCCAC CACCCCCTGCTAATTTTTGTAATTTTAGTGGAGACACGGTTTCGCCATGTTGGCCGGGCTCGTCTTGAGC TCCTGGCCTCAGGTGATCCGCCCGCCTCGGCCTCCCAAAGTGCTGGGATTGCAGCCGTGAGCCTCCACAC CCGGCCTATTTATTTATTTTTAAATTGGCTGCTCTTAGAAAGGCATACCATGTTTCTGGATGGGAAGGCT TATTAATTCACCCTAATTTAATGTATAAATTTGATGCAATCATAGTCACAGTCCCAGTGGAATTTTTTAA CTTGGTAAGATGTTCTAAAATTAATGAGAGAACTTGAATTACCAGGTATTGAAACACTGTAAAGCCACAA TCATGTAAACAGTATGTTTATAACCATGGGAATAGAGGTCTGTGATACAGCAGAAAAAGTGAAAAAAGA ATAACTGTATTCATAAAAATTTAAATGTGGAGTCACTGGGGGAAAGGATTAAATATTCGATAATGTAGAA ACAACTCAACTATTTGGAGAAATGTAAATTTAGAGCCTTATCTCATGCCATATACCAAAATACTATTTAG ATTTGATTAAAAAATAAAAAAAAAAAAAAAAAA | |
| BC035498 | GCGGCCGCCAGCGCGGTGTAGGGGGCAGGCGCGGATCCCGCCACCGCCGCGCGCTCGGCCCGCCGACTCC CGGCGCCGCCGCCGCCACTGCCGTCGCCGCCGCCGCCTGCCGGGACTGGAGCGCGCCGTCCGCCGCGGAC AAGACCCTGGCCTCAGGCCGGAGCAGCCCCATCATGCCGAGGGAGCGCAGGGAGCGGGATGCGAAGGAGC GGGACACCATGAAGGAGGACGGCGGCGCGGAGTTCTCGGCTCGCTCCAGGAAGAGGAAGGCAAACGTGAC CGTTTTTTTGCAGGATCCAGATGAAGAAATGGCCAAAATCGACAGGACGGCGAGGGACCAGTGTGGGAGC CAGCCTTGGGACAATAATGCAGTCTGTGCAGACCCCTGCTCCCTGATCCCCACACCTGACAAAGAAGATG ATGACCGGGTTTACCCAAACTCAACGTGCAAGCCTCGGATTATTGCACCATCCAGAGGCTCCCCGCTGCC TGTACTGAGCTGGGCAAATAGAGAGGAAGTCTGGAAAATCATGTTAAACAAGGAAAAGACATACTTAAGG GATCAGCACTTTCTTGAGCAACACCCTCTTCTGCAGCCAAAAATGCGAGCAATTCTTCTGGATTGGTTAA TGGAGGTGTGTGAAGTCTATAAACTTCACAGGGAGACCTTTTACTTGGCACAAGATTTCTTTGACCGGTA TATGGCGACACAAGAAATGTTGTAAAAACTCTTTTACAGCTTATTGGGATTTCATCTTTATTTATTGCA GCCAAACTTGAGGAAATCTATCCTCCAAAGTTGCACCAGTTTGCGTATGTGACAGATGGAGCTTGTTCAG GAGATGAAATTCTCACCATGGAATTAATGATTATGAAGGCCCTTAAGTGGCGTTTAAGTCCCCTGACTAT TGTGTCCTGGCTGAATGTATACATGCAGGTTGCATATCTAAATGACTTACATGAAGTGCTACTGCCGCAG TATCCCCAGCAAATCTTTATACAGATTGCAGAGCTGTTGGATCTCTGTGTCCTGGATTGTGACTGCCTTG AATTTCCTTATGGTATACTTGCTGCTTCGGCCTTTGATCATTTCTCGTCATCTGAATTGATGCAAAAGGT TTCAGGGTATCAGTGGTGCGACATAGAGAACTGTGTCAAGTGGATGGTTCCATTTGCCATGGTTATAAGG GAGACGGGGAGCTCAAAACTGAAGCACTTCAGGGGCGTCGCTGATGAAGATGCACACAACATACAGACCC ACAGAGACAGCTTGGATTTGCTGGACAAAGCCCGAGCAAAGAAAGCCATGTTGTCTGAACAAAATAGGGC TTCTCCTCTCCCCAGTGGGCTCCTCACCCCGCCACAGAGCGGTAAGAAGCAGAGCAGCGGGCCGGAAATG GCGTGACCACCCCATCCTTCTCCACCAAAGACAGTTGCGCGCCTGCTCCACGTTCTCTTCTGTCTGTTGC AGCGGAGGCGTGCGTTTGCTTTTACAGATATCTGAATGGAAGAGTGTTTCTTCCACAACAGAAGTATTTC TGTGGATGGCATCAAACAGGGCAAAGTGTTTTTTATTGAATGCTTATAGGTTTTTTTTAAATAAGTGGGT CAAGTACACCAGCCACCTCCAGACACCAGTGCGTGCTCCCGATGCTGCTATGGAAGGTGCTACTTGACCT AAGGGACTCCCACAACAACAAAAGCTTGAAGCTGTGGAGGGCCACGGTGGCGTGGCTCTCCTCGCAGGTG TTCTGGGCTCCGTTGTACCAAGTGGAGCAGGTGGTTGCGGGCAAGCGTTGTGCAGAGCCCATAGCCAGCT GGGCAGGGGGCTGCCCTCTCCACATTATCAGTTGACAGTGTACAATGCCTTTGATGAACTGTTTTGTAAG TGCTGCTATATCTATCCATTTTTTAATAAAGATAATACTGTTTTTGAAAAAAAAAAAAAAAAAAAAAAAA AAAAAAAAAAAAAAAAAAAAAAAAAAAAAAAAAAAAAAAAAAAAAAAAAA | 99 |

(continued)

| GENBANK ACCESSION NUMBER | SEQUENCE | SEQ ID NO: |
|---|---|---|
| BG256659 | GAGGGCACGGGCTCCGTAGGCACCAACTGCAAGGACCCCTCCCCCTGCGGGCGCTCCCATGGCACAGTTC GCGTTCGAGAGTGACCTGCACTCGCTGCTTCAGCTGGATGCACCCATCCCCAATGCACCCCCTGCGCGCT GGCAGCGCAAAGCCAAGGAAGCCGCAGGCCCCGGCCCCCTCACCCATGCGGGCCGCCAACCGATCCCACAG CGCCGGCAGGACTCCGGGCCGAACTCCTGGCAAATCCAGTTCCAAGGTTCAGACCACTCCTAGCAAACCT GGCGGTGACCGCTATATCCCCCATCGCAGTGCTGCCCAGATGGGAGGTGGCCAGCTTCCTCCTGAGCAAGG AGAACCAGCCTGAAAACAGCCAGACGCCCACCAAGAAGGAACATCAGAAAGCCTGGGCTTTGAACCTGAA CGGTTTTGATGTAGAGGAAGCCAAGATCCTTCGGCTCAGTGGAAAAACCACAAAAATGCGCCAGAGGGTT ATCACGAACAGACTGAAAGTACTCTACAGCCAAAAGGCCACTCCTGGCTCCAGCCGGAAGACCTGCCGTT TACATTCCTTCCCTGCCAAGACCGTATCCTGGATGCGCCTGAAATCGAATGACTATTAACTGAACCTGTG GGACTGGCAGTCCGGGGAATGTCCGGGCCGGGCCACGGCCACGAGGTGTTCCGTGTGGAGTGCAAGCTGG GACACACCGTGCCGCTTGTGCACAGGGCCACGCGGGGAAATAATCCCGGGGCGCGCAAAGCGGCACTGGC GAGAGCCGCACGGGCCGGTGCTGGGGGTGGTACAACAGGCCAAAACAACACACAAGGCCAACAAGACATA CGCGCGCTGACACCACGGTGCAAAGCGCTCAGACGAGTAGTAACCGGCACTGTGGTTGCTGCCTCCCCAC CTCTCCCGCTCTCAGCGTAAGATAAAAGAAAGAAGAGCAAAAAGCAAAGAAAGAAGACGAGACGAGACAC ACAGGAACGAACAGTAAAGCAAGCTAAAGCAAACGCAAGACCAGACAACAGAAATAGAAAGAACCAACAG AGAGGAGACAGAACAGGACGCCAGCAACATAGCAACAAACGAACAGAAGAGAGCACTAAACAAAAGCAGC AGCAAGACGAGACAGGAGAGAAGGAGGAAGGAGGGCCGAGCGAGCAGGGAGCGCGAGCAGCGAGGCGAAG CAGCAGACAAGGGCAGGCGAAGGGCAACGAGAGGAGGCACCACACAAAAAGGAGAGGGGACAGGAGAAGC AGCGAGAGAAGCGGAGGAGCAACAAGAGGAAGAAAAGGAGAGGGGAGAGGAGGGAGAGAGCGGAAGGAGGA AGAAACAGCACGAGGCGACGAAGGGGGGAGACGCGGGGGCAGGAAAAGACACAGGAAGGCAGCGCGGAGG AGGAGAAGGGGAAGCAGGAAGGAGACGAAGGAGACAGGGAGAGGAGCAGCGCAAGAGAGCGCGCGCGGC GACAGCGAGGGACGGAGCGAGAGAGAGGAAACGGAAAGCGAGAGGGAAGAGGAGAGGCAACGCAGCGAAC CAACCGAAAACAGCAGAAAGAGAGGAGAAGGACGCGCAAAGAGGCAAGCGCAAGACGACAGGAAACGAAG CGAGAGACGAGAAGCCGGTGACGAGCAGGAGAAAGGGAAGGCAGGAGACAGGACAGGCGGAAGAGAGACA CGCGAGACGCAAAGAGTGAGCAGAACGAAGCGAAGAGCAACGCACGAGAGAAACGAC | 100 |

(continued)

| GENBANK ACCESSION NUMBER | SEQUENCE | SEQ ID NO: |
|---|---|---|
| NM_001254 | GAGCGCGGCTGGAGTTTGCTGCTGCCGCTGTGCAGTTTGTTCAGGGGCTTGTGGTGGTGAGTCCGAGAGG<br>CTGCGTGTGAGAGACGTGAGAAGGATCCTGCACTGAGGAGGTGGAAAGAAGAGGATTGCTCGAGGAGGCC<br>TGGGGTCTGTGAGGCAGCGGAGCTGGGTGAAGGCTGCGGGTTCCGGCGAGGCCTGAGCTGTGCTGTCGTC<br>ATGCCTCAAACCCGATCCCAGGCACAGGCTACAATCAGTTTTCCAAAAAGGAAGCTGTCTCGGGCATTGA<br>ACAAAGCTAAAAAACTCCAGTGATGCCAAACTAGAACCAACAAATGTCCAAACCGTAACCTGTTCTCCTCG<br>TGTAAAAGCCCTGCCTCTCAGCCCCAGGAAACGTCTGGGCGATGACAACCTATGCAACACTCCCCATTTA<br>CCTCCTTGTTCTCCACCAAAGCAAGGCAAGAAAGAGAATGGTCCCCCTCACTCACATACACTTAAGGGAC<br>GAAGATTGGTATTTGACAATCAGCTGACAATTAAGTCTCCTAGCAAAAGAGAACTAGCCAAAGTTCACCA<br>AAACAAAATACTTTCTTCAGTTAGAAAAAGTCAAGAGATCACAACAAATTCTGAGCAGAGATGTCCACTG<br>AAGAAAGAATCTGCATGTGTGAGACTATTCAAGCAAGAAGGCACTTGCTACCAGCAAGCAAAGCTGGTCC<br>TGAACACAGCTGTCCCAGATCGGCTGCCTGCCAGGGAAAGGGAGATGGATGTCATCAGGAATTTCTTGAG<br>GGAACACATCTGTGGGAAAAAAGCTGGAAGCCTTTACCTTTCTGGTGCTCCTGGAACTGGAAAAACTGCC<br>TGCTTAAGCCGGATTCTGCAAGACCTCAAGAAGGAACTGAAAGGCTTTAAAACTATCATGCTGAATTGCA<br>TGTCCTTGAGGACTGCCCAGGCTGTATTCCCAGCTATTGCTCAGGAGATTTGTCAGGAAGAGGTATCCAG<br>GCCAGCTGGGAAGGACATGATGAGGAAATTGGAAAAACATATGACTGCAGAGAAGGGCCCCATGATTGTG<br>TTGGTATTGGACGAGATGGATCAACTGGACAGCAAAGGCCAGGATGTATTGTACACGCTATTTGAATGGC<br>CATGGCTAAGCAATTCTCACTTGGTGCTGATTGGTATTGCTAATACCCTGGATCTCACAGATAGAATTCT<br>ACCTAGGCTTCAAGCTAGAGAAAAATGTAAGCCACAGCTGTTGAACTTCCCACCTTATACCAGAAATCAG<br>ATAGTCACTATTTTGCAAGATCGACTTAATCAGGTATCTAGAGATCAGGTTCTGGACAATGCTGCAGTTC<br>AATTCTGTGCCCGCAAAGTCTCTGCTGTTTCAGGAGATGTTCGCAAAGCACTGGATGTTTGCAGGAGAGC<br>TATTGAAATTGTAGAGTCAGATGTCAAAAGCCAGACTATTCTCAAACCACTGTCTGAATGTAAATCACCT<br>TCTGAGCCTCTGATTCCCAAGAGGGTTGGTCTTATTCACATATCCCAAGTCATCTCAGAAGTTGATGGTA<br>ACAGGATGACCTTGAGCCAAGAAGGAGCACAAGATTCCTTCCCTCTTCAGCAGAAGATCTTGGTTTGCTC<br>TTTGATGCTCTTGATCAGGCAGTTGAAAATCAAAGAGGTCACTCTGGGGAAGTTATATGAAGCCTACAGT<br>AAAGTCTGTCGCAAACAGCAGGTGGCGGCTGTGGACCAGTCAGAGTGTTTGTCACTTTCAGGGCTCTTGG<br>AAGCCAGGGGCATTTTAGGATTAAAGAGAAACAAGGAAACCCGTTTGACAAAGGTGTTTTTCAAGATTGA<br>AGAGAAAGAAATAGAACATGCTCTGAAAGATAAAGCTTTAATTGGAAATATCTTAGCTACTGGATTGCCT<br>TAAATTCTTCTCTTACACCCCACCCGAAAGTATTCAGCTGGCATTTAGAGAGCTACAGTCTTCATTTTAG<br>TGCTTTACACATTCGGGCCTGAAAACAAATATGACCTTTTTTACTTGAAGCCAATGAATTTTAATCTATA<br>GATTCTTTAATATTAGCACAGAATAATATCTTTGGGTCTTACTATTTTTACCCATAAAAGTGACCAGGTA<br>GACCCTTTTTAATTACATTCACTACTTCTACCACTTGTGTATCTCTAGCCAATGTGCTTGCAAGTGTACA<br>GATCTGTGTAGAGGAATGTGTGTATATTTACCTCTTCGTTTGCTCAAACATGAGTGGGTATTTTTTTGTT<br>TGTTTTTTTGTTGTTTGTTTTTGAGGCGCGTCTCACCCTGTTGCCCAGGCTGGAGTGCAATGGCGCG<br>TTCTCTGCTCACTACAGCACCCGCTTCCCAGGTTGAAGTGATTCTCTTGCCTCAGCCTCCCGAGTAGCTG<br>GGATTACAGGTGCCCACCACCGCGCCCAGCTAATTTTTTAATTTTTAGTAGAGACAGGGTTTTACCATGT<br>TGGCCAGGCTGGTCTTGAACTCCTGACCCTCAAGTGATCTGCCCACCTTGGCCTCCCTAAGTGCTGGGAT<br>TATAGGCGTGAGCCACCATGCTCAGCCATTAAGGTATTTGTTAAGAACTTTAAGTTTAGGGTAAGAAGA<br>ATGAAAATGATCCAGAAAAATGCAAGCAAGTCCACATGGAGATTTGGAGGACACTGGTTAAAGAATTTAT<br>TTCTTTGTATAGTATACTATGTTCATGGTGCAGATACTACAACATTGTGGCATTTTAGACTCGTTGAGTT<br>TCTTGGGCACTCCCAAGGGCGTTGGGGTCATAAGGAGACTATAACTCTACAGATTGTGAATATATTTATT<br>TTCAAGTTGCATTCTTTGTCTTTTTAAGCAATCAGATTTCAAGAGAGCTCAAGCTTTCAGAAGTCAATGT<br>GAAAATTCCTTCCTAGGCTGTCCCACAGTCTTTGCTGCCCTTAGATGAAGCCACTTGTTTCAAGATGACT<br>ACTTTGGGGTTGGGTTTTCATCTAAACACATTTTTCCAGTCTTATTAGATAAATTAGTCCATATGGTTGG<br>TTAATCAAGAGCCTTCTGGGTTTGGTTTGGTGGCATTAAATGG | 101 |
| NM_031423 | GCGGAATGGGGCGGGACTTCCAGTAGGAGGCGGCAAGTTTGAAAAGTGATGACGGTTGACGTTTGCTGAT<br>TTTTGACTTTGCTTGTAGCTGCTCCCCGAACTCGCCGTCTTCCTGTCGGCGGCCGGCACTGTAGATTAAC<br>AGGAAACTTCCAAGATGGAAACTTTGTCTTTCCCCAGATATAATGTAGCTGAGATTGTGATTCATATTCG<br>CAATAAGATCTTAACAGGAGCTGATGGTAAAAACCTCACCAAGAATGATCTTTATCCAAATCCAAAGCCT | 102 |

(continued)

| GENBANK ACCESSION NUMBER | SEQUENCE | SEQ ID NO: |
|---|---|---|
| | GAAGTCTTGCACATGATCTACATGAGAGCCTTACAAATAGTATATGGAATTCGACTGGAACATTTTTACA<br>TGATGCCAGTGAACTCTGAAGTCATGTATCCACATTTAATGGAAGGCTTCTTACCATTCAGCAATTTAGT<br>TACTCATCTGGACTCATTTTTGCCTATCTGCCGGGTGAATGACTTTGAGACTGCTGATATTCTATGTCCA<br>AAAGCAAAACGGACAAGTCGGTTTTTAAGTGGCATTATCAACTTTATTCACTTCAGAGAAGCATGCCGTG<br>AAACGTATATGGAATTTCTTTGGCAATATAAATCCTCTGCGGACAAAATGCAACAGTTAAACGCCGCACA<br>CCAGGGAGGCATTAATGAAACTGGAGAGACTTGATTCTGTTCCAGTTGAAGAGCAAGAAGAGTTCAAGCAG<br>CTTTCAGATGGAATTCAGGAGCTACAACAATCACTAAATCAGGATTTTCATCAAAAAACGATAGTGCTGC<br>AAGAGGGAAATTCCCAAAAGAAGTCAAATATTTCAGAGAAAACCAAGCGTTTGAATGAACTAAAATTGTC<br>GGTGGTTTCTTTGAAAGAAATACAAGAGAGTTTGAAGGAAATTGTGGATTCTCCAGAGAAGTTAAAG<br>AATTATAAAGAAAAAATGAAAGATACGGTCCAGAAGCTTAAAAATGCCAGACAAGAAGTGGTGGAGAAAT<br>ATGAAATCTATGGAGACTCAGTTGACTGCCTGCCTTCATGTCAGTTGGAAGTGCAGTTATATCAAAAGAA<br>AATACAGGACCTTTCAGATAATAGGGAAAAATTAGCCAGTATCTTAAAGGAGAGCCTGAACTTGGAGGAC<br>CAAATTGAGAGTGATGAGTCAGAACTGAAGAAATTGAAGACTGAAGAAAATTCGTTCAAAAGACTGATGA<br>TTGTGAAGAAGGAAAAACTTGCCACAGCACAATTCAAAATAAATAAGAAGCATGAAGATGTTAAGCAATA<br>CAAACGCACAGTAATTGAGGATTGCAATAAAGTTCAAGAAAAAAGAGGTGCTGTCTATGAACGAGTAACC<br>ACAATTAATCAAGAAATCCAAAAAATTAAACTTGGAATTCAACAACTAAAAGATGCTGCTGAAAGGGGAGA<br>AACTGAAGTCCCAGGAAATATTTCTAAACTTGAAAACTGCTTTGGAGAAATACCACGACGGTATTGAAAA<br>GGCAGCAGAGGACTCCTATGCTAAGATAGATGAGAAGACAGCTGAACTGAAGAGGAAGATGTTCAAAATG<br>TCAACCTGATTAACAAAATTACATGTCTTTTTGTAAATGGCTTGCCATCTTTTAATTTTCTATTTAGAAA<br>GAAAAGTTGAAGCGAATGGAAGTATCAGAAGTACCAAATAATGTTGGCTTCATCAGTTTTTATACACTCT<br>CATAAGTAGTTAATAAGATGAATTTAATGTAGGCTTTTATTAATTTATAATTAAAATAACTTGTGCAGCT<br>ATTCATGTCTCTACTCTGCCCCTTGTTGTAAATAGTTTGAGTAAAACAAAACTAGTTACCTTTGAAATAT<br>ATATATTTTTTTCTGTTACTATC | |
| BC041846 | GGCTAGCGCGGGAGGTGGAGAAAGAGGCTTGGGCGGCCCCGCTGTAGCCGCGTGTGGGAGGACGCACGGG<br>CCTGCTTCAAAGCTTTGGGATAACAGCGCCTCCGGGGGATAATGAATGCGGAGCCTCCGTTTTCAGTCGA<br>CTTCAGATGTGTCTCCACTTTTTTCCGCTGTAGCCGCAAGGCAAGGAAACATTTCTCTTCCCGTACTGAG<br>GAGGCTGAGGAGTGCACTGGGTGTTCTTTTCTCCTCTAACCCAGAACTGCGAGACAGAGGCTGAGTCCCT<br>GTAAAGAACAGCTCCAGAAAAGCCAGGAGAGCGCAGGAGGGCATCCGGGAGGCCAGGAGGGGTTCGCTGG<br>GGCCTCAACCGCACCCACATCGGTCCCACCTGCGAGGGGCGGGACCTCGTGGCGCTGGACCAATCAGCA<br>CCCACCTGCGCTCACCTGGCCTCCTCCCGCTGGCTCCCGGGGGCTGCGGTGCTCAAAGGGGCAAGAGCTG<br>AGCGGAACACCGGCCCGCCGTCGCGGCAGCTGCTTCACCCCTCTCTCTGCAGCCATGGGGCTCCCTCGTG<br>GACCTCTCGCGTCTCTCCTCCTTCTCCAGGTTTGCTGGCTGCAGTGCGCGGCCTCCGAGCCGTGCCGGGC<br>GGTCTTCAGGGAGGCTGAAGTGACCTTGGAGGCGGGAGGCGCGGAGCAGGAGCCCGGCCAGGCGCTGGGG<br>AAAGTATTCATGGGCTGCCCTGGGCAAGAGCCAGCTCTGTTTAGCACTGATAATGATGACTTCACTGTGC<br>GGAATGGCGAGCAGTCCAGGAAAGAAGGTCACTGAAGGAAAGGAATCCATTGAAGATCTTCCCATCCAA<br>ACGTATCTTACGAAGACACAAGAGAGATTGGGTGGTTGCTCCAATATCTGTCCCTGAAAATGGCAAGGGT<br>CCCTTCCCCCAGAGACTGAATCAGCTCAAGTCTAATAAAGATAGAGACACCAAGATTTTCTACAGCATCA<br>CGGGGCCGGGGGCAGACAGCCCCCCTGAGGGTGTCTTCGCTGTAGAGAAGGAGACAGGCTGGTTGTTGTT<br>GAATAAGCCACTGGACCGGGAGGAGATTGCCAAGTATGAGCTCTTTGGCCACGCTGTGTCAGAGAATGGT<br>GCCTCAGTGGAGGACCCCATGAACATCTCCATCATAGTGACCGACCAGAATGACCACAAGCCCAAGTTTA<br>CCCAGGACACCTTCCGAGGGAGTGTCTTAGAGGGAGTCCTACCAGGTACTTCTGTGATGCAGATGACAGC<br>CACAGATGAGGATGATGCCATCTACACCTACAATGGGGTGGTTGCTTACTCCATCCATAGCCAAGAACCA<br>AAGGACCCACACGACCTCATGTTCACAATTCACCGGAGCACAGGCACCATCAGCGTCATCTCCAGTGGCC<br>TGGACCGGGAAAAAGTCCCTGAGTACACACTGACCATCCAGGCCACAGACATGGATGGGGACGGCTCCAC<br>CACCACGGCAGTGGCAGTAGTGGAGATCCTTGATGCCAATGACAATGCTCCCATGTTTGACCCCCAGAAG<br>TACGAGGCCCATGTGCCTGAGAATGCAGTGGGCCATGAGGTGCAGAGGCTGACGGTCACTGATCTGGACG<br>CCCCCAACTCACCAGCGTGGCGTGCCACCTACCTTATCATGGGCGGTGACGACGGGGACCATTTTACCAT<br>CACCACCCACCCTGAGAGCAACCAGGGCATCCTGACAACCAGGAAGGGTTTGGATTTTGAGGCCAAAAAC<br>CAGCACACCCTGTACGTTGAAGTGACCAACGAGGCCCCTTTTGTGCTGAAGCTCCCAACCTCCACAGCCA<br>CCATAGTGGTCCACGTGGAGGATGTGAATGAGGCACCTGTGTTTGTCCCACCCTCCAAAGTCGTTGAGGT<br>CCAGGAGGGCATCCCCACTGGGGAGCCTGTGTGTGTCTACACTGCAGAAGACCCTGACAAGGAGAATCAA<br>AAGATCAGCTACCGCATCCTGAGAGACCCAGCAGGGTGGCTAGCCATGGACCCAGACAGTGGGCAGGTCA<br>CAGCTGTGGGCACCCTCGACCGTGAGGATGAGCAGTTTGTGAGGAACAACATCTATGAAGTCATGGTCTT<br>GGCCATGGACAATGGAAGCCCTCCCACCACTGGCACGGGAACCCTTCTGCTAACACTGATTGATGTCAAC<br>GACCATGGCCCAGTCCCTGAGCCCCGTCAGATCACCATCTGCAACCAAAGCCCTGTGCGCCAGGTGCTGA<br>ACATCACGGACAAGGACCTGTCTCCCCACACCTCCCCTTTCCAGGCCCAGCTCACAAGTGACTCAGACAT<br>CTACTGGACGGCAGAGGTCAACGAGGAAGGTGACACAGTGGTCTTGTCCCTGAAGAAGTTCCTGAAGCAG<br>GATACATATGACGTGCACCTTTCTCTGTCTGACCATGGCAACAAAGAGCAGCTGACGGTGATCAGGGCCA<br>CTGTGTGCGACTGCCATGGCCATGTCGAAACCTGCCCTGGACCCTGGAAAGGAGGTTTCATCCTCCCTGT<br>GCTGGGGGCTGTCCTGGCTCTGCTGTTCCTCCTGCTGGTGCTGCTTTTGTTGGTGAGAAAGAAGCGGAAG<br>ATCAAGGAGCCCCTCCTACTCCCAGAAGATGACACCCGTGACAACGTCTTCTACTATGGCGAAGAGGGGG<br>GTGGCGAAGAGGACCAGGACTATGACATCACCCAGCTCCACCGAGGTCTGGAGGCCAGGCCGGAGGTGGT<br>TCTCCGCAATGACGTGGCGCACCAACCATCATCCCGACACCCATGTACCGTCCTAGGCCAGCCAACCCAGAT<br>GAAATCGGCAACTTTATAATTGAGAACCTGAAGGCGGCTAACACAGACCCCACAGCCCCGCCCTACGACA<br>CCCTCTTGGTGTTCGACTATGAGGGCAGCGGCTCCGACGCCGCGTCCCTGAGCTCCCTCACCTCCTCCGC<br>CTCCGACCAAGACCAAGATTACGATTATCTGAACGAGTGGGGCAGCCGCTTCAAGAAGCTGGCAGACATG<br>TACGGTGGCGGGGAGGACGACTAGGCGGCCTGCCTGCAGGGCTGGGGACCAAACGTCAGGCCACAGAGCA<br>TCTCCAAGGGGTCTCAGTTCCCCCTTCAGCTGAGGACTTCGGAGCTTGTCAGGAAGTGGCCGTAGCAACT | 103 |

(continued)

| GENBANK ACCESSION NUMBER | SEQUENCE | SEQ ID NO: |
|---|---|---|
| | TGGCGGAGACAGGCTATGAGTCTGACGTTAGAGTGGTTGCTTCCTTAGCCTTTCAGGATGGAGGAATGTG<br>GGCAGTTTGACTTCAGCACTGAAAACCTCTCCACCTGGGCCAGGGTTGCCTCAGAGGCCAAGTTTCCAGA<br>AGCCTCTTACCTGCCGTAAAATGCTCAACCCTGTGTCCTGGGCCTGGGCCTGCTGTGACTGACCTACAGT<br>GGACTTTCTCTCTGGAATGGAACCTTCTTAGGCCTCCTGGTGCAACTTAATTTTTTTTTTTAATGCTATC<br>TTCAAAACGTTAGAGAAAGTTCTTCAAAAGTGCAGCCCAGAGCTGCTGGGCCCACTGGCCGTCCTGCATT<br>TCTGGTTTCCAGACCCCAATGCCTCCCATTCGGATGGATCTCTGCGTTTTTATACTGAGTGTGCCTAGGT<br>TGCCCCTTATTTTTTATTTTCCCTGTTGCGTTGCTATAGATGAAGGGTGAGGACAATCGTGTATATGTAC<br>TAGAACTTTTTTATTAAAGAAACTTTTCCCAAAAAAAAAAAAAAAA | |
| NM_016343 | GAGACCAGAAGCGGGCGAATTGGGCACCGGTGGCGGCTGCGGGCAGTTTGAATTAGACTCTGGGCTCCAG<br>CCCGCCGAAGCCGCGCCAGAACTGTACTCTCCGAGAGGTCGTTTTCCCGTCCCCGAGAGCAAGTTTATTT<br>ACAAATGTTGGAGTAATAAAGAAGGCAGAACAAAATGAGCTGGGCTTTGGAAGAATGGAAAGAAGGGCTG<br>CCTACAAGAGCTCTTCAGAAAATTCAAGAGCTTGAAGGACAGCTTGACAAACTGAAGAAGGAAAAGCAGC<br>AAAGGCAGTTTCAGCTTGACAGTCTCGAGGCTGCGCTGCAGAAGCAAAAACAGAAGGTTGAAAATGAAAA<br>AACCGAGGGTACAAACCTGAAAAGGGAGAATCAAAGATTGATGGAAATATGTGAAAGTCTGGAGAAAACT<br>AAGCAGAAGATTTCTCATGAACTTCAAGTCAAGGAGTCACAAGTGAATTTCCAGGAAGGCAACTGAATT<br>CAGGCAAAAAACAAATAGAAAAACTGGAACAGGAACTTAAAAGGTGTAAATCTGAGCTTGAAAGAAGCCA<br>ACAAGCTGCGCAGTCTGCAGATGTCTCTCTGAATCCATGCAATACACCACAAAAAATTTTTACAACTCCA<br>CTAACACCAAGTCAATATTATAGTGGTTCCAAGTATGAAGATCTAAAAGAAAAATATAATAAAGAGGTTG<br>AAGAACGAAAAAGATTAGAGGCAGAGGTTAAAGCCTTGCAGGCTAAAAAAGCAAGCCAGACTCTTCCACA<br>AGCCACCATGAATCACCGCGACATTGCCCGGCATCAGGCTTCATCATCTGTGTTCTCATGGCAGCAAGAG<br>AAGACCCCAAGTCATCTTTCATCTAATTCTCAAAGAACTCCAATTAGGAGAGATTTCTCTGCATCTTACT<br>TTTCTGGGGAACAAGAGGTGACTCCAAGTCGATCAACTTTGCAAATAGGGAAAAGAGATGCTAATAGCAG<br>TTTCTTTGACAATTCTAGCAGTCCTCATCTTTTGGATCAATTAAAAGCGCAGAATCAAGAGCTAAGAAAC<br>AAGATTAATGAGTTGGAACTACGCCTGCAAGGACATGAAAAAGAAATGAAAGGCCAAGTGAATAAGTTTC<br>AAGAACTCCAACTCCAACTGGAGAAAGCAAAAGTGGAATTAATTGAAAAAGAGAAAGTTTTGAACAAATG<br>TAGGGATGAACTAGTGAGAACAACAGCACAATACGACCAGGCGTCAACCAAGTATACTGCATTGGAACAA<br>AAACTGAAAAAATTGACGGAAGATTTGAGTTGTCAGCGACAAAATGCAGAAAGTGCCAGATGTTCTCTGG<br>AACAGAAAATTAAGGAAAAAGAAAAGGAGTTTCAAGGAGGAGCTCTCCCGTCAACAGCGTTCTTTCCAAAC<br>ACTGGACCAGGAGTGCATCCAGATGAAGGCCAGACTCACCCAGGAGTTACAGCAAGCCAAGAATATGCAC<br>AACGTCCTGCAGGCTGAACTGGATAAACTCACATCAGTAAAGCAACAGCTAGAAAACAATTTGGAAGAGT<br>TTAAGCAAAAGTTGTGCAGAGCTGAACAGGCGTTCCAGGCGAGTCAGATCAAGGAGAATGAGCTGAGGAG<br>AAGCATGGAGGAAATGAAGAAGGAAAACAACCTCCTTAAGAGTCACTCTGAGCAAAAGGCCAGAGAAGTC<br>TGCCACCTGGAGGCAGAACTCAAGAACATCAAACAGTGTTTAAATCAGAGCCAGAATTTTGCAGAAGAAA<br>TGAAAGCGAAGAATACCTCTCAGGAAACCATGTTAAGAGATCTTCAAGAAAAAATAAATCAGCAAGAAAA<br>CTCCTTGACTTTAGAAAAACTGAAGCTTGCTGTGGCTGATCTGGAAAAGCAGCGAGATTGTTCTCAAGAC<br>CTTTTGAAGAAAAGAGAACATCACATTGAACAACTTAATGATAAGTTAAGCAAGACAGAGAAAGAGTCCA<br>AAGCCTTGCTGAGTGCTTTAGAGTTAAAAAAGAAAGAATATGAAGAATTGAAAGAAGAGAAAACTCTGTT<br>TTCTTGTTGGAAAAGTGAAAACGAAAAACTTTTAACTCAGATGGAATCAGAAAAGGAAAACTTGCAGAGT<br>AAAATTAATCACTTGGAAACTTGTCTGAAGACACAGCAAATAAAAAGTCATGAATCAACGAGAGAGTAA<br>GAACGCTGGAGATGGACAGAGAAAACCTAAGTGTCGAGATCAGAAACCTTCACAACGTGTTAGACAGTAA<br>GTCAGTGGAGGTAGAGACCCAGAAACTAGCTTATATGGAGCTACAGCAGAAAGCTGAGTTCTCAGATCAG<br>AAACATCAGAAGGAAATAGAAAATATGTGTTTGAAGACTTCTCAGCTTACTGGGCAAGTTGAAGATCTAG<br>AACACAAGCTTCAGTTACTGTCAAATGAAATAATGGACAAAGACCGGTGTTACCAAGACTTGCATGCCGA<br>ATATGAGAGCCTCAGGGATCTGCTAAAATCCAAAGATGCTTCTCTGGTGACAAATGAAGATCATCAGAGA<br>AGTCTTTTGGCTTTTGATCAGCAGCCTGCCATGCATCATTCCTTTGCAAATATAATTGGAGAACAAGGAA<br>GCATGCCTTCAGAGAGGAGTGAATGTCGTTTAGAAGCAGACCAAAGTCCGAAAAATTCTGCCATCCTACA<br>AAATAGAGTTGATTCACTTGAATTTTCATTAGAGTCTCAAAAACAGATGAACTCAGACCTGCAAAAGCAG<br>TGTGAAGAGTTGGTGCAAATCAAAGGAGAAATAGAAGAAAATCTCATGAAAGCAGAACAGATGCATCAAA<br>GTTTTGTGGCTGAAACAAGTCAGCGCATTAGTAAGTTACAGGAAGACACTTCTGCTCACCAGAATGTTGT<br>TGCTGAAACCTTAAGTGCCCTTGAGAACAAGGAAAAAGAGCTGCAACTTTTAAATGATAAGGTAGAAACT<br>GAGCAGGCAGAGATTCAAGAATTAAAAAAGAGCAACCATCTACTTGAAGACTCTCTAAAGGAGCTACAAC<br>TTTTATCCGAAACCCTAAGCTTGGAGAAGAAAGAAATGAGTTCCATCATTTCTCTAAATAAAAGGGAAAT<br>TGAAGAGCTGACCCAAGAGAATGGGACTCTTAAGGAAATTAATGCATCCTTAAATCAAGAGAAGATGAAC<br>TTAATCCAGAAAGTGAGAGTTTTGCAAACTATATAGATGAAAGGGAGAAAAGCATTTCAGAGTTATCTG<br>ATCAGTACAAGCAAGAAAAACTTATTTTACTACAAAGATGTGAAGAAACCGGAAATGCATATGAGGATCT<br>TAGTCAAAAATACAAAGCAGCACAGGAAAAGAATTCTAAATTAGAATGCTTGCTAAATGAATGCACTAGT<br>CTTTGTGAAAATAGGAAAGGAGTTGGAACAGCTAAAGGAAGCATTTGCAAAGGAACCAAGAATTCT<br>TAACAAAATTAGCATTTGCTGAAGAAAGAAATCAGAATCTGATGCTAGAGTTGGAGACAGTGCAGCAAGC<br>TCTGAGATCTGAGATGACAGATAACCAAAACAATTCTAAGAGCGAGGCTGGTGGTTAAAGCAAGAAATC<br>ATGACTTTAAAGGAAGAACAAAACAAAATGCAAAGGGAAGTTAATGACTTATTACAAGAGAATGAACAGC<br>TGATGAAGGTAATGAAGACTAAACATGAATGTCAAAATCTAGAATCAGAACCAATTAGGAACTCTGTGAA<br>AGAAAGAGAGAGTGAGAGAAATCAATGTAATTTTAAACCTCAGATGGATCTTGAAGTTAAAGAAATTTCT<br>CTAGATAGTTATAATGCGCAGTTGGTGCAATTAGAAGCTATGCTAAGAAATAAGGAATTAAAACTTCAGG<br>AAAGTGAGAAGGAGAAGGAGAGTGCCTGCAGCATGAATTACAGCAATTAGAGGAGATCTTGAAACCAGCAA<br>TTTGCAAGACATGCAGTCACAAGAAATTAGTGGCCTTAAAGACTGTGAAATAGATGCGGAAGAAAAGTAT<br>ATTTCAGGGCCTCATGAGTTGTCAACAAGTCAAAACGACAATGCACACCTTCAGTGCTCTCTGCAAACAA<br>CAATGAACAAGCTGAATGAGCTAGAGAAAATATGTGAAATACTGCAGGCTGAAAAGTATGAACTCGTAAC<br>TGAGCTGAATGATTCAAGGTCAGAATGTATCACAGCAACTAGGAAAATGGCAGAAGAGGTAGGGAAACTA | 104 |

(continued)

| GENBANK ACCESSION NUMBER | SEQUENCE | SEQ ID NO: |
|---|---|---|
| | CTAAATGAAGTTAAAATATTAAATGATGACAGTGGTCTTCTCCATGGTGAGTTAGTGGAAGACATACCAG<br>GAGGTGAATTTGGTGAACAACCAAATGAACAGCACCCTGTGTCTTTGGCTCCATTGGACGAGAGTAATTC<br>CTACGAGCACTTGACATTGTCAGACAAAGAAGTTCAAATGCACTTTGCCGAATTGCAAGAGAAATTCTTA<br>TCTTTACAAAGTGAACACAAAATTTTACATGATCAGCACTGTCAGATGAGCTCTAAAATGTCAGAGCTGC<br>AGACCTATGTTGACTCATTAAAGGCCGAAAATTTGGTCTTGTCAACGAATCTGAGAAACTTTCAAGGTGA<br>CTTGGTGAAGGAGATGCAGCTGGGCTTGGAGGAGGGGCTCGTTCCATCCCTGTCATCCTCTTGTGTGCCT<br>GACAGCTCTAGTCTTAGCAGTTTGGGAGACTCCTCCTTTTACAGAGCTCTTTTAGAACAGACAGGAGATA<br>TGTCTCTTTTGAGTAATTTAGAAGGGGCTGTTTCAGCAAACCAGTGCAGTGTAGGATGAAGTATTTTGCAG<br>CAGTCTGCAGGAGGAGAATCTGACCAGGAAAGAAACCCCTTCGGCCCCAGCGAAGGGTGTTGAAGAGCTT<br>GAGTCCCTCTGTGAGGTGTACCGGCAGTCCCTCGAGAAGCTAGAAGAGAAAATGGAAAGTCAAGGGATTA<br>TGAAAAATAAGGAAATTCAAGAGCTCGAGCAGTTATTAAGTTCTGAAAGGCAAGAGCTTGACTGCCTTAG<br>GAAGCAGTATTTGTCAGAAAATGAACAGTGGCAACAGAAGCTGACAAGCGTGACTCTGGAGATGGAGTCC<br>AAGTTGGCGGCAGAAAAGAAACAGACGGAACAACTGTCACTTGAGCTGGAAGTAGCACGACTCCAGCTAC<br>AAGGTCTGGACTTAAGTTCTCGGTCTTTGCTTGGCATCGACACAGAAGATGCTATTCAAGGCCGAAATGA<br>GAGCTGTGACATATCAAAAGAACATACTTCAGAAACTACAGAAAGAACACCAAAGCATGATGTTCATCAG<br>ATTTGTGATAAAGATGCTCAGCAGGACCTCAATCTAGACATTGAGAAAATAACTGAGACTGGTGCAGTGA<br>AACCCACAGGAGAGTGCTCTGGGGAACAGTCCCCAGATACCAATTATGAGCCTCCAGGGGAAGATAAAAC<br>CCAGGGCTCTTCAGAATGCATTTCTGAATTGTCATTTTCTGGTCCTAATGCTTTGGTACCTATGGATTTC<br>CTGGGGAATCAGGAAGATATCCATAATCTTCAACTGCGGGTAAAAGAGACATCAAATGAGAATTTGAGAT<br>TACTTCATGTGATAGAGGACCGTGACAGAAAGTTGAAAGTTTGCTAAATGAAATGAAATGAAGAATTAGACTC<br>AAAACTCCATTTACAGGAGGTACAACTAATGACCAAAATTGAAGCATGCATAGAATTGGAAAAAATAGTT<br>GGGGAACTTAAGAAAGAAAACTCAGATTTAAGTGAAAAATTGGAATATTTTTCTTGTGATCACCAGGAGT<br>TACTCCAGAGAGTAGAAACTTCTGAAGGCCTCAATTCTGATTTAGAAATGCATGCAGATAAATCATCACG<br>TGAAGATATTGGAGATAATGTGGCCAAGGTGAATGACAGCTGGAAGGAGAGATTCTTGATGTGGAAAAT<br>GAGCTGAGTAGGATCAGATCGGAGAAAGCTAGCATTGAGCATGAAGCCCTCTACCTGGAGGCTGACTTAG<br>AGGTAGTTCAAACAGAGAAGCTATGTTTAGAAAAAGACAATGAAAATAAGCAGAAGGTTATTGTCTGCCT<br>TGAAGAAGAACTCTCAGTGGTCACAAGTGAGAGAAACCAGCTTCGTGGAGAATTAGATACTATGTCAAAA<br>AAAACCACGGCACTGGATCAGTTGTCTGAAAAAATGAAGGAGAAAACACAAGAGCTTGAGTCTCATCAAA<br>GTGAGTGTCTCCATTGCATTCAGGTGGCAGAGGCAGAGGTGAAGGAAAAGACGGAACTCCTTCAGACTTT<br>GTCCTCTGATGTGAGTGAGCTGTTAAAAGACAAAACTCATCTCCAGGAAAAGCTGCAGAGTTTGGAAAAG<br>GACTCACAGGCACTGTCTTTGACAAAATGTGAGCTGGAAAACCAAATTGCACAACTGAATAAAGAGAAAG<br>AATTGCTTGTCAAGGAATCTGAAAGCCTGCAGGCCAGACTGAGTGAATCAGATTATGAAAAGCTGAATGT<br>CTCCAAGGCCTTGGAGGCCGCACTGGTGGAGAAAGGTGAGTTCGCATTGAGGCTGAGCTCAACACAGGAG<br>GAAGTGCATCAGCTGAGAAGAGGCATCGAGAAACTGAGAGTTCGCATTGAGGCCGATGAAAAGAAGCAGC<br>TGCACATCGCAGAGAAACTGAAAGAACGCGAGCGGGAGAATGATTCACTTAAGGATAAAGTTGAGAACCT<br>TGAAAGGGAATTGCAGATGTCAGAAGAAAACCAGGAGCTAGTGATTCTTGATGCCGAGAATTCCAAAGCA<br>GAAGTAGAGACTCTAAAAACACAAATAGAAGAGATGGCCAGAAGCCTGAAAGTTTTTGAATTAGACCTTG<br>TCACGTTAAGGTCTGAAAAAGAAAATCTGACAAAAACAAATACAAGAAAAACAAGGTCAGTTGTCAGAACT<br>AGACAAGTTACTCTCTTCATTTAAAAGTCTGTTAGAAGAAAAGGAGCAAGCAGAGATACAGATCAAAGAA<br>GAATCTAAAACTGCAGTGGAGATGCTTCAGAATCAGTTAAAGGAGCTAAATGAGGCAGTAGCAGCCTTGT<br>GTGGTGACCAAGAAATTATGAAGGCCACAGAACAGAGTCTAGACCCACCAATAGAGGAAGAGCATCAGCT<br>GAGAAATAGCATTGAAAAGCTGAGAGCCCGCCTAGAAGCTGATGAAAAGAAGCAGCTCTGTGTCTTACAA<br>CAACTGAAGGAAAGTGAGCATCATGCAGATTTACTTAAGGGTAGAGTGGAGAACCTTGAAAGAGAGCTAG<br>AGATAGCCAGGACAAACCAAGAGCATGCAGCTCTTGAGGCAGGACATTCCAAAGGAGAGGTAGAGACCCT<br>AAAAGCAAAAATAGAAGGGATGACCCAAAGTCTGAGAGGTCTGGAATTAGATGTTGTTACTATAAGGTCA<br>GAAAAAGAAAATCTGACAAATGAATTACAAAAAGAGCAAGAGCGAATATCTGAATTAGAAATAATAAATT<br>CATCATTTGAAAATATTTTGCAAGAAAAAGAGCAAGAGAAAGTACAGATGAAAGAAAAATCAAGCACTGC<br>CATGGAGATGCTTCAAACACAATTAAAAGAGCTCAATGAGAGAGTGGCAGCCCTGCATAATGACCAAGAA<br>GCCTGTAAGGCCAAAGAGCAGAATCTTAGTAGTCAAGTAGAGTGTCTTGAACTTGAGAAGGCTCAGTTGC<br>TACAAGGCCTTGATGAGGCCAAAAATAATTATATTGTTTTGCAATCTTCAGTGAATGGCCTCATTCAAGA<br>AGTAGAAGATGGCAAGCAGAAACTGGAGAAGAAGGATGAAGAAATCAGTAGACTGAAAAAATCAAATTCAA<br>GACCAAGAGCAGCTTGTCTCTAAACTGTCCCAGGTGGAAGGAGAGCACCAACTTTGGAAGGAGCAAAACT<br>TAGAACTGAGAAATCTGACAGTGGAATTGGAGCAGAAGATCCAAGTGCTACAATCCAAAAATGCCTCTTT<br>GCAGGACACATTAGAAGTGCTGCAGAGTTCTTACAAGAATCTAGAGAATGAGCTTGAATTGACAAAAATG<br>GACAAAATGTCCTTTGTTGAAAAAGTAAACAAAATGACTGCAAAGGAAACTGAGCTGCAGAGGGAAATGC<br>ATGAGATGGCACAGAAAAACAGCAGAGCTGCAAGAAGAACTCAGTGGAGAGAAAAATAGGCTAGCTGGAGA<br>GTTGCAGTTACTGTTGGAAGAAATAAAAGAGCAGCAAAGATCAATTGAAGGAGCTCACACTAGAAAATAGT<br>GAATTGAAGAAGAGCCTAGATTGCATGCACAAAGACCAGGTGGAAAAGGAAGGGAAAGTGAGAGAGGAAA<br>TAGCTGAATATCAGCTACGGCTTCATGAAGCTGAAAAGAAACACCAGGCTTTGCTTTTGGACACAAACAA<br>ACAGTATGAAGTAGAAATCCAGACATACCGAGAGAAATTGACTTCTAAAGAAGAATGTCTCAGTTCACAG<br>AAGCTGGAGATAGACCTTTTAAAGTCTAGTAAAGAAGAGCTCAATAATTCATTGAAAGCTACTACTCAGA<br>TTTTGGAAGAATTGAAGAAAACCAAGATGGACAATCTAAAATATGTAAATCAGTTGAAGGAGAAAATGA<br>ACGTGCCCAGGGGAAAATGAAGTTGTTGATCAAATCCTGTAAACAGCCTGGAGAGGAAAAGGGAGATACTG<br>CAGAAAGAACTCTCTCAACTTCAAGCTGCACAGGAGAAGCAGAAAACAGGTACTGTTATGGATACCAAGG<br>TCGATGAATTAACAACTGAGATCAAAGAACTGAAAGAAACTCTTGAAGAAAAACCAAGGAGGCAGATGA<br>ATACTTGGATAAGTACTGTTCCTTGCTTATAAGCCATGAAAAGTTAGAGAAAGCTAAAGAGATGTTAGAG<br>ACACAAGTGGCCCATCTGTGTTCACAGCAATCTAAACAAGATTCCCGAGGGTCTCCTTTGCTAGGTCCAG<br>TTGTTCCAGGACCATCTCCAATCCCTTCTGTTACTGAAAAGAGGTTATCATCTGGCCAAAATAAAGCTTC | |

(continued)

| GENBANK ACCESSION NUMBER | SEQUENCE | SEQ ID NO: |
|---|---|---|
| | AGGCAAGAGGCAAAGATCCAGTGGAATATGGGAGAATGGTAGAGGACCAACACCTGCTACCCCAGAGAGC TTTTCTAAAAAAAGCAAGAAAGCAGTCATGAGTGGTATTCACCCTGCAGAAGACACGGAAGGTACTGAGT TTGAGCCAGAGGGACTTCCAGAAGTTGTAAAGAAAGGGTTTGCTGACATCCCGACAGGAAAGACTAGCCC ATATATCCTGCGAAGAACAACCATGGCAACTCGGACCAGCCCCCGCCTGGCTGCACAGAAGTTAGCGCTA TCCCCACTGAGTCTCGGCAAAGAAAATCTTGCAGAGTCCTCCAAACCAACAGCTGGTGGCAGCAGATCAC AAAAAGGTCAAAGTTGCTCAGCGGAGCCCAGTAGATTCAGGCACCATCCTCCGAGAACCCACCACGAAATC CGTCCCAGTCAATAATCTTCCTGAGAGAAGTCCGACTGACAGCCCCAGAGAGGGCCTGAGGGTCAAGCGA GGCCGACTTGTCCCCAGCCCCAAAGCTGGACTGGAGTCCAACGGCAGTGAGAACTGTAAGGTCCAGTGAA GGCACTTTGTGTGTGCAGTACCCCTGGGAGGTGCCAGTCATTGAATAGATAAGGCTGTGCCTACAGGACTT CTCTTTAGTCAGGGCATGCTTTATTAGTGAGGAGAAAACAATTCCTTAGAAGTCTTAAATATATTGTACT CTTTAGATCTCCCATGTGTAGGTATTGAAAAAGTTTGGAAGCACTGATCACCTGTTAGCATTGCCATTCC TCTACTGCAATGTAAATAGTATAAAGCTATGTATATAAAGCTTTTTGGTAATATGTTACAATTAAAATGA CAAGCACTATATCACAATCTCTGTTTGTATGTGGGTTTTACACTAAAAAAATGCAAAACACATTTTATTC TTCTAATTAACAGCTCCTAGGAAAATGTAGACTTTTGCTTTATGATATTCTATCTGTAGTATGAGGCATG GAATAGTTTTGTATCGGGAATTTCTCAGAGCTGAGTAAAATGAAGGAAAAGCATGTTATGTGTTTTTAAG GAAAATGTGCACACATATACATGTAGGAGTGTTTATCTTTCTCTTACAATCTGTTTTAGACATCTTTGCT TATGAAACCTGTACATATGTGTGTGTGGGTATGTGTTTATTTCCAGTGAGGGCTGCAGGCTTCCTAGAGG TGTGCTATACCATGCGTCTGTCGTTGTGCTTTTTTCTGTTTTTAGACCAATTTTTTACAGTTCTTTGGTA AGCATTGTCGTATCTGGTGATGGATTAACATATAGCCTTTGTTTTCTAATAAAATAGTCGCCTTCGTTTT CTGTAAAAAAAAAAAAAAAAAAAAAAAA | |
| AB091343 | GGCACGAGGGGCCGACGCGAGCGCCGCGCTTCGCTTCAGCTGCTAGCTGGCCCAAGGGAGGCGACCGCGG AGGGTGGCGAGGGGCGGCCAGGACCCGCAGCCCCGGGGCCGGGCCGGTCCGGACCGCCAGGGAGGGCAGG TCAGTGGGCAGATCGCGTCCGCGGGATTCAATCTCTGCCCGCTCTGATAACAGTCCTTTTCCCTGGCGCT CACTTCGTGCCTGGCACCCGGCTGGGCGCCTCAAGACCGTTGTCTCTTCGATCGCTTCTTTGGACTTGGC GACCATTTCAGAGATGTCTTCCAGAAGTACCAAAGATTTAATTAAAAGTAAGTGGGGATCGAAGCCTAGT AACTCCAAATCCGAAACTACATTAGAAAAATTAAAGGGAGAAATTGCACACTTAAAGACATCAGTGGATG AAATCACAAGTGGGAAAGGAAAGCTGACTGATAAAGAGAGACACAGACTTTTGGAGAAAATTCGAGTCCT TGAGGCTGAGAGGGAGAAGAATGCCTTATCAACTCACAGAGAAGGACAAAGAAAATACAGCGACTGAGAGAC CAACTGAAGGCCAGATATAGTACTACCGCATTGCTTGAACAGCTGGAAGAGACAACGAGAGAAGGAGAAA GGAGGGAGCAGGTGTTGAAAGCCTTATCTGAAGAGAAAGACGTATTGAAACAACAGTTGTCTGCTGCAAC CTCACGAATTGCTGAACTTGAAAGCAAAACCAATACACTCCGTTTATCACAGACTGTGGCTCCAAACTGC TTCAACTCATCAATAAATAATATTCATGAAATGGAAATACAGCTGAAAGATGCTCTGGAGAAAAATCAGC AGTGGCTCGTGTATGATCAGCAGCGGGAAGTCTATGTAAAAGGACTTTTAGCAAAGATCTTTGAGTTGGA AAAGAAAACGGAAACAGCTGCTCATTCACTCCCACAGCAGACAAAAAAGCCTGAATCAGAAGGTTATCTT CAAGAAGAGACGAAATGTTACAACGATCTCTTGGCAAGTGCAAAAAAGATCTTGAGGTTGAACGAC AAACCATAACTCAGCTGAGTTTTGAACTGAGTGAATTTCGAAGAAAATATGAAGAAACCCAAAAAGAAGT TCACAATTTAAATCAGCTGTTGTATTCACAAAGAAGGGCAGATGTGCAACATCTGGAAGATGATAGGCAT AAAACAGAGAAGATACAAAAACTCAGGGAAGAGAATGATATTGCTAGGGGAAAACTTGAAGAAGAGAAGA AGAGATCCGAAGAGCTCTTATCTCAGGTCCAGTTTCTTTACACATCTCTGCTAAAGCAGCAAGAAGAACA AACAAGGGTAGCTCTGTTGGAACAACAGATGCAGGCATGTACTTTAGACTTTGAAAATGAAAAACTCGAC CGTCAACATGTGCAGCATCAATTGCATGTAATTCTTAAGGAGCTCCGAAAAGCAAGAAATCAAATAACAC AGTTGGAATCCTTGAAACAGCTTCATGAGTTTGCCATCACAGAGCCATTAGTCACTTTCCAAGGAGAGAC TGAAAACAGAGAAAAAGTTGCCGCCTCACCAAAAAGTCCCACTGCTGCACTCAATGAAAGCCTGGTGGAA TGTCCCAAGTGCAATATACAGTATCCAGCCACTGAGCATCGCGATCTGCTTGTCCATGTGGAATACTGTT CAAAGTAGCAAAATAAGTATTTGTTTTGATATTAAAAGATTCAATACTGTATTTTCTGTTAGCTTGTGGG CATTTTGAATTATATATTTCACATTTTGCATAAAACTGCCTATCTACCTTTGACACTCCAGCATGCTAGT GAATCATGTATCTTTTAGGCTGCTGTGCATTTCTCTTGGCAGTGATACCTCCCTGACATGGTTCATCATC AGGCTGCAATGACAGAATGTGGTGAGCAGCGTCTACTGAGACTACTAACATTTTGCACTGTCAAAATACT TGGTGAGGAAAAGATAGCTCAGGTTATTGCTAATGGGTTAATGCACCAGCAAGCAAAATATTTTATGTTT TGGGGGTTTGAAAAATCAAAGATAATTAACCAAGGATCTTAACTGTGTTCGCATTTTTTATCCAAGCACT TAGAAAACCTACAATCCTAATTTTGATGTCCATTGTTAAGAGGTGGTGATAGATACTATTTTTTTTTTCA TATTGTATAGCGGTTATTAGAAAAGTTGGGGATTTTCTTGATCTTATTGCTGCTTACCATTGAAACTTA ACCCAGCTGTGTTCCCCAACTCTGTTCTGCGCACGAAACAGTATCTGTTTGAGGCATAAATCTTAAGTGGC CACACACAATGTTTTCTCTTATGTTATCTGGCAGTAACTGTAACTTGAATTACATTAGCACATTCTGCTT AGCTAAAATTGTTAAAATAAACTTTAATAAACCCATGTAGCCCTCTCATTTGATTGACAGTATTTTAGTT ATTTTTGGCATTCTTAAAGCTGGGCAATGTAATGATCAGATCTTTGTTTGTCTGAACAGGTATTTTTATA CATGCTTTTTGTAAACCAAAAACTTTTAAATTTCTTCAGGTTTTCTAACATGCTTACCACTGGGCTACTG TAAATGAGAAAAGAATAAAATTATTTAATGTTTTAAAAAAAAAAAAAAA | 105 |

(continued)

| GENBANK ACCESSION NUMBER | SEQUENCE | SEQ ID NO: |
|---|---|---|
| BC006428 | GGCGGCTGAGCCTGAGCGGGGATGTAGAGGCGGCGGCAGCAGAGGCGGCACTGGCGGCAAGAGCAGACGC<br>CCGAGCCGAGCGAGAAGAGCGGCAGAGCCTTATCCCCTGAAGCCGGGCCCCGCGTCCCAGCCCTGCCCAG<br>CCCGCGCCCAGCCATGCGCGCCGCCTGCTGAGTCCGGGCGCCGCACGCTGAGCCCTCCGCCCGCGAGCCG<br>CGCTCAGCTCGGGGGTGATTAGTTGCTTTTTGTTGTTTTTTAATTTGGGCCGCGGGGAGGGGGAGGAGGG<br>GCAGGTGCTGCAGGCTCCCCCCCCTCCCCGCCTCGGGCCCAGCCGCGGCGGCGCGACTCGGGCTCCGGACC<br>CGGGCACTGCTGGCGGCTGGAGCGGAGCGCACCGCGGCGGTGGTGCCCAGAGCGGAGCGCAGCTCCCTGC<br>CCCGCCCCTCCCCCTCGGCCTCGCGGCGACGGCGGCGGTGGCGGCTTGGACGACTCGGAGAGCCGAGTGA<br>AGACATTTCCACCTGGACACCTGACCATGTGCCTGCCCTGAGCAGCGAGGCCCACCAGGCATCTCTGTTG<br>TGGGCAGCAGGGCCAGGTCCTGGTCTGTGGACCCTCGGCAGTTGGCAGGCTCCCTCTGCAGTGGGGTCTG<br>GGCCTCGGCCCCACCATGTCGAGCCTCGGCGGTGGCTCCCAGGATGCCGGCGGCAGTAGCAGCAGCAGCA<br>CCAATGGCAGCGGTGGCAGTGGCAGCAGTGGCCCAAAGGCAGGAGCAGCAGACAAGAGTGCAGTGGTGGC<br>TGCCGCCGCACCAGCCTCAGTGGCAGATGACACACCACCCCCCGAGCGTCGGAACAAGAGCGGTATCATC<br>AGTGAGCCCCTCAACAAGAGCCTGCCGCCGCTCCCGCCCGCTCTCCCACTACTCTTCTTTTGGCAGCAGTG<br>GTGGTAGTGGCGGTGGCAGCATGATGGGCGGAGAGTCTGCTGACAAGGCCACTGCGGCTGCAGCCGCTGC<br>CTCCCTGTTGGCCAATGGGCATGACCTGGCGGCGGCCATGGCGGTGGACAAAAGCAACCCTACCTCAAAG<br>CACAAAAGTGGTGCTGTGGCCAGCCTGCTGAGCAAGGCAGAGCGGGCCACGGAGCTGGCAGCCGAGGGAC<br>AGCTGACGCTGCAGCAGTTTGCGCAGTCCACAGAGATGCTGAAGCGCGTGGTGCAGGAGCATCTCCCGCT<br>GATGAGCGAGGCGGGTGCTGGCCTGCCTGACATGGAGGCTGTGGCAGGTGCCGAAGCCCTCAATGGCCAG<br>TCCGACTTCCCCTACCTGGGCGCTTTCCCCATCAACCCAGGCCTCTTCATTATGACCCCGGCAGGTGTGT<br>TCCTGGCCGAGAGCGCGCTGCACATGGCGGGCCTGGCTGAGTACCCCATGCAGGGAGAGCTGGCCTCTGC<br>CATCAGCTCCGGCAAGAAGAAGCGGAAACGCTGCGGCATGTGCGCGCCCTGCCGGCGGCGCATCAACTGC<br>GAGCAGTGCAGCAGTTGTAGGAATCGAAAGACTGGCCATCAGATTTGCAAATTCAGAAAATGTGAGGAAC<br>TCAAAAAGAAGCCTTCCGCTGCTCTGGAGAAGGTGATGCTTCCGACGGGAGCCGCCTTCCGGTGGTTTCA<br>GTGACGGCGGCGGAACCCAAAGCTGCCCTCTCCGTGCAATGTCACTGCTCGTGTGGTCTCCAGCAAGGGA<br>TTCGGGCGAAGACAAACGGATGCACCCGTCTTTAGAACCAAAAATATTCTCTCACAGATTTCATTCCTGT<br>TTTTATATATATATTTTTTGTTGTCGTTTTAACATCTCCACGTCCCTAGCATAAAAAGAAAAAGAAAAAA<br>ATTTAAACTGCTTTTTCGGAAGAACAACAACAAAAAAGAGGTAAAGACGAATCTATAAAGTACCGAGACT<br>TCCTGGGCAAAGAATGGACAATCAGTTTCCTTCCTGTGTCGATGTCGATGTTGTCTGTGCAGGAGATGCA<br>GTTTTTGTGTAGAGAATGTAAATTTTCTGTAACCTTTTGAAATCTAGTTACTAATAAGCACTACTGTAAT<br>TTAGCACAGTTTAACTCCACCCTCATTTAAACTTCCTTTGATTCTTTCCGACCATGAAATAGTGCATAGT<br>TTGCCTGGAGAATCCACTCACGTTCATAAAGAGAATGTTGATGGCGCCGTGTAGAAGCCGCTCTGTATCC<br>ATCCACGCGTGCAGAGCTGCCAGCAGGGAGCTCACAGAAGGGGAGGGAGCACCAGGCCAGCTGAGCTGCA<br>CCCACAGTCCCGAGACTGGGATCCCCCACCCCAACAGTGATTTTGGAAAAAAAAATGAAAGTTCTGTTCG<br>TTTATCCATTGCGATCTGGGGAGCCCCATCTCGATATTTCCAATCCTGGCTACTTTTCTTAGAGAAAATA<br>AGTCCTTTTTTTCTGGCCTTGCTAATGGCAACAGAAGAAAGGGCTTCTTTGCGTGGTCCCCTGCTGGTGG<br>GGGTGGGTCCCCAGGGGGCCCCCTGCGGCCTGGGCCCCCCTGCCCACGGCCAGCTTCCTGCTGATGAACA<br>TGCTGTTTGTATTGTTTTAGGAAACCAGGCTGTTTTGTGAATAAAACGAATGCATGTTTGTGTCACGAAA<br>AAAAAAAAAAAAAAAAAAAAAAAAAAAAAAAAAAAAAA | 106 |

(continued)

| GENBANK ACCESSION NUMBER | SEQUENCE | SEQ ID NO: |
|---|---|---|
| NM_005228 | CCCCGGCGCAGCGCGGCCGCAGCAGCCTCCGCCCCCCGCACGGTGTGAGCGCCCGACGCGGCCGAGGCGG<br>CCGGAGTCCCGAGCTAGCCCCGGCGGCCGCCGCCGCCCAGACCGGACGACAGGCCACCTCGTCGGCGTCC<br>GCCCGAGTCCCCGCCTCGCCGCCAACGCCACAACCACCGCGCACGGCCCCCTGACTCCGTCCAGTATTGA<br>TCGGGAGAGCCGGAGCGAGCTCTTCGGGGAGCAGCGATGCGACCCTCCGGGACGGCCGGGGCAGCGCTCC<br>TGGCGCTGCTGGCTGCGCTCTGCCCGGCGAGTCGGGCTCTGGAGGAAAAGAAAGTTTGCCAAGGCACGAG<br>TAACAAGCTCACGCAGTTGGGCACTTTTGAAGATCATTTTCTCAGCCTCCAGAGGATGTTCAATAACTGT<br>GAGGTGGTCCTTGGGAATTTGGAAATTACCTATGTGCAGAGGAATTATGATCTTTCCTTCTTAAAGACCA<br>TCCAGGAGGTGGCTGGTTATGTCCTCATTGCCCTCAACACAGTGGAGCGAATTCCTTTGGAAAACCTGCA<br>GATCATCAGAGGAAATATGTACTACGAAAATTCCTATGCCTTAGCAGTCTTATCTAACTATGATGCAAAT<br>AAAACCGGACTGAAGGAGCTGCCCATGAGAAATTTACAGGAAATCCTGCATGGCGCCGTGCGGTTCAGCA<br>ACAACCCTGCCCTGTGCAACGTGGAGAGCATCCAGTGGCGGGACATAGTCAGCAGTGACTTTCTCAGCAA<br>CATGTCGATGGACTTCCAGAACCACCTGGGCAGCTGCCAAAAGTGTGATCCAAGCTGTCCCAATGGGAGC<br>TGCTGGGGTGCAGGAGAGGAGAACTGCCAGAAACTGACCAAAATCATCTGTGCCCAGCAGTGCTCCGGGC<br>GCTGCCGTGGCAAGTCCCCCAGTGACTGCTGCCACAACCAGTGTGCTGCAGGCTGCACAGGCCCCCGGGA<br>GAGCGACTGCCTGGTCTGCCGCAAATTCCGAGACGAAGCCACGTGCAAGGACACCTGCCCCCCACTCATG<br>CTCTACAACCCCACCACGTACCAGATGGATGTGAACCCCGAGGGCAAATACAGCTTTGGTGCCACCTGCG<br>TGAAGAAGTGTCCCCGTAATTATGTGGTGACAGATCACGGCTCGTGCGTCCGAGCCTGTGGGGCCGACAG<br>CTATGAGATGGAGGAAGACGGCGTCCGCAAGTGTAAGAAGTGCGAAGGGCCTTGCCGCAAAGTGTGTAAC<br>GGAATAGGTATTGGTGAATTTAAAGACTCACTCTCCATAAATGCTACGAATATTAAACACTTCAAAAACT<br>GCACCTCCATCAGTGGCGATCTCCACATCCTGCCGGTGGCATTTAGGGGTGACTCCTTCACACATACTCC<br>TCCTCTGGATCCACAGGAACTGGATATTCTGAAAACCGTAAAGGAAATCACAGGGTTTTTGCTGATTCAG<br>GCTTGGCCTGAAAACAGGACGGACCTCCATGCCTTTGAGAACCTAGAAATCATACGCGGCAGGACCAAGC<br>AACATGGTCAGTTTTCTCTTGCAGTCGTCAGCCTGAACATAACATCCTTGGGATTACGCTCCCTCAAGGA<br>GATAAGTGATGGAGATGTGATAATTTCAGGAAACAAAAATTTGTGCTATGCAAATACAATAAACTGGAAA<br>AAACTGTTTGGGACCTCCGGTCAGAAAACCAAAATTATAAGCAACAGAGGTGAAAACAGCTGCAAGGCCA<br>CAGGCCAGGTCTGCCATGCCTTGTGCTCCCCCGAGGGCTGCTGGGGCCCGGAGCCCAGGGACTGCGTCTC<br>TTGCCGGAATGTCAGCCGAGGCAGGGAATGCGTGGACAAGTGCAACCTTCTGGAGGGTGAGCCAAGGGAG<br>TTTGTGGAGAACTCTGAGTGCATACAGTGCCACCCAGAGTGCCTGCCTCAGGCCATGAACATCACCTGCA<br>CAGGACGGGGACCAGACAACTGTATCCAGTGTGCCCACTACATTGACGGCCCCCACTGCGTCAAGACCTG<br>CCCGGCAGGAGTCATGGGAGAAAACAACACCCTGGTCTGGAAGTACGCAGACGCCGGCCATGTGTGCCAC<br>CTGTGCCATCCAAACTGCACCTACGGATGCACTGGGCCAGGTCTTGAAGGCTGTCCAACGAATGGGCCTA<br>AGATCCCGTCCATCGCCACTGGGATGGTGGGGGCCCTCCTCTTGCTGCTGGTGGTGGCCCTGGGGATCGG<br>CCTCTTCATGCGGAGGCGCCACATCGTTCGGAAGCGCACGCTGCGGAGGCTGCTGCAGGAGAGGGAGCTT<br>GTGGAGCCTCTTACACCCAGTGGAGAAGCTCCCAACCAAGCTCTCTTGAGGATCTTGAAGGAAACTGAAT<br>TCAAAAAGATCAAAGTGCTGGGCTCCGGTGCGTTCGGCACGGTGTATAAGGGACTCTGGATCCCAGAAGG<br>TGAGAAAGTTAAAATTCCCGTCGCTATCAAGGAATTAAGAGAAGCAACATCTCCGAAAGCCAACAAGGAA<br>ATCCTCGATGAAGCCTACGTGATGGCCAGCGTGGACAACCCCCACGTGTGCCGCCTGCTGGGCATCTGCC<br>TCACCTCCACCGTGCAGCTCATCACGCAGCTCATGCCCTTCGGCTGCCTCCTGGACTATGTCCGGGAACA<br>CAAAGACAATATTGGCTCCCAGTACCTGCTCAACTGGTGTGTGCAGATCGCAAAGGGCATGAACTACTTG<br>GAGGACCGTCGCTTGGTGCACCGCGACCTGGCAGCCAGGAACGTACTGGTGAAAACACCGCAGCATGTCA | 107 |

(continued)

| GENBANK ACCESSION NUMBER | SEQUENCE | SEQ ID NO: |
|---|---|---|
| | AGATCACAGATTTTGGGCTGGCCAAACTGCTGGGTGCGGAAGAGAAAGAATACCATGCAGAAGGAGGCAA AGTGCCTATCAAGTGGATGGCATTGGAATCAATTTTACACAGAATCTATACCCACCAGAGTGATGTCTGG AGCTACGGGGTGACCGTTTGGGAGTTGATGACCTTTGGATCCAAGCCATATGACGGAATCCCTGCCAGCG AGATCTCCTCCATCCTGGAGAAAGGAGAACGCCTCCCTCAGCCACCCATATGTACCATCGATGTCTACAT GATCATGGTCAAGTGCTGGATGATAGACGCAGATAGTCGCCCAAAGTTCCGTGAGTTGATCATCGAATTC TCCAAAATGGCCCGAGACCCCCAGCGCTACCTTGTCATTCAGGGGGATGAAAGAATGCATTGCCAAGTC CTACAGACTCCAACTTCTACCGTGCCCTGATGGATGAAGAAGACATGGACGACGTGGTGGATGCCGACGA GTACCTCATCCCACAGCAGGGCTTCTTCAGCAGCCCCTCCACGTCACGGACTCCCCTCCTGAGCTCTCTG AGTGCAACCAGCAACAATTCCACCGTGGCTTGCATTGATAGAAATGGGCTGCAAAGCTGTCCCATCAAGG AAGACAGCTTCTTGCAGCGATACAGCTCAGACCCCACAGGCGCCTTGACTGAGGACAGCATAGACGACAC CTTCCTCCCAGTGCCTGAATACATAAAACCAGTCCGTTCCCAAAAGGCCCGCTGGCTCTGTGCAGAATCCT GTCTATCACAATCAGCCTCTGAACCCCGCGCCCAGCAGAGACCCACACTACCAGGACCCCCACAGCACTG CAGTGGGCAACCCCGAGTATCTCAACACTGTCCAGCCCACCTGTGTCAACAGCACATTCGACAGCCCTGC CCACTGGGCCCAGAAAGGCAGCCACCAAATTAGCCTGGACAACCCTGACTACCAGCAGGACTTCTTTCCC AAGGAAGCCAAGCCAAATGGCATCTTTAAGGGCTCCACAGCTGAAAATGCAGAATACCTAAGGGTCGCGC CACAAAGCAGTGAATTTATTGGAGCATGACCACGGAGGATAGTAGCTGAGCCCTAAAAATCCAGACTCTTTC GATACCCAGGACCAAGCCACAGCAGGTCCTCCATCCCAACAGCCATGCCCGCCATTAGCTCTTAGACCCAC AGACTGGTTTTGCAACGTTTACACCGACTAGCCAGGAAGTACTTCCACCTCGGGCACATTTTGGGAAGTT GCATTCCTTTGTCTTCAAACTGTGAAGCATTTACAGAAACGCATCCAGCAAGAATATTGTCCCTTTGAGC AGAAATTTATCTTTCAAAGAGGTATATTTGAAAAAAAAAAAAAAGTATATGTGAGGATTTTTATTGATTGG GGATCTTGGAGTTTTTCATTGTCGCTATTGATTTTTACTTCAATGGGCTCTTCCAACAAGGAAGAAGCTT GCTGGTAGCACTTGCTACCCTGAGTTCATCCAGGCCCAACTGTGAGCAAGGAGCACAAGCCACAAGTCTT CCAGAGGATGCTTGATTCCAGTGGTTCTGCTTCAAGGCTTCCACTGCAAAACACTAAAGATCCAAGGAAGG CCTTCATGGCCCCAGCAGGCCGGATCGGTACTGTATCAAGTCATGGCAGGTACAGTAGGATAAGCCACTC TGTCCCTTCCTGGGCAAAGAAGAAACGGAGGGGATGGAATTCTTCCTTAGACTTACTTTTGTAAAAATGT CCCCACGGTACTTACTCCCCACTGATGGACCAGTGGTTTCCAGTCATGAGCGTTAGACTGACTTGTTTGT CTTCCATTCCATTGTTTTGAAACTCAGTATGCTGCCCCTGTCTTGCTGTCATGAAATCAGCAAGAGAGGA TGACACATCAAATAATAACTCGGATTCCAGCCCACATTGGATTCATCAGCATTTGGACCAATAGCCCACA GCTGAGAATGTGGAATACCTAAGGATAGCACCGCTTTTGTTCTCGCAAAAACGTATCTCCTAATTTGAGG CTCAGATGAAATGCATCAGGTCCTTTGGGGCATAGATCAGAAGACTACAAAAATGAAGCTGCTCTGAAAT CTCCTTTAGCCATCACCCCAACCCCCCAAAATTAGTTTGTTTACTTATGGAAGATAGTTTTCTCCTTTT ACTTCACTTCAAAAGCTTTTTACTCAAAGAGTATATGTTCCCTCCAGGTCAGCTGCCCCCAAACCCCCTC CTTACGCTTTGTCACACAAAAAGTGTCTCTGCCTTGAGTCATCTATTCAAGCACTTACAGCTCTGGCCAC AACAGGGCATTTTACAGGTGCGAATGACAGTAGCATTATGAGTAGTGTGGAATTCAGGTAGTAAATATGA AACTAGGGTTTGAAATTGATAATGCTTTCACAACATTTGCAGATGTTTTAGAAGGAAAAAAGTTCCTTCC TAAAATAATTTCTCTACAATTGGAAGATTGGAAGATTCAGCTAGTTAGGAGCCCACCTTTTTTCCTAATC TGTGTGTGCCCTGTAACCTGACTGGTTAACAGCAGTCCTTTGTAAACAGTGTTTTAAACTCTCCTAGTCA ATATCCACCCCATCACCCCAACCCCCCAAAATTAGTTCAGAAAAATATTTTCAGCCTACAGTTATGTTCA GTCACACACACATACAAAATGTTCCTTTTGCTTTTAAAGTAATTTTTGACTCCCAGATCAGTCAGAGCCC CTACAGCATTGTTAAGAAAGTATTTGATTTTTGTCTCAATGAAAATAAAACTATATTCATTTCCACTCTA AAAAAAAAAAAAAAAA | |
| NM_001005862 | GTTCCCGGATTTTTGTGGGCGCCTGCCCCGCCCCTCGTCCCCCTGCTGTGTCCATATATCGAGGCGATAG GGTTAAGGGAAGGCGGACGCCTGATGGGTTAATGAGCAAACTGAAGTGTTTTCCATGATCTTTTTTGAGT CGCAATTGAAGTACCACCTCCCGAGGGTGATTGCTTCCCCATGCGGGGTAGAACCTTTGCTGTCCTGTTC ACCACTCTACCTCCAGCACAGAATTTGGCTTATGCCTACTCAATGTGAAGATGATGAGGATGAAAACCTT TGTGATGATCCACTTCCACTTAATGAATGGTGGCAAAGCAAAGCTATATTCAAGACCACATGCAAAGCTA CTCCCTGAGCAAAGAGTCACAGATAAAACGGGGGCACCAGTAGAATGGCCAGGACAAACGCAGTGCAGCA CAGAGACTCAGACCCTGGCAGCCATGCCTGCGCAGGCAGTGATGAGAGTGACATGTACTGTTGTGGGACAT GCACAAAAGTGAGTGTGCACCGGCACAGACATGAAGCTGCGGGCTCCCTGCCAGTCCCGAGACCCACCTGG ACATGCTCCGCCACCTCTACCAGGGCTGCCAGGTGGTGCAGGGAAACCTGGAACTCACCTACCTGCCCCAC CAATGCCAGCCTGTCCTTCCTGCCAGGATATCCAGGAGGTGCAGGGCTACGTGCTCATCGCTCACAACCAA GTGAGGCAGGTCCCACTGCAGAGGCTGCGGATTGTGCGAGGCACCCAGCTCTTTGAGGACAACTATGCCC TGGCCGTGCTAGACAATGGAGACCCGCTGAACAATACCACCCCTGTCACAGGGGCCTCCCCAGGAGGCCT GCGGGAGCTGCAGCTTCGAAGCCTCACAGAGATCTTGAAAGGAGGGGTCTTGATCCAGCGGAACCCCCAG CTCTGCTACCAGGACACGATTTTGTGGAAGGACATCTTCCACAAGAACAACCAGCTGGCTCTCACACTGA TAGACACCAACCGCTCTCGGGCCTGCCACCCCTGTTCTCCGATGTGTAAGGGCTCCCGCTGCTGGGGAGA GAGTTCTGAGGATTGTCAGAGCCTGACGCGCACTGTTGTGGCCCGGCTGCACGGGCCCCAAGCACTCTGACTGCCTGG CCTGCCTCCACTTCAACCACAGTGGCATCTGTGAGCTGCACTGCCCAGCCCTGGTCACCTACAACACAGA CACGTTTGAGTCCATGCCCAATCCCGAGGGCCGGTATACATTCGGCGCCAGCTGTGTGACTGCCTGTCCC TACAACTACCTTTCTACGGACGTGGGATCCTGCACCTCGTCTGCCCCCTGCACAACCAAGAGGTGACAG CAGAGGATGGAACACAGCGGTGTGAGAAGTGCAGCAAGCCCTGTGCCCGAGTGTGCTATGGTCTGGGCAT GGAGCACTTGCGAGAGGTGAGGGCAGTTACCAGTGCCAATATCCAGGAGTTTGCTGGCTGCAAGAAGATC TTTGGGAGCCTGGCATTTCTGCCGGAGAGCTTTGATGGGGACCCAGCCTCCAACACTGCCCCGCTCCAGC CAGAGCAGCTCCAAGTGTTTGAGACTCTGGAAGAGATCACAGGTTACCTATACATCTCAGCATGGCCGGA CAGCCTGCCTGACCTCAGCGTCTTCCAGAACCTGCAAGTAATCCGGGGACGAATTCTGCACAATGGCGCC TACTCGCTGACCCTGCAAGGGCTGGGCATCAGCTGGCTGGGGCTGCGCTCACTGAGGGAACTGGGCAGTG GACTGGCCCTCATCCACCATAACACCCACCTCTGCTTCGTGCACACGGTGCCCTGGGACCAGCTCTTTCG | 108 |

**EP 2 852 689 B1**

(continued)

| GENBANK ACCESSION NUMBER | SEQUENCE | SEQ ID NO: |
|---|---|---|
| | GAACCCGCACCAAGCTCTGCTCCACACTGCCAACCGGCCAGAGGACGAGTGTGTGGGCGAGGGCCTGGCC<br>TGCCACCAGCTGTGCGCCCGAGGGCACTGCTGGGGTCCAGGGCCCCACCCAGTGTGTCAACTGCAGCCAGT<br>TCCTTCGGGGCCAGGAGTGCGTGGAGGAATGCCGAGTACTGCAGGGGCTCCCCAGGGAGTATGTGAATGC<br>CAGGCACTGTTTGCCGTGCCACCCTGAGTGTCAGCCCCAGAATGGCTCAGTGACCTGTTTTGGACCGGAG<br>GCTGACCAGTGTGTGGCCTGTGCCCACTATAAGGACCCTCCCTTCTGCGTGGCCCGCTGCCCCAGCGGTG<br>TGAAACCTGACCTCTCCTACATGCCCATCTGGAAGTTTCCAGATGAGGAGGGCGCATGCCAGCCTTGCCC<br>CATCAACTGCACCCACTCCTGTGTGGACCTGGATGACAAGGGCTGCCCCGCCGAGCAGAGAGCCAGCCCT<br>CTGACGTCCATCATCTCTGCGGTGGTTGGCATTCTGCTGGTCGTGGTCTTGGGGGTGGTCTTTGGGATCC<br>TCATCAAGCGACGGCAGCAGAAGATCCGGAAGTACACGATGCGGAGACTGCTGCAGGAAACGGAGCTGGT<br>GGAGCCGCTGACACCTAGCGGAGCGATGCCCAACCAGGCGCAGATGCGGGATCCTGAAAGAGACGGAGCTG<br>AGGAAGGTGAAGGTGCTTGGATCTGGCGCTTTTGGCACAGTCTACAAGGGCATCTGGATCCCTGATGGGG<br>AGAATGTGAAAATTCCAGTGGCCATCAAAGTGTTGAGGGAAAACACATCCCCAAAGCCAACAAAGAAAT<br>CTTAGACGAAGCATACGTGATGGCTGGTGTGGGCTCCCCATATGTCTCCCGCCTTCTGGGCATCTGCCTG<br>ACATCCACGGTGCAGCTGGTGACACAGCTTATGCCCTATGGCTGCCTCTTAGACCATGTCCGGGAAAACC<br>GCGGACGCCTGGGCTCCCAGGACCTGCTGAACTGGTGTATGCAGATTGCCAAGGGGATGAGCTACCTGGA<br>GGATGTGCGGCTCGTACACAGGGACTTGGCCGCTCGGAACGTGCTGGTCAAGAGTCCCAACCATGTCAAA<br>ATTACAGACTTCGGGCTGGCTCGGCTGCTGGACATTGACGAGACAGAGTACCATGCAGATGGGGGCAAGG<br>TGCCCATCAAGTGGATGGCGCTGGAGTCCATTCTCCGCCGGCGGTTCACCCACCAGAGTGATGTGTGGAG<br>TTATGGTGTGACTGTGTGGGAGCTGATGACTTTTGGGGCCAAACCTTACGATGGGATCCCAGCCCGGGAG<br>ATCCCTGACCTGCTGGAAAAGGGGGAGCGGCTGCCCCAGCCCCCCATCTGCACCATTGATGTCTACATGA<br>TCATGGTCAAATGTTGGATGATTGACTCTGAATGTCGGCCAAGATTCCGGGAGTTGGTGTCTGAATTCTC<br>CCGCATGGCCAGGGACCCCCAGCGCTTTGTGGTCATCCAGAATGAGGACTTGGGCCCAGCCAGTCCCTTG<br>GACAGCACCTTCTACCGCTCACTGCTGGAGGACGATGACATGGGGGACCTGGTGGATGCTGAGGAGTATC<br>TGGTACCCCAGCAGGGCTTCTTCTGTCCAGACCCTGCCCCGGGCGCTGGGGGCATGGTCCACCACAGGCA<br>CCGCAGCTCATCTACCAGGAGTGGCGGTGGGGACCTGACACTAGGGCTGGGAGCCCTCTGAAGAGGAGCC<br>CCCAGGTCTCCACTGGCACCCTCCGAAGGGGCTGGCTCCGATGTATTTGATGGTGACCTGGGAATGGGGG<br>CAGCCAAGGGGCTGCAAAGCCTCCCCACACATGACCCCAGCCCTCTACAGCGGTACAGTGAGGACCCCAC<br>AGTACCCCTGCCCTCTGAGACTGATGGCTACGTTGCCCCCCTGACCTGCAGCCCCCAGCCTGAATATGTG<br>AACCAGCCAGATGTTCGGCCCCAGCCCCCTTCGCCCCGAGAGGGCCCTCTGCCTGCTGCCCGACCTGCTG<br>GTGCCACTCTGGAAAGGCCCAAGACTCTCTCCCCAGGGAAGAATGGGGTCGTCAAAGACGTTTTTGCCTT<br>TGGGGGTGCCGTGGAGAACCCCGAGTACTTGACACCCCAGGGAGGAGCTGCCCCTCAGCCCCACCCTCCT<br>CCTGCCTTCAGCCCAGCCTTCGACAACCTCTATTACTGGGACCAGGACCCACCAGAGCGGGGGGCTCCAC<br>CCAGCACCTTCAAAGGGACACCTACGGCAGAGAACCCAGAGTACCTGGGTCTGGACGTGCCAGTGTGAAC<br>CAGAAGGCCAAGTCCGCAGAAGCCCTGATGTGTCCTCAGGGAGCAGGGAAGGCCTGACTTCTGCTGGCAT<br>CAAGAGGTGGGAGGGCCCTCCGACCACTTCCAGGGGAACCTGCCATGCCAGGAACCTGTCCTAAGGAACC<br>TTCCTTCCTGCTTGAGTTCCCAGATGGCTGGAAGGGGTCCAGCCTCGTTGGAAGAGGAACAGCACTGGGG<br>AGTCTTTGTGGATTCTGAGGCCCTGCCCAATGAGACTCTAGGGTCCAGTGGATGCCACAGCCCAGCTTGG<br>CCCTTTCCTTCCAGATCCTGGGTACTGAAAGCCTTAGGGAAGCTGGCCTGAGAGGGGAAGCGGCCCTAAG<br>GGAGTGTCTAAGAACAAAAGCGACCCATTCAGAGACTGTCCCTGAAACCTAGTACTGCCCCCCCATGAGGA<br>AGGAACAGCAATGGTGTCAGTATCCAGGCTTTGTACAGAGTGCTTTTCTGTTTAGTTTTTACTTTTTTTG<br>TTTTGTTTTTTTAAAGATGAAATAAAGACCCAGGGGGAGAATGGGTGTTGTATGGGGAGGCAAGTGTGGG<br>GGGTCCTTCTCCACACCCACTTTGTCCATTTGCAAATATATTTTGGAAAACAGCTA | |
| NM_001122742 | ATGGTCATAACAGCCTCCTGTCTACCGACTCAGAACGGATTTTACCAAAACTGAAAATGCAGGCTCCATG<br>CTCAGAAGCTCTTTAACAGGCTCGAAAGGTCCATGCTCCTTTCTCCTGCCCATTCTATAGCATAAGAAGA<br>CAGTCTCTGAGTGATAATCTTCTCTTCAAGAAGAAGAAAACTAGGAAGGAGTAAGCACAAAGATCTCTTC<br>ACATTCTCCGGGACTGCGGTACCAAATATCAGCACAGCACTTCTTGAAAAAGGATGTAGATTTTAATCTG<br>AACTTTGAACCATCACTGAGGTGGCCCGCCGGTTTCTGAGCCTTCTGCCCTGCGGGGACACGGTCTGCAC<br>CCTGCCCGCGGCCACGGACCATGACCATGACCCTCCACACCAAAGCATCTGGGATGGCCCTACTGCATCA<br>GATCCAAGGGAACGAGCTGGAGCCCCTGAACCGTCCGCAGCTCAAGATCCCCCTGGAGCGGCCCCCTGGGC<br>GAGGTGTACCTGGACAGCAGCAAGCCCGCCGTGTACAACTACCCCGAGGGCGCCGCCTACGAGTTCAACG<br>CCGCGGCCGCCGCCAACGCGCAGGTCTACGGTCAGACCGGCCTCCCCTACGGCCCCGGGTCTGAGGCTGC<br>GGCGTTCGGCTCCAACGGCCTGGGGGGTTTCCCCCCACTCAACAGCGTGTCTCCGAGCCCGCTGATGCTA<br>CTGCACCCGCCGCCGCAGCTGTCGCCCTTCCTGCAGCCCCACGGCCAGCAGGTGCCCTACTACCTGGAGA<br>ACGAGCCCAGCGGCTACACGGTGCGCGAGGCCGGCCCGCCGGCATTCTACAGGCCAAATTCAGATAATCG<br>ACGCCAGGGTGGCAGAGAAAGATTGGCCAGTACCAATGACAAGGGAAGTATGGCTATGGAATCTGCCAAG<br>GAGACTCGCTACTGTGCAGTGTGCAATGACTATGCTTCAGGCTACCATTATGGAGTCTGGTCCTGTGAGG<br>GCTGCAAGACCTTCTTCAAGAGAAGTATTCAAGGACATAAGACTATATGTGTCCAGCCACCAACCAGTG<br>CACCATTGATAAAAACAGGAGGAAGAGCTGCCAGGCCTGCCGGCTCCGCAAATGCTACGAAGTGGGAATG<br>ATGAAAGGTGGGATACGAAAAGACCGAAGAGGAGGGAGAATGTTGAAACACAAGCGCCAGAGAGATGATG<br>GGGAGGGCAGGGGTGAAGTGGGGTCTGCTGGAGACATGAGAGCTGCCAACCTTTGGCCAAGCCCGCTCAT<br>GATCAAACGCTCTAAGAAGAACAGCCTGGCCTTGTCCCTGACGGCCGACCAGATGGTCAGTGCCTTGTTG<br>GATGCTGAGCCCCCCATACTCTATTCCGAGTATGATCCTACCAGACCCTTCAGTGAAGCTTCGATGATGG<br>GCTTACTGACCAACCTGGCAGACAGGGAGCTGGTTCACATGATCAACTGGGCGAAGAGGGTGCCAGGCTT<br>TGTGGATTTGACCCTCCATGATCAGGTCCACCTTCTAGAAATGTGCCTGGCTAGAGATCCTGATGATTGGT<br>CTCGTCTGGCGCTCCATGGAGCACCCAGGGAAGCTACTGTTTGCTCCTAACTTGCTCTTGGACAGGAACC<br>AGGGAAAATGTGTAGAGGGCATGGTGGAGATCTTCGACATGCTGCTGGCTACATCATCTCGGTTCCGCAT<br>GATGAATCTGCAGGGAGAGGAGTTTGTGTGCCTCAAATCTATTATTTTGCTTAATTCTGGAGTGTACACA<br>TTTCTGTCCAGCACCCTGAAGTCTCTGGAAGAGAAGGACCATATCCACCGAGTCCTGGACAAGATCACAG | 109 |

26

(continued)

| GENBANK ACCESSION NUMBER | SEQUENCE | SEQ ID NO: |
|---|---|---|
| | ACACTTTGATCCACCTGATGGCCAAGGCAGGCCTGACCCTGCAGCAGCAGCACCAGCGGCTGGCCCAGCT CCTCCTCATCCTCTCCCACATCAGGCACATGAGTAACAAAGGCATGGAGCATCTGTACAGCATGAAGTGC AAGAACGTGGTGCCCCTCTATGACCTGCTGCTGGAGATGCTGGACGCCCACCGCCTACATGCGCCCACTA GCCGTGGAGGGGCATCCGTGGAGGAGACGGACCAAAGCCACTTGGCCACTGCGGGCTCTACTTCATCGCA TTCCTTGCAAAAGTATTACATCACGGGGGGAGGCAGAGGGTTTCCCTGCCACGGTCTGAGAGCTCCCTGGC TCCCACACGGTTCAGATAATCCCTGCTGCATTTTACCCTCATCATGCACCACTTTAGCCAAATTCTGTCT CCTGCATACACTCCGGCATGCATCCAACACCAATGGCTTTCTAGATGAGTGGCCATTCATTTGCTTGCTC AGTTCTTAGTGGCACATCTTCTGTCTTCTGTTGGGAACAGCCAAAGGGATTCCAAGGCTAAATCTTTGTA ACAGCTCTCTTTCCCCCTTGCTATGTTACTAAGCGTGAGGATTCCCGTAGCTCTTCACAGCTGAACTCAG TCTATGGGTTGGGGCTCAGATAACTCTGTGCATTTAAGCTACTTGTAGAGACCCAGGCCTGGAGAGTAGA CATTTTGCCTCTGATAAGCACTTTTTAAATGGCTCTAAGAATAAGCCACAGCAAAGAATTTAAAGTGGCT CCTTTAATTGGTGACTTGGAGAAAGCTAGGTCAAGGGTTTATTATAGCACCCTCTTGTATTCCTATGGCA ATGCATCCTTTTATGAAAGTGGTACACCTTAAAGCTTTTATATGACTGTAGCAGAGTATCTGGTGATTGT CAATTCATTCCCCCTATAGGAATACAAGGGGCACACAGGGAAGGCAGATCCCCTAGTTGGCAAGACTATT TTAACTTGATACACTGCAGATTCAGATGTGCTGAAAGCTCTGCCTCTGGCTTTCCGGTCATGGGTTCCAG TTAATTCATGCCTCCCATGGACCTATGGAGAGCAGCAAGTTGATCTTAGTTAAGTCTCCCTATATGAGGG ATAAGTTCCTGATTTTTGTTTTTATTTTTGTGTTACAAAAGAAAGCCCTCCCTCCCTGAACTTGCAGTAA GGTCAGCTTCAGGACCTGTTCCAGTGGGCACTGTACTTGGATCTTCCCGGCGTGTGTGTGCCTTACACAG GGGTGAACTGTTCACTGTGGTGATGCATGATGAGGGTAAATGGTAGTTGAAAGGAGCAGGGGCCCTGGTG TTGCATTTAGCCCTGGGGCATGGAGCTGAACAGTACTTGTGCAGGATTGTTGTGGCTACTAGAGAACAAG AGGGAAAGTAGGGCAGAAACTGGATACAGTTCTGAGGCACAGCCAGACTTGCTCAGGGTGGCCCTGCCAC AGGCTGCAGCTACCTAGGAACATTCCTTGCAGACCCCGCCATTGCCCTTTGGGGGTGCCCTGGGATCCCTG GGGTAGTCCAGCTCTTCTTCATTTCCCAGCGTGGCCCTGGTTGGAAGAAGCAGCTGTCACAGCTGCTGTA GACAGCTGTGTTCCTACAATTGGCCCAGCACCCTGGGGCACGGGAGAAGGGTGGGGACCGTTGCTGTCAC TACTCAGGCTGACTGGGGCCTGGTCAGATTACGTATGCCCTTGGTGGTTTAGAGATAATCCAAAATCAGG GTTTGGTTTGGGGAAGAAAATCCTCCCCCTTCCTCCCCCGCCCCGTTCCCTACCGCCTCCACTCCTGCCA GCTCATTTCCTTCAATTTCCTTTGACCTATAGGCTAAAAAAGAAAGGCTCATTCCAGCCACAGGGCAGCC TTCCCTGGGCCTTTGCTTCTCTAGCACAATTATGGGTTACTTCCTTTTTCTTAACAAAAAAGAATGTTTG ATTTCCTCTGGGTGACCTTATTGTCTGTAATTGAAACCCTATTGAGAGGTGATGTCTGTGTTAGCCAATG ACCCAGGTGAGCTGCTCGGGCTTCTCTTGGTATGTCTTGTTTGGAAAAGTGGATTTCATTCATTTCTGAT TGTCCAGTTAAGTGATCACCAAAGGACTGAGAATCTGGGAGGGCAAAAAAAAAAAAAAAAAGTTTTTATGTG CACTTAAATTTGGGGACAATTTTATGTATCTGTGTTAAGGATATGTTTAAGAACATAATTCTTTTGTTGC TGTTTGTTTAAGAAGCACCTTAGTTTGTTTAAGAAGCACCTTATATAGTATAATATATATTTTTTTGAAA TTACATTGCTTGTTTATCAGACAATTGAATGTAGTAATTCTGTTCTGGATTTAATTTGACTGGGTTAACA TGCAAAAACCAAGGAAAAATATTTAGTTTTTTTTTTTTTTTTTTGTATACTTTTCAAGCTACCTTGTCATG TATACAGTCATTTATGCCTAAAGCCTGGTGATTATTCATTTAAATGAAGATCACATTTCATATCAACTTT TGTATCCACAGTAGACAAAATAGCACTAATCCAGATGCCTATTGTTGGATACTGAATGACAGACAATCTT ATGTAGCAAAGATTATGCCTGAAAAGGAAAATTATTCAGGGCAGCTAATTTTGCTTTTACCAAAATATCA GTAGTAATATTTTTGGACAGTAGCTAATGGGTCAGTGGGTTCTTTTTAATGTTTATACTTAGATTTTCTT TTAAAAAAATTAAAATAAAACAAAAAAAAATTTCTAGGACTAGACGATGTAATACCAGCTAAAGCCAAAC AATTATACAGTGGAAGGTTTTACATTATTCATCCAATGTGTTTCTATTCATGTTAAGATACTACTACATT TGAAGTGGGCAGAGAACATCAGATGATTGAAATGTTCGCCCAGGGGTCTCCAGCAACTTTGGAAATCTCT TTGTATTTTTACTTGAAGTGCCACTAATGGACAGCAGATATTTTCTGGCTGATGTTGGTATTGGGTGTAG GAACATGATTTAAAAAAAAAACTCTTGCCTCTGCTTTCCCCCACTCTGAGGCAAGTTAAAATGTAAAGAT GTGATTTATCTGGGGGGGCTCAGGTATGGTGGGGAAGTGGATTCAGGAATCTGGGGAATGGCAAATATATT AAGAAGAGTATTGAAAGTATTTGGAGGAAAATGGTTAATTCTGGGTGTGCACCAGGGTTCAGTAGAGTCC ACTTCTGCCCTGGAGACCACAAATCAACTAGCTCCATTTACAGCCATTTCTAAAATGGCAGCTTCAGTTC TAGAGAAGAAAGAACAACATCAGCAGTAAAGTCCATGGAATAGCTAGTGGTCTGTGTTTCTTTTCGCCAT TGCCTAGCTTGCCGTAATGATTCTATAATGCCATCATGCAGCAATTATGAGAGGCTAGGTCATCCAAAGA GAAGACCCTATCAATGTAGGTTGCAAAATCTAACCCCTAAGGAAGTGCAGTCTTTGATTTGATTTCCCTA GTAACCTTGCAGATATGTTTAACCAAGCCATAGCCCATGCCTTTTGAGGGCTGAACAAATAAGGGACTTA CTGATAATTTACTTTTGATCACATTAAGGTGTTCTCACCTTGAAATCTTATACACTGAAATGGCCATTGA TTTAGGCCACTGGCTTGAGACTACCTTCCCCTGCATGACACTGATTACAAAATACTTTCCTATTCATACT TTCCAATTATGAGATGGACTGTGGGTACGTGGGAGTGATCACTAACACCATAGTAATGTCTAATATTCACA GGCAGATCTGCTTGGGGAAGCTAGTTATGTGAAAGGCAAATAGAGTCATACAGTAGCTCAAAAGGCAACC ATAATTCTCTTTGGTGCAGGTCTTGGGAGCGTGATCTAGATTACACTGCACCATTCCCAAGTTAATCCCC TGAAAACTTACTCTCAACTGGAGCAAATGAACTTTGGTCCCAAATATCCATCTTTTCAGTAGCGTTAATT ATGCTCTGTTTCCAACTGCATTTCCTTTCCAATTGAATTAAAGTGTGGCCTCGTTTTTAGTCATTTAAAA TTGTTTTCTAAGTAATTGCTGCCTCTATTATGGCACTTCAATTTTGCACTGTCTTTTGAGATTCAAGAAA AATTTCTATTCTTTTTTTTGCATCCAATTGTGCCTGAACTTTTAAAATATGTAAATGCTGCCATGTTCCA AACCCATCGTCAGTGTGTGTGTTTAGAGCTGTGCACCCTAGAAACAACATATTGTCCCATGAGCAGGTGC CTGAGACACAGACCCCTTTGCATTCACAGAGAGGTCATTGGTTATAGAGACTTGAATTAATAAGTGACAT TATGCCAGTTTCTGTTCTCTCACAGGTGATAAACAATGCTTTTTGTGCACTACATACTCTTCAGTGTAGA GCTCTTGTTTATGGGAAAAGGCTCAAATGCCAAATTGTGTTTGATGGATTAATATGCCCTTTTGCCGAT GCATACTATTACTGATGTGACTCGGTTTTGTCGCAGCTTTGCTTTGTTTAATGAAACACACTTGTAAACC TCTTTTGCACTTTGAAAAGAATCCAGCGGGATGCTCGAGCACCTGTAAACAATTTTCTCAACCTATTTG ATGTTCAAATAAAGAATTAAACTAAA | |
| NM_130398 | AAATTGAAAGGTCAGCCTTTCGCGCGCTGTGTAGGCAAGTTACCCGTGTTCTGCGTTGCCGGCCGTGGGT | 110 |

| GENBANK ACCESSION NUMBER | SEQUENCE | SEQ ID NO: |
|---|---|---|
| | GCTCTGGCCACAGTGAGTTAGGGGCGTCGGAGCGGGTTTCTCCAACCGCAATCGGCTCCGCTCAAGGGGA<br>GGAGGAGAGTCCCTTCTCGGAAGGCCTAAGGAAACGTGTCGTCTGGAATGGGCTTGGGGGCCACGCCTGC<br>ACATCTCCGCGGAGCAGAGGGATAAAGTGAAGATGGTGCTGTTATTGTTACCTCGAGTGCCACATGCGAC<br>CTCTGAGATATGTACACAGTCATTCTTACTATCGCACTCAGCCATTCTTACTACGCTAAAGAAGAAATAA<br>TTATTCGAGGATATTTGCCTGGCCCAGAAGAAACTTATGTAAATTTCATGAACTATTATATCCGTTTTCC<br>TCGGAGTGAGAGAAAACTCTTTTTAGATATCATCTGAGAGAACTAGTGAATCCCAGTCACTGAGTGGAGT<br>TGAGAGTCTAAGAACCTCTGAAATTTGAGAACTGCTGGACCAGAGCCTTTAGAGCTCTGATAAGGTGTCA<br>ACAGGGTAGTTAATTTGGCACCATGGGGATACAGGGATTGCTACAATTTATCAAAGAAGCTTCAGAACCC<br>ATCCATGTGAGGAAGTATAAAGGGCAGGTAGTAGCTGGGATACATATTGCTGGCTTCACAAAGGAGCTA<br>TTGCTTGTGCTGAAAAACTAGCCAAAGGTGAACCTACTGATAGGTATGTAGGGATTTTGTATGAAATTTGT<br>AAATATGTTACTATCTCATGGGATCAAGCCTATTCTCGTATTTGATGGATGTACTTTACCTTCTAAAAAG<br>GAAGTAGAGAGATCTAGAAGAGAAAGACGACAAGCCAATCTTCTTAAGGGAAAGCAACTTCTTCGTGAGG<br>GGAAAGTCTCGGAAGCTCGAGAGTGTTTCACCCGGTCTATCAATATCACACATGCCATGGCCCACAAAGT<br>AATTAAAGCTGCCCGGTCTCAGGGGGTAGATTGCCTCGTGGCTCCCTATGAAGCTGATGCGCAGTTGGCC<br>TATCTTAACAAAGCGGGAATTGTGCAAGCCATAATTACAGAGGACTCGGATCTCCTAGCTTTTGGCTGTA<br>AAAAGGTAATTTTAAAGATGGACCAGTTTGGAAATGGACTTGAAATTGATCAAGCTCGGCTAGGAATGTG<br>CAGACAGCTTGGGGATGTATTCACGGAAGAGAAGTTTCGTTACATGTGTATTCTTTCAGGTTGTGACTAC<br>CTGTCATCACTGCGTGGGATTGGATTAGCAAAGGCATGCAAAGTCCTAAGACTAGCCAATAATCCAGATA<br>TAGTAAAGGTTATCAAGAAAATTGGACATTATCTCAAGATGAATATCACGGTACCAGAGGATTACATCAA<br>CGGGTTTATTCGGGCCAACAATACCTTCCTCTATCAGCTAGTTTTTGATCCCATCAAAAGGAAACTTATT<br>CCTCTGAACGCCTATGAAGATGATGTTGATCCTGAAACACTAAGCTACGCTGGGCAATATGTTGATGATT<br>CCATAGCTCTTCAAATAGCACTTGGAAATAAAGATATAAATACTTTTGAACAGATCGATGACTACAATCC<br>AGACACTGCTATGCCTGCCCATTCAAGAAGTCATAGTTGGGATGACAAAACATGTCAAAAGTCAGCTAAT<br>GTTAGCAGCATTTGGCATAGGAATTACTCTCCCAGACCAGAGTCGGGTACTGTTTCAGATGCCCCACAAT<br>TGAAGGAAAATCCAAGTACTGTGGGAGTGGAACGAGTGATTAGTACTAAAGGGTTAAATCTCCCAAGGAA<br>ATCATCCATTGTGAAAAGACCAAGAAGTGCAGAGCTGTCAGAAGATGACCTGTTGAGTCAGTATTCTCTT<br>TCATTTACGAAGAAGACCAAGAAAAATAGCTCTGAAGGCAATAAATCATTGAGCTTTTCTGAAGTGTTTG<br>TGCCTGACCTGGTAAATGGACCTACTAACAAAAAGAGTGTAAGCACTCCACCTAGGACGAGAAATAAATT<br>TGCAACATTTTTACAAAGGAAAAATGAAGAAAGTGGTGCAGTTGTGGTTCCAGGGACCAGAAGCAGGTTT<br>TTTTGCAGTTCAGATTCTACTGACTGTGTATCAAACAAAGTGAGCATCCAGCCTCTGGATGAAACTGCTG<br>TCACAGATAAAGAGAACAATCTGCATGAATCAGAGTATGGAGACCAAGAAGGCAAGAGACTGGTTGACAC<br>AGATGTAGCACGTAATTCAAGTGATGACATTCCGAATAATCATATTCCAGGTGATCATATTCCAGACAAG<br>GCAACAGTGTTTACAGATGAAGAGTCCTACTCTTTTGAGAGCAGCAAATTTACAAGGACCATTTCACCAC<br>CCACTTTGGGAACACTAAGAAGTTGTTTTAGTTGGTCTGGAGGTCTTGGAGATTTTTCAAGAACGCCGAG<br>CCCCTCTCCAAGCACAGCATTGCAGCAGTTCCGAAGAAAGAGCGATTCCCCCACCTCTTTGCCTGAGAAT<br>AATATGTCTGATGTGTCGCAGTTAAAGAGCGAGGAGTCCAGTGACGATGAGTCTCATCCCTTACGAGAAG<br>AGGCATGTTCTTCACAGTCCCAGGAAAGTGGAGAATTCTCACTGCAGAGTTCAAATGCATCAAAGCTTTC<br>TCAGTGCTCTAGTAAGGACTCTGATTCAGAGGAATCTGATTGCAATATTAAGTTACTTGACAGTCAAAGT<br>GACCAGACCTCCAAGCTACGTTTATCTCATTTCTCAAAAAAAGACACACCTCTAAGGAACAAGGTTCCTG<br>GGCTATATAAGTCCAGTTCTGCAGACTCTCTTTCTACAACCAAGATCAAACCTCTAGGACCTGCCAGAGC<br>CAGTGGGCTGAGCAAGAAGCCGGCAAGCATCCAGAAGAGAAAGCATCATAATGCCGAGAACAAGCCGGGG<br>TTACAGATCAAACTCAATGAGCTCTGGAAAAACTTTGGATTTAAAAAAGATTCTGAAAAGCTTCCTCCTT<br>GTAAGAAACCCCTGTCCCCAGTCAGAGATAACATCCAACTAACTCCAGAAGCGGAAGAGGATATATTTAA<br>CAAACCTGAATGTGGCCGTGTTCAAAGAGCAATATTCCAGTAAATGCAGACTGCTGCAAAGCTTTTGCCT<br>GCAAGAGAATCTGATCAATTTGAAGTCCCTGTTTGGGAATGAGGCACTTATCAGCATGAAGAATTTTTTC<br>TCATTCTGTGCCATTTTAAAAATAGAATACATTTTGTATATTAACTTTATAATTGGGTTGTGGTTTTTTT<br>GCTCAGCTTTTTATATTTTTATAAGAAGCTAAATAGAAGAATAATTGTATCTCTGACAGGTTTTTGGAGG<br>TTTTAGTGTTAATTGGGAAAATCCTCTGGAGTTTATAAAAGTCTACTCTAAATATTTCTGTAATGTTGTC<br>AAGTAGAAAGATAGTAAATGGAGAAACTACAAAAAAAAAAAAAAAAAAAAA | |
| AB209631 | CCATGACCTGCCTTGAGAAGGGGCAGGGGAAGCCAGATGGACTGGAAGTGGAGTGGCAGTGACCAAGGAG<br>GAGGAGGTGTGATAGGCTTCCCACGCAGGGTAGATCCAGAGACACCAGTGCCACCCATAGGCCCCTAGGA<br>CTGCAGTGGTCACCCGATTCCTTTGTCCCAGCTGAGACTCAGTTCTGAGTGTTCTATTTTGGGGAACAGA<br>GGCGTCCTTGGTAGCATTTGGAAGAGGATAGCCAGCTGGGGTGTGTGTACATCACAGCCTGACAGTAACA<br>GCATCCGAACCAGAGGTGACTGGCTAAGGGCAGACCCAGGGCAACAGGTTAACCGTTCTAGGGCCGGGCA<br>CAGGGAGGAGAACATTCCAACACTCTGTGTGCCCAGTGCCGACGCACGTTCTCTCTTTTATCCTCAAAAC<br>AGTCCTATGAGGATATAAGCCAGAGAGAGACAGAGACAAGGAATTACAAGTTGGTGAGAGTCAGGATTTG<br>AACTTGGCTCTGGCAGATGGAAAATTAGGGTCTGTATTCTTTACAAAACCGTGGTGTGCCTCAGATGGAGT<br>TGGTGCATAACAAGCAGAGGTATCCAGGGTCGCGGTCCTGCTTGCCACGGAAGGGGCCGCCTTGTCAGTT<br>GTGACCACCCAGCCCTGGAAATGTCAGTAATGCTGTAAGGAGTGGGGATCGGATCAGATGCCATCCAGAT<br>GCTGAAGTTTGACCTTGTGTCATTTTTCACTTTCTTTTTTGGCTCTTCTGCAATCAATTCATTTATTTAG<br>CAAAAAGAAATTATGTGTGCCGAGAGCATGCAGAAGATATGTCTCCGTTCTCTGCTTCCCTCCAAAAAA<br>GAATCCCAAAACTGCTTTCTGTGAACGTGTGCCAGGGTCCCAGCAGGACTCAGGGAGAGCAGGAAGCCCA<br>GCCCAGACCCCTTGCACAACCTACCGTGGGGAGGCCTTAGGCTCTGGCTACTACAGAGCTGGTTCCAGTC<br>TGCACTGCCACAGCCTGGCCAGGGACTTGGACACATCTGCTGGCCACTTCCTGTCTCAGTTTCCTTATCT<br>GCAAAATAAGGGAAAAGCCCCCACAAAGGTGCACGTGTAGCAGGAGTCTCTTTTCCCTCCCTATTTTAGGA<br>AGGCAGTTGGTGGGAAGTCCAGCTTGGGTCCCTGAGAGCTGTGAGAAGGAGATGCGGCTGCTGCTGGCCC<br>TGTTGGGGGTCCTGCTGAGTGTGCCTGGGCCTCCAGTCTTGTCCCTGGAGGCCTCTGAGGAAGTGGAGCT<br>TGGTATGGCTTCTGAGGTGGGAGAGGGTGGCAGGGGTGGGAAGAGTGGGCACCAGGAGGGGGCTGCTGGG | 111 |

| GENBANK ACCESSION NUMBER | SEQUENCE | SEQ ID NO: |
|---|---|---|
| | CTGAGCAAAGCTGGAAAGGATCCTTGCCCAGGCCCTGAGAAGGTGGCGGCAGGGCAGGGCTCAACCACTG<br>AGACTCAGTCAGTGCCTGGCTTCCAGCAAGCATTCATCTATCACTGTGTCTGCGAGAGAGGACTGGCCTT<br>GCAGGGCGCAGGGCCCTAAGCTGGGCTGCAGAGCTGGTGGTGAGCTCCTTGCCTGGGTGTGTGTGCGTGT<br>GTGTGTGTGTTCGTGCACTGGGTGTGTGACCTAGGAGGTCCAGGCAGCATGTGTGGTATAAGCATTATG<br>AGGGTGATATGCCCCGGTGCAGCATGACCCTGTATGTGGCACCAACAGCATGTGCCTTGTGTGTGTGTGT<br>GTCCGTATGTGTGTGTGTGTATGCGTGTGTGTGTGTGTGTGTGTGTCTTGGCCACTGTCATGTGCACT<br>AAATGCTGTGTGTGTGACATGCCCCAAGAGTGTGGCATTTGCCCTGGGTGTGGCATCCGCAGCATGTGGC<br>TGTGTGGGTGTCAAGGAGTGGTGGCTCCTTCAGCATGCGTTGCGAAGTGCTTGTGCCCTGCATGTGCGGT<br>GTGTTCTCTGTACACAGGAGGCTGCCTCAGATGGGGCTGCGGGGTCTGCTGACCTCTGCCCTCTGCCCAC<br>AGAGCCCTGCCTGGCTCCCAGCCTGGAGCAGCAAGAGCAGGAGCTGACAGTAGCCCTTGGGCAGCCTGTG<br>CGGCTGTGCTGTGGGCGGGCTGAGCGTGGTGGCCACTGGTACAAGGAGGGCAGTCGCCTGGCACCTGCTG<br>GCCGTGTACGGGGCTGGAGGGGCCGCCTAGAGATTGCCAGCTTCCTACCTGAGGATGCTGGCCGCTACCT<br>CTGCCTGGCACGAGGCTCCATGATCGTCCTGCAGAATCTCACCTTGATTACAGGTGACTCCTTGACCTCC<br>AGCAACGATGATGAGGACCCCAAGTCCCATAGGGACCTCTCGAATAGGCACAGTTACCCCCAGCAAGGTC<br>AGTAGGTCTCCAAGGACTTGTGTCCCCGCTGCTGCTCATCTGATCACTGAGAAGAGGAGGCCTGTGTGGG<br>AACACACGGTCATTCTAGGGGCCTTCCCCTGCCCTCCAGCACCCTACTGGACACACCCCCAGCGCATGGA<br>GAAGAAACTGCATGCAGTACCTGCGGGGAACACCGTCAAGTTCCGCTGTCCCAGCTGCAGGCAACCCCACG<br>CCCACCATCCGCTGGCTTAAGGATGGACAGGCCTTTCATGGGGAGAACCGCATTGGAGGCATTCGGCTGC<br>GCCATCAGCACTGGAGTCTCGTGATGGAGAGCGTGGTGCCCTCGGACCGCGGCACATACACCTGCCTGGT<br>AGAGAACGCTGTGGGCAGCATCCGTTATAACTACCTGCTAGATGTGCTGGAGCGGTCCCCGCACCGGCCC<br>ATCCTGCAGGCCGGGCTCCCGGCCAACACCACAGCCGTGGTGGGCAGCGACGTGGAGCTGCTGTGCAAGG<br>TGTACAGCGATGCCCAGCCCCACATCCAGTGGCTGAAGCACATCGTCATCAACGGCAGCAGCTTCGGAGC<br>CGACGGTTTCCCCTATGTGCAAGTCCTAAAGACTGCAGACATCAATAGCTCAGAGGTGGAGGTCCTGTAC<br>CTGCGGAACGTGTCAGCCGAGGACGGCAGGCGAGTACACCTGCCTCGCCAGGCAATTCCATCGGCCTCTCCT<br>ACCAGTCTGCCTGGCTCACGGTGCTGCCAGGTGAGCACCTGAAGGGCCAGGAGATGCTGCGAGATGCCCC<br>TCTGGGCCAGCAGTGGGGGCTGTGGCCTGTTGGGTGGTCAGTCTCTGTTGGCCTGTGGGGTCTGGCCTGG<br>GGGGCAGTGTGTGGATTTGTGGGTTTGAGCTGTATGACAGCCCCTCTGTGCCTCTCCACACGTGGCCGTC<br>CATGTGACCGTCTGCTGAGGTGTGGGTGCCTGGGACTGGGCATAACTACAGCTTCCTCCGTGTGTGTCCC<br>CACATATGTTGGGGAGCTGGGAGGGACTGAGTTAGGGTGCACGGGGCGGCCAGTCTCACCACTGACCAGTT<br>TGTCTGTCTGTGTGTGTCCATGTGCGAGGGCAGAGGAGGACCCCACATGGACCGCAGCAGCGCCCGAGGC<br>CAGGTATACGGACATCATCCTGTACGCGTCGGGCTCCCTGGCCTTGGCTGTGCTCCTGCTGCTGGCCAGG<br>CTGTATCGAGGGCAGGCGCTCCACGGCCGGCACCCCCGCCCGCCACTGTGCAGAAGCTCTCCCGCT<br>TCCCTCTGGCCCGACAGTTCTCCCTGGAGTCAGGCTCTTCCGGCAAGTCAAGCTCATCCCTGGTACGAGG<br>CGTGCGTCTCTCCTCCAGCGGCCCCGCCTTGCTCGCCGGCCTCGTGAGTCTAGATCTACCTCTCGACCCA<br>CTATGGGAGTTCCCCCGGGACAGGCTGGTGCTTGGGAAGCCCCTAGGCGAGGGCTGCTTTGGCCAGGTAG<br>TACGTGCAGAGGCCTTTGGCATGGACCCTGCCCGGCCTGACCAAGCCAGCACTGTGGCCGTCAAGATGCT<br>CAAAGACAACGCCTCTGACAAGGACCTGGCCGACCTGGTCTCGGAGATGGAGGTGATGAAGCTGATCGGC<br>CGACACAAGAACATCATCAACCTGCTTGGTGTCTGCACCCAGGAAGGGCCCCTGTACGTGATCGTGGAGT<br>GCGCCGCCAAGGGAAACCTGCGGGAGTTCCTGCGGGCCCGGCGCCCCCAGGCCCCGACCTCAGCCCCGA<br>CGGTCCTCGGAGCAGTGAGGGGCCGCTCTCCTTCCCAGTCCTGGTCTCCTGCGCCTACCAGGTGGCCCGA<br>GGCATGCAGTATCTGGAGTCCCGGAAGTGTATCCACCGGGACCTGGCTGCCCGCAATGTGCTGGTGACTG<br>AGGACAATGTGATGAAGATTGCTGACTTTGGGCTGGCCCGCGGCGTCCACCACATTGACTACTATAAGAA<br>AACCAGCAACGGCCGCCTGCCTGTGAAGTGGATGGCGCCCGAGGCCTTGTTTGACCGGGTGTACACACAC<br>CAGAGTGACGTGTGGTCTTTTGGGATCCTGCTATGGGAGATCTTCACCCTCGGGGGCTCCCCGTATCCTG<br>GCATCCCGGTGGAGGAGTCGTTCTCGCTGCTGCGGGAGGGGACATCGGATGGACCGGACCCCCCACACTGCCC<br>CCCAGAGCTGTACGGGCTGATGCGTGAGTGCTGGCACGCAGCGCCCTCCCAGAGGCCTACCTTCAAGCAG<br>CTGGTGGAGGCGCTGGACAAGGTCCTGCTGGCCGTCTCTGAGGAGTACCTCGACCTCCGCCTGACCTTCG<br>GACCCTATTCCCCCTCTGGTGGGGACGCCAGCAGCACCTGCTCCTCCAGCGATTCTGTCTTCAGCCACGA<br>CCCCCTGCCATTGGGATCCAGCTCCTTCCCCTTGGGTCTGGGGTGCAGACATGAGCAAGGCTCAAGGCT<br>GTGCAGGCACATAGGCTGGTGGCCTTGGGCCTTGGGGCTCAGCCACAGCCTGACACAGTGCTCGACCTTG<br>ATAGCATGGGGCCCCTGGCCCAGAGTTGCTGTGCCGTGTCCAAGGGCCGTGCCCTTGCCCTTGGAGCTGC<br>CGTGCCTGTGTCCTGATGGCCCAAATGTCAGGGTTCTGCTCGGCTTCTTGGACCTTGGCGCTTAGTCCCC<br>ATCCCGGGTTTGGCTGAGCCTGGCTGGGAGAGCTGCTATGCTAAACCTCCTGCCTCCCAATACCAGCAGGA<br>GGTTCTGGGCCTCTGAACCCCCTTTCCCCACACCTCCCCCTGCTGCTGCGCCCCAGCGTCTTGACGGGA<br>GCATTGGCCCCTGAGCCCAGAGAAGCTGGAAGCCTGCCGAAAACAGGAGCAAATGGCGTTTTATAAATTA<br>TTTTTTGAAAT | |
| NM_004496 | TAAGATCCACATCAGCTCAACTGCACTTGCCTCGCAGAGGCAGCCCGCTCACTTCCCGCGGAGGCGCTCC<br>CCGGCGCCGCGCTCCGCGGCAGCCGCCTGCCCCCGGCGCTGCCCCCGCCCGCCGCGCCGCCGCCGCCGCC<br>GCGCACGCCGCGCCCCGCAGCTCTGGGCTTCCTCTTCGCCCGGGTGGCGTTGGGCCCGCGCGGGCGCTCG<br>GGTGACTGCAGCTGCTCAGCTCCCCTCCCCCGCCCCGCGCCGCGCGGCCGCCCGTCGCTTCGCACAGGGC<br>TGGATGGTTGTATTGGGCAGGGTGGCTCCAGGATGTTAGGAACTGTGAAGATGGAAGGGCATGAAACCAG<br>CGACTGGAACAGCTACTACGCAGACACGCAGGAGGCCTACTCCTCCGTCCCGGTCAGCAACATGAACTCA<br>GGCCTGGGCTCCATGAACTCCATGAACACCTACATGACCATGAACACCATGACTACGAGCGGCAACATGA<br>CCCCGGCGTCCTTCAACATGTCCTATGCCAACCCGGGCCTAGGGGCCGGCCTGAGTCCCGGCGCAGTAGC<br>CGGCATGCCGGGGGGCTCGGCGGGCGCCATGAACAGCATGACTGCGGCCGGCGTGACGGCCATGGGTACG<br>GCGCTGAGCCCGAGCGGCATGGGCGCCATGGGTGCGCAGCAGGCGGCCTCCATGAATGGCCTGGGCCCCT<br>ACGCGGCCGCCATGAACCCCGTGCATGAGCCCCATGGCGTACGCGCCGTCCAACCTGGGCCGCAGCCGCGC<br>GGGCGGCGGCGGCGACGCCAAGACGTTCAAGCGCAGCTACCCGCACGCCAAGCCGCCCTACTCGTACATC | 112 |

(continued)

| GENBANK ACCESSION NUMBER | SEQUENCE | SEQ ID NO: |
|---|---|---|
| | TCGCTCATCACCATGGCCATCCAGCAGGCGCCCAGCAAGATGCTCACGCTGAGCGAGATCTACCAGTGGA<br>TCATGGACCTCTTCCCCTATTACCGGCAGAACCAGCAGCGCTGGCAGAACTCCATCCGCCACTCGCTGTC<br>CTTCAATGACTGCTTCGTCAAGGTGGCACGCTCCCCGGACAAGCCGGGCAAGGGCTCCTACTGGACGCTG<br>CACCCGGACTCCGGCAACATGTTCGAGAACGGCTGCTACTTGCGCCGCCAGAAGCGCTTCAAGTGCGAGA<br>AGCAGCCGGGGGCCGGCGGCGGGGGCGGGAGCGGAAGCGGGGGCAGCGGCGCCAAGGGCGGCCCTGAGAG<br>CCGCAAGGACCCCTCTGGCGCCTCTAACCCCAGCGCCGACTCGCCCCTCCATCGGGGTGTGCACGGGAAG<br>ACCGGCCAGCTAGAGGGCGCGCCGGCCCCCGGGCCCGCCGCCAGCCCCCAGACTCTGGACCACAGTGGGG<br>CGACGGCGACAGGGGGCGCCTCGGAGTTGAAGACTCCAGCCTCCTCAACTGCGCCCCCCATAAGCTCCGG<br>GCCCGGGGCGCTGGCCTCTGTGCCCGCCTCTCACCCGGCACACGGCTTGGCACCCCACGAGTCCCAGCTG<br>CACCTGAAAGGGGACCCCCACTACTCCTTCAACCACCCGTTCTCCATCAACAACCTCATGTCCTCCTCGG<br>AGCAGCAGCATAAGCTGGACTTCAAGGCATACGAACAGGCACTGCAATACTCGCCTTACGGCTCTACGTT<br>GCCCGCCAGCCTGCCTCTAGGCAGCGCCTCGGTGACCACCAGGAGCCCCATCGAGCCCTCAGCCCTGGAG<br>CCGGCGTACTACCAAGGTGTGTATTCCAGACCCGTCCTAAACACTTCCTAGCTCCCGGGACTGGGGGGTT<br>TGTCTGGCATAGCCATGCTGGTAGCAAGAGAGAAAAAATCAACAGCAAACAAAACCACACAAACCAAACC<br>GTCAACAGCATAATAAAATCCCAACAACTATTTTTATTTCATTTTTCATGCACAACCTTTCCCCCAGTGC<br>AAAAGACTGTTACTTTATTATTGTATTCAAAATTCATTGTGTATATTACTACAAAGACAACCCCAAACCA<br>ATTTTTTTCCTGCGAAGTTTAATGATCCACAAGTGTATATATGAAATTCTCCTCCTTCCTTGCCCCCCTC<br>TCTTTCTTCCCTCTTTCCCCTCCAGACATTCTAGTTTGTGGAGGGTTATTTAAAAAAACAAAAAAGGAAG<br>ATGGTCAAGTTTGTAAAATATTTGTTTGTGCTTTTTCCCCCTCCTTACCTGACCCCCTACGAGTTTACAG<br>GTCTGTGGCAATACTCTTAACCATAAGAATTGAAATGGTGAAGAACAAGTATACACTAGAGGCTCTTAA<br>AAGTATTGAAAGACAATACTGCTGTTATATAGCAAGACATAAACAGATTATAAACATCAGAGCCATTGC<br>TTCTCAGTTTACATTTCTGATACATGCAGATAGCAGATGTCTTTAAATGAAATACATGTATATTGTGTAT<br>GGACTTAATTATGCACATGCTCAGATGTGTAGACATCCTCCGTATATTTACATAACATATAGAGGTAATA<br>GATAGGTGATATACATGATACATTCTCAAGAGTTGCTTGACCGAAAGTTACAAGGACCCCAACCCCTTTG<br>TCCTCTCTACCCACAGATGGCCCTGGGAATCAATTCCTCAGGAATTGCCCTCAAGAACTCTGCTTCTTGC<br>TTTGCAGAGTGCCATGGTCATGTCATTCTGAGGTCACATAACACATAAAATTAGTTTCTATGAGTGTATA<br>CCATTTAAAGAATTTTTTTTTCAGTAAAAGGGAATATTACAATGTTGGAGGAGAGATAAGTTATAGGGAG<br>CTGGATTTCAAAACGTGGTCCAAGATTCAAAAATCCTATTGATAGTGGCCATTTTAATCATTGCCATCGT<br>GTGCTTGTTTCATCCAGTGTTATGCACTTTCCACAGTTGGACATGGTGTTAGTATAGCCAGACGGGTTTC<br>ATTATTATTTCTCTTTGCTTTCTCAATGTTAATTTATTGCATGGTTTATTCTTTTTCTTTACAGCTGAAA<br>TTGCTTTAAATGATGGTTAAAATTACAAATTGTTAATTTTTATCAATGTGATTGTAATTAAAAAT<br>ATTTTGATTTAAATAACAAAAATAATACCAGATTTTAAGCCGTGGAAAATGTTCTTGATCATTTGCAGTT<br>AAGGACTTTAAATAAATCAAATGTTAACAAAAAAAAAAAAAAAAA | |
| NM_001453 | ATGCAGGCGCGCTACTCCGTGTCCAGCCCCAACTCCCTGGGAGTGGTGCCCTACCTCGGCGGCGAGCAGA<br>GCTACTACCGCGCGGCGGCCGCGGCGGCCGGGGGCGGCTACACCGGCCATGCCGGCCCCCATGAGCGTGTA<br>CTCGCACCCTGCGCACGCCGAGCAGTACCCGGGCGGCATGGCCCGCGCCTACGGGCCCTACACGCCGCAG<br>CCGCAGCCCAAGGACATGGTGAAGCCGCCCTATAGCTACATCGCGCTCATCACCATGGCCATCCAGAACG<br>CCCCGGACAAGAAGATCACCCTGAACGGCATCTACCAGTTCATCATGGACCGCTTCCCCTTCTACCGGGA<br>CAACAAGCAGGGCTGGCAGAACAGCATCCGCCACAACCTCTCGCTCAACGAGTGCTTCGTCAAGGTGCCG<br>CGCGACGACAAGAAGCCGGGCAAGGGCAGCTACTGGACGCTGGACCCGGACTCCTACAACATGTTCGAGA<br>ACGGCAGCTTCCTGCGGCGGCGGCGGCGCTTCAAGAAGAAGGACGCGGTGAAGGACAAGGAGGAGAAGGA<br>CAGGCTGCACCTCAAGGAGCCGCCCCCGCCCGGCCGCCAGCCCCCGCCCGCCGGAGCAGGCCGAC<br>GGCAACGCGCCCGGTCCGCAGCCGCGCCCGTGCGCATCCAGGACATCAAGACCGAGAACGGTACGTGCC<br>CCTCGCCGCCCCAGCCCCTGTCCCGGCCGCCGCCCTGGGCAGCGGCAGCGCCGCCGCGGTGCCCAAGAT<br>CGAGAGCCCCGACAGCAGCAGCAGCAGCCTGTCCAGCGGGAGCAGCCCCCCGGGCAGCCTGCCGTCGGCG<br>CGGCCGCTCAGCCTGGACGGTGCGGATTCCGCGCCGCCGCCGCCCGCGCCCTCCGCCCCGCCGCCGCACC<br>ATAGCCAGGGCTTCAGCGTGGACAACATCATGACGTCGCTGCGGGGGTCGCCGCAGAGCGCGGCCGCGGA<br>GCTCAGCTCCGGCCTTCTGGCCTCGGCGGCCGCGTCCTCGCGCGCGGGGATCGCACCCCCGCTGGCGCTC<br>GGCGCCTACTCGCCCCGGCCCAGAGCTCCCTCTACAGCTCCCCCTGCAGCCAGACCTCCAGCGCGGGCAGCT<br>CGGGCGGCGGCGGCGGCGGCGCGGGGGCCGCGGGGGGGCGCGGGCGGCGGCGCCGGGACCTACCACTGCAACCT<br>GCAAGCCATGAGCCTGTACGCGGCCGGCGAGCGCGGGGGCCACTTGCAGGGCGCGCCCGGGGGCGCGGGC<br>GGCTCGGCCGTGGACGACCCCCTGCCCGACTACTCTCTGCCTCCGGTCACCAGCAGCAGCTCGTCGTCCC<br>TGAGTCACGGCGGCGGCGGCGGCGGCGGCGGGGGAGGCCAGGAGGCCGGCCACCACCCTGCGGCCCACCA<br>AGGCCGCCTCACCTCGTGGTACCTGAACCAGGCGGGCGGAGACCTGGGCCACTTGGCGAGCGCGGCGGCG<br>GCGGCGGCGGCCGCAGGCTACCCGGGCCAGCAGCAGAACTTCCACTCGGTGCGGGAGATGTTCGAGTCAC<br>AGAGGATCGGCTTGAACAACTCTCCAGTGAACGGGAATAGTAGCTGTCAAATGGCCTTCCCTTCCAGCCA<br>GTCTCTGTACCGCACGTCCGGAGCTTTCGTCTACGACTGTAGCAAGTTTTGACACACCCTCAAAGCCGAA<br>CTAAATCGAACCCCAAAGCAGGAAAAGCTAAAGGAACCCATCAAGGCAAAATCGAAACTAAAAAAAAAAA<br>ATCCAATTAAAAAAAAACCCCTGAGAATATTCACCACACCAGCGAACAGAATATCCCTCCAAAAATTCAGC<br>TCACCAGCACCAGCACGAAGAAAACTCTATTTTCTTAACCGATTAATTCAGAGCCACTCCACTTTGCCT<br>TGTCTAAATAAACAAACCCGTAAACTGTTTTATACAGAGACAGCAAAATCTTGGTTTATTAAAGGACAGT<br>GTTACTCCAGATAACACGTAAGTTTCTTCTTGCTTTTCAGAGACCTGCTTTCCCCTCCTCCCGTCTCCCC<br>TCTCTTGCCTTCTTCCTTGCCTCTCACCTGTAAGATATTATTTTATCCTATGTTGAAGGGAGGGGGGAAAG<br>TCCCCGTTTATGAAAGTCGCTTCTTTTTATTCATGGACTTGTTTTAAAATGTAAATTGCAACATAGTAA<br>TTTATTTTTAAATTTGTAGTTGGATGTCGTGGACCAAACGCCAGAAAGTGTTCCCAAAAACCTGACGTTAAA<br>TTGCCTGAAACTTTAAATTGTGCTTTTTTTCTCATTATAAAAAGGGAAACTGTATTAATCTTATTCTATC<br>CTCTTTTCTTTCTTTTTGTTGAACATATTCATTGTTTGTTTATTAATAAATTACCATTCAGTTTGAATGA<br>GACCTATATGTCTGGATACTTTAATAGAGCTTTAATTATTACGAAAAAAGATTTCAGAGATAAAACACTA | 113 |

(continued)

| GENBANK ACCESSION NUMBER | SEQUENCE | SEQ ID NO: |
|---|---|---|
| | GAAGTTACCTATTCTCCACCTAAATCTCTGAAAAATGGAGAAACCCTCTGACTAGTCCATGTCAAATTTT ACTAAAAGTCTTTTGTTTAGATTTATTTTCCTGCAGCATCTTCTGCAAAATGTACTATATAGTCAGCTT GCTTTGAGGCTAGTAAAAAGATATTTTTCTAAACAGATTGGAGTTGGCATATAAACAAATACGTTTTCTC ACTAATGACAGTCCATGATTCGGAAATTTTAAGCCCATGAATCAGCCGCGGTCTTACCACGGTGATGCCT GTGTGCCGAGAGATGGGACTGTGCGGCCAGATATGCACAGATAAATATTTGGCTTGTGTATTCCATATAA AATTGCAGTGCATATTATACATCCCTGTGAGCCAGATGCTGAATAGATATTTTCCTATTATTTCAGTCCT TTATAAAAGGAAAAATAAACCAGTTTTTAAATGTATGTATATAATTCTCCCCCATTTACAATCCTTCATG TATTACATAGAAGGATTGCTTTTTTAAAAATATACTGCGGGTTGGAAAGGGATATTTAATCTTTGAGAAA CTATTTTAGAAAATATGTTTGTAGAACAATTATTTTTGAAAAAGATTTAAAGCAATAACAAGAAGGAAGG CGAGAGGAGCAGAACATTTTGGTCTAGGGTGGTTTCTTTTTAAACCATTTTTTCTTGTTAATTTACAGTT AAACCTAGGGGCAATCCGGATTGGCCCTCCCCCTTTTGTAAATAACCCAGGAAATGTAATAAATTCATT ATCTTAGGGTGATCTGCCCTGCCAATCAGACTTTGGGGAGATGGCGATTTGATTACAGACGTTCGGGGGG GTGGGGGGCTTGCAGTTTGTTTTGGAGATAATACAGTTTCCTGCTATCTGCCGCTCCTATCTAGAGGCAA CACTTAAGCAGTAATTGCTGTTGCTTGTTGTCAAAATTTGATCATTGTTAAAGGATTGCTGCAAATAAAT ACACTTTAATTTCAGTCAAAAA | |
| AJ249248 | GTGGCCTCGAGGTGGTGGCAGGGCCGCCCCCTGCAGTCCGGAGACGAACGCACGGACCGGGCCTCCGGAG GCAGGTTCGGCTGGAAGGAACCGCTCTCGCTTCGTCCTACACTTGCGCAAATGTCTCCGAGCTTACTCAC ATAGCATATTGGTATATCAAAATGAAATGCAAGGAACCAAAAATAACATAATTGAAGGCAGTAAAAGTGA AATTAAATAGGAAGATCATCAGTCAAGGAAGACCCACTGGAGAGGACAGAAATGAAGCAGTGTTTTATC ATGTGTATTTCAGCAGGTCTTCTTGAAATTTAACTAAAAATATGACTGCTCTCTCTTCAGAGAACTGCTC TTTTCAGTACCAGTTACGTCAAACAAACCAGCCCCTAGACGTTAACTATCTGCTATTCTTGATCATACTT GGGAAAATATTATTAAATATCCTTACACTAGGAATGAGAAGAAAAACACCTGTCAAAATTTTATGGAAT ATTTTTGCATTTCACTAGCATTCGTTGATCTTTTACTTTTGGTAAACATTTCCATTATATTGTATTTCAG GGATTTGTACTTTTAAGCATTAGGTTCACTAAATACCACATCTGCCTATTTACTCAAATTATTTCCTTT ACTTATGGCTTTTTGCATTATCCAGTTTTCCTGACAGCTTGTATAGATTATTGCCTGAATTCTCTAAAA CAACCAAGCTTTCATTTAAGTGTCAAAAATTATTTTATTTCTTTACAGTAATTTTAATTTGGATTTCAGT CCTTGCTTATGTTTTGGGAGACCCAGCCATCTACCAAAGCCTGAAGGCACAGAATGCTTATTCTCGTCAC TGTCCTTTCTATGTCAGCATTCAGAGTTACTGGCTGTCATTTTTCATGGTGATGATTTTATTTGTAGCTT TCATAACCTGTTGGGAAGAAGTTACTACTTTGGTACAGGCTATCAGGATAACTTCCTATATGAATGAAAC TATCTTATATTTTCCTTTTTCATCCCACTCCAGTTATACTGTGAGATCTAAAAAAAATATTCTTATCCAAG CTCATTGTCTGTTTTCTCAGTACCTCGGTTACCATTTGTACTACTTCAGGTAATCATTGTTTTACTTAAAG TTCAGATTCCAGCATATATTGAGATGAATATTCCCTGGTTATACTTTGTCAATAGTTTTCTCATTGCTAC AGTGTATTGGTTTAATTGTCACAAGCTTAATTTAAAAGACATTGGATTACCTTTGGATCCATTGTCAAC TGGAAGTGCTGCTTCATTCCACTTACAATTCCTAATCTTGAGCAAATTGAAAAGCCTATATCAATAATGA TTTGTTAATATTATTAATTAAAAAGTTACAGCTGTCATAAGATCATAATTTTATGAACAGAAAGAACTCAG GACATATTAAAAAAATAAACTGAACTAAAACAACTTTTGCCCCCTGACTGATAGCATTTCAGAATGTGTCT TTTGAAGGGCTATACCAGTTATTAAATAGTGTTTTTATTTTAAAAACAAAATAATTCCAAGAAGTTTTTAT AGTTATTCAGGGACACTATATTACAAATATTACTTTGTTATTAACACAAAAAGTGATAAGAGTTAACATT TGGCTATACTGATGTTTGTGTTACTCAAAAAAAACTACTGGATGCAAACTGTTATGTAAATCTGAGATTTC ACTGACAACTTTAAGATATCAACCTAAACATTTTTATTAAATGTTCAAATGTAAGCAAGAAAAAAAAAAA | 114 |
| NM_014176 | AGTCAGAGGTCGCGCAGGCGCTGGTACCCCGTTGGTCCGCGCGTTGCTGCGTTGTGAGGGGTGTCAGCTC AGTGCATCCCAGGCAGCTCTTAGTGTGGAGCAGTGAACTGTGTGTGGTTCCTTCTACTTGGGGATCATGC AGAGAGCTTCACGTCTGAAGAGAGAGCTGCACATGTTAGCCACAGAGCCACCCCCCAGGCATCACATGTTG GCAAGATAAAGACCAAATGGATGACCTGCGAGCTCAAATATTAGGTGGAGCCAACACACCTTATGAGAAA GGTGTTTTTAAGCTAGAAGTTATCATTCCTGAGAGGTACCCATTTGAACCTCCTCAGATCCGATTTCTCA CTCCAATTTATCATCCAAACATTGATTCTGCTGGAAGGATTTGTCTGGATGTTCTCAAATTGCCACCAAA AGGTGCTTGGAGACCATCCCTCAACATCGCAACTGTGTTGACCTCTATTCAGCTGCTCATGTCAGAACCC AACCCTGATGACCCGCTCATGGCTGACATATCCTCAGAATTTAAATATAATAAGCCAGCCTTCCTCAAGA ATGCCAGACAGTGGACAGAGAAGCATGCAAGACAGAAACAAAAGGCTGATGAGGAAGAGATGCTTGATAA TCTACCAGAGGCTGGTGACTCCAGAGTACACAACTCAACACAGAAAAGGAAGGCCAGTCAGCTAGTAGGC ATAGAAAAGAAATTTCATCCTGATGTTTAGGGGACTTGTCCTGGTTCATCTTAGTTAATGTGTTCTTTGC CAAGGTGATCTAAGTTGCCTACCTTGAATTTTTTTTTAAATATATTTGATGACATAATTTTTGTGTAGTT TATTTATCTTGTACATGTATTTTGAAATCTTTTAAACCTGAAAAATAAATAGTCATTTAATGTTGAAA AAAAAAAAAAAAAAAAAAAAAAAA | 115 |

(continued)

| GENBANK ACCESSION NUMBER | SEQUENCE | SEQ ID NO: |
|---|---|---|
| NM_006845 | ACGCTTGCGCGCGGGATTTAAACTGCGGCGGTTTACGCGGCGTTAAGACTTCGTAGGGTTAGCGAAATTG AGGTTTCTTGGTATTGCGCGTTTCTCTTCCTTGCTGACTCTCCGAATGGCCATGGACTCGTCGCTTCAGG CCCGCCTGTTTCCCGGTCTCGCTATCAAGATCCAACGCAGTAATGGTTTAATTCACAGTGCCAATGTAAG GACTGTGAACTTGGAGAAATCCTGTGTTTCAGTGGAATGGGCAGAAGGAGGTGCCACAAAGGGCAAAGAG ATTGATTTTGATGATGTGGCTGCAATAAACCCAGAACTCTTACAGCTTCTTCCCTTACATCCGAAGGACA ATCTGCCCTTGCAGGAAAATGTAACAATCCAGAAACAAAAACGGAGATCCGTCAACTCCAAAATTCCTGC TCCAAAAGAAAGTCTTCGAAGCCGCTCCACTCGCATGTCCACTGTCTCAGAGCTTCGCATCACGGCTCAG GAGAATGACATGGAGGTGGAGCTGCCTGCAGCTGCAAACTCCCGCAAGCAGTTTTCAGTTCCTCCTGCCC CCACTAGGCCTTCCTGCCCTGCAGTGGCTGAAATACCATTGAGGATGGTCAGCGAGGAGATGGAAGAGCA AGTCCATTCCATCCGAGGCAGCTCTTCTGCAAACCCTGTGAACTCAGTTCGGAGGAAATCATGTCTTGTG AAGGAAGTGGAAAAAATGAAGAACAAGCGAGAAGAGAAGAAGGCCCAGAACTCTGAAATGAGAATGAAGA GAGCTCAGGAGTATGACAGTAGTTTTCCAAACTGGGAATTTGCCCGAATGATTAAAGAATTTCGGGCTAC TTTGGAATGTCATCCACTTACTATGACTGATCCTATCGAAGAGCACAGAATATGTGTCTGTGTTAGGAAA CGCCCACTGAATAAGCAAGAATTGGCCAAGAAAGAAATTGATGTGATTTCCATTCCTAGCAAGTGTCTCC TCTTGGTACATGAACCCAAGTTGAAAGTGGACTTAACAAAGTATCTGGAGAACCAAGCATTCTGCTTTGA CTTTGCATTTGATGAAACAGCTTCGAATGAAGTTGTCTACAGGTTCACAGCAAGGCCACTGGTACAGACA ATCTTTGAAGGTGGAAAAGCAACTTGTTTTGCATATGGCCAGACAGGAAGTGGCAAGACACATACTATGG GCGGAGACCTCTCTGGGAAAGCCCAGAATGCATCCAAAGGGATCTATGCCATGGCCTCCCGGGACGTCTT CCTCCTGAAGAATCAACCCTGCTACCGGAAGTTGGGCCTGGAAGTCTATGTGACATTCTTCGAGATCTAC AATGGGAAGCTGTTTGACCTGCTCAACAAGAAGGCCAAGCTGCGCGTGCTGGAGGACGGCAAGCAACAGG TGCAAGTGGTGGGGCTGCAGGAGCATCTGGTTAACTCTGCTGATGATGTCATCAAGATGATCGACATGGG CAGCGCCTGCAGAACCTCTGGGCAGACATTTGCCAACTCCAATTCCTCCCGCTCCCACGCGTGCTTCCAA ATTATTCTTCGAGCTAAAGGGAGAATGCATGGCAAGTTCTCTTTGGTAGATCTGGCAGGGAATGAGCGAG GCGCGGACACTTCCAGTGCTGACCGGCAGACCCGCATGGAGGGCGCAGAAATCAACAAGAGTCTCTTAGC CCTGAAGGAGTGCATCAGGGCCCTGGGACAGAACAAGGCTCACACCCCGTTCCGTGAGAGCAAGCTGACA CAGGTGCTGAGGGACTCCTTCATTGGGGAGAACTCTAGGACTTGCATGATTGCCACGATCTCACCAGGCA TAAGCTCCTGTGAATATACTTTAAACACCCTGAGATATGCAGACAGGGTCAAGGAGCTGAGCCCCCACAG TGGGCCCAGTGGAGAGCAGTTGATTCAAATGGAAACAGAAGAGATGGAAGCCTGCTCTAACGGGGCGCTG ATTCCAGGCAATTTATCCAAGGAAGAGGAGGAACTGTCTTCCCAGATGTCCAGCTTTAACGAAGCCATGA CTCAGATCAGGGAGCTGGAGGAGAAGGCTATGGAAGAGCTCAAGGAGATCATACAGCAAGGACCAGACTG GCTTGAGCTCTCTGAGATGACCGAGCAGCCAGACTATGACCTGGAGACCTTTGTGAACAAAGCGGAATCT GCTCTGGCCCAGCAAGCCAAGCATTTCTCAGCCCTGCGAGATGTCATCAAGGCCTTGCGCCTGGCCATGC AGCTGGAAGAGCAGGCTAGCAGACAAATAAGCAGCAAGAAACGGCCCCAGTGACGACTGCAAATAAAAAT CTGTTTGGTTTGACACCCAGCCTCTTCCCTGGCCCTCCCCAGAGAACTTTGGGTACCTGGTGGGTCTAGG CAGGGTCTGAGCTGGGACAGGTTCTGGTAAATGCCAAGTATGGGGGCATCTGGGCCCAGGGCAGCTGGGG AGGGGGTCAGAGTGACATGGGACACTCCTTTTCTGTTCCTCAGTTGTCGCCCTCACGAGAGGAAGGAGCT CTTAGTTACCCTTTTGTGTTGCCCTTCTTTCCATCAAGGGGAATGTTCTCAGCATAGAGCTTTCTCCGCA GCATCCTGCCTGCGTGGACTGGCTGCTAATGGAGAGCTCCCTGGGGTTGTCCTGGCTCTGGGGAGAGAGA CGGAGCCTTTAGTACAGCTATCTGCTGGCTCTAAACCTTCTACGCCTTTGGGCCGAGCACTGAATGTCTT GTACTTTAAAAAAAATGTTTCTGAGACCTCTTTCTACTTTACTGTCTCCCTAGAGATCCTAGAGGATCCCT ACTGTTTTCTGTTTTATGTGTTTATACATTGTATGTAACAATAAAGAGAAAAAATAAATCAGCTGTTTAA GTGTGTGGAAAAAAAAAAAAAAAAAA | 116 |

| GENBANK ACCESSION NUMBER | SEQUENCE | SEQ ID NO: |
|---|---|---|
| NM_006101 | ACTGCGCGCGTCGTGCGTAATGACGTCAGCGCCGGCGGAGAATTTCAAATTCGAACGGCTTTGGCGGGCC<br>GAGGAAGGACCTGGTGTTTTGATGACCGCTGTCCTGTCTAGCAGATACTTGCACGGTTTACAGAAATTCG<br>GTCCCTGGGTCGTGTCAGGAAACTGGAAAAAAGGTCATAAGCATGAAGCGCAGTTCAGTTTCCAGCGGTG<br>GTGCTGGCCGCCTCTCCATGCAGGAGTTAAGATCCCAGGATGTAAATAAACAAGGCCTCTATACCCCTCA<br>AACCAAAGAGAAACCAACCTTTGGAAAGTTGAGTATAAACAAACCGACATCTGAAAGAAAAGTCTCGCTA<br>TTTGGCAAAAGAACTAGTGGACATGGATCCCGGAATAGTCAACTTGGTATATTTTCCAGTTCTGAGAAAA<br>TCAAGGACCCGAGACCACTTAATGACAAAGCATTCATTCAGCAGTGTATTCGACAACTCTGTGAGTTTCT<br>TACAGAAAATGGTTATGCACATAATGTGTCCATGAAATCTCTACAAGCTCCCTCTGTTAAAGACTTCCTG<br>AAGATCTTCACATTTCTTTATGGCTTCCTGTGCCCCTCATACGAACTTCCTGACACAAAGTTTGAAGAAG<br>AGGTTCCAAGAATCTTTAAAGACCTTGGGTATCCTTTTGCACTATCCAAAAGCTCCATGTACACAGTGGG<br>GGCTCCTCATACATGGCCTCACATTGTGGCAGCCTTAGTTTGGCTAATAGACTGCATCAAGATACATACT<br>GCCATGAAAGAAAGCTCACCTTTATTGATGATGGGCAGCCTTGGGGAGAAGAAACTGAAGATGGAATTA<br>TGCATAATAAGTTGTTTTTGGACTACACCATAAAATGCTATGAGAGTTTTATGAGTGGTGCCGACAGCTT<br>TGATGAGATGAATGCAGAGCTGCAGTCAAAACTGAAGGATTTATTTAATGTGGATGCTTTTAAGCTGGAA<br>TCATTAGAAGCAAAAAACAGAGCATTGAATGAACAGATTGCAAGATTGGAACAAGAAAGAGAAAAAGAAC<br>CGAATCGTCTAGAGTCGTTGAGAAAACTGAAGGCTTCCTTACAAGGAGATGTTCAAAAGTATCAGGCATA<br>CATGAGCAATTTGGAGTCTCATTCAGCCATTCTTGACCAGAAATTAAATGGTCTCAATGAGGAAATTGCT<br>AGAGTAGAACTAGAATGTGAAACAATAAAACAGGAGAACACTCGACTACAGAATATCATTGACAACCAGA<br>AGTACTCAGTTGCAGACATTGAGCGAATAAATCATGAAAGAAATGAATTGCAGCAGACTATTAATAAATT<br>AACCAAGGACCTGGAAGCTGAACAACAGAAGTTGTGGAATGAGGAGTTAAAATATGCCAGAGGCAAAGAA<br>GCGATTGAAACACAATTAGCAGAGTATCACAAATTGGCTAGAAAATTAAAACTTATTCCTAAAGGTGCTG<br>AGAATTCCAAAGGTTATGACTTTGAAATTAAGTTTAATCCCGAGGCTGGTGCCAACTGCCTTGTCAAATA<br>CAGGGCTCAAGTTTATGTACCTCTTAAGGAACTCCTGAATGAAACTGAAGAAGAAATTAATAAAGCCCTA<br>AATAAAAAAATGGGTTTGGAGGATACTTTAGAACAATTGAATGCAATGATAACAGAAAGCAAGAGAAGTG<br>TGAGAACTCTGAAAGAAGAAGTTCAAAAGCTGGATGATCTTTACCAACAAAAAATTAAGGAAGCAGAGGA<br>AGAGGATGAAAAATGTGCCAGTGAGCTTGAGTCCTTGGAGAAACACAAGCACCTGCTAGAAAGTACTGTT<br>AACCAGGGGCTCAGTGAAGCTATGAATGAATTAGATGCTGTTCAGCGGGAATACCAACTAGTTGTGCAAA<br>CCACGACTGAAGAAAGACGAAAAGTGGGAAATAACTTGCAACGTCTGTTAGAGATGGTTGCTACACATGT<br>TGGGTCTGTAGAGAAACATCTTGAGGAGCAGATTGCTAAAGTTGATAGAGAATATGAAGAATGCATGTCA<br>GAAGATCTCTCGGAAAATATTAAAGAGATTAGAGATAAGTATGAGAAGAAAGCTACTCTAATTAAGTCTT<br>CTGAAGAATGAAGATAAAATGTTGATCATGTATATATATCCATAGTGAATAAAATTGTCTCAGTAAAGTG<br>TAAAAAAAAAAAAAAAAAAAAAAAAAAAAAAAAAAAA | 117 |
| BC042437 | CTCCCTCCTCTGCACCATGACTACCTGCAGCCGCCAGTTCACCTCCTCCAGCTCCATGAAGGGCTCCTGC<br>GGCATCGGGGGCGGCATCGGGGGCGGCTCCAGCCGCATCTCCTCCGTCCTGGCCGGAGGGTCCTGCCGCG<br>CCCCCAGCACCTACGGGGGCGGCCTGTCTGTCTCATCCTCCCGCTTCTCCTCTGGGGGAGCCTATGGGTT<br>GGGGGGCGGCTATGGCGGTGGCTTCAGCAGCAGCAGCAGCAGCTTTGGTAGTGGCTTTGGGGGAGGATAT<br>GGTGGTGGCCTTGGTGCTGGCCTTGGGTGGTGGCTTTGGTGGTGGCTTTGCTGGTGGTGATGGGCTTCTGG<br>TGGGCAGTGAGAAGGTGACCATGCAGAACCTCAACGACCGCCTGGCCTCCTACCTGGACAAGGTGCGTGC<br>TCTGGAGGAGGCCAACGCCGACCTGGAAGTGAAGATCCGTGACTGGTACCAGAGGCAGCGGCCTGCTGAG<br>ATCAAAGACTACAGTCCCTACTTCAAGACCATTGAGGACCTGAGGAACAAGATTCTCACAGCCACAGTGG<br>ACAATGCCAATGTCCTTCTGCAGATTGACAATGCCCGTCTGGCCGCGGATGACTTCCGCACCAAGTATGA<br>GACAGAGTTGAACCTGCGCATGAGTGTGGAAGCCGACATCAATGGCCTGCGCAGGGTGCTGGACGAACTG<br>ACCCTGGCCAGAGCTGACCTGGAGATGCAGATTGAGAGCCTGAAGGAGGAGCTGGCCTACCTGAAGAAGA<br>ACCACGAGGAGGAGATGAATGCCCTGAGAGGCCAGGTGGGTGGAGATGTCAATGTGGAGATGGACGCTGC<br>ACCTGGCGTGGACCTGAGCCGCATTCTGAACGAGATGCGTGACCAGTATGAGAAGATGGCAGAGAAGAAC<br>CGCAAGGATGCCGAGGAATGGTTCTTCACCAAGACAGAGGAGCTGAACCGCGAGGTGGCCACCAACAGCG<br>AGCTGGTGCAGAGCGGCAAGAGCGAGATCTCGGAGCTCCGGCGCACCATGCAGAACCTGGAGATTGAGCT<br>GCAGTCCCAGCTCAGCATGAAAGCATCCCTGGAGAACAGCCTGGAGGAGACCAAAGGTCGCTACTGCATG<br>CAGCTGGCCCAGATCCAGGAGATGATTGGCAGCGTGGAGGAGCAGCTGGCCCAGCTCCGCTGCGAGATGG<br>AGCAGCAGAACCAGGAGTACAAGATCCTGCTGGACGTGAAGACGCGGCTGGAGCAGGAGATCGCCACCTA<br>CCGCCGCCTGCTGGAGGGCGAGGACGCCCACCTCTCCTCCTCCCAGTTCTCCTCTGGATCGCAGTCATCC<br>AGAGATGTGACCTCCTCCAGCCGCCAAATCCGCACCAAGGTCATGGATGTGCACGATGGCAAGGTGGTGT<br>CCACCCACGAGCAGGTCCTTCGCACCAAGAACTGAGGCTGCCCAGCCCCGCTCAGGCCTAGGAGGCCCCC<br>CGTGTGGACACAGATCCCACTGGAAGATCCCCTCTCCTGCCCAAGCACTTCACAGCTGGACCCTGCTTCA<br>CCCTCACCCCCTCCTGGCAATCAATACAGCTTCATTATCTGAGTTGCATAAAAAAAAAAAAAAAAAAAAAA<br>AAAAAAAAAAAAAAAAAAAAAAAAAAAAAAAAAAAAAAAAAAAAAAAAAAAAAAAAAAAAAAAAAAAAAAAA<br>AAAAAAAAAAAAAAAAAAAAAAAAAAAAAAAAAAAAAAAAAAAAAAAAAAAAAAAAAA | 118 |

(continued)

| GENBANK ACCESSION NUMBER | SEQUENCE | SEQ ID NO: |
|---|---|---|
| AK095281 | CTCTTTTGCAGGGGCCGTTCCTCGGGGCATGACGCTGGCTCCTGCACAGATCCTGCTCCTCTGTGGCCTT CCTGGGCTGCCCTCCCCTCCTCCGGGACTGCTCTGGACTGACACTGCTCAGGTTCGGATTCCCTCAAAGA CTTTGGGAGACAAGACTTGGTCCCCCTTTTACAAACAAGGGAACGGAGGCTCTAGAACTGACTTCCTGAA AGGCTTGGATCCAAAGCTCCCTCAGTTCAGCGGCCACGTCTATTTCCCTCAGACACAGGGATCCTTGAAC CTGTGGGCTGTATCTCCCCGCGGACTTGGAAGAATCCCAAGAGAGTGGGGCTCCCACAGGCTGGAGTGCA ATGGTGTGATCTCGGCTCACTGCAACCTCCACCTCCCAGGTTCAAGCTATTCTCCTGCCTCAGCCTCCTG AGTAGCTGGGATTACAGATCCTGGTGGCTGTGGTCGGTAATTCCAGCTTCGTGCTGGCTACAGGTGGATG ATGCCCACCTGGCTGCCGATGACCTCTGCACCAAGTGAGGCTGGGTCTCTGGAGCTGCCCCAGGGGCTGG ACAAGCTGACCCTGGCCGGGGCCAACCTGGAGATGCAGATTGAGAACCTCAAGGAGGACCTGGTCTACCT GAAGAAGAACCACAAGCAGGAAATGAACGTCCTTTGAGGTCAGGTGGATGAGGATGTCAGTGTGAAGATG GACACTGTGCCTGGAGTGAACCTGAGCTGCATCCTGAATGAGATGCGTGACCAGGACAAGACATTGGTGG AGAAGAGCTGCAAGGATGCCGAGGGCTGGTTCTTCAGCATGGTGGGTGGCCGTGCGTAAGCAGGTGTGTA CACGTGTGGGCACATGTGCTGCATGCTGGTGCAGCTGGAGCACTGGCAGATCCACAGGCTGTCCCAGTTG GAAGGACTTTTGGAAACCAGTTGGACCAGCCCCTCATGTTTTAGATGTAAAACGTGAGGCTCAGAGAGGA CTCAAGCTCACACAGCCCTTCACTGTGGCCTGCAAAATAGATCCAGGTCTCTACAAGTCTGGTCTTGGGT TTCCACCACAGCTGTTTACAGGATGTGCGTATTTGAATACATATGTATACCCTTGGCAAGCACAGGCTGA GTATCTCCGGTATCCTAGGGACAGCAACAGGCGCAAAAGAATAACACCCAGTGCCTGTCTTTGAGGTGCT GCAGTTCAGTAGGAAAAAGAAATGCAAATGACCGCAGAGCAGGCTGAATTCCTCCAAGTTCCAATGTGGG TGCAGAGGCTCTCTGTGTGCAGAAAGAGGGGCTGAACTGCGAGGTGGCCACCAACACAGAGGCCCTGCAG AGTGGCTGGATAGAGATATGGAGCTCTACGTCTCTGTGCAGAACCTGAGCCGTCCCAGCTCAGCAAGAAA GCATCGCTGGAGGGCAGCCTGGTGGAGATGGAGGTGTGTTACAGGACCCTGCCGGCCCAGCTGCAGGGGC TTAACAGAAGCATGGAGCAGCAGCTGTGCGAGCTCTGCTGCGACACGGAGCACCAGGACCACAAGCACAG GTCCTTCTGGACGTGAAGACGTGGCTGGAGCAGGAGATCGCCACCTACCGCCGCTTGCTGGAGGTTGAGG ACGCCCAGAGGTGATACTGACGATGCAGGCTGGAGTCTGGCTGAGGAGCCTTGAATGCCAAGTTAAAGCG TCTGGACTAGATCACGTAGGCAATGGGGAGCCATGGAGGGATTTGGAGCAGGAGAGTGAAATGAACATCA AGAGATTTTAGAACATTCACTCTGGCTGCAGAGGGAAAATGGATCAGAGGGGTCAGGGCGGGGCCAGAG AGATGTGTCAGGGGGCTGGAGCAGGGAGTCTGGCCAGAGAAGTCCCGTGCGGTGGTGGGTAGTGGGGCAG GGGAAGGAAGGTGGTGCACGCAGAAGAGAGGTTATAGCTCAAAACAGCGGGACTGGATGCCTGGATCTCG GGGTAAGCATGGCTCACAGTCAGGACTCAGTAAGTGTCGGGAGAACACATGAAGGAGCAGGCATTGATGG CCCTGGGTTTCTGGTTCTGATGACTGTGTGAGTGGTGAAGAGCAAGGTGGGTGGTGGTTGGGTTTGCAGT TGGGAAGGGTGATCAGGCCTTCAGCTGAGAGTGTCCCGGAGTCTCCATGCTTAGTCACACGTTGCAGCTT TTTGCTCCCCGGAAATGGTGAAGTCCATCTATAGTCTAACAACAGTCTCTCCTGCTTTAATTGGGTCTAT TTGTTGGGCCCTCTGGGTTATGGAAAAACCAACTTGCTCAGCTTCTCCTTGTAAATTCCTGGTGAGTAGCC ACAGAGTGCCGCCAGACCTACTGCTGTGCTGTTTCTTTTTCTTCTTCCTGCTGTGCTGAACCCCTGCCCT TTCATTCTTGGGCCTGCGCTAATTTCTGTGCATTCCCAACTGTGATTTTTCACCAATTTAGGGGAACCTC CTCTGCCAGGGCCTACTTCTCCCCAGCAGTGCTTGCAGGTGCCTGGGCTGGCTGGCATCCCTGGGCTGAT GGGTGCTTCTCTCCCTGCAGGCTGGCCACTCAGTACTCCTTGTCCCTGGCCTCGCAGCCCACCCGGGAAG CCACAGTGACCAGCCACCAGGTGTGCCATCGTGGAGGAAGTCCAGGTTGGAGAGGTGGTCTTCTTCTGTG AGCAGGTCCACTTCTCCACCCACTGAGACCCCTTTCTGTCTGCGACAGCCCCACCTCGAGGGCCACGGCA CAGCCATCAGCTCCAGCTCCCAGCATGCTACTGCCACGCCCCGAGTGTCCGTCTGGGCCCCGGTGCATGG CCTGTTGTCTTTCTGTATCTACTTTCTGCAGCCCCTCACTGAGGAGGCCTCCTGGGTTTGTCCAGTGCCT ACTATTAAAGCTTTGCTCCAAGTTC | 119 |
| M21389 | GCATCCTTTTTGGGCTGCTCACAGCCCCCAGCCTCTATGGTGAAGACATACTTGCTAGCAGCGTCACCAA CTTGCTGCCAAGAGATCAGTGCTGCAAGGCAAGGTTATTTCTAACTGAGCAGAGCCTGCCAGGAAGAAAG CGTTTGCACCCCACACCACTGTGCAGGTGTGACCGGTGAGCTCACAGCTGCCCCCCAGGCATGCCCAGCC CACTTAATCATTCACAGCTCGACAGCTCTCTCGCCCAGCCCAGTTCTGGAAGGGATAAAAAGGGGGCATC ACCGTTCCTGGGTAACAGAGCCACCTTCTGCGTCCTGCTGAGCTCTGTTCTCTCCAGCACCTCCCAACCC ACTAGTGCCTGGTTCTCTTGCTCCACCAGGAACAAGCCACCATGTCTCGCCAGTCAAGTGTGTCCTTCCG GAGCGGGGGCAGTCGTAGCTTCAGCACCGCCTCTGCCATCACCCCGTCTGTCTCCCGCACCAGCTTCACC TCCGTGTCCCGGTCCGGGGGTGGCGGTGGTGGTGGCTTCGGCAGGGTCAGCCTTGCGGGTGCTTGTGGAG TGGGTGGCTATGGCAGCCGGAGCCTCTACAACCTGGGGGGCTCCAAGAGGATATCCATCAGCACTAGAGG | 120 |

(continued)

| GENBANK ACCESSION NUMBER | SEQUENCE | SEQ ID NO: |
|---|---|---|
| | AGGCAGCTTCAGGAACCGGTTTGGTGCTGGTGCTGGAGGCGGCTATGGCTTTGGAGGTGGTGCCGGTAGT<br>GGATTTGGTTTCGGCGGTGGAGCTGGTGGTGGCTTTGGGCTCGGTGGCGGAGCTGGCTTTGGAGGTGGCT<br>TCGGTGGCCCTGGCTTTCCTGTCTGCCCTCCTGGAGGTATCCAAGAGGTCACTGTCAACCAGAGTCTCCT<br>GACTCCCCTCAACCTGCAAATCGACCCCAGCATCCAGAGGGTGAGGACCGAGGAGCGCGAGCAGATCAAG<br>ACCCTCAACAATAAGTTTGCCTCCTTCATCGACAAGGTGCGGTTCCTGGAGCAGCAGAACAAGGTTCTGG<br>ACACCAAGTGGACCCTGCTGCAGGAGCAGGGCACCAAGACTGTGAGGCAGAACCTGGAGCCGTTGTTCGA<br>GCAGTACATCAACAACCTCAGGAGGCAGCTGGACAGCATCGTGGGGGAACGGGGCCGCCTGGACTCAGAG<br>CTGAGAAACATGCAGGACCTGGTGGAAGACTTCAAGAACAAGTATGAGGATGAAATCAACAAGCGTACCA<br>CTGCTGAGAATGAGTTTGTGATGCTGAAGAAGGATGTAGATGCTGCCTACATGAACAAGGTGGAGCTGGA<br>GGCCAAGGTTGATGCACTGATGGATGAGATTAACTTCATGAAGATGTTCTTTGATGCGGAGCTGTCCCAG<br>ATGCAGACGCATGTCTCTGACACCTCAGTGGTCCTCTCCATGGACAACAACCGCAACCTGGACCTGGATA<br>GCATCATCGCTGAGGTCAAGGCCCAGTATGAGGAGATTGCCAACCGCAGCCGGACAGAAGCCGAGTCCTG<br>GTATCAGACCAAGTATGAGGAGCTGCAGCAGACAGCTGGCCGGCATGGCGATGACCTCCGCAACACCAAG<br>CATGAGATCACAGAGATGAACCGGATGATCCAGAGGCTGAGAGCCGAGATTGACAATGTCAAGAAACAGT<br>GCGCCAATCTGCAGAACGCCATTGCGGATGCCGAGCAGCGTGGGGAGCTGGCCCTCAAGGATGCCAGGAA<br>CAAGCTGGCCGAGCTGGAGGAGGCCCTGCAGAAGGCCAAGCAGGACATGGCCCGGCTGCTGCGTGAGTAC<br>CAGGAGCTCATGAACACCAAGCTGGCCCTGGACGTGGAGATCGCCACTTACCGCAAGCTGCTGGAGGGCG<br>AGGAATGCAGACTCAGTGGAGAAGGAGTTGGACCAGTCAACATCTCTGTTGTCACAAGCAGTGTTTCCTC<br>TGGATATGGCAGTGGCAGTGGCTATGGCGGTGGCCTCGGTGGAGGTCTTGGCGGCGGCCTCGGTGGAGGT<br>CTTGCCGGAGGTAGCAGTGGAAGCTACTACTCCAGCAGCAGTGGGGGTGTCGGCCTAGGTGGTGGGCTCA<br>GTGTGGGGGGGCTCTGGCTTCAGTGCAAGCAGTGGCCGAGGGCTGGGGGTGGGCTTTGGCAGTGGCGGGGG<br>TAGCAGCTCCAGCGTCAAATTTGTCTCCACCACCTCCTCCTCCCGGAAGAGCTTCAAGAGCTAAGAACCT<br>GCTGCAAGTCACTGCCTTCCAAGTGCAGCAACCCAGCCCATGGAGATTGCCTCTTCTAGGCAGTTGCTCA<br>AGCCATGTTTTATCCTTTTCTGGAGAGTAGTCTAGACCAAGCCAATTGCAGAACCACATTCTTTGGTTCC<br>CAGGAGAGCCCCATTCCCAGCCCCTGGTCTCCCGTGCCGCAGTTCTATATTCTGCTTCAAATCAGCCTTC<br>AGGTTTCCCACAGCATGGCCCCTGCTGACACGAGAACCCAAAGTTTTCCCAAATCTAAATCATCAAAACA<br>GAATCCCCACCCCAATCCCAAATTTTGTTTTGGTTCTAACTACCTCCAGAATGTGTTCAATAAAATGCTT<br>TTATAATAT | |
| NM_<br>001123066 | GGACGGCCGAGCGGCAGGGCGCTCGCGCGCGCCCACTAGTGGCCGGAGGAGAAGGCTCCCGCGGAGGCCG<br>CGCTGCCCGCCCCCTCCCCTGGGGAGGCTCGCGTTCCCGCTGCTCGCGCCTGCGCCGCCCGCCGGCCTCA<br>GGAACGCGCCCTCTTCGCCGGCGCGCGCCCTCGCAGTCACCGCCACCCACCAGCTCCGGCACCAACAGCA<br>GCGCCGCTGCCACCGCCCACCTTCTGCCGCCGCCACCACAGCCACCTTCTCCTCCTCCGCTGTCCTCTCC<br>CGTCCTCGCCTCTGTCGACTATCAGGTGAACTTTGAACCAGGATGGCTGAGCCCCGCCAGGAGTTCGAAG<br>TGATGGAAGATCACGCTGGGACGTACGGGTTGGGGGACAGGAAAGATCAGGGGGGCTACACCATGCACCA<br>AGACCAAGAGGGTGACACGGACGCTGGCCTGAAAGAATCTCCCCTGCAGACCCCCACTGAGGACGGATCT<br>GAGGAACCGGGCTCTGAAACCTCTGATGCTAAGAGCACTCCAACAGCGGAAGATGTGACAGCACCCTTAG<br>TGGATGAGGGAGCTCCCGGCAAGCAGGCTGCCGCGCAGCCCCACACGGGAGATCCCAGAAGGAACCACAGC<br>TGAAGAAGCAGGCATTGGAGACACCCCCAGCCTGGAAGACGAAGCTGCTGGTCACGTGACCCAAGAGCCT<br>GAAAGTGGTAAGGTGGTCCAGGAAGGCTTCCTCCGAGAGCCAGGCCCCCCAGGTCTGAGCCACCAGCTCA<br>TGTCCGGCATGCCTGGGGCTCCCCTCCTGCCTGAGGGCCCCAGAGAGGCCACACGCCAACCTTCGGGGAC<br>AGGACCTGAGGACACAGAGGGCGGCCGCCACGCCCCTGAGCTGCTCAAGCACCAGCTTCTAGGAGACCTG<br>CACCAGGAGGGGCCGCCGCTGAAGGGGGCAGGGGCAAAGAGAGGCCGGGGAGCAAGGAGGAGGTGGATG<br>AAGACCGCGACGTCGATGAGTCCTCCCCCCCAAGACTCCCCTCCCTCCAAGGCCTCCCCAGCCCAAGATGG<br>GCGGCCTCCCCAGACAGCCGCCAGAGAAGCCACCAGCCATCCCAGGCTTCCCAGCGGAGGGTGCCATCCCC<br>CTCCCTGTGGATTTCCTCTCCAAAGTTTCCACAGAGATCCCAGCCTCAGAGCCCGACGGGCCCAGTGTAG<br>GGCGGGCCAAAGGGCAGGATGCCCCCCTGGAGTTCACGTTTCACGTGGAAATCACACCCAACGTGCAGAA<br>GGAGCAGGCGCACTCGGAGGAGCATTGGGAAGGGCTGCATTTCCAGGGGCCCCTGGAGAGGGGCCAGAG<br>GCCCGGGGCCCCTCTTTGGGAGAGGACACAAAAGAGGCTGACCTTCCAGAGCCCTCTGAAAAGCAGCCTG<br>CTGCTGCTCCGCGGGGAAGCCCGTCAGCCGGGTCCCTCAACTCAAAGCTCGCATGGTCAGTAAAAGCAA<br>AGACGGGACTGGAAGCGATGACAAAAAAGCCAAGACATCCACACGTTCCTCTGCTAAAACCTTGAAAAAT<br>AGGCCTTGCCTTAGCCCCAAACACCCCACTCCTGGTAGCTCAGACCCTCTGATCCAACCCTCCAGCCCTG<br>CTGTGTGCCCAGAGCCACCTTCCTCTCCTAAATACGTCTCTTCTGTCACTTCCCGAACTGGCAGTTCTGG<br>AGCAAAGGAGATGAAACTCAAGGGGGCTGATGGTAAAACGAAGATCGCCACACCGCGGGGAGCAGCCCT<br>CCAGGCCAGAAGGGCCAGGCCAACGCCACCAGGATTCCAGCAAAAACCCCGCCCGCTCCAAAGACACCAC<br>CCAGCTCTGCGACTAAGCAAGTCCAGAGAAGACCACCCCCTGCAGGGCCCAGATCTGAGAGAGGTGAACC<br>TCCAAAATCAGGGGATCGCAGCGGGTCTACAGCAGCCCCGGCTCCCCAGGCACTCCCGGCCAGCCGTCCCGC<br>ACCCCGTCCCTTCCAACCCCACCCACCCGGGAGCCCAAGAAGGTGGCAGTGGTCCGTACTCCACCCAAGT<br>CGCCGTCTTCCGCCAAGAGCCGCCTGCAGACAGCCCCCGTGCCCATGCCAGACCTGAAGAATGTCAAGTC<br>CAAGATCGGCTCCACTGAGAACCTGAAGCACCAGCCGGGAGGCGGGAAGGTGCAGATAATTAATAAGAAG<br>CTGGATCTTAGCAACGTCCAGTCCAAGTGTGGCTCAAAGGATAATATCAAACACGTCCCGGGAGGCGGCA<br>GTGTGCAAATAGTCTACAAACCAGTTGACCTGAGCAAGGTGACCTCCAAGTGTGGCTCATTAGGCAACAT<br>CCATCATAAACCAGGAGGTGGCCAGGTGGAAGTAAAATCTGAGAAGCTTGACTTCAAGGACAGAGTCCAG<br>TCGAAGATTGGGTCCCTGGACAATATCACCCACGTCCCTGGCGGAGGGAAATAAAAAGATTGAAACCCACA<br>AGCTGACCTTCCGCGAGAACGCCCAAAGCCAAGACAGAAGCCGGGGCGGGAGATCGTGTACAAGTCGCCAGT<br>GGTGTCTGGGGACACGTCTCCACGGCATCTCAGCAATGTCTCCTCCACCGGCAGCATCGACATGGTAGAC<br>TCGCCCCAGCTCGCCACGCTAGCTGACGAGGTGTCTGCCTCCCTGGCCAAGCAGGGTTTGTGATCAGGCC<br>CCTGGGGCGGTCAATAATTGTGGAGAGGAGAGAATGAGAGAGTGTGGAAAAAAAAAGAATAATGACCCGG<br>CCCCCGCCCTCTGCCCCCAGCTGCTCCTCGCAGTTCGGTTAATTGGTTAATCACTTAACCTGCTTTTGTC | 121 |

(continued)

| GENBANK ACCESSION NUMBER | SEQUENCE | SEQ ID NO: |
|---|---|---|
| | ACTCGGCTTTGGCTCGGGACTTCAAAATCAGTGATGGGAGTAAGAGCAAATTTCATCTTTCCAAATTGAT<br>GGGTGGGCTAGTAATAAAATATTTAAAAAAAAAACATTCAAAAACATGGCCACATCCAACATTTCCTCAGG<br>CAATTCCTTTTGATTCTTTTTTCTTCCCCCTCCATGTAGAAGAGGGGAGAAGGAGAGGCTCTGAAAGCTGC<br>TTCTGGGGGATTTCAAGGGACTGGGGGTGCCAACCACCTCTGGCCCTGTTGTGGGGGGTGTCACAGAGGCA<br>GTGGCAGCAACAAAGGATTTGAAACTTGGTGTGTTCGTGGAGCCACAGGCAGACGATGTCAACCTTGTGT<br>GAGTGTGACGGGGGTTGGGGTGGGGCGGGAGGCCACGGGGGAGGCCGAGGCAGGGGCTGGGCAGAGGGGA<br>GAGGAAGCACAAGAAGTGGGAGTGGGAGAGGAAGCCACGTGCTGGAGAGTAGACATCCCCCTCCTTGCCG<br>CTGGGAGAGCCAAGGCCTATGCCACCTGCAGCGTCTGAGCGGCCGCCTGTCCTTGGTGGCCGGGGGTGGG<br>GGCCTGCTGTGGGTCAGTGTGCCACCCTCTGCAGGGCAGCCTGTGGGAGAAGGGACAGCGGGTAAAAAGA<br>GAAGGCAAGCTGGCAGGAGGGTGGCACTTCGTGGATGACCTCCTTAGAAAAGACTGACCTTGATGTCTTG<br>AGAGCGCTGGCCTCTTCCTCCCTCCCTGCAGGGTAGGGGGCCTGAGTTGAGGGGCTTCCCTCTGCTCCAC<br>AGAAACCCTGTTTTATTGAGTTCTGAAGGTTGGAACTGCTGCCATGATTTTGGCCACTTTGCAGACCTGG<br>GACTTTAGGGCTAACCAGTTCTCTTTGTAAGGACTTGTGCCTCTTGGGAGACGTCCACCCGTTTCCAAGC<br>CTGGGCCACTGGCATCTCTGGAGTGTGTGGGGGGTCTGGGAGGCAGGTCCCGAGCCCCCTGTCCTTCCCAC<br>GGCCACTGCAGTCACCCCGTCTGCGCCGCTGTGCTGTTGTCTGCCGTGAGAGCCCAATCACTGCCTATAC<br>CCCTCATCACACGTCACAATGTCCCGAATTCCCAGCCTCACCACCCCTTCTCAGTAATGACCCTGGTTGG<br>TTGCAGGAGGTACCTACTCCATACTGAGGGTGAAATTAAGGGAAGGCAAAGTCCAGGCACAAGAGTGGGA<br>CCCCAGCCTCTCACTCTCAGTTCCACTCATCCAACTGGGACCCTCACCCACGAATCTCATGATCTGATTCG<br>GTTCCCTGTCTCCTCCTCCCGTCACAGATGTGAGCCAGGGCACTGCTCAGCTGTGACCCTAGGTGTTTCT<br>GCCTTGTTGACATGGAGAGAGCCCTTTCCCCTGAGAAGGCCTGGCCCCTTCCTGTGCTGAGCCCACAGCA<br>GCAGGCTGGGTGTCTTGGTTGTCAGTGGTGGCACCAGGATGGAAGGGCAAGGCACCCAGGGCAGGCCCAC<br>AGTCCCGCTGTCCCCCACTTGCACCCTAGCTTGTAGCTGCCAACCTCCCAGCACAGCCCAGCCCGCTGCTC<br>AGCTCCACATGCATAGTATCAGCCCTCCACACCCGACAAAGGGGAACACACCCCCTTGGAAATGGTTCTT<br>TTCCCCCAGTCCCAGCTGGAAGCCATGCTGTCTGTTCTGCTGGAGCAGCTGAACATATACATAGATGTTG<br>CCCTGCCCTCCCCATCTGCACCCTGTTGAGTTGTAGTTGGATTTGTCTGTTTATGCTTGGATTCACCAGA<br>GTGACTATGATAGTGAAAAGAAAAAAAAAAAAAAAAAAAAGGACGCATGTATCTTGAAATGCTTGTAAAGAG<br>GTTTCTAACCCACCCTCACGAGGTGTCTCTCACCCCCCACACTGGGACTCGTGTGGCCTGTGTGGTGCCAC<br>CCTGCTGGGGCCTCCCAAGTTTTGAAAGGCTTTCCTCAGCACCTGGGACCCAACAGAGACCAGCTTCTAG<br>CAGCTAAGGAGGCCGTTCAGCTGTGACGAAGGCCTGAAGCACAGGATTAGGACTGAAGCGATGATGTCCC<br>CTTCCCTACTTCCCCTTGGGGCTCCCTGTGTCAGGGCACAGACTAGGTCTTGTGGCTGGTCTGGCTTGCG<br>GCGCGAGGATGGTTCTCTCTGGTCATAGCCCGAAGTCTCATGGCAGTCCCAAAGGAGGCTTACAACTCCT<br>GCATCACAAGAAAAAGGAAGCCACTGCCAGCTGGGGGGATCTGCAGCTCCCAGAAGCTCCGTGAGCCTCA<br>GCCACCCCTCAGACTGGGTTCCTCTCCAAGCTCGCCCTCTGGAGGGGCAGCGCAGCCTCCCACCAAGGGC<br>CCTGCGACCACAGCAGGGATTGGGATGAATTGCCTGTCCTGGATCTGCTCTAGAGGCCCAAGCTGCCTGC<br>CTGAGGAAGGATGACTTGACAAGTCAGGAGACACTGTTCCCAAAGCCTTGACCAGAGCACCTCAGCCCGC<br>TGACCTTGCACAAACTCCATCTGCTGCCATGAGAAAAGGGAAGCCGCCTTTGCAAAACATTGCTGCCTAA<br>AGAAACTCAGCAGCCTCAGGCCCAATTCTGCCACTTCTGGTTTGGGTACAGTTAAAGGCAACCCTGAGGG<br>ACTTGGCAGTAGAAATCCAGGGCCTCCCCTGGGGCTGGCAGCTTCGTGTGCAGCTAGAGCTTTACCTGAA<br>AGGAAGTCTCTGGGCCCAGAACTCTCCACCAAGAGCCTCCCTGCCGTTCGCTGAGTCCCAGCAATTCTCC<br>TAAGTTGAAGGGATCTGAGAAGGAGAAGGAAATGTGGGGTAGATTTGGTGGTGGTTAGAGATATGCCCCC<br>CTCATTACTGCCAACAGTTTCGGCTGCATTTCTTCACGCACCTCGGTTCCTCTTCCTGAAGTTCTTGTGC<br>CCTGCTCTTCAGCACCATGGGCCTTCTTATACGGAAGGCTCTGGGATCTCCCCCTTGTGGGGCAGGCTCT<br>TGGGGCCAGCCTAAGATCATGGTTTAGGGTGATCAGTGCTGGCAGATAAATTGAAAAGGCACGCTGGCTT<br>GTGATCTTAAATGAGGACAATCCCCCCAGGGCTGGGCACTCCTCCCCTCCCCTCACTTCTCCCACCTGCA<br>GAGCCAGTGTCCTTGGGTGGGCTAGATAGGATATACTGTATGCCCGGCTCCTTCAAGCTGCTGACTCACTT<br>TATCAATAGTTCCATTTAAATTGACTTCAGTGGTGAGACTGTATCCTGTTTGCTATTGCTTGTTGTGCTA<br>TGGGGGGAGGGGGGAGGAATGTGTAAGATAGTTAACATGGGCAAAGGGAGATCTTGGGGTGCAGCACTTA<br>AACTGCCTCGTAACCCTTTTCATGATTTCAACCACATTTGCTAGAGGGAGGGAGCAGCCACGGAGTTAGA<br>GGCCCTTGGGGTTTCTCTTTTCCACTGACAGGCTTTCCCAGGCAGCTGGCTAGTTCATTCCCTCCCCAGC<br>CAGGTGCAGGCGTAGGAATATGGACATCTGGTTGCTTTGGCCTGCTGCCCTCTTTCAGGGGTCCTAAGCC<br>CACAATCATGCCTCCCTAAGACCTTGGCATCCTTCCCTCTAAGCCGTTGGCACCTCTGTGCCACCTCTCA<br>CACTGGCTCCAGACACACAGCCTGTGCTTTTGGAGCTGAGATCACTCGCTTCACCCTCCTCATCTTTGTT<br>CTCCAAGTAAAGCCACGAGGTCGGGGCGAGGGCAGAGGTGATCACCTGCGTGTCCCATCTACAGACCTGC<br>AGCTTCATAAAACTTCTGATTTCTCTTCAGCTTTGAAAAGGGTTACCCTGGGCACTGGCCTAGAGCCTCA<br>CCTCCTAATAGACTTAGCCCCATGAGTTTGCCATGTTGAGCAGGACTATTCTGGCACTTGCAAGTCCCA<br>TGATTTCTTCGGTAATTCTGAGGGTGGGGGGAGGGACATGAAATCATCTTAGCTTAGCTTTCTGTCTGTG<br>AATGTCTATATAGTGTATTGTGTGTTTTAACAAATGATTTACACTGACTGTTGCTGTAAAAGTGAATTTG<br>GAAATAAAGTTATTACTCTGATTAAA | |
| M92424 | GCACCGCGCGAGCTTGGCTGCTTCTGGGGCCTGTGTGGCCCTGTGTGTCGGAAAGATGGAGCAAGAAGCC<br>GAGCCCGAGGGGCGGCCGCGACCCCTCTGACCGAGATCCTGCTGCTTTCGCAGCCAGGAGCACCGTCCCT<br>CCCCGGATTAGTGCGTACGAGCGCCCAGTGCCCTGGCCCGGAGAGTGGAATGATCCCCGAGGCCCAGGGC<br>GTCGTGCTTCCGCAGTAGTCAGTCCCCGTGAAGGAAACTGGGGAGTCTTGAGGGACCCCCGACTCCAAGC<br>GCGAAAACCCCGGATGGTGAGGAGCAGGCAAATGTGCAATACCAACATGTCTGTACCTACTGATGGTGCT<br>GTAACCACCTCACAGATTCCAGCTTCGGAACAAGAGACCCTGGTTAGACCAAAGCCATTGCTTTTGAAGT<br>TATTAAAGTCTGTTGGTGCACAAAAAGACACTTATACTATGAAAGAGGTTCTTTTTTATCTTGGCCAGTA<br>TATTATGACTAAACGATTATATGATGAGAAGCAACAACATATTGTATATTGTTCAAATGATCTTCTAGGA<br>GATTTGTTTGGCGTGCCAAGCTTCTCTGTGAAAGAGCACAGGAAAAATATATACCATGATCTACAGGAACT<br>TGGTAGTAGTCAATCAGCAGGAATCATCGGACTCAGGTACATCTGTGAGTGAGAACAGGTGTCACCTTGA | 122 |

(continued)

| GENBANK ACCESSION NUMBER | SEQUENCE | SEQ ID NO: |
|---|---|---|
|  | AGGTGGGAGTGATCAAAAGGACCTTGTACAAGAGCTTCAGGAAGAGAAACCTTCATCTTCACATTTGGTT TCTAGACCATCTACCTCATCTAGAAGGAGAGCAATTAGTGAGACAGAAGAAAATTCAGATGAATTATCTG GTGAACGACAAAGAAAACGCCACAAATCTGATAGTATTTCCCTTTCCTTTGATGAAAGCCTGGCTCTGTG TGTAATAAGGGGAGATATGTTGTGAAAGAAGCAGTAGCAGTGAATCTACAGGGACGCCATCGAATCCGGAT CTTGATGCTGGTGTAAGTGAACATTCAGGTGATTGGTTGGATCAGGATTCAGTTTCAGATCAGTTTAGTG TAGAATTTGAAGTTGAATCTCTCGACTCAGAAGATTATAGCCTTAGTGAAGAAGGACAAGAACTCTCAGA TGAAGATGATGAGGTATATCAAGTTACTGTGTATCAGGCAGGGGAGAGTGATACAGATTCATTTGAAGAA GATCCTGAAATTTCCTTAGCTGACTATTGGAAATGCACTTCATGCAATGAAATGAATCCCCCCCTTCCAT CACATTGCAACAGATGTTGGGCCCTTCGTGAGAATTGGCTTCCTGAAGATAAAGGGAAAGATAAAGGGGA AATCTCTGAGAAAGCCAAACTGGAAAACTCAACACAAGCTGAAGAGGGCTTTGATGTTCCTGATTGTAAA AAAACTATAGTGAATGATTCCAGAGAGTCATGTGTTGAGGAAAATGATGATAAAATTACACAAGCTTCAC AATCACAAGAAAGTGAAGACTATTCTCAGCCATCAACTTCTAGTAGCATTATTTATAGCAGCCAAGAAGA TGTGAAAGAGTTTGAAAGGGAAGAAACCCAAGACAAAGAAGAGAGTGTGGAATCTAGTTTGCCCCTTAAT GCCATTGAACCTTGTGTGATTTGTCAAGGTCGACCTAAAAATGGTTGCATTGTCCATGGCAAAACAGGAC ATCTTATGGCCTGCTTTACATGTGCAAAGAAGCTAAAGAAAAGGAATAAGCCCTGCCCAGTATGTAGACA ACCAATTCAAATGATTGTGCTAACTTATTTCCCCTAGTTGACCTGTCTATAAGAGAATTATATATTTCTA ACTATATAACCCTAGGAATTTAGACAACCTGAAATTTATTCACATATATCAAAGTGAGAAAATGCCTCAA TTCACATAGATTTCTTCTCTTTAGTATAATTGACCTACTTTGGTAGTGGAATAGTGAATACTTACTATAA TTTGACTTGAATATGTAGCTCATCCTTTACACCAACTCCTAATTTTAAATAATTTCTACTCTGTCTTAAA TGAGAAGTACTTGGTTTTTTTTTTCTTAAATATGTATATGACATTTAAATGTAACTTATTATTTTTTTTG AGACCGAGTCTTGCTCTGTTACCCAGGCTGGAGTGCAGTGGGTGATCTTGGCTCACTGCAAGCTCTGCCC TCCCCGGGTTCGCACCATTCTCCTGCCTCAGCCTCCCAATTAGCTTGGCCTACAGTCATCTGCCACCACA CCTGGCTAATTTTTTGTACTTTTAGTAGAGACAGGGTTTCACCGTGTTAGCCAGGATGGTCTCGATCTCC TGACCTCGTGATCCGCCCACCTCGGCCTCCCAAAGTGCTGGGATTACAGGCATGAGCCACCG |  |
| NM_014791 | GAGATTTGATTCCCTTGGCGGGCGGAAGCGGCCACAACCCGGCGATCGAAAAGATTCTTAGGAACGCCGT ACCAGCCGCGTCTCTCAGGACAGCAGGCCCCTGTCCTTCGTCGGGCGCCGCTCAGCCGTGCCCTCCGCC CCTCAGGTTCTTTTTCTAATTCCAAATAAACTTGCAAGAGGACTATGAAAGATTATGATGAACTTCTCAA ATATTATGAATTACATGAAACTATTGGGACAGCAGGCTTTGCAAAGGTCAAACTTGCCTGCCATATCCTT ACTGGAGAGATGGTAGCTATAAAAATCATGGATAAAAACACACTAGGGAGTGATTTGCCCCGGATCAAAA CGGAGATTGAGGCCTTGAAGAACCTGAGACATCAGCATATATGTCAACTCTACCATGTGCTAGAGACAGC CAACAAAATATTCATGGTTCTTGAGTACTGCCCTGGAGGAGAGCTGTTTGACTATATAATTCCCAGGAT CGCCTGTCAGAAGAGGAGACCCGGGTTGTCTTCCGTCAGATAGTATCTGCTGTTGCTTATGTGCACAGCC AGGGCTATGCTCACAGGGACCTCAAGCCAGAAAATTTGCTGTTTGATGAATATCATAAATTAAAGCTGAT TGACTTTGGTCTCTGTGCAAAACCCAAGGGTAACAAGGATTACCATCTACAGACATGCTGTGGGAGTCTG GCTTATGCAGCACCTGAGTTAATACAAGGCAAATCATATCTTGGATGACAGAGGCAGATGTTTGGAGCATGG GCATACTGTTATATGTTCTTATGTGTGGATTTCTACCATTTGATGATGATAATGTAATGGCTTTATACAA GAAGATTATGAGAGGAAAATATGATGTTCCCAAGTGGCTCTCTCCCAGTAGCATTCTGCTTCTTCAACAA ATGCTGCAGGTGGACCCAAAGAAACGGATTTCTATGAAAAATCTATTGAACCATCCCTGGATCATGCAAG ATTACAACTATCCTGTTGAGTGGCAAAGCAAGAATCCTTTTATTCACCTCGATGATGATTGCGTAACAGA ACTTTCTGTACATCACAGAAACAACAGGCAAACAATGGAGGATTAATTTCACTGTGGCAGTATGATCAC CTCACGGCTACCTATCTTCTGCTTCTAGCCAAGAAGGCTCGGGGAAAACCAGTTCGTTTAAGGCTTTCTT CTTTCTCCTGTGGACAAGCCAGTGCTACCCCATTCACAGACATCAAGTCAAATAATTGGAGTCTGGAAGA TGTGACCGCAAGTGATAAAAATTATGTGGCGGGATTAATAGACTATGATTGGTGTGAAGATGATTTATCA ACAGGTGCTGCTACTCCCCGAACATCACAGTTTACCAAGTACTGGACAGAATCAAATGGGGTGGAATCTA AATCATTAACTCCAGCCTTATGCAGAACACCTGCAAATAAATTAAAGAACAAAGAAAATGTATATACTCC TAAGTCTGCTGTAAAGAATGAAGAGTACTTTATGTTTCCTGAGCCAAAGACTCCAGTTAATAAGAACCAG CATAAGAGAGAAATACTCACTACGCCAAATCGTTACACTACACCCTCAAAAGCTAGAAACCAGTGCCTGA AAGAAACTCCAATTAAAATACCAGTAAATTCAACAGGAACAGACAAGTTAATGACAGGTGTCATTAGCCC TGAGAGGCGGTGCCGCTCAGTGGAATTGGATCTCAACCAAGCACATATGGAGGAGACTCCAAAAAGAAAG GGAGCCAAAGTGTTTGGGAGCCTTGAAAGGGGGTTGGATAAGGTTATCACTGTGCTCACCAGGAGCAAAA GGAAGGGTTCTGCCAGAGACGGGCCCAGAAGACTAAAGCTTCACTATAACGTGACTACAACTAGATTAGT GAATCCAGATCAACTGTTGAATGAAATAATGTCTATTCTTCCAAAGAAGCATGTTGACTTTGTACAAAAG GGTTATACACTGAAGTGTCAAACACAGTCAGATTTTGGGAAAGTGACAATGCAATTGAATTAGAAGTGT GCCAGCTTCAAAAACCCGATGTGGTGGGTATCAGGAGGCAGCGGCTTAAGGGCGATGCCTGGGTTTACAA AAGATTAGTGGAAGACATCCTATCTAGCTGCAAGGTATAATTGATGGATTCTTCCATCCTGCCGGATGAG TGTGGGTGTGATACAGCCTACATAAAGACTGTTATGATCGCTTTGATTTTAAAGTTCATTGGAACTACCA ACTTGTTTCTAAAGAGCTATCTTAAGACCAATATCTCTTTGTTTTAAACAAAAGATATTATTTTGTGTA TGAATCTAAATCAAGCCCATCTGTCATTATGTTACTGTCTTTTTTAATCATGTGGTTTTGTATATTAATA ATTGTTGACTTTCTTAGATTCACTTCCATATGTGAATGTAAGCTCTTAACTATGTCTCTTTGTAATGTGT AATTTCTTTCTGAAATAAAACCATTTGTGAATATAG | 123 |

(continued)

| GENBANK ACCESSION NUMBER | SEQUENCE | SEQ ID NO: |
|---|---|---|
| BG765502 | GCAGCGGAGGAGCCCAGTCCACGATGGCCCGGTCCCTGGTGTGCCTTGGTGTCATCATCTTGCTGTCTGC<br>CTTCTCCGGACCTGGTGTCAGGGGTGGTCCTATGCCCAAGCTGGCTGACCGGAAGCTGTGTGCGGACCAG<br>GAGTGCAGCCACCCTATCTCCATGGCTGTGGCCCTTCAGGACTACATGGCCCCCGACTGCCGATTCCTGA<br>CCATTCACCGGGGCCAAGTGGTGTATGTCTTCTCCAAGCTGAAGGGCCGTGGGCGGCTCTTCTGGGGAGG<br>CAGCGTTCAGGGAGATTACTATGGAGATCTGGCTGCTCGCCTGGGCTATTTCCCCAGTAGCATTGTCCGA<br>GAGGACCAGACCCTGAAACCTGGCAAAGTCGATGTGAAGACAGACAAATGGGATTTCTACTGCCAGTGAG<br>CTCAGCCTACCGCTGGCCCTGCCGTTTCCCCTCCTTGGGTTTATGCAAATACAATCAGCCCAGTGCAAAA<br>AAAAAAAAAAAAAAAAAAAAACTTCGGAGAAGAGATAGCAACAAAAGGCCGCTTGTGTGAAGGCGCCAAAA<br>GTTTTCGCCCAAGAGACCTTCGGCCTCCCCCAGGGCGCGCGCAAAGGCGCCTTGTTTTGACAACCTCTTG<br>GACAACCGGAGGGGCTACCGCCCGGAGACCCCTGTGGTGGACCCCCCGGGCAACCCGGTGTGACAGGGTA<br>CTCACCCCCACGGCTTTGTCGGGGGTCCCACCAAAGGCCCCAAAGAGGCTCTTTCAAGGCACTATTCCTT<br>GTTGTAGACCTTGTGTGTGCCACAGGCGCCAAAGAAACCTCGGGGGGCTAACAAACGCACGTGCTTGGCA<br>GCTCCGAGAAGGCTCTCTCCCACCCGAGGGGTGGACGCAACAGGGGGAATGGGCCATCATATTGTTGCCC<br>CCGGTGGGCACCAACTCTTTTTCCCCCATAGAGAGGCCTTAGCACACTATGTGGGGCACGTTATTGCCGC<br>CTAGAGAAACCGAGCGCCAGAAAATTTCGAAGGGGGGGGCGCTTCTCATCATTTTGCGCAAAACCCCCTT<br>GTGGGAGTATGCCCCGAACTCCTCTGGAACACACAAGCGACACTTGCGCGGGGTCTGCAAAAAACCTCCT<br>GTTGGGAAGCCGGCTTCACN | 124 |

(continued)

| GENBANK ACCESSION NUMBER | SEQUENCE | SEQ ID NO: |
|---|---|---|
| NM_002417 | TACCGGGCGGAGGTGAGCGCGGCGCCGGCTCCTCCTGCGGCGGACTTTGGGTGCGACTTGACGAGCGGTG<br>GTTCGACAAGTGGCCTTGCGGGCCGGATCGTCCCAGTGGAAGAGTTGTAAATTTGCTTCTGGCCTTCCCC<br>TACGGATTATACCTGGCCTTCCCCTACGGATTATACTCAACTTACTGTTTAGAAAATGTGGCCCACGAGA<br>CGCCTGGTTACTATCAAAAGGAGCGGGGTCGACGGTCCCCACTTTCCCCTGAGCCTCAGCACCTGCTTGT<br>TTGGAAGGGGTATTGAATGTGACATCCGTATCCAGCTTCCTGTTGTGTCAAAACAACATTGCAAAATTGA<br>AATCCATGAGCAGGAGGCAATATTACATAATTTCAGTTCCACAAATCCAACACAAGTAAATGGGTCTGTT<br>ATTGATGAGCCTGTACGGCTAAAACATGGAGATGTAATAACTATTATTGATCGTTCCTTCAGGTATGAAA<br>ATGAAAGTCTTCAGAATGGAAGGAAGTCAACTGAATTTCCAAGAAAAATACGTGAACAGGAGCCAGCACG<br>TCGTGTCTCAAGATCTAGCTTCTCTTCTGACCCTGATGAGAAAGCTCAAGATTCCAAGGCCTATTCAAAA<br>ATCACTGAAGGAAAAGTTTCAGGAAATCCTCAGGTACATATCAAGAATGTCAAAGAAGACAGTACCGCAG<br>ATGACTCAAAAGACAGTGTTGCTCAGGGAACAACTAAGTTCATTCCTCAGAACATGCTGGACGTAATGG<br>CAGAAATGCAGCTGATCCCATTTCTGGGGATTTTAAAGAAATTTCCAGCGTTAAATTAGTGAGCCGTTAT<br>GGAGAATTGAAGTCTGTTCCCACTACACAATGTCTTGACAATAGCAAAAAAAATGAATCTCCCTTTTGGA<br>AGCTTTATGAGTCAGTGAAGAAGAGTTGGATGTAAAATCACAAAAAGAAAATGTCCTACAGTATTGTAG<br>AAAATCTGGATTACAAACTGATTACGCAACAGAGAAAGAAAGTGCTGATGGTTTACAGGGGGAGACCCAA<br>CTGTTGGTCTCGCGTAAGTCAAGACCCAAAATCTGGTGGGAGCGGCCACGCTGTGGCAGAGCCTGCTTCAC<br>CTGAACAAGAGCTTGACCAGAACAAGGGGAAGGGAAGAGACGTGGAGTCTGTTCAGACTCCCAGCAAGGC<br>TGTGGGCGCCAGCTTTCCTCTCTATGAGCCGGCTAAAATGAAGACCCCTGTACAATATTCACAGCAACAA<br>AATTCTCCACAAAAACATAAGAACAAAGACCTGTATACTACTGGTAGAAGAGAATCTGTGAATCTGGGTA<br>AAAGTGAAGGCTTCAAGGCTGGTGATAAAACTCTTACTCCCAGGAAGCTTTCAACTAGAAATCGAACACC<br>AGCTAAAGTTGAAGATGCAGCTGACTCTGCCACTAAGCCAGAAAATCTCTCTTCCAAAACCAGAGGAAGT<br>ATTCCTACAGATGTGGAAGTTCTGCCTACGGAAACTGAAATTCACAATGAGCCATTTTTAACTCTGTGGC<br>TCACTCAAGTTGAGAGGAAGATCCAAAAGGATTCCCTCAGCAAGCCTGAGAAATTGGGCACTACAGCTGG<br>ACAGATGTGCTCTGGGTTACCTGGTCTTAGTTCAGTTGATATCAACAACTTTGGTGATTCCATTAATGAG<br>AGTGAGGGAATACCTTTGAAAAGAAGGCGTGTGTCCTTTGGTGGGCACCTAAGACCTGAACTATTTGATG<br>AAAACTTGCCTCCTAATACGCCTCTCAAAAGGGGAGAAGCCCCAACCAAAAGAAAGTCTCTGGTAATGCA<br>CACTCCACCTGTCCTGAAGAAAATCATCAAGGAACAGCCTCAACCATCAGGAAAACAAGAGTCAGGTTCA<br>GAAATCCATGTGGAAGTGAAGGCACAAAGCTTGGTTATAAGCCCTCCAGCTCCTAGTCCTAGGAAAACTC<br>CAGTTGCCAGTGATCAACGCCGTAGGTCCTGCAAAAACAGCCCCTGCTTCCAGCAGCAAATCTCAGACAGA<br>GGTTCCTAAGAGAGGAGGGAGAAAGAGTGGCAACCTGCCTTCAAAGAGAGTGTCTATCAGCCGAAGTCAA<br>CATGATATTTTACAGATGATATGTTCCAAAAGAAGAAGTGGTGCTTCGGAAGCAAATCTGATTGTTGCAA<br>AATCATGGGCAGATGTAGTAAAACTTGGTGCAAAACAAACACAAACTAAAGTCATAAAACATGGTCCTCA<br>AAGGTCAATGACAAAAGGCAAAGAACCTGCTACTCCAAAGAAGCCTGTGGGCGAAGTTCACAGTCAA<br>TTTAGTACAGGCCACGCAAACTCTCCTTGTACCATAATAATAGGGAAAGCTCATACTGAAAAAGTACATG<br>TGCCTGCTCGACCCTACAGAGTGCTCAACAACTTCATTTCCAACCAAAAAATGGACTTTAAGGAAGATCT<br>TTCAGGAATAGCTGAAATGTTCAAGACCCCAGTGAAGGAGCAACCGCAGTTGACAAGCACATGTCACATC<br>GCTATTTCAAATTCAGAGAATTTGCTTGGAAAACAGTTTCAAGGAACTGATTCAGGAGAAGAACCTCTGC<br>TCCCCACCTCAGAGAGTTTTGGAGGAAATGTGTTCTTCAGTGCACAGAATGCAGCAAAACAGCCATCTGA<br>TAAATGCTCTGCAAGCCCTCCCTTAAGACGGCAGTGTATTAGAGAAAATGGAAACGTAGCAAAAACGCCC<br>AGGAACACCTACAAAATGACTTCTCTGGAGACAAAAACTTCAGATACTGAGACAGAGCCTTCAAAAACAG<br>TATCCACTGCAAACAGGTCAGGAAGGTCTACAGAGTTCAGGAATATACAGAAGCTACCTGTGGAAAGTAA<br>GAGTGAAGAAACAAATACAGAAATTGTTGAGTGCATCCTAAAAAGAGGTCAGAAGGCAACACTACTACAA<br>CAAAGGAGAGAAGGGAGAGATGAAGGGAAATAGAAAGACCTTTTGAGACATATAAGGAAAATATTGAATTAA<br>AAGAAAACGATGAAAAGATGAAAGCAATGAAGAGATCAAGAACTTGGGGGCAGAAATGTGCACCAATGTC<br>TGACCTGACAGACCTCAAGAGCTTGCCTGATACAGAACTCATGAAAGACACGGCACGTGGCCAGAATCTC<br>CTCCAAACCCAAGATCATGCCAAGGCACCAAAGAGTGAGAAAGGCAAAATCACTAAAATGCCCTGCCAGT<br>CATTACAACCAGAACCAATAAACACCCCAACACACACAAACAACAGTTGAAGGCATCCCTGGGGAAAGT<br>AGGTGTGAAGAAGAGCTCCTAGCAGTCGGCAAGTTCACACGGACGTCAGGGGGAGACCACGCACACGCAC<br>AGAGAGCCAGCAGGAGATGGCAAGAGCATCAGAACGTTTAAGGAGTCTCCAAAGCAGATCCTGGACCCAG<br>CAGCCCGTGTAACTGGAATGAAGAAGTGGCCAAGAACGCCTAAGGAAGAGCCCAGTCACTAGAAGACCT<br>GGCTGGCTTCAAAGAGCTCTTCCAGACACCAGGTCCCTCTGAGGAATCAATGACTGATGAGAAAACTACC<br>AAAATAGCCTGCAAATCTCCACCACCAGAATCAGTGGACACTCCAACAAGCACAAAGCAATGGCCTAAGA<br>GAAGTCTCAGGAAAGCAGATGTAGAGGAAGAATTCTTAGCACTCAGGAAACTAACACCATCAGCAGGGAA<br>AGCCATGCTTACGCCCAAACCAGCAGGAGGTGATGAGAAAGCATTAAAGCATTTATGGGAACTCCAGTG<br>CAGAAACTGGACCTGGCAGGAACTTTACCTGGCAGCAAAAGACAGCTACAGACTCCTAAGGAAAAGGCCC<br>AGGCTCTAGAAGACCTGGCTGGCTTTAAAGAGCTCTTCCAGACTCCTGGTCACACCGAGGAATTAGTGGC<br>TGCTGGTAAAACCACTAAAATACCCTGCGACTCTCCACAGTCAGACCCAGTGGACACCCCAACAAGCACA<br>AAGCAACGACCCAAGAGAAGTATCAGGAAAGCAGATGTAGAGGGAGAACTCTTAGCGTGCAGGAATCTAA<br>TGCCATCAGCAGGCAAAGCCATGCACACGCCTAAACCATCAGTAGGTGAAGAGAAAGACATCATCATATT | 125 |

| GENBANK ACCESSION NUMBER | SEQUENCE | SEQ ID NO: |
|---|---|---|
| | TGTGGGAACTCCAGTGCAGAAACTGGACCTGACAGAGAACTTAACCGGCAGCAAGAGACGGCCACAAACT CCTAAGGAAGAGGCCCAGGCTCTGGAAGACCTGACTGGCTTTAAAGAGCTCTTCCAGACCCCTGGTCATA CTGAAGAAGCAGTGGCTGCTGGCAAAACTACTAAAATGCCCTGCGAATCTTCTCCACCAGAATCAGCAGA CACCCCAACAAGCACAAGAAGGCAGCCCAAGACACCTTTGGAGAAAAGGGACGTACAGAAGGAGCTCTCA GCCCTGAAGAAGCTCACACAGACATCAGGGGAAACCACACACACAGATAAAGTACCAGGAGGTGAGGATA AAAGCATCAACGCGTTTAGGGAAACTGCAAAACAGAAACTGGACCCAGCAGCAAGTGTAACTGGTAGCAA GAGGCACCCAAAAACTAAGGAAAAGGCCCAACCCCTAGAAGACCTGGCTGGCTTGAAAGAGCTCTTCCAG ACACCAGTATGCACTGACAAGCCCACGACTCACGAGAAAACTACCAAAATAGCCTGCAGATCACAACCAG ACCCAGTGGACACACCAACAAGCTCCAAGCCACAGTCCAAGAGAAGTCTCAGGAAAGTGGACGTAGAAGA AGAATTCTTCGCACTCAGGAAACGAACACCATCAGCAGGCAAAGCCATGCACACACCCAAACCAGCAGTA AGTGGTGAGAAAAACATCTACGCATTTATGGGAACTCCAGTGCAGAAACTGGACCTGACAGAGAACTTAA CTGGCAGCAAGAGACGGCTACAAACTCCTAAGGAAAAGGCCCAGGCTCTAGAAGACCTGGCTGGCTTTAA AGAGCTCTTCCAGACACGAGGTCACACTGAGGAATCAATGACTAACGATAAAACTGCCAAAGTAGCCTGC AAATCTTCACAACCAGACCCAGACAAAAACCCAGCAAGCTCCAAGCGACGGCTCAAGACATCCCTGGGGA AAGTGGGCGTGAAAGAAGAGCTCCTAGCAGTTGGCAAGCTCACACAGACATCAGGAGAGACTACACACAC ACACACAGAGCCAACAGGAGATGGTAAGAGCATGAAAGCATTTATGGAGTCTCCAAAGCAGATCTTAGAC TCAGCAGCAAGCTCTAACTGGCAGCAAGAGGCAGCTGAGAACTCCTAAGGGAAAGTCTGAAGTCCCTGAAG ACCTGGCCGGCTTCATCGAGCTCTTCCAGACACCAAGTCACACTAAGGAATCAATGACTAACGAAAAAAC TACCAAAGTATCCTACAGAGCTTCACAGCCAGACCTAGTGGACACCCCAACAAGCTCCAAGCCACAGCCC AAGAGAAGTCTCAGGAAAGCAGACACTGAAGAAGAATTTTTAGCATTTAGGAAAACAAACGCCATCAGCAG GCAAAGCCATGCACACACCCAAACCAGCAGTAGGTGAAGAGAAAGACATCAACACGTTTTTGGGAACTCC AGTGCAGAAACTGGACCAGCCAGGAAATTTACCTGGCAGCAATAGACGGCTACAAACTCGTAAGGAAAAG GCCCAGGCTCTAGAAGAACTGACTGGCTTCAGAGAGCTTTTCCAGACACCATGCACTGATAACCCCACGA CTGATGAGAAAACTACCAAAAAAAATACTCTGCAAATCTCCGCAATCAGACCCAGCGGACACCCCAACAAA CACAAAGCAACGGCCCAAGAGAAGCCTCAAGAAAGCAGACGTAGAGGAAGAATTTTTAGCATTCAGGAAA CTAACACCATCAGCAGGCAAAGCCATGCACACGCCTAAAGCAGCAGTAGGTGAAGAGAAAGACATCAACA CATTTGTGGGGACTCCAGTGGAGAAACTGGACCTGCTAGGAAATTTACCTGGCAGCAAGAGACGGCCACA AACTCCTAAAGAAAAGGCCAAGGCTCTAGAAGATCTGGCTGGCTTCAAAGAGCTCTTCCAGACACCAGGT CACACTGAGGAATCAATGACCGATGACAAAATCACAGAAGTATCCTGCAAATCTCCACAACCAGACCCAG TCAAAACCCCAACAAGCTCCAAGCAACGACTCAAGATATCCTTGGGGAAAGTAGGTGTGAAAGAAGAGGT CCTACCAGTCGGCAAGCTCACACAGACGTCAGGGAAGACCACACAGACACACAGAGAGACAGCAGGAGAT GGAAAGAGCATCAAAGCGTTTAAGGAATCTGCAAACAGATGCTGAGACCCAGCAAACTATGGAACTGGGA TGGAGAGGTGGCCAAGAACACCTAAGGAAGAGGCCCAATCACTAGAAGACCTGGCCGGCTTCAAAGAGCT CTTCCAGACACCAGACCACACTGAGGAATCAACAACTGATGACAAAACTACCAAAATAGCCTGCAAATCT CCACCACCAGAATCAATGGACACTCCAACAAGCACAAGGAGGCGGCCCAAAACACCTTTGGGGAAAAGGG ATATAGTGGAAGAGCTCTCAGCCCTGAAGCAGCTCACACAGACCACACACACAGACAAAGTACCAGGAGA TGAGGATAAAAGGCATCAACGTGTTCAGGGAAACTGCAAAACAGAAACTGGACCCAGCAGCAAGTGTAACT GGTAGCAAGAGGCAGCCCAAGAACTCCTAAGGGAAAAGCCCAACCCCTAGAAGACTTGGCTGGCTTGAAAG AGCTCTTCCAGACACCAATATGCACTGACAAGCCCACGACTCATGAGAAAACTACCAAAATAGCCTGCAG ATCTCCACAACCAGACCCAGTGGGTACCCCAACAATCTTCAAGCCACAGTCCAAGAGAAGTCTCAGGAAA GCAGACGTAGAGGAAGAATCCTTAGCACTCAGGAAACGAACACCATCAGTAGGGAAAGCTATGGACACAC CCAAACCAGCAGGAGGTGATGAGAAAGACATGAAAGCATTTATGGGAACTCCAGTGCAGAAATTGGACCT GCCAGGAAATTTACCTGGCAGCAAAAGATGGCCACAAACTCCTAAGGAAAAGGCCCAGGCTCTAGAAGAC CTGGCTGGCTTCAAAGAGCTCTTCCAGACACCAGGCACTGACAAGCCCACGACTGATGAGAAAACTACCA AAATAGCCTGCAAATCTCCACAACCAGACCCAGTGGACACCCAGCAAGCACAAAGCAACGGCCCAAGAG AAACCTCAGGAAAGCAGACGTAGAGGAAGAATTTTTAGCACTCAGGAAACGAACACCATCAGCAGGCAAA GCCATGGACACACCAAAACCAGCAGTAAGTGATGAGAAAAATATCAACACATTTGTGGAAACTCCAGTGC AGAAACTGGACCTGCTAGGAAATTTACCTGGCAGCAAGAGACAGCCACAGACTCCTAAGGAAAAGGCTGA GGCTCTAGAGGACCTGGTTGGCTTCAAAGAACTCTTCCAGACACCAGGTCACACTGAGGAATCAATGACT GATGACAAAATCACAGAGTATCCTGTAAATCTCCACAGACCAGATCATTCAAAAACCTCAAGAAGCTCCA AGCAAAGGCTCAAGATACCCCTGGTGAAAGTGGACATGAAAGAAGAGCCCCTAGCAGTCAGCAAGCTCAC ACGGACATCAGGGGAGACTACGCAAACACACACAGAGCCAACAGGAGATAGTAAGAGCATCAAAGCGTTT AAGGAGTCTCCAAAGCAGATCCTGGACCCAGCAGCAAGTGTAACTGGTAGCAGGAGGCAGCTGAGAACTC GTAAGGAAAAGGCCCGTGCTCTAGAAGACCTGGTTGACTTCAAAGAGCTCTTCTCAGCACCAGGTCACAC TGAAGAGTCAATGACTATTGACAAAAACACAAAAATTCCCTGCAAATCTCCCCCACCAGAACTAACAGAC ACTGCCACGAGCACAAAGAGATGCCCCAAGACACGTCCCAAGGAAAGAAGTAAAAGAGGAGCTCTCAGCAG TTGAGAGGCTCACGCAAACATCAGGGCAAAGCACACACACACACAAAGAACCAGCAAGCGGTGATGAGGG CATCAAAGTATTGAAGCAACGTGCAAAGAAGAAACCAAACCCAGTAGAAGAGGAACCCAGCAGGAGAAGG CCAAGAGCACCTAAGGAAAAGGCCCAACCCCTGGAAGACCTGGCCGGCTTCACAGAGCTCTCTGAAACAT CAGGTCACACTCAGGAATCACTGACTGCTGGCAAAGCCACTAAAATACCCTGCGAATCTCCCCCACTAGA AGTGGTAGACACCACAGCAAGCACAAAGAGGCATCTCAGGACACGTGTGCAGAAGGTACAAGTAAAAGAA GAGCCTTCAGCAGTCAAGTTCACACAAACATCAGGGGAAACCACGGATGCAGACAAAGAAACCAGCAGGTG AAGATAAAGGCATCAAAGCATTGAAGGAATCTGCAAAACAGACACCGGCTCCAGCAGCAAGTGTAACTGG CAGCAGGAGACGGCCAAGAGCACCCAGGGAAAGTGCCCAAGCCATAGAAGACCTAGCTGGCTTCAAAGAC CCAGCAGCAGGTCACACTGAAGAATCAATGACTGATGACAAAACCACTAAAATACCCTGCAAATCATCAC CAGAACTAGAAGACACCGCAACAAGCTCAAAGAGACGGCCCAGGACACGTGCCCAGAAAGTAGAAGTGAA GGAGGAGCTGTTAGCAGTTGGCAAGCTCACACAAACCTCAGGGGAGACCACGCACACCGACAAAGAGCCG GTAGGTGAGGGCAAAGGCACGAAAGCATTTAAGCAACCTGCAAAGCGGAAGCTGGACGCAGAAGATGTAA | |

(continued)

| GENBANK ACCESSION NUMBER | SEQUENCE | SEQ ID NO: |
|---|---|---|
| | TTGGCAGCAGGAGACAGCCAAGAGCACCTAAGGAAAAAGGCCCAACCCCTGGAAGATCTGGCCAGCTTCCA AGAGCTCTCTCAAACACCAGGCCACACTGAGGAACTGGCAAATGGTGCTGCTGATAGCTTTACAAGCGCT CCAAAGCAAACACCTGACAGTGGAAAACCTCTAAAAATATCCAGAAGAGTTCTTCGGGCCCCTAAAGTAG AACCCGTGGGAGACGTGGTAAGCACCAGAGACCCTGTAAAATCACAAAGCAAAAGCAACACTTCCCTGCC CCCACTGCCCTTCAAGAGGGGAGGTGGCAAAGATGGAAGCGTCACGGGAACCAAGAGGCTGCGCTGCATG CCAGCACCAGAGGAAATTGTGGAGGAGCTGCCAGCCAGCAAGAAGCAGAGGGTTGCTCCCAGGGCAAGAG GCAAATCATCCGAACCCGTGGTCATCATGAAGAGAAGTTTGAGGACTTCTGCAAAAAGAATTGAACCTGC GGAAGAGCTGAACAGCAACGACATGAAAACCAACAAAGAGGAACACAAATTACAAGACTCGGTCCCTGAA AATAAGGGAATATCCCTGCGCTCCAGACGCCAAAATAAGACTGAGGCAGAACAGCAAATAACTGAGGTCT TTGTATTAGCAGAAAGAATAGAAATAAACAGAAATGAAAAGAAGCCCATGAAGACCTCCCCAGAGATGGA CATTCAGAATCCAGATGATGGAGCCCGGAAACCCATACCTAGAGACAAAGTCACTGAGAACAAAAGGTGC TTGAGGTCTGCTAGACAGAATGAGAGCTCCCAGCCTAAGGTGGCAGAGGAGAGCGGAGGGCAGAAGAGTG CGAAGGTTCTCATGCAGAATCAGAAAGGGAAAGGAGAAGCAGGAAATTCAGACTCCATGTGCCTGAGATC AAGAAAGACAAAAAGCCAGCCTGCAGCAAGCACTTTGGAGAGCAAATCTGTGCAGAGAGTAACGCGGAGT GTCAAGAGGTGTGCAGAAAATCCAAAGAAGGCTGAGGACAATGTGTGTGTCAAGAAAATAAGAACCAGAA GTCATAGGGACAGTGAAGATATTTGACAGAAAAATCGAACTGGGAAAAATATAATAAAGTTAGTTTTGTG ATAAGTTCTAGTGCAGTTTTTGTCATAAATTACAAGTGAATTCTGTAAGTAAGGCTGTCAGTCTGCTTAA GGGAAGAAAACTTTGGATTTGCTGGGTCTGAATCGGCTTCATAAACTCCACTGGGAGCACTGCTGGGCTC CTGGACTGAGAATAGTTGAACACCGGGGGCTTTGTGAAGGAGTCTGGGCCAAGGTTTGCCCTCAGCTTTG CAGAATGAAGCCTTGAGGTCTGTCACCACCCACAGCCACCCTACAGCAGCCTTAACTGTGACACTTGCCA CACTGTGTCGTCGTTTGTTTGCCTATGTCCTCCAGGGCACGGTGGCAGGAACAACTATCCTCGTCTGTCC CAACACTGAGCAGGCACTCGGTAAACACGAATGAATGGATGAGCGCACGGATGAATGGAGCTTACAAGAT CTGTCTTTCCAATGGCCGGGGGCATTTGGTCCCCAAATTAAGGCTATTGGACATCTGCACAGGACAGTCC TATTTTTGATGTCCTTTCCTTTCTGAAAATAAAGTTTGTGCTTTGGAGAATGACTCGTGAGCACATCTT TAGGGACCAAGAGTGACTTTCTGTAAGGAGTGACTCGTGGCTTGCCTTGGTCTCTTGGGAATACTTTTCT AACTAGGGTTGCTCTCACCTGAGACATTCTCCACCCGCGGAATCTCAGGGTCCCAGGCTGTGGGCCATCA CGACCTCAAACTGGCTCCTAATCTCCAGCTTTCCTGTCATTGAAAGCTTCGGAAGTTTACTGGCTCTGCT CCCGCCTGTTTTCTTTCTGACTCTATCTGGCAGCCCGATGCCACCCAGTACAGGAAGTGACACCAGTACT CTGTAAAGCATCATCATCCTTGGAGAGACTGAGCACTCAGCACCTTCAGCCACGATTTCAGGATCGCTTC CTTGTGAGCCGCTGCCTCCGAAATCTCCTTTGAAGCCCAGACATCTTTCTCCAGCTTCAGACTTGTAGAT ATAACTCGTTCATCTTCATTTACTTTCCACTTTGCCCCCTGTCCTCTCTGTTCCCCAAATCAGAGAAT AGCCCGCCATCCCCCAGGTCACCTGTCTGGATTCCTCCCCATTCACCCACCTTGCCAGGTGCAGGTGAGG ATGGTGCACCAGACAGGGTAGCTGTCCCCCAAAATGTGCCCTGTGCGGGCAGTGCCCTGTCTCCACGTTT GTTTCCCCAGTGTCTGGCGGGGAGCCAGGTGACATCATAAATACTTGCTGAATGAATGCAGAAATCAGCG GTACTGACTTGTACTATATTGGCTGCCATGATAGGGTTCTCACAGCGTCATCCATGATCGTAAGGGAGAA TGACATTCTGCTTGAGGGAGGGAATAGAAAGGGGCAGGGAGGGGACATCTGAGGGCTTCACAGGGCTGCA AAGGGTACAGGGATTGCACCAGGGCAGAACAGGGGAGGGTGTTCAAGGAAGAGTGGCTCTTAGCAGAGGC ACTTTGGAAGGTGTGAGGCATAAATGCTTCCTTCTACGTAGGCCAACCTCAAAACTTTCAGTAGGAATGT TGCTATGATCAAGTTGTTCTAACACTTTAGACTTAGTAGTAATTATGAACCTCACATAGAAAAATTTCAT CCAGCCATATGCCTGTGGAGTGGAATATTCTGTTTAGTAGAAAAATCCTTTAGAGTTCAGCTCTAACCAG AAATCTTGCTGAAGTATGTCAGCACCTTTTCTCACCCTGGTAAGTACAGTATTTCAAGAGCACGCTAAGG GTGGTTTTCATTTTACAGGGCTGTTGATGATGGGTTAAAAATGTTCATTTAAGGGCTACCCCCGTGTTTA ATAGATGAACACCACTTCTACACAACCCTCCTTGGTACTGGGGGAGGGAGAGATCTGACAAATACTGCCC ATTCCCCTAGGCTGACTGGATTTGAGAACAAATACCCACCCATTTCCACCATGGTATGGTAACTTCTCTG AGCTTCAGTTTCCAAGTGAATTTCCATGTAATAGGACATTCCCATTAAATACAAGCTGTTTTTACTTTT CGCCTCCCAGGGCCTGTGGGATCTGGTCCCCCAGCCTCTCTTGGGCTTTCTTACACTAACTCTGTACCTA CCATCTCCTGCCTCCCTTAGGCAGGCACCTCCAACCACCACACACTCCCTGCTGTTTTCCCTGCCTGGAA CTTTCCCTCCTGCCCCACCAAGATCATTTCATCCAGTCCTGAGCTCAGCTTAAGGGAGGCTTCTTGCCTG TGGGTTCCCTCACCCCCATGCCTGTCCTCCAGGCTGGGGCAGGTTCTTAGTTTGCCTGGAATTGTTCTGT ACCTCTTTGTAGCACGTAGTGTTGTGGAAACTAAGCCACTAATTGAGTTTCTGGCTCCCCTCCTGGGGTT GTAAGTTTTGTTCATTCATGAGGGCCGACTGCATTTCCTGGTTACTCTATCCCAGTGACCAGCCACAGGA GATGTCCAATAAAGTATGTGATGAAATGGTCTTAAAAAAAAAAAAAAA | |
| NM_024101 | GCGCCGGGACGTGGCCAGTTGCCCGCCTGCCCCGGAGAGCCAGGCGCTAACCAGCCGCTCTGCGCCCCGC GCCCTGCTTGCCCCCATTATCCAGCCTTGCCCCGGCGCCCTGACCTGACGCCCTGGCCTGACGCCCTGCT TCGTCGCCTCCTTTCTCTCCCAGGTGCTGGACCAGGGACTGAGCGTCCCCCGGAGAGGGTCCGGTGTGAC CCCGACAAGAAGCAGAAATGGGAAGAAACTGGATCTTTCCAAGCTCATGATGAAGAGGCCCAGCATGT CTTGGAAGTTGTTCAACGAGATTTTGACCTCCGAAGGAAAGAAGAGGAACGGCTAGAGGCGTTGAAGGGC AAGATTAAGAAGGAAAGCTCCAAGAGGGAGCTGCTTTCCGACACTGCCCATCTGAACGAGACCCACTGCG CCCGCTGCCTGCAGCCCTACCAGCTGCTTGTGAATAGCAAAAGGCAGTGCCTGGAATGTGGCCTCTTCAC CTGCAAAAGCTGTGGCCGCGTCCACCGGAGGAGCAGGGCTGGATCTGTGACCCCTGCCATCTGGCCAGA GTCGTGAAGATCGGCTCACTGGAGTGGTACTATGAGCATGTGAAAGCCCGCTTCAAGAGGTTCGGAAGTG CCAAGGTCATCCGGTCCCTCCACGGGCGGCTGCAGGGTGGAGCTGGGCCTGAACTGATATCTGAAGAGAG AAGTGGAGACAGCGACGCAGACATGAGGATGGAGAACCTGGCTCAGAGGCCCAGGCCCAGGCCCAGCCC TTTGGCAGCAAAAAAAAGCGCCTCCTCTCCGTCCACGACTTCGACTTCGAGGGAGACTGACGATGACTCCA CTCAGCCTCAAGGTCACTCCCTGCACCTGTCCTCAGTCCCTGAGGCCAGGGACAGCCCACAGTCCCTCAC AGATGAGTCCTGCTCAGAGAAGGCAGCCCCTCACAAGGCTGAGGGCCTGGAGGAGGCTGATACTGGGGCC TCTGGGTGCCACTCCCATCCGGAAGAGCAGCCGACCAGCATCTCACCTTCCAGACACGGCGCCCTGGCTG AGCTCTGCCCGCCTGGAGGCTCCCACAGGATGGCCCTGGGGACTGCTGCTGCACTCGGGTCGAATGTCAT | 126 |

| GENBANK ACCESSION NUMBER | SEQUENCE | SEQ ID NO: |
|---|---|---|
| | CAGGAATGAGCAGCTGCCCCTGCAGTACTTGGCCGATGTGGACACCTCTGATGAGGAAAGCATCCGGGCT CACGTGATGGCCTCCCACCATTCCAAGCGGAGAGGCCGGGCGTCTTCTGAGAGTCAGATCTTTGAGCTGA ATAAGCATATTTCAGCTGTGGAATGCCTGCTGACCTACCTGGAGAACACAGTTGTGCCTCCCTTGGCCAA GGGTCTAGGTGCTGGAGTGCGCACGGAGGCCGATGTAGAGGAGGAGGCCCTGAGGAGGAAGCTGGAGGAG CTGACCAGCAACGTCAGTGACCAGGAGACCTCGTCCGAGGAGGAGGAAGCCAAGGACGAAAAGGCAGAGC CCAACAGGGACAAATCAGTTGGGCCTCTCCCCCAGGCGGACCCGGAGGTGGGCACGGCTGCCCATCAAAC CAACAGACAGGAAAAAAGCCCCCAGGACCCTGGGGACCCCGTCCAGTACAACAGGACCACAGATGAGGAG CTGTCAGAGCTGGAGGACAGAGTGGCAGTGACGGCCTCAGAAGTCCAGCAGGCAGAGAGCGAGGTTTCAG ACATTGAATCCAGGATTGCAGCCCTGGAGGGCCGCAGGGCTCACGGTGAAGCCCTCGGGAAAGCCCCGGAG GAAGTCAAACCTCCCGATATTTCTCCCTCGAGTGGCTGGGAAACTTGGCAAGAGACCCAGAGGACCCAAAT GCAGACCCTTCAAGTGAGGCCAAGGCAATGGCTGTGCCCTATCTTCTGAGAAGAAAGTTCAGTAATTCCC TGAAAAGTCAAGGTAAAGATGATGATTCTTTTGATCGGAAATCAGTGTACCGAGGCTCGCTGACACAGAG AAACCCCAACGCGAGGAAAGGAATGGCCAGCCACACCTTCGCGAAACCTGTGGTGGCCCACCAGTCCTAA CGGGACAGGACAGAGAGACAGAGCAGCCCTGCACTGTTTTCCCTCCACCACAGCCATCCTGTCCCTCATT GGCTCTGTGCTTTCCACTATACACAGTCACCGTCCCAATGAGAAACAAGAAGGAGCACCCTCCACATGGA CTCCCACCTGCAAGTGGACAGCGACATTCAGTCCTGCACTGCTCACCTGGGTTTACTGATGACTCCTGGC TGCCCCACCATCCTCTCTGATCTGTGAGAAACAGCTAAGCTGCTGTGACTTCCCTTTAGGACAATGTTGT GTAAATCTTTGAAGGACACACCGAAGACCTTTATACTGTGATCTTTTACCCCTTTCACTCTTGGCTTTCT TATGTTGCTTTCATGAATGGAATGGAAAAAAGATGACTCAGTTAAGGCACCAGCCATATGTGTATTCTTG ATGGTCTATATCGGGGTGTGAGCAGATGTTTGCGTATTTCTTGTGGGTGTGACTGGATATTAGACATCCG GACAAGTGACTGAACTAATGATCTGCTGAATAATGAAGGAGGAATAGACACCCCAGTCCCCACCCTACGT GCACCCGCTCTGCAAGTTCCCATGTGATCTGTAGACCAGGGGAAATTACACTGCGGTCAAGGGCAGAGCC TGCACATGACAGCAAGTGAGCCATTTGATAGATGCTCAGATGCTAGTGCAGAGAGCGTGCTGGGAGACGAA GAGACAGCAGGCAGAGCTCCAGATGGGCAAGGAAGAGGCTTGGTTCTAGCCTGGCTCTGCCCCTCCACTGC AGTGGATCCAGTGGGGCAGAGGCAGAGGGTCACAACCAATGAGGGATGTCTGCCAAGGATGGGGGTGCA GAGGCCACAGGAGTCAGCTTGCCACTCGCCCATTGGTTACATAGATGATCTCTCAGACAGGCTGGGACTC AGAGTTATTTCCTAGTATCGGTGTGCCCCATCCAGTTTTAAGTGGAGCCCTCCAAGACTCTCCAGAGCTG CCTTTGAACATCCTAACAGTAATCACATCTCACCCTCCCTGAGGTTCACTTTAGACAGGACCCAATGGCT GCACTGCCTTTGTCAGAGGGGGTGCTGAGAGGAGTGGCTTCTTTTAGAATCAAACAGTAGAGACAAGAGT CAAGCCTTGTGTCTTCAAGCATTGACCAAGTTAAGTGTTTCCTTCCCTCTCTCAATAAGACACTTCCAGG AGCTTTCCAATCTCTCACTTAAAACTAAGGTTTGAATCTCAAAGTGTTGCTGGGAGGCTGATACTCCTGC AACTTCAGGAGACCTGTGAGCACACATTAGCAGCTGTTTCTCTGACTCCTTGTGGCATCAGATAAAAACG TGGGAGTTTTTCCATATAATTCCCAGCCTTACTTATAAATTCTATTCTTTGAAAAAATTATTCAGGCTAG GTAAGGTGGCTCATACCTATAATCCCAGCCCTTTGAGAGGCCAAGGTGGGAGAATTGCTTGAGGCCAGGA GTTTGAGACCTCCTGGGCAACATAGTGAGATCCCATCTCTACAAAAAACAAAACAAAAAAATTACCCAAG CATGATGGTATATGCCTGTAGTCGTACCTACTTACTTAGGAGGCTGAGGCAGGAGGATCACTTGAGCCCT GGAGGTTGGGGCTGCAGTGAGCCATGATCGCATCACTATACTCGAGCCTGGGCAACAGAGTGAGACCTTG TCTCTTAAAAAAATTAATAATAAATAAATGAAATAATTCTTCAGAAAAAAAAAAAAAAAAA | |
| NM_005940 | AAGCCCAGCAGCCCCGGGGCGGATGGCTCCGGCCGCCTGGCTCCGCAGCGCGGCCGCGCGCGCCCTCCTG CCCCCGATGCTGCTGCTGCTGCTCCAGCCGCCGCCGCTGCTGGCCCGGGCTCTGCCGCCGGACGCCCACC ACCTCCATGCCGGAGAGGAGGGGGCCACAGCCCTGGCATGCAGCCCTGCCCAGTAGCCCGGCACCTGCCCC TGCCACGCAGGAAGCCCCCCGGCCTGCCAGCAGCCTCAGGCCTCCCCGCTGTGGCGTGCCCGACCCCATCT GATGGGCTGAGTGCCCGCAACCGACAGAAGAGGTTCGTGCTTTCTGGCGGGCGTGGGAGGAAGACGGACC TCACCTACAGGATCCTTCGGTTCCCCATGGCAGTTGGTGCAGGAGCAGGTGCGGCAGACGATGGCAGAGGC CCTAAAGGTATGGAGCGATGTGACGCCACTCACCTTTACTGAGGTGCACGAGGGCCGTGCTGACATCATG ATCGACTTCGCCAGGTACTGGCATGGGGACGACCTGCCGTTTGATGGGCCTGGGGGGCATCCTGGCCCATG CCTTCTTCCCCAAGACTCACCGAGAAGGGGATGTCCACTTCGACTATGATGAGACCTGGACTATCGGGGA TGACCAGGGCACAGACCTGCTGCAGGTGGCAGCCCATGAATTTGGCCACGTGCTGGGGCTGCAGCACACA ACAGCAGCCAAGGCCCTGATGTCCGCCTTCTACACACCTTTCGCTACCCACTGAGTCTCAGCCCAGATGACT GCAGGGGCGTTCAACACCTATATGGCCAGCCCTGGCCCACTGTCACCTCCAGGACCCCAGCCCTGGGCCC CCAGGCTGGGATAGACACCAATGAGATTGCACCGCTGGAGCCAGACGCCCCGCCAGATGCCTGTGAGGCC TCCTTTGACGCGGTCTCCACCATCCGAGGCGAGCTCTTTTTTCTTCAAAGCGGGCTTTGTGTGGCGCCTCC GTGGGGGCCAGCTGCAGCCCGGCTACCCAGCATTGGCCTCTCGCCACTGGCAGGGACTGCCCAGCCCTGT GGACGCTGCCTTCGAGGATGCCCAGGGCCACATTTGGTTCTTCCAAGGTGCTCAGTACTGGGTGTACGAC GGTGAAAAGCCAGTCCTGGGCCCCGCACCCCTCACCGAGCTGGGCCTGGTGAGGTTCCCGGTCCATGCTG CCTTGGTCTGGGGTCCCGAGAAGAACAAGATCTACTTCTTCCGAGGCAGGGACTACTGGCGTTTCCACCC CAGCACCCGGCGTGTAGACAGTCCCGTGCCCCGCAGGGCCCACTGACTGGAGAGGGGTGCCCTCTGAGATC GACGCTGCCTTCCAGGATGCTGATGGCTATGCCTACTTCCTGCGCGGCCGCCTCTACTGGAAGTTTGACC CTGTGAAGGTGAAGGCTCTGGAAGGCTTCCCCCGTCTCGTGGGTCCTGACTTCTTTGGCTGTGCCGAGCC TGCCAACACTTTCCTCTGACCATGGCTTGGATGCCCAGGGGTGCTGACCCCTGCCAGGCCACGAATAT CAGGCTAGAGACCCATGGCCATCTTTGTGGCTGTGGGCACCAGGCATGGGACTGAGCCCATGTCTCCTCA GGGGGATGGGGTGGGGTACAACCACCATGACAACTGCCGGGAGGGCCACGCAGGTCGTGGTCACCTGCCA GCGACGTGTCTCAGACTGGGCAGGGAGGCTTTGGCATGACTTAAGAGGAAGGGCAGTCTTGGGCCCGCTAT GCAGGTCCTGGCCAAAACCTGGCTGCCCTGTCTCCATCCCCTGTCCCTCAGGGTAGCACCATGGCAGGACTGG GGGAACTGGAGTGTCCTTGCTGTATCCCTGTTGTGAGGTTCCTTCCAGGGGCTGGCACTGAAGCAAGGGT GCTGGGGCCCCATGGCCTTCAGCCCTGGCTGAGCAACTGGGCTGTAGGGCAGGGCCACTTCCTGAGGTCA GGTCTTGGTAGGTGCCTGCATCTGTCTGCCTTCTGGCTGACAATCCTGGAAATCTGTTCTCCAGAATCCA GGCCAAAAAGTTCACAGTCAAATGGGGAGGGGTATTCTTCATGCAGGAGACCCCAGGCCCTGGAGGCTGC | 127 |

(continued)

| GENBANK ACCESSION NUMBER | SEQUENCE | SEQ ID NO: |
|---|---|---|
| | AACATACCTCAATCCTGTCCCAGGCCGGATCCTCCTGAAGCCCTTTTCGCAGCACTGCTATCCTCCAAAG<br>CCATTGTAAATGTGTGTACAGTGTGTATAAACCTTCTTCTTCTTTTTTTTTTTTTTAAACTGAGGATTGTC | |
| NM_002467 | GACCCCCGAGCTGTGCTGCTCGCGGCCGCCACCGCCGGGCCCCGGCCGTCCCTGGCTCCCCTCCTGCCTC<br>GAGAAGGGCAGGGCTTCTCAGAGGCTTGGCGGGAAAAAGAACGGAGGGAGGGATCGCGCTGAGTATAAAA<br>GCCGGTTTTCGGGGCTTTATCTAACTCGCTGTAGTAATTCCAGCGAGAGGCAGAGGGAGCGAGCGGGCGG<br>CCGGCTAGGGTGGAAGAGCCGGGCGAGCAGAGCTGCGCTGCGGGCGTCCTGGGAAGGGAGATCCGGAGCG<br>AATAGGGGGCTTCGCCTCTGGCCCAGCCCTCCCGCTGATCCCCCAGCCAGCGGTCCGCAACCCTTGCCGC<br>ATCCACGAAACTTTGCCCATAGCAGCGGGCGGGCACTTTGCACTGGAACTTACAACACCCGAGCAAGGAC<br>GCGACTCTCCCGACGCGGGGAGGCTATTCTGCCCATTTGGGGACACTTCCCCGCCGCTGCCAGGACCCGC<br>TTCTCTGAAAGGCTCTCCTTGCAGCTGCTTAGACGCTGGATTTTTTTCGGGTAGTGGAAAACCAGCAGCC<br>TCCCGCGACGATGCCCCTCAACGTTAGCTTCACCAACAGGAACTATGACCTCGACTACGACTCGGTGCAG<br>CCGTATTTCTACTGCGACGAGGAGGAGAACTTCTACCAGCAGCAGCAGCAGAGCGAGCTGCAGCCCCCGG<br>CGCCCAGCGAGGATATCTGGAAGAAATTCGAGCTGCTGCCCACCCCGCCCCTGTCCCCTAGCCGCCGCTC<br>CGGGCTCTGCTCGCCCTCCTACGTTGCGGTCACACCCTTCTCCCTTCGGGGAGACAACGACGGCGGTGGC<br>GGGAGCTTCTCCACGGCCGACCAGCTGGAGATGGTGACCGAGCTGCTGGGAGGAGACATGGTGAACCAGA<br>GTTTCATCTGCGACCCGGACGACGAGACCTTCATCAAAAACATCATCATCCAGGACTGTATGTGGAGCGG<br>CTTCTCGGCCGCCGCCAAGCTCGTCTCAGAGAAGCTGGCCTCCTACCAGGCTGCGCGCAAAGACAGCGGC<br>AGCCCGAACCCCGCCCGCGGCCACAGCGTCTGCTCCACCTCCAGCTTGTACCTGCAGGATCTGAGCGCCG<br>CCGCCTCAGAGTGCATCGACCCCTCGGTGGTCTTCCCCTACCCTCTCAACGACAGCAGCTCGCCCAAGTC<br>CTGCGCCTCGCAAGACTCCAGCGCCTTCTCTCCGTCCTCGGATTCTCTGCTCTCCTCGACGGAGTCCTCC<br>CCGCAGGGCAGCCCCGAGCCCCTGGTGCTCCATGAGGAGACACCGCCCACCACCAGCAGCGACTCTGAGG<br>AGGAACAAGAAGATGAGGAAGAAATCGATGTTGTTTCTGTGGAAAAGAGGCAGGCTCCTGGCAAAAGGTC<br>AGAGTCTGGATCACCTTCTGCTGGAGGCCACAGCAAACCTCCTCACAGCCCACTGGTCCTCAAGAGGTGC<br>CACGTCTCCACACATCAGCACAACTACGCAGCGCCTCCCTCCACTCGGAAGGACTATCCTGCTGCCAAGA<br>GGGTCAAGTTGGACAGTGTCAGAGTCCTGAGACAGATCAGCAACAACCGAAAATGCACCAGCCCCAGGTC<br>CTCGGACACCGAGGAGAATGTCAAGAGGCGAACACACAACGTCTTGGAGCGCCAGAGGAGGAACGAGCTA<br>AAACGGAGCTTTTTTGCCCTGCGTGACCAGATCCCGGAGTTGGAAAACAATGAAAAGGCCCCCAAGGTAG<br>TTATCCTTAAAAAAGCCACAGCATACATCCTGTCCGTCCAAGCAGAGGAGCAAAAGCTCATTTCTGAAGA<br>GGACTTGTTGCGGAAACGACGAGAACAGTTGAAACACAAACTTGAACAGCTACGGAACTCTTGTGCGTAA<br>GGAAAAGTAAGGAAAACGATTCCTTCTAACAGAAATGTCCTGAGCAATCACCTATGAACTTGTTTCAAAT<br>GCATGATCAAATGCAACCTCACAACCTTGGCTGAGTCTTGAGACTGAAAGATTTAGCCATAATGTAAACT<br>GCCTCAAATTGGACTTTGGGCATAAAAGAACTTTTTTATGCTTACCATCTTTTTTTTTTCTTTAACAGAT<br>TTGTATTTAAGAATTGTTTTTAAAAAATTTTAAGATTTACACAATGTTTCTCTGTAAATATTGCCATTAA<br>ATGTAAATAACTTTAATAAAACGTTTATAGCAGTTACACAGAATTTCAATCCTAGTATATAGTACCTAGT<br>ATTATAGGTACTATAAACCCTAATTTTTTTTATTTAAGTACATTTTGCTTTTTAAAGTTGATTTTTTTCT<br>ATTGTTTTTAGAAAAAATAAAATAACTGGCAAATATATCATTGAGCCAAATCTTAAAAAAAAAAAAAAAA | 128 |
| BC013732 | GTGGGAGGATTGCATTCAGTCTAGTTCCTGGTTGCCGGCTGAAATAACCTGCTCTCCAAAATGTCCACAA<br>AAGTGACTTAAGTCAGGTTCCCCCAAAACCAGACACCAAGACAAGAATCCATGTGTGTGTGACTGAAGGAA<br>GTGCTGGGAGGACCCCAGCTGCAGCCTGGATGTGAACTGCAACTCCAAAGTGTGTCCAGACTCAAGGCAA<br>GGGCACTAGGCTTTCCAGACCTCCTACTAAGTCATTGATCCAGCACTGCCCTGCCAGGACATAAATCCCT<br>GGCACCTCTTGCTCTCTGCAAAGGAGGGCAAAGCAGCTTCAGGAGCCCTTGGGAGTCCTCCAAAGAGAGT<br>CTAGGGTACAGGTCCGAAAGTAGAAGAACACAGAAGGCAGGCCAGGGGCACTGTGAGATGGTAAAAGAGA<br>TCTGAAGGGATCCAGAATTCAAGCCAGGAAGAAGCAGCAATCTGTCTTCTGGATTAAAACTGAAGATCAA<br>CCTACTTTCAACTTACTAAGAAAGGGGATCATGGACATTGAAGCATATCTTGAAAGAATTGGCTATAAGA<br>AGTCTAGGAACAAATTGGACTTGGAAACATTAACTGATATTCTTCAACACCAGATCCGAGCTGTTCCCTT<br>TGAGAACCTTAACATCCATTGTGGGGATGCCATGGACTTAGGCTTAGAGGCCATTTTGATCAAGTTGTG<br>AGAAGAAATCGGGGTGGATGGTGTCTCCAGGTCAATCATCTTCTGTACTGGGCTCTGACCACTATTGGTT<br>TTGAGACCACGATGTTGGGAGGGTATGTTTACAGCACTCCAGCCAAAAAATACAGCACTGGCATGATTCA<br>CCTTCTCCTGCAGGTGACCATTGATGGCAGGAACTACATTGTCGATGCTGGGTTTGGACGCTCATACCAG<br>ATGTGGCAGCCTCTGGAGTTAATTTCTGGGAAGGATCAGCCTCAGGTGCCTTGTGTCTTCCGTTTGACGG<br>AAGAGAATGGATTCTGGTATCTAGACCAAATCAGAAGGGAACAGTACATTCCAAATGAAGAATTTCTTCA<br>TTCTGATCTCCTAGAAGACAGCAAATACCGAAAAATCTACTCCTTTACTCTTAAGCCTCGAACAATTGAA<br>GATTTTGAGTCTATGAATACATACCTGCAGACATCTCCATCATCTGTGTTTACTAGTAAATCATTTTGTT<br>CCTTGCAGACCCCAGATGGGGTTCACTGTTTGGTGGGCTTCACCCTCACCCATAGGAGATTCAATTATAA<br>GGACAATACAGATCTAATAGAGTTCAAGACTCTGAGTGAGGAAGAAATAGAAAAAGTGCTGAAAAATATA<br>TTTAATATTTCCTTGCAGAGAAAGCTTGTGCCCAAACATGGTGATAGATTTTTTACTATTTAGAATAAGG<br>AGTAAAACAATCTTGTCTATTTGTCATCCAGCTCACCAGTTATCAACTGACGACCTATCATGTATCTTCT<br>GTACCCTTACCTTATTTTGAAGAAAATCCTAGACATCAAATCATTTCACCTATAAAAATGTCATCATATA<br>TAATTAAACAGCTTTTTAAAGAAACATAACCACAAACCTTTTCAAATAATAATAATAATAATAATAATAA<br>ATGTCTTTTAAAGATGGCCTGTGGTTATCTTGGAAATTGGTGATTTATGCTAGAAAGCTTTTAATGTTGG<br>TTTATTGTTGAATTCCTAGAAAAGTTTTATGGGTAGATGAGTAAATAAAATATTGTAAAAAAACTTATTG<br>TCTATAAAGTATATTAAAACATTGTTGGCTAATATAAAAAAAAAAAAAAAA | 129 |

(continued)

| GENBANK ACCESSION NUMBER | SEQUENCE | SEQ ID NO: |
|---|---|---|
| NM_014321 | GCGCGCGGGTTTCGTTGACCCGCGGCGTTCACGGGAATTGTTCGCTTTAGTGCCGGCGCCATGGGGTCGG AGCTGATCGGGCGCCTAGCCCCGCGCCTGGGCCTCGCCGAGCCCGACATGCTGAGGAAAGCAGAGGAGTA CTTGCGCCTGTCCCGGGTGAAGTGTGTCGGCCTCTCCGCACGCACCACGGAGACCAGCAGTGCAGTCATG TGCCTGGACCTTGCAGCTTCCTGGATGAAGTGCCCCTTGGACAGGGCTTATTTAATTAAACTTTCTGGTT TGAACAAGGAGACATATCAGAGCTGTCTTAAATCTTTTGAGTGTTTACTGGGCCTGAATTCAAATATTGG AATAAGAGACCTAGCTGTACAGTTTAGCTGTATAGAAGCAGTGAACATGGCTTCAAAGATACTAAAAAGC TATGAGTCCAGTCTTCCCCAGACACAGCAAGTGGATCTTGACTTATCCAGGCCACTTTTCACTTCTGCTG CACTGCTTTCAGCATGCAAGATTCTAAAGCTGAAAGTGGATAAAAACAAAATGGTAGCCACATCCGGTGT AAAAAAAGCTATATTTGATCGACTGTGTAAACAACTAGAGAAGATTGGACAGCAGGTCGACAGAGAACCT GGAGATGTAGCTACTCCACCACGGAAGAGAAAGAAGATAGTGGTTGAAGCCCCAGCAAAGGAAATGGAGA AGGTAGAGGAGATGCCACATAAACCACAGAAAGATGAAGATCTGACACAGGATTATGAAGAATGGAAAAG AAAAATTTTGGAAAATGCTGCCAGTGCTCAAAAGGCTACAGCAGAGTGATTTCAGCTTCCAAACTGGTAT ACATTCCAAACTGATAGTACATTGCCATCTCCAGGAAGACTTGACGGCTTTGGGATTTTGTTTAAACTTT TATAATAAGGATCCTAAGACTGTTGCCTTTAAATAGCAAAGCAGCCTACCTGGAGGCTAAGTCTGGGCAG TGGGCTGGCCCCTGGTGTGAGCATTAGACCAGCCACAGTGCCTGATTGGTATAGCCTTATGTGCTTTCCT ACAAAATGGAATTGGAGGCCGGGCGCAGTGGCTCACGCCTGTAATCCCAGCACTTTGGGAGGCCAAGGTG GGTGGATCACCTGAGGTCAGGAGCTCGAGACCAGCCTGGCCAACATGGTGAAACCCCATCTCTACTAAAA ATACAAAAATTAGCCAGGTGTGATGGTGCATGCCTGTAATCCCAGCTCCTCAGTAGGCTGAGACAGGAGC ATCACTTGAACGTGGGAGGCAGAGGTTGCAGTGAGCCGAGATTGCACCACCGCACTCCAGCCTGGGTGAC AGAGCGAGACTTATCTCATAAATAAATAGATAGATACTCCAGCCTGGGTGACAGAGCGAGACTTATAGAT AGATAGATAGATAGATGGATAGATAGATAGATAGATAGATAGATAGATAAACGGAATTGGAGCCATTTTG CTTTAAGTGAATGGCAGTCCCTTGTCTTATTCAGAATATAAAATTCAGTCTGAATGGCATCTTACAGATT TTACTTCAATTTTTGTGTACGGTATTTTTTATTTGACTAAATCAATATATTGTACAGCCTAAGTTAATAA ATGTTATTTATATATGCAAAAAAAAAAAAAAAAA | 130 |

(continued)

| GENBANK ACCESSION NUMBER | SEQUENCE | SEQ ID NO: |
|---|---|---|
| NM_000926 | AGTCCACAGCTGTCACTAATCGGGGTAAGCCTTGTTGTATTTGTGCGTGTGGGTGGCATTCTCAATGAGA<br>ACTAGCTTCACTTGTCATTTGAGTGAAATCTACAACCCGAGGCGGCTAGTGCTCCCGCACTACTGGGATC<br>TGAGATCTTCGGAGATGACTGTCGCCCGCAGTACGGAGCCAGCAGAAGTCCGACCCTTCCTGGGAATGGG<br>CTGTACCGAGAGGTCCGACTAGCCCCAGGGTTTTAGTGAGGGGGCAGTGGAACTCAGCGAGGGACTGAGA<br>GCTTCACAGCATGCACGAGTTTGATGCCAGAGAAAAAGTCGGGAGATAAAGGAGCCGCGTGTCACTAAAT<br>TGCCGTCGCAGCCGCAGCCACTCAAGTGCCGGACTTGTGAGTACTCTGCGTCTCCAGTCCTCGGACAGAA<br>GTTGGAGAACTCTCTTGGAGAACTCCCCGAGTTAGGAGACGAGATCTCCTAACAATTACTACTTTTTCTT<br>GCGCTCCCCACTTGCCGCTCGCTGGGACAAACGACAGCCACAGTTCCCCTGACGACAGGATGGAGGCCAA<br>GGGCAGGAGCTGACCAGCGCCGCCCTCCCCCGCCCCCGACCCAGGAGGTGGAGATCCCTCCGGTCCAGCC<br>ACATTCAACACCCACTTTCTCCTCCCTCTGCCCCTATATTCCCGAAACCCCCTCCTCCTTCCCTTTTCCC<br>TCCTCCTGGAGACGGGGGAGGAGAAAAGGGGAGTCCAGTCGTCATGACTGAGCTGAAGGCAAAGGGTCCC<br>CGGGCTCCCCACGTGGCGGGCGGCCCGCCCTCCCCCGAGGTCGGATCCCCACTGCTGTGTCGCCCAGCCG<br>CAGGTCCGTTCCCGGGGAGCCAGACCTCGGACACCTTGCCTGAAGTTTCGGCCATACCTATCTCCCTGGA<br>CGGGCTACTCTTCCCTCGGCCCTGCCAGGGACAGGACCCCTCCGACGAAAAGACGCAGGACCAGCAGTCG<br>CTGTCGGACGTGGAGGGCGCATATTCCAGAGCTGAAGCTACAAGGGGTGCTGGAGGCAGCAGTTCTAGTC<br>CCCCAGAAAAGGACAGCGGACTGCTGGACAGTGTCTTGGACACTCTGTTGGCGCCCTCAGGTCCCGGGCA<br>GAGCCAACCCAGCCCTCCCGCCTGCGAGGTCACCAGCTCTTGGTGCCTGTTTGGCCCCGAACTTCCCGAA<br>GATCCACCGGCTGCCCCCGCCACCCAGCGGGTGTTGTCCCCGCTCATGAGCCGGTCCGGGTGCAAGGTTG<br>GAGACAGCTCCGGGACGGCAGCTGCCCATAAAGTGCTGCCCCGGGGCCTGTCACCAGCCCGGCAGCTGCT<br>GCTCCCGGCCTCTGAGAGCCCTCACTGGTCCGGGGCCCCAGTGAAGCCGTCTCCGCAGGCCGCTGCGGTG<br>GAGGTTGAGGAGGAGGATGGCTCTGAGTCCGAGGAGTCTGCGGGTCCGCTTCTGAAGGGCAAACCTCGGG<br>CTCTGGGTGGCGCGGCGGCTGGAGGAGGAGCCGCGGCTGTCCCGCCGGGGGCGGCAGCAGGAGGCGTCGC<br>CCTGGTCCCCAAGGAAGATTCCCGCTTCTCAGCGCCCAGGGTCGCCCTGGTGGAGCAGGACGCGCCGATG<br>GCGCCCGGGCGCTCCCCGCTGGCCACCACGGTGATGGATTTCATCCACGTGCCTATCCTGCCTCTCAATC<br>ACGCCTTATTGGCAGCCCGCACTCGGCAGCTGCTGGAAGACGAAAGTTACGACGGCGGGGCCGGGGCTGC<br>CAGCGCCTTTGCCCCGCCGCGGAGTTCACCCTGTGCCTCGTCCCACCCCGGTCGCTGTAGGCGACTTCCCC<br>GACTGCGCGTACCCGCCCGACGCCGAGCCCAAGGACGACGCGTACCCTCTCTATAGCGACTTCCAGCCGC<br>CCGCTCTAAAGATAAAGGAGGAGGAGGAAGGCGCGGAGGCCTCCGCGCGCTCCCCGCGTTCCTACCTTGT<br>GGCCGGTGCCAACCCCGCAGCCTTCCCGGATTTCCCGTTGGGGCCACCGCCCCCGCTGCCGCCGCGAGCG<br>ACCCCATCCAGACCCGGGGAAGCGGCGGTGACGGCCGCACCCGCCAGTGCCTCAGTCTCGTCTGCGTCCT<br>CCTCGGGGTCGACCCTGGAGTGCATCCTGTACAAAGCGGAGGGCGCGCCGCCCCAGCAGGGCCCGTTCGC<br>GCCGCCGCCCTGCAAGGCGCCGGGCGCGAGCGGCTGCCTGCTCCCGCGGGACGGCCTGCCCTCCACCTCC<br>GCCTCTGCCGCCGCCGGGGCGGCCCCCGCGCTCTACCCTGCACTCGGGCCTCAACGGGCTCCCGCCAGC<br>TCGGCTACCAGGCCGCCGTGCTCAAGGAGGGCCTGCCGCAGGTCTACCCGCCCTATCTCAACTACCTGAG<br>GCCGGATTCAGAAGCCAGCCAGAGCCCACAATACAGCTTCGAGTCATTACCTCAGAAGATTTGTTTAATC<br>TGTGGGGATGAAGCATCAGGCTGTCATTATGGTGTCCTTACCTGTGGGAGCTGTAAGGTCTTCTTTAAGA<br>GGGCAATGGAAGGGCAGCACAACTACTTATGTGCTGGAAGAAATGACTGCATCGTTGATAAAATCCGCAG<br>AAAAAACTGCCCAGCATGTCGCCTTAGAAAGTGCTGTCAGGCTGGCATGGTCCTTGGAGGTCGAAAATTT<br>AAAAAGTTCAATAAAGTCAGAGTTGTGAGAGCACTGGATGCTGTTGCTCTCCCACAGCCAGTGGGCGTTC<br>CAAATGAAAGCCAAGCCCTAAGCCAGAGATTCACTTTTTCACCAGGTCAAGACATACAGTTGATTCCACC<br>ACTGATCAACCTGTTAATGAGCATTGAACCAGATGTGATCTATGCAGGACATGACAACACAAAACCTGAC<br>ACCTCCAGTTCTTTGCTGACAAGTCTTAATCAACTAGGCGAGAGGCAACTTCTTTCAGTAGTCAAGTGGT<br>CTAAATCATTGCCAGGTTTTCGAAACTTACATATTGATGACCAGATAACTCTCATTCAGTATTCTTGGAT<br>GAGCTTAATGGTGTTTGGTCTAGGATGGAGATCCTACAAACACGTCAGTGGGCAGATGCTGTATTTTGCA<br>CCTGATCTAATACTAAATGAACAGCGGATGAAAGAATCATCATTCTATTCATTATGCCTTACCATGTGGC<br>AGATCCCACAGGAGTTTGTCAAGCTTCAAGTTAGCCAAGAAGAGTTCCTCTGTATGAAAGTATTGTTACT<br>TCTTAATACAATTCCTTTGGAAGGGCTACGAAGTCAAACCCAGTTTGAGGAGATGAGGTCAAGCTACATT<br>AGAGAGCTCATCAAGGCAATTGGTTTGAGGCAAAAAGGAGTTGTGTCGAGCTCACAGCGTTTCTATCAAC<br>TTACAAAACTTCTTGATAACTTGCATGATCTTGTCAAACAACTTCATCTGTACTGCTTGAATACATTTAT<br>CCAGTCCCGGGCACTGAGTGTTGAATTTCCAGAAATGATGTCTGAAGTTATTGCTGCACAATTACCCAAG | 131 |

(continued)

| GENBANK ACCESSION NUMBER | SEQUENCE | SEQ ID NO: |
|---|---|---|
|  | ATATTGGCAGGGATGGTGAAACCCCTTCTCTTTCATAAAAAGTGAATGTCATCTTTTTCTTTTAAAGAAT<br>TAAATTTTGTGGTATGTCTTTTTGTTTTGGTCAGGATTATGAGGTCTTGAGTTTTTATAATGTTCTTCTG<br>AAAGCCTTACATTTATAACATCATAGTGTGTAAATTTAAAAGAAAAATTGTGAGGTTCTAATTATTTTCT<br>TTTATAAAGTATAATTAGAATGTTTAACTGTTTTGTTTACCCATATTTTCTTGAAGAATTTACAAGATTG<br>AAAAAGTACTAAAATTGTTAAAGTAAACTATCTTATCCATATTATTTCATACCATGTAGGTGAGGATTTT<br>TAACTTTTGCATCTAACAAATCATCGACTTAAGAGAAAAAATCTTACATGTAATAACACAAAGCTATTAT<br>ATGTTATTTCTAGGTAACTCCCTTTGTGTCAATTATATTTCCAAAAATGAACCTTTAAAATGGTATGCAA<br>AATTTTGTCTATATATATTTGTGTGAGGAGGAAATTCATAACTTTCCTCAGATTTTCAAAAGTATTTTTA<br>ATGCAAAAAATGTAGAAAGAGTTTAAAACCACTAAAATAGATTGATGTTCTTCAAACTAGGCAAAACAAC<br>TCATATGTTAAGACCATTTTCCAGATTGGAAACACAAATCTCTTAGGAAGTTAATAAGTAGATTCATATC<br>ATTATGCAAATAGTATTGTGGGTTTTGTAGGTTTTTAAAATAACCTTTTTTGGGGAGAGAATTGTCCTCT<br>AATGAGGTATTGCGAGTGGACATAAGAAATCAGAAGATTATGGCCTAACTGTACTCCTTACCAACTGTGG<br>CATGCTGAAAGTTAGTCACTCTTACTGATTCTCAATTCTCTCACCTTTGAAAGTAGTAAAATATCTTTCC<br>TGCCAATTGCTCCTTTGGGTCAGAGCTTATTAACATCTTTTCAAATCAAAGGAAAGAAGAAAGGGAGAGG<br>AGGAGGAGGGAGGTATCAATTCACATACCTTTCTCCTCTTTATCCTCCACTATCATGAATTCATATTATG<br>TTTCAGCCATGCAAATCTTTTTACCATGAAATTTCTTCCAGAATTTTCCCCCTTTGACACAAATTCCATG<br>CATGTTTCAACCTTCGAGACTCAGCCAAATGTCATTCTGTAAAATCTTCCCTGAGTCTTCCAAGCAGTA<br>ATTTGCCTTCTCCTAGAGTTTACCTGCCATTTTGTGCACATTTGAGTTACAGTAGCATGTTATTTTACAA<br>TTGTGACTCTCCTGGGAGTCTGGGAGCCATATAAAGTGGTCAATAGTGTTTGCTGACTGAGAGTTGAATG<br>ACATTTTCTCTCTGTCTTGGTATTACTGTAGATTTCGATCATTCTTTGGTTACATTTCTGCATATTTCTG<br>TACCCATGACTTTATCACTTTCTTCTCCCATGCTTTATCTCCATCAATTATCTTCATTACTTTTAAATTT<br>TCCACCTTTGCTTCCTACTTTGTGAGATCTCTCCCTTTACTGACTATAACATAGAAGAATAGAAGTGTAT<br>TTTATGTGTCTTAAGGACAATACTTTAGATTCCTTGTTCTAAGTTTTTAAACTGAATGAATGGAATATTA<br>TTTCTCTCCCTAAGCAAAATTCCACAAAACAATTATTTCTTATGTTTATGTAGCCTTAAATTGTTTTGTA<br>CTGTAAACCTCAGCATAAAAACTTTCTTCATTTCTAATTTCATTCAACAAATATTGATTGAATACCTGGT<br>ATTAGCACAAGAAAAATGTGCTAATAAGCCTTATGAGAATTTGGAGCTGAAGAAAGACATATAACTCAGG<br>AAAGTTACAGTCCAGTAGTAGGTATAAATTACAGTGCCTGATAAATAGGCATTTTAATATTTGTACACTC<br>AACGTATACTAGGTAGGTGCAAAACATTTACATATAATTTTACTGATACCCATGCAGCACAAAGGTACTA<br>ACTTTAAATATTAAATAACACCTTTATGTGTCAGTAATTCATTTGCATTAAATCTTATTGAAAAGGCTTT<br>CAATATATTTTCCCCACAAATGTCATCCCAAGAAAAAAGTATTTTTAACATCTCCCAAATATAATAGTTA<br>CAGGAAATCTACCTCTGTGAGAGTGACACCTCTCAGAATGAACTGTGTGACACAAGAAAATGAATGTAGG<br>TCTATCCAAAAAAAACCCCAAGAAACAAAAACAAATTATTAGCCCTTTATGCTTAAGTGATGGACTCAG<br>GGAACAGTTGATGTTGTGATCATTTTATTATCTGATTCTTGTTACTTTGAATTAAACCAATATTTTGATG<br>ATATAAATCATTTCCACCAGCATATATTTAATTTCCATAATAACTTTAAAATTTTCTAATTTCACTCAAC<br>TATGAGGGAATAGAATGTGGTGGCCACAGGTTTGGCTTTTGTTAAAATGTTTGATATCTTCGATGTTGAT<br>CTCTGTCTGCAATGTAGATGTCTAAACACTAGGATTTAATATTTAAGGCTAAGCTTTAAAAATAAAGTAC<br>CTTTTTAAAAAGAATATGGCTTCACCAAATGGAAAATACCTAATTTCTAAATCTTTTTCTCTACAAAGTC<br>CTATCTACTAATGTCTCCATTACTATTTAGTCATCATAACCATTATCTTCATTTTACATGTCGTGTTCTT<br>TCTGGTAGCTCTAAAATGACACTAAATCATAAGAAGACAGGTTACATATCAGGAAATACTTGAAGGTTAC<br>TGAAATAGATTCTTGAGTTAATGAAAATATTTTCTGTAAAAAGGTTTGAAAAGCCATTTGAGTCTAAAGC<br>ATTATACCTCCATTATCAGTAGTTATGTGACAATTGTGTGTGTGTTTAATGTTTAAAGATGTGGCACTTT<br>TTAATAAGGCAATGCTATGCTATTTTTTCCCATTTAACATTAAGATAATTTATTGCTATACAGATGATAT<br>GGAAATATGATGAACAATATTTTTTTTGCCAAAACTATGCCTTGTAAGTAGCCATGGAATGTCAACCTGT<br>AACTTAAATTATCCACAGATAGTCATGTGTTTGATGATGGGCACTGTGGAGATAAGTTGACATAGGACTGT<br>GCCCCCCTTCTCTGCCACTTACTAGCTGGATGAGATTAAGCAAGTCATTTAACTGCTCTGATTAAACCTG<br>CCTTTCCCAAGTGCTTTGTAATGAATAGAAATGGAAACCAAAAAAAACGTATACAGGCCTTCAGAAATAG<br>TAATTGCTACTATTTTGTTTTCATTAAGCCATAGTTCTGGCTATAATTTTATCAAACTCACCAGCTATAT<br>TCTACAGTGAAAGCAGGATTCTAGAAAGTCTCACTGTTTTATTTATGTCACCATGTGCTATGATATATTT<br>GGTTGAATTCATTTGAAATTAGGGCTGGAAGTATTCAAGTAATTTCTTCTGCTGAAAAAAATACAGTGTTT<br>TGAGTTTAGGGCCTGTTTTATCAAAGTTCTAAAGAGCCTATCACTCTTCCATTGTAGACATTTTAAAATA<br>ATGACACTGATTTTAACATTTTTAAGTGTCTTTTTAGAACAGAGAGCCTGACTAGAACACAGCCCCTCCA<br>AAAACCCATGCTCAAATTATTTTTACTATGGCAGCAATTCCACAAAAGGGAACAATGGGTTTAGAAATTA<br>CAATGAAGTCATCAACCCAAAAAACATCCCTATCCCTAAGAAGGTTATGATATAAAATGCCCACAAGAAA<br>TCTATGTCTGCTTTAATCTGTCTTTTATTGCTTTGGAAGGATGGCTATTACATTTTTAGTTTTTGCTGTG<br>AATACCTGAGCAGTTTCTCTCATCCATACTTATCCTTCACACATCAGAAGTCAGGATAGAATATGAATCA<br>TTTAAAAACTTTTACAACTCCAGAGCCATGTGCATAAGAAGCATTCAAAACTTGCCAAAACATACATTT<br>TTTTTCAAATTTAAAGATACTCTATTTTTGTATTCAATAGCTCAACAACTGTGGTCCCCACTGATAAAGT<br>GAAGTGGACAAGGAGACAAGTAATGGCATAAGTTTGTTTTTCCCAAAGTATGCCTGTTCAATAGCCATTG<br>GATGTGGGAAATTCTACATCTCTTAAAAATTTTACAGAAAATACATAGCCAGATAGTCTAGCAAAAGTTC<br>ACCAAGTCCTAAATTGCTTATCCTTACTTCACTAAGTCATGAAATCATTTTAATGAAAAGAACATCACCT<br>AGGTTTTGTGGTTTCTTTTTTTCTTATTCATGGCTGAGTGAAAACAACAATCTCTGTTTCTCCCTAGCAT<br>CTGTGGACTATTTAATGTACCATTATTCCACACTCTATGGTCCTTACTAAATGAAAATTGAACAAAAAG<br>CAGTAAAACAACTGACTCTTCACCCATATTATAAAAATATAATCCAAGCCAGATTAGTCAACATCCATAAG<br>ATGAATCCAAGCTGAACTGGGCCTAGATTATTGAGTTCAGGTTGGATCACATCCCTATTTATTAATAAAC<br>TTAGGAAAGAAGGCCTTACAGACCATCAGTTAGCTGGAGCTAATAGAACCTACACTTCTAAAGTTCGGCC<br>TAGAATCAATGTGGCCTTAAAAGCTGAAAAGAAGCAGGAAAGAACAGTTTTCTTCAATAATTTGTCCACC<br>CTGTCACTGGAGAAAATTTAAGAATTTGGGGGTGTTGGTAGTAAGTTAAACACAGCAGCTGTTCATGGCA<br>GAAATTATTCAATACATACCTTCTCTGAATATCCTATAACCAAAGCAAAGAAAAACACCAAGGGGTTTGT |  |

(continued)

| GENBANK ACCESSION NUMBER | SEQUENCE | SEQ ID NO: |
|---|---|---|
| | TCTCCTCCTTGGAGTTGACCTCATTCCAAGGCAGAGCTCAGGTCACAGGCACAGGGGCTGCGCCCAAGCT<br>TGTCCGCAGCCTTATGCAGCTGTGGAGTCTGGAAGACTGTTGCAGGACTGCTGGCCTAGTCCCAGAATGT<br>CAGCCTCATTTTCGATTTACTGGCTCTTGTTGCTGTATGTCATGCTGACCTTATTGTTAAACACAGGTTT<br>GTTTGCTTTTTTTCCACTCATGGAGACATGGGAGAGGCATTATTTTTTAAGCTGGTTGAAAGCTTTAACCG<br>ATAAAGCATTTTTAGAGAAATGTGAATCAGGCAGCTAAGAAAGCATACTCTGTCCATTACGGTAAAGAAA<br>ATGCACAGATTATTAACTCTGCAGTGTGGCATTAGTGTCCTGGTCAATATTCGGATAGATATGAATAAAA<br>TATTTAAATGGTATTGTAAATAGTTTTCAGGACATATGCTATAGCTTATTTTTATTATCTTTTGAAATTG<br>CTCTTAATACATCAAATCCTGATGTATTCAATTTATCAGATATAAATTATTCTAAATGAAGCCCAGTTAA<br>ATGTTTTTGTCTTGTCAGTTATATGTTAAGTTTCTGATCTCTTTGTCTATGACGTTTACTAATCTGCATT<br>TTTACTGTTATGAATTATTTTAGACAGCAGTGGTTTCAAGCTTTTTGCCACTAAAAATACCTTTTATTTT<br>CTCCTCCCCCAGAAAAGTCTATACCTTGAAGTATCTATCCACCAAACTGTACTTCTATTAAGAAATAGTT<br>ATTGTGTTTTCTTAATGTTTTGTTATTCAAAGACATATCAATGAAAGCTGCTGAGCAGCATGAATAACAA<br>TTATATCCACACAGATTTGATATATTTGTGCAGCCTTAACTTGATAGTATAAAATGTCATTGCTTTTTA<br>AATAATAGTTAGTCAATGGACTTCTATCATAGCTTTCCTAAACTAGGTTAAGATCCAGAGCTTTGGGGTC<br>ATAATATATTACATACAATTAAGTTATCTTTTTCTAAGGGCTTTAAAATTCATGAGAATAACCAAAAAAG<br>GTATGTGGAGAGTTAATACAAACATACCATATTCTTGTTGAAACAGAGATGTGGCTCTGCTTGTTCTCCA<br>TAAGGTAGAAATACTTTCCAGAATTTGCCTAAACTAGTAAGCCCTGAATTTGCTATGATTAGGGATAGGA<br>AGAGATTTTCACATGGCAGACTTTAGAATTCTTCACTTTAGCCAGTAAAGTATCTCCTTTTGATCTTAGT<br>ATTCTGTGTATTTTAACTTTTCTGAGTTGTGCATGTTTATAAGAAAAATCAGCACAAAGGGTTTAAGTTA<br>AAGCCTTTTTACTGAAATTTGAAAGAAACAGAAGAAAAATATCAAAGTTCTTTGTATTTTGAGAGGATTAA<br>ATATGATTTACAAAAGTTACATGGAGGGCTCTCTAAAACATTAAATTAATTATTTTTTGTTGAAAAGTCT<br>TACTTTAGGCATCATTTTATTCCTCAGCAACTAGCTGTGAAGCCTTTACTGTGCTGTATGCCAGTCACTC<br>TGCTAGATTGTGGAGATTACCAGTGTTCCCGTCTTCTCCGAGCTTAGAGTTGGATGGGGAATAAAGACAG<br>GTAAACAGATAGCTACAATATTGTACTGTGAATGCTTATGCTGGAGGAAGTACAGGGAACTATTGGAGCA<br>CCTAAGAGGAGCACCTACCTTGAATTTAGGGGTTAGCAGAGGCATCCTGAAAAAAGTCAAAGCTAAGCCA<br>CAATCTATAAGCAGTTTAGGAATTAGCAGAACGTGCGTGGTGAGGAGATGCCAAAGGCAAGAAGAGAAGA<br>GTATTCCAAACAGGAGGGATTCCAAAGAGAGAAGAGTATCCCAAACAACATTTGCACAAACCTGATGGGG<br>AGAGAGAATGTGGGGTGGGGATGGATGATGAGACTGAAGAAGAAAGCCAGGTCTAGATAATCAGTGGCCT<br>TGTACACCATGTTAAAGAGTGTAGACTTGATTCTGTTGTAAACAGGAAAGCAGCACAATTCATATGAATA<br>TTTTAGAAGACTCCCACTGGAATATGGAGAATAAAGTTGGAGATGACTAATCCTGGAAGCAGGGAGAACA<br>TTTTTGAGGAAGTTGCACTATTTTGGTGAAAATGATGATCATAAACATGAAGAATTGTAGGTGATCATGA<br>CCTCCTCTCTAATTTTCCAGAAGGGTTTTGGAAGATATAACATAGGAACATTGACAGGACTGACGAAAGG<br>AGATGAAATACACCATATAAATTGTCAAACACAAGGCCAGATGTCTAATTATTTTGCTTATGTGTTGAAA<br>TTACAAATTTTTCATCAGGAAACCAAAAACTACAAAACTTAGTTTTCCCAAGTCCCAGAATTCTATCTGT<br>CCAAACAATCTGTACCACTCCACCTATATCCCTACCTTTGCATGTCTGTCCAACCTCAAAGTCCAGGTCT<br>ATACACACGGGTAAGACTAGAGCAGTTCAAGTTTCAGAAAATGAGAAAGAGGAACTGAGTTGTGCTGAAC<br>CCATACAAAATAAACACATTCTTTGTATAGATTCTTGGAACCTCGAGAGGAATTCACCTAACTCATAGGT<br>ATTTGATGGTATGAATCCATGGCTGGGCTCGGCTTTTAAAAAGCCTTATCTGGGATTCCTTCTATGGAAC<br>CAAGTTCCATCAAAGCCCATTTAAAAGCCTACATTAAAAACAAAATTCTTGCTGCATTGTATACAAATAA<br>TGATGTCATGATCAAATAATCAGATGCCATTATCAAGTGGAATTACAAAATGGTATACCCACTCCAAAAA<br>AAAAAAAAAAGCTAAATTCTCAGTAGAACATTGTGACTTCATGAGCCCTCCACAGCCTTGGAGCTGAGGA<br>GGGAGCACTGGTGAGCAGTAGGTTGAAGAGAAAACTTGGCGCTTAATAATCTATCCATGTTTTTTCATCT<br>AAAAGAGCCTTCTTTTTGGATTACCTTATTCAATTTCCATCAAGGAAATTGTTAGTTCCACTAACCAGAC<br>AGCAGCTGGGAAGGCAGAAGCTTACTGTATGTACATGGTAGCTGTGGGAAGGAGGTTTCTTTCTCCAGGT<br>CCTCACTGGCCATACACCAGTCCCTTGTTAGTTATGCCTGGTCATAGACCCCCGTTGCTATCATCTCATA<br>TTTAAGTCTTTGGCTTGTGAATTTATCTATTCTTTCAGCTTCAGCACTGCAGAGTGCTGGGACTTTGCTA<br>ACTTCCATTTCTTGCTGGCTTAGCACATTCCTCATAGGCCCAGCTCTTTTCTCATCTGGCCCTGCTGTGG<br>AGTCACCTTGCCCCTTCAGGAGAGCCATGGCTTACCACTGCCTGCTAAGCCTCCACTCAGCTGCCACCAC<br>ACTAAATCCAAGCTTCTCTAAGATGTTGCAGACTTTACAGGCAAGCATAAAAGGCTTGATCTTCCTGGAC<br>TTCCCTTTACTTGTCTGAATCTCACCTCCTTCAACTTTCAGTCTCAGAATGTAGGCATTTGTCCTCTTTG<br>CCCTACATCTTCCTTCTTCTGAATCATGAAAGCCTCTCACTTCCTCTTGCTATGTGCTGGAGGCTTCTGT<br>CAGGTTTTAGAATGAGTTCTCATCTAGTCCTAGTAGCTTTTGATGCTTAAGTCCACCTTTTAAGGATACC<br>TTTGAGATTTAGACCATGTTTTTCGCTTGAGAAAGCCCTAATCTCCAGACTTGCCTTTCTGTGGATTTCA<br>AAGACCAACTGAGGAAGTCAAAAGCTGAATGTTGACTTTCTTTGAACATTTCCGCTATAACAATTCCAAT<br>TCTCCTCAGAGCAATATGCCTGCCTCCAACTGACCAGGAGAAAGGTCCAGTGCCAAAGAGAAAAACACAA<br>AGATTAATTATTTCAGTTGAGCACATACTTTCAAAGTGGTTTGGGTATTCATATGAGGTTTTCTGTCAAG<br>AGGGTGAGACTCTTCATCTATCCATGTGTGCCTGACAGTTCTCCTGGCACTGGGTGGTAACAGATGCAAA<br>ACTGTAAAAATTAAGTGATCATGTATTTTAACGATATCATCACATACTTATTTTCTATGTAATGTTTTAA<br>ATTTCCCCTAACATACTTTGACTGTTTTGCACATGGTAGATATTCACATTTTTTTGTGTTGAAGTTGATG<br>CAATCTTCAAAGTTATCTACCCCGTTGCTTATTAGTAAAACTAGTGTTAATACTTGGCAAGAGATGCAGG<br>GAATCTTTCTCATGACTCACGCCCTATTTAGTTATTAATGCTACTACCCTATTTTGAGTAAGTAGTAGGT<br>CCCTAAGTACATTGTCCAGAGTTATACTTTAAAGATATTTAGCCCCATATACTTCTTGAATCTAAAGTC<br>ATACACCTTGCTCCTCATTTCTGAGTGGGAAAGACATTTGAGAGTATGTTGACAATTGTTCTGAAGGTTT<br>TTGCCAAGAAGGTGAAACTGTCCTTTCATCTGTGTATGCCTGGGGCTGGGTCCCTGGCAGTGATGGGGTG<br>ACAATGCAAAGCTGTAAAAACTAGGTGCTAGTGGGCACCTAATATCATCATCATATACTTATTTTCAAGC<br>TAATATGCAAAATCCCATCTCTGTTTTTAAACTAAGTGTAGATTTCAGAGAAAATATTTTGTGGTTCACA<br>TAAGAAAACAGTCTACTCAGCTTGACAAGTGTTTTATGTTAAATTGGCTGGTGGTTTGAAATGAATCATC<br>TTCACATAATGTTTTCTTTAAAAATATTGTGAATTTAACTCTAATTCTTGTTATTCTGTGTGATAATAAA<br>GAATAAACTAATTTCTA | |

(continued)

| GENBANK ACCESSION NUMBER | SEQUENCE | SEQ ID NO: |
|---|---|---|
| AK093306 | ATTCTATGCTGCAGCCTAAGCATCATTCCTCTTCTCTTCTTAGTGGAGATAAAATTACCCACTGCTCTCC<br>TTACATTTACTTTGTCCATATTTGCTCCTATGCTCTAGGCTCGTGCACAACAAACACAGTGTGGGCCCTT<br>ACCCTAGAAGCCAACTTCTCATGACCTTTCTCTATCTCCAGAATCCATGCAGTGGGAATGAAGGTAAAAG<br>AAGGTTTTCATGGGATCCAGCTGAGAGCTCTACGGGGAAAATGGATCTGAGGAGCCATGTGCTCCATCTC<br>TTTTATTTTACAGGTAGAGACTAGGGGTATAGAGTGAGGTGAATTACCGCAGTGACCCACACATTGTTGG<br>CAGACCTAGGATTAGAACTCTGTCTTCCTGGTTCCCAGCTTGGTGCTTTTGAAAGCATACTTGCTGCTTT<br>CTTACCGGCCTGGTGTCTGCCACTTTGGGACAGAGTGTGGACTTGCTCACCTGCCCCATTTCTTAGGGAT<br>TCTCATTCTGTGTTTGAGCAAGAATATTCTTATTCTGGAAAGAACCACATACCACAGGATTCTGGGTGAG<br>CATAAGGAAGATTGTCTTGGGGATCTGACTTAGCTCACGTATAGTGGCTATGATGAATTCAGTGTCTTAT<br>TTTTTGCATATGTATATTTTTAGTCTAATATTGCCTGGGTGTCTGAGCAAGTCTAGATGAATTTAATTGC<br>TCTCATTTTTCCCCTGCCCCTCTTCCTTTGGTCTCTCTTTTAGGAAATGTTTTTCTTTCAACATTCGTTT<br>CATTCATTATTTACTCATTCGGCCAACCAACATTATTGAGTGCCTTCCCTGTATCAGGGACAGGGGCTT<br>ACAAAGTAGAATTTGATCCCACCTCTGCCCTCAGTAGCTCAGTGTCTAATGGAGGTAGTGATGTTCATTA<br>AGCGTCGCCAGATACTGTGCTAGGTGCTGTGCCTGTTCTCTCTCGCTTGTTCCTCACACACTTGAGAAGG<br>CCGAAGCTGATTCATAGCTTGGAAGGCAGGGGCCTTGGATTTGAACCCAGGCCTGACCAATGGCAGAACC<br>TATCAGATGTGTGGACAGATGACATTGCCTTTCTTTCTTTGGATATATCAAAATCAGCCAGCAGGCAGGA<br>ACTCCCATTTTGAGCAAGCAATGTGCAGGAATGATAGGGTATACAGAGAGGAACAGGAGATGGCCCCTGA<br>CTTCCAGCATGTGTCTGATGGACATCCAGGCTGCAGGCATCATGGTGCTGTCTAGAGAGATGAGCCAGGT<br>GCCCAGAGCCCATGGGCCAATGCTGCCCTTTCTTGAGCATGCCAAACAAAGCGGTTGGTGTGTTAGAGGC<br>ACAGTCTCCTCCACTCTAAGTAAAAATCAGCATGAGTCCTAGCCCACATTTCCCTAGTGAGTACACCAAA<br>GATATCTATGAACTGGCAGTCATCAGTGACTTCCTAAGGTTCCGGAAATGCATCTCTTACTCAGGAGTAA<br>GCAATGATGTGCCTGCGGCTTTACGAGTTCTCACAGAATGACTTTCTGGACCCAAATGTTTTTTCTGCTT<br>CAGGACTGTGAAGGCCTTATTGTTCGCTCTGCCACCAAGGTGACCGCTGATGTCATCAACGCAGCTGAGA<br>AACTCCAGGTGGTGGGCAGGGCTGGCACAGGTGTGGACAATGTGGATCTGGAGGCCGCAACAAGGAAGGG<br>CATCTTGGTTATGAACACCCCCAATGGGAACAGCCTCAGTGCCGCAGAACTCACTTGTGGAATGATCATG<br>TGCCTGGCCAGGCAGATTCCCCAGGCGACGGCTTCGATGAAGGACGGCAAATGGGAGCGGGAAGAAGTTCA<br>TGGGAACAGAGCTGAATGGAAAGACCCTGGGAATTCTTGGCCTGGGCAGGATTGGGAGAGAGGTAGCTAC<br>CCGGATGCAGTCCTTTGGGATGAAGACTATAGGGTATGACCCCATCATTTCCCCAGAGGTCTCGGCCTCC<br>TTTGGTGTTCAGCAGCTGCCCCTGGAGGAGATCTGGCCTCTCTGTGATTTCATCACTGTGCACACTCCTC<br>TCCTGCCCTCCACGACAGGCTTGCTGAATGACAACACCTTTGCCCAGTGCAAGAAGGGGGTGCGTGTGGT<br>GAACTGTGCCCGTGGGAGGGATCGTGGACGAAGGCGCCCTGCTCCGGGCCCTGCAGTCTGGCCAGTGTGCC<br>GGGGCTGCACTGGACGTGTTTACGGAAGAGCCGCCACGGGACCGGGCCTTGGTGGACCATGAGAATGTCA<br>TCAGCTGTCCCCACCTGGGTGCCAGCACCAAGGAGGCTCAGAGCCGCTGTGGGGAGGGAAATTGCTGTTCA<br>GTTCGTGGACATGGTGAAGGGGAAATCTCTCACGGGGGTTGTGAATGCCCAGGCCCTTACCAGTGCCTTC<br>TCTCCACACACCAAGCCTTGGATTGGTCTGGCAGAAGTCTGGGGACACTGATGCGAGCCTGGGCTGGGT<br>CCCCCAAAGGGACCATCCAGGTGATAACACAGGGAACATCCCTGAAGAATGCTGGGAACTGCCTAAGCCC<br>CGCAGTCATTGTCGGCCTCCTGAAAGAGGCTTCCAAGCAGGCGGATGTGAACTTGGTGAACGCTAAGCTG<br>CTGGTGAAAGAGGCTGGCCTCAATGTCACCACCTCCCACAGCCCTGCTGCACCAGGGGGGCAAGGCTTCG<br>GGGAATGCCTCCTGGCCGTGGCCCTGGCAGGCGCCCCTTACCAGGCTGTGGGCTTGGTCCAAGGCACTAC<br>ACCTGTACTGCAGGGGCTCAATGGACGTGTCTTCAGGCCAGAAGTGCCTCTTCCGCAGGGACCTGCCCCTG<br>CTCCTATTCCGGGACTCAGACCTCTGACCCTGCAATGCTGCCTACCATGATTGGCCTCCTGGCAGAGGCAG<br>GCGTGCGGCTGCTGTCCTACCAGACTTCACTGGTGTCAGATGGGGAGACCTGGCACGTCATGGGCATCTC<br>CTCCTTGCTGCCCAGCCTGGAAGCGTGGAAGCAGCATGTGACTGAAGCCTTCCAGTTCCACTTCTAACCT<br>TGGAGCTCACTGGTCCCTGCCTCTGGGGCTTTTCTGAAGAAACCCACCCACTGTGATCAATAGGGAGAGA<br>AAATCCACATTCTTGGGCTGAACGCGAGCCTCTGACACTGCTTACACTGCACTCTGACCCTGTAGTACAG<br>CAATAACCGTCTAATAAAGAGCCTACCCCC | 132 |
| BE904476 | CAAAACAAAAACAGCCAAGCTTTTCTGCCAAAAAGATGACTGAGAAGACTGTTAAAGCAAAAAGCTCTGTT<br>CCTGCCTCAGATGATGCCTATCCAGAAATAGAAAAAATTCTTTCCCTTCAATCCTCTAGACTTTGAGAGTT<br>TTGACCTGCCTGAAGAGCACCAGATTGCGCACCTCCCCTTGAGTGGAGTGCCTCTCATGATCCTTGACGA<br>GGAGAGAGAGCTTGAAAAGCTGTTTCAGCTGGGCCCCCCTTCACCTGTGAAGATGCCCTCTCCACCATGG<br>GAATCCAATCTGTTGCAGTCTCCTTCAAGCATTCTGTCGACCCTGGATGTTGAATTGCCACCTGTTTGCT<br>GTGACATAGATATTTAAATTTCTTAGTGCTTCAGAGTCTGTGTGTATTTGTATTAATAAAGCATTCTTTA<br>ACAGAAAAAAAAAAAAAAAAAAAAAAAAAAAAAAAAAAAAAAAAAAGGGGGGGGGAGACACAAAAA<br>GAATTCCCCAAGAGGGGGCCACAAGATAATCAGAGGATATCACACAAGATCTCTCGGCGCACCAACGACG<br>GGGGCCCCAAATAAGGGAGAGACCCAGAATCACAACAGCCAAGACACGGTGGACACGACGGAAACAAACA<br>CACAGCCCAGACACGGGGGCAAACACGCGCGCACACCGCGGACACCATGGGACAAAGCAGACACCACCCA<br>CAAAACAACACCGCGGAGGGGGAAGAACAACAAAACAAGTGCGCAAACAGAACACAACCACAGAAAGAGA<br>AAAATTAAAACGGCCCCCAAGACGGCGACAACACAACAAAACAACCACTACAGAGCGCTCAACAGCCGAG<br>TAAAAACACAACAGGACAACTAACACACAAAGGAATGAAAACGGGGCACACACCGACACCACCGGA<br>AATCCGGCGAACAACTCACACCGAGCGAGGGTCCCAGACAACAAATCACAGACAACGAAACCGAGAAAC<br>AAGACCAGCAAGACGAGCAGGCAAAAGACAAACAAGACAGAGGAGACGACGACGAACGCAAAGGACAAGA<br>GGACACAACGACGCGAGGAGCGAGAGCGAGAGGAAGAGACAACAAAAAGACACAAAAGAACAACAAGCAA<br>GCAGCGAAGAACGACACACAACCACACGAGACAGCAGGAGCAGAGGCGGAGAAAACACAACGAGCAAGCC<br>AAGACCAAGAGAGGAGAACAAAATAAAAAAATACGAGAGCAGGCGGACGAGAGCACGAGACGAACAGACA<br>AACGGGAATCAGAAGCATAACGATCCGCGACGCGAACAACN | 133 |
| AK123010 | GTGCACCCTGTCCCAGCCGTCCTGTCCTGGCTGCTCGCTCTGCTTCGCTGCGCCTCCACTATGCTCTCCC<br>TCCGTGTCCCGCTCGCGCCCATCACGGACCCGCAGCAGCTGCAGCTCTCGCCGCTGAAGGGGCTCAGCTT | 134 |

(continued)

| GENBANK ACCESSION NUMBER | SEQUENCE | SEQ ID NO: |
|---|---|---|
| | GGTCGACAAGGAGAACACGCCGCCGGCCCTGAGCGGGACCCGCGTCCTGGCCAGCAAGACCGCGAGGAGG<br>ATCTTCCAGGAGAAAACCCCCGCCGCTTTGTCATCTTCCCCATCGAGTACCATGATATCTGGCAGATGTA<br>TAAGAAGGCAGAGGCTTCCTTTTGGACCGCCGAGGAGGTGGACCTCTCCAAGGACATTCAGCACTGGGAA<br>TCCCTGAAACCCGAGGAGAGATATTTTATATCCCATGTTCTGGCTTTCTTTGCAGCAAGCGATGGCATAG<br>TAAATGAAAACTTGGTGGAGCGATTTAGCCAAGAAGTTCAGATTACAGAAGCCCGCTGTTTCTATGGCTT<br>CCAAATTGCCATGGAAAACATACATTCTGAAATGTATAGTCTTCTTATTGACACTTACATAAAAGATCCC<br>AAAGAAAGGGAATTTCTCTTCAATGCCATTGAAACGATGCCTTGTGTCAAGAAGAAGGCAGACTGGGCCT<br>TGCGCTGGATTGGGGACAAAGAGGCTACCTATGGTGAACGTGTTGTAGCCTTTGCTGCAGTGGAAGGCAT<br>TTTCTTTTCCGGTTCTTTTGCGTCGATATTCTGGCTCAAGAAACGAGGACTGATGCCTGGCCTCACATTT<br>TCTAATGAACTTATTAGCAGAGATGAGGGTTTACACTGTGATTTTGCTTGCCTGATGTTCAAACACCTGG<br>TACACAAACCATCGGAGGAGAGAGTAAGAGAAATAATTATCAATGCTGTTCGGATAGAACAGGAGTTCCT<br>CACTGAGGCCTTGCCTGTGAAGCTCATTGGGATGAATTGCACTCTAATGAAGCAATACATTGAGTTTGTG<br>GCAGACAGACTTATGCTGGAACTGGGTTTTAGCAAGGTTTTCAGAGTAGAGAACCCATTTGACTTTATGG<br>AGAATATTTCACTGGAAGGAAAGACTAACTTCTTTGAGAAGAGAGTAGGCGAGTATCAGAGGATGGGAGT<br>GATGTCAAGTCCAACAGAGAATTCTTTTACCTTGGATGCTGACTTCTAAATGAACTGAAGATGTGCCCTT<br>ACTTGGCTGATTTTTTTTTTTCCATCTCATAAGAAAAATCAGCTGAAGTGTTACCAACTAGCCACACCAT<br>GAATTGTCCGTAATGTTCATTAACAGCATCTTTAAAACTGTGTAGCTACCTCACAACCAGTCCTGTCTGT<br>TTATAGTGCTGGTAGTATCACCTTTTGCCAGAAGGCCTGGCTGGCTGTGACTTACCATAGCAGTGACAAT<br>GGCAGTCTTGGCTTTAAAGTGAGGGGTGACCCTTTAGTGAGCTTAGCACAGCGGGATTAAACAGTCCTTT<br>AACCAGCACAGCCAGTTAAAAGATGCAGCCTCACTGCTTCAACGCAGATTTTAATGTTTACTTAAATATA<br>AACCTGGCACTTTACAAACAAATAAACATTGTTTGTACTCACAAGGCGATAATAGCTTGATTTATTTGGT<br>TTCTACACCAAATACATTCTCCTGACCACTAATGGGAGCCAATTCACAATTCACTAAGTGACTAAAGTAA<br>GTTAAACTTGTGTAGACTAAGCATGTAATTTTTAAGTTTTATTTTAATGAATTAAAATATTTGTTAACCA<br>ACTTTAAAGTCAGTCCTGTGTATACCTAGATATTAGTCAGTTGGTGCCAGATAGAAGACAGGTTGTGTTT<br>TTATCCTGTGGCTTGTGTAGTGTCCTGGGATTCTCTGCCCCCTCTGAGTAGAGTGTTGTGGGATAAAGGA<br>ATCTCTCAGGGCAAGGAGCTTCTTAAGTTAAATCACTAGAAATTTAGGGGTGATCTGGGCCTTCATATGT<br>GTGAGAAGCCGTTTCATTTATTTCTCACTGTATTTTCCTCAACGTCTGGTTGATGAGAAAAAATTCTTG<br>AAGAGTTTTCATATGTGGGAGCTAAGGTAGTATTGTAAAATTTCAAGTCATCCTTAAACAAAATGATCCA<br>CCTAAGATCTTGCCCCTGTTAAGTGGTGAAATCAACTAGAGGTGGTTCCTACAAGTTGTTCATTCTAGTT<br>TTGTTTGGTGTAAGTAGGTTGTGTGAGTTAATTCATTTATATTTACTATGTCTGTTAAATCAGAAATTTT<br>TTATTATCTATGTTCTTCTTAGATTTTACCTGTAGTTCATACTTCAGTCACCCAGTGTCTTATTCTGGCAT<br>TGTCTAAATCTGAGCATTGTCTAGGGGGATCTTAAACTTTAGTAGGAAACCATGAGCTGTTAATACAGTT<br>TCCATTCAAATATTAATTTCAGAATGAAACATAATTTTTTTTTTTTTTTTGAGATGGAGTCTCGCTCT<br>GTTGCCCAGGCTGGAGTGCAGTGGCGCGATTTTGGCTCACTGTAACCTCCATCTCCTGGGTTCAAGCAAT<br>TCTCCTGTCTCAGCCTCCCTAGTAGCTGGGACTGCAGGTATGTGCTACCACACCTGGCTAATTTTTGTAT<br>TTTTAGTAGAGATGGAGTTTCACCATATTGGTCAGGCTGGTCTTGAACTCCTGACCTCAGGTGATCCACC<br>CACCTCGGCCTCCCAAAGTGCTGGGATTGCAGGCGTGATAAACAAATATTCTTAATAGGGCTACTTTGAA<br>TTAATCTGCCTTTATGTTTGGGGAGAAGAAAGCTGAGACATTGCATGAAAGATGATGAGAGATAAAATGTTG<br>ATCTTTTGGCCCCATTTGTTAATTGTATTCAGTATTTGAACGTCGTCCTGTTTATTGTTAGTTTTCTTCA<br>TCATTTATTGTATAGACAATTTTTAAAATCTCTGTAATATGATACATTTTCCTATCTTTTAAGTTATTGTT<br>ACCTAAAGTTAATCCAGATTATATGGTCCTTATATGTGTACAACATTAAAATGAAAGGCTTTGTCTTGCA<br>TTGTGAGGTACAGGCGGAAGTTGGAATCAGGTTTTAGGATTCTGTCTCTCATTAGCTGAATAATGTGAGG<br>ATTAACTTCTGCCAGCTCAGACCATTTCCTAATCAGTTGAAAGGGAAACAAGTATTTCAGTCTCAAAATT<br>GAATAATGCACAAGTCTTAAGTGATTAAAATAAAACTGTTCTTATGTCAGTTT | |
| BC036503 | AGCGGGGGCACTCCAGCCCTGCAGCCTCCGGAGTCAGTGCCGCGCGCCCGCCGCCCCGCGCCTTCCTGCT<br>CGCCGCACCTCCGGGAGCCGGGGCGCACCCAGCCCGCAGCGCCGCCTCCCCGCCCGCGCCGCCTCCGACC<br>GCAGGCCGAGGGCCGCCACTGGCCGGGGGGACCGGGCAGCAGCTTGCGGCCGCGGAGCCGGGCAACGCTG<br>GGGACTGCGCCTTTTGTCCCCGGAGGTCCCTGGAAGTTTGCGGCAGGACGCGCGCGGGGAGGCGGCGGAG<br>GCAGCCCCGACGTCGCGGAGAACAGGGCGCAGAGCCGGCATGGGCATCGGGCGCAGCGAGGGGGGCCGCC<br>GCGGGGCAGCCTGGGCGTGCTGCTGCCGGCGCGGCGCTTCTGGCCGTGGCCTCGGCCGAGCGAGTA<br>CGACTACGTGAGCTTCCAGTCGGACATCGGCCCGTACCAGAGCGGGCGCTTCTACACCAAGCCACCTCAG<br>TGCGTGGACATCCCCGCGGACCTGCGGCTGTGCCACAACGTGGGCTACAAGAAGATGGTGCTGCCCAACC<br>TGCTGGAGCACGAGACCATGGCGGAGGTGAAGCAGCAGGCCAGCAGCTGGGTGCCCCTGCTCAACAAGAA<br>CTGCCACGCCGGCACCCAGGTCTTCCTCTGCTCGCTCTTCGCGCCCGTCTGCCTGGACCGGCCCATCTAC<br>CCGTGTCGCTGGCTCTGCGAGGCCGTGCGCGACTCGTGCGAGCCGGTCATGCAGTTCTTCGGCTTCTACT<br>GGCCCGAGATGCTTAAGTGTGACAAGTTCCCCGAGGGGGACGTCTGCATCGCCATGACGCCGCCCAATGC<br>CACCGAAGCCTCCAAGCCCCAAGGCACAACGGTGTGTCCTCCCTGTGACAACGAGTTGAAATCTGAGGCC<br>ATCATTGAACATCTCTGTGCCAGCGAGTTTGCACTGAGGATGAAAATAAAAGAAGTGAAAAAAGAAAATG<br>GCGACAAGAAGATTGTCCCCAAGAAGAAGAAGCCCCTGAAGTTGGGGCCCATCAAGAAGAAGGACCTGAA<br>GAAGCTTGTGCTGTACCTGAAGAATGGGGCTGACTGTCCCTGCCACCAGCTGGACAACCTCAGCCACCAC<br>TTCCTCATCATGGGCCGCAAGGTGAAGAGCCAGTACTTGCTGACGGCCATCCACAAGTGGGACAAGAAA<br>ACAAGGAGTTCAAAAACTTCATGAAGAAAATGAAAAACCATGAGTGCCCCACCTTTCAGTCCGTGTTTAA<br>GTGATTCTCCCGGGGGCAGGGTGGGGAGGGAGCCTCGGGTGGGGTGGGAGCGGGGGGGGACAGTGCCCCGG<br>GAACCCGGTGGGTCACACACACGCACTGCGCCTGTCAGTAGTGGACATTTAATCCAGTCGGCTTGTTCTT<br>GCAGCATTCCCGCTCCCTTCCCTCCCATAGCCACGCTCCAAACCCCAGGGTAGCCATGGCCGGGTAAAGCA<br>AGGGCCATTTAGATTAGGGAAGGTTTTTAAGATCCGCAATGTGGAGCAGCAGCCACTGCACAGGAGGAGGT<br>GACAAACCATTTCCAACAGCAACACAGCCACTAAAACACAAAAAGGGGGATTGGGCGGAAAGTGAGAGCC<br>AGCAGCAAAAACTACATTTTGCAACTTGTTGGTGTGGATCTATTGGCTGATCTATGCCTTTCAACTAGAA | 135 |

| GENBANK ACCESSION NUMBER | SEQUENCE | SEQ ID NO: |
|---|---|---|
| | AATTCTAATGATTGGCAAGTCACGTTGTTTTCAGGTCCAGAGTAGTTTCTTTCTGTCTGCTTTAAATGGA<br>AACAGACTCATACCACACTTACAATTAAGGTCAAGCCCAGAAAGTGATAAGTGCAGGGAGGAAAAGTGCA<br>AGTCCATTATGTAATAGTGACAGCAAAGGGACCAGGGGAGAGGCATTGCCTTCTCTGCCCACAGTCTTTC<br>CGTGTGATTGTCTTTGAATCTGAATCAGCCAGTCTCAGATGCCCCAAAGTTTCGGTTCCTATGAGCCCGG<br>GGCATGATCTGATCCCCAAGACATGTGGAGGGGCAGCCTGTGCCTGCCTTTGTGTCAGAAAAAGGAAACC<br>ACAGTGAGCCTGAGAGAGACGGCGATTTTCGGGCTGAGAAGGCAGTAGTTTTCAAAACACATAGTTAAAA<br>AAGAAACAAATGAAAAAAATTTTAGAACAGTCCAGCAAATTGCTAGTCAGGGTGAATTGTGAAATTGGGT<br>GAAGAGCTTACGATTCTAATCTCATGTTTTTTCCTTTTCACATTTTTAAAAGAACAATGACAAACACCCA<br>CTTATTTTTCAAGGTTTTAAAACAGTCTACATTGAGCATTTGAAAGGTGTGCTAGAACAAGGTCTCCTGA<br>TCCGTCCGAGGCTGCTTCCCAGAGGAGCAGCTCTCCCCAGGCATTTGCCAAGGGAGGCGGGATTTCCCTGG<br>TAGTGTAGCTGTGTGGCTTTCCTTCCTGAAGAGTCCGTGGTTGCCCTAGAACCTAACACCCCCTAGCAAA<br>ACTCACAGAGCTTTCCGTTTTTTTCTTTCCTGTAAAGAAACATTTCCTTTGAACTTGATTGCCTATGGAT<br>CAAAGAAATTCAGAACAGCCTGCCTGTCCCCCCGCACTTTTTACATATATTTGTTTCATTTCTGCAGATG<br>GAAAGTTGACATGGGTGGGGTGTCCCCATCCAGCGAGAGAGTTTAAAAAGCAAAACATCTCTGCAGTTTT<br>TCCCAAGTGCCCTGAGATACTTCCCAAAGCCCTTATGTTTAATCAGCGATGTATATAAGCCAGTTCACTT<br>AGACAACTTTACCCTTCTTGTCCAATGTACAGGAAGTAGTTCTAAAAAAAATGCATATTAATTTCTTCCC<br>CCAAAGCCGGATTCTTAATTCTCTGCAACACTTTGAGGACATTTATGATTGTCCCTCTGGGCCAATGCTT<br>ATCCCAGTGAGGATGCTGCAGTGAGGCTGTAAAGTGGCCCCCTGCGGCCCTAGCCTGACCCGGAGGAAA<br>GGATGGTAGATTCTGTTAACTCTTGAAGACTCCAGTATGAAAATCAGCATGCCCGCCTAGTTACCTACCG<br>GAGAGTTATCCTGATAAATTAACCTCTCACAGTTAGTGATCCTGTCCTTTTAACACCTTTTTTGTGGGGT<br>TCTCTCTGACCTTTCATCGTAAAGTGCTGGGGACCTTAAGTGATTTGCCTGTAATTTTGGATGATTAAAA<br>AATGTGTATATATATTAGCTAATTAGAAATATTCTACTTCTCTGTTGTCAAACTGAAATTCAGAGCAAGT<br>TCCTGAGTGCGTGGATCTGGGTCTTAGTTCTGGTTGATTCACTCAAGAGTTCAGTGCTCATACGTATCTG<br>CTCATTTTGACAAAGTGCCTCATGCAACCGGGCCCTCTCTCTGCGGCAGAGTCCTTAGTGGAGGGGTTTA<br>CCTGGAACATTAGTAGTTACCACAGAATACGGAAGAGCAGGTGACTGTGCTGTGCAGCTCTCTAAATGGG<br>AATTCTCAGGTAGGAAGCAACAGCTTCAGAAAGAGCTCAAAATAAATTGGAAATGTGAATCGCAGCTGTG<br>GGTTTTACCACCGTCTGTCTCAGAGTCCCAGGACCTTGAGTGTCATTAGTTACTTTATTGAAGGTTTTAG<br>ACCCATAGCAGCTTTGTCTCTGTCACATCAGCAATTTCAGAACCAAAAGGGAGGCTCTCTGTAGGCACAG<br>AGCTGCACTATCACGAGCCTTTGTTTTTCTCCACAAAGTATCTAACAAAACCAATGTGCAGACTGATTGG<br>CCTGGTCATTGGTCTCCGAGAGAGGAGGTTTGCCTGTGATTTCCTAATTATCGCTAGGGCCAAGGTGGGA<br>TTTGTAAAGCTTTACAATAATCATTCTGGATAGAGTCCTGGGAGGTCCTTGGCAGAACTCAGTTAAATCT<br>TTGAAGAATATTTGTAGTTATCTTAGAAGATAGCATGGGAGGTGAGGATTCCAAAAACATTTTATTTTTA<br>AAATATCCTGTGTAACACTTGGCTCTTGGTACCTGTGGGTTAGCATCAAGTTCTCCCCAGGGTAGAATTC<br>AATCAGAGCTCCAGTTTGCATTTGGATGTGTAAATTACAGTAATCCCATTTCCCAAACCTAAAATCTGTT<br>TTTCTCATCAGACTCTGAGTAACTGGTTGCTGTGTCATAACTTCATAGATGCAGGAGGCTCAGGTGATCT<br>GTTTGAGCAGAGCACCCTAGGCAGCCTGCAGGGAATAACATACTGGCCGTTCTGACCTGTTGCCAGCAGA<br>TACACAGGACATGGATGAAATTCCCGTTTCCTCTAGTTTCTTCCTGTAGTACTCCTCTTTTAGATCCTAA<br>GTCTCTTACAAAAGCTTTGAATACTGTGAAAATGTTTTACATTCCATTTCATTTGTGTTGTTTTTTTAAC<br>TGCATTTTACCAGATGTTTTGATGTTATCGCTTATGTTAATAGTAATTCCCGTACGTGTTCATTTTATTT<br>TCATGCTTTTTCAGCCATGTATCAATATTCACTTGACTAAAATCACTCAATTAATCAAAAAAAAAAAAAA<br>AA | |
| NM_012319 | AGTCCTGGGCGAAGGGGGCGGTGGTTCCCCGCGGCGCTGCGCGCGGCGGTAATTAGTGATTGTCTTCCAG<br>CTTCGCGAAGGCTAGGGGCGCGGCTGCCGGGTGGCTGCGCGGCGCTGCCCCCGGACCGAGGGGCAGCCAA<br>CCCAATGAAACCACCGCGTGTTCGCGCCTGGTAGAGATTTCTCGAAGACACCAGTGGGCCCGTTCCGAGC<br>CCTCTGGACCGCCCGTGTGGAACCAAACCTGCGCGCGTGGCCGGGCCGTGGGACAACGAGGCCGCGGAGA<br>CGAAGGCGCAATGGCGAGGAAGTTATCTGTAATCTTGATCCTGACCTTTGCCCTCTCTGTCACAAATCCC<br>CTTCATGAACTAAAAGCAGCTGCTTTCCCCCAGACCACTGAGAAAATTAGTCCGAATTGGGAATCTGGCA<br>TTAATGTTGACTTGGCAATTTCCACACGGCAATATCATCTACAACAGCTTTTCTACCGCTATGGAGAAAA<br>TAATTCTTTGTCAGTTGAAGGGTTCAGAAAATTACTTCAAAATATAGGCATAGATAAGATTAAAAGAATC<br>CATATACACCATGACCACGACCATCACTCAGACCACGAGCATCACTCAGACCACATGAGCGTCACTCAGACC<br>ATGAGCATCACTCAGACCACGAGCATCACTCTGACCATGATCATCACTCTCACCATAATCATGCTGCTTC<br>TGGTAAAAATAAGCGAAAAGCTCTTTGCCCAGACCATGACTCAGATAGTTCAGGTAAAGATCCTAGAAAC<br>AGCCAGGGGAAAGGAGCTCACCGACCAGAACATGCCAGTGGTAGAAGGAATGTCAAGGACAGTGTTAGTG<br>CTAGTGAAGTGACCTCAACTGTGTACAACACTGTCTCTGAAGGAACTCACTTCTAGAGACAATAGAGAC<br>TCCAAGACCTGGAAAACTCTTCCCCAAAGATGTAAGCAGCTCCACTCCACCCAGTGTCACATCAAAGAGC<br>CGGGTGAGCCGGCTGGCTGGTAGGAAAACAAATGAATCTGTGAGTGAGCCCCGAAAAGGCTTTATGTATT<br>CCAGAAACACAAATGAAAATCCTCAGAGTGTTTCAATGCATCACAGCTACTGACATCTCATGGCATGGG<br>CATCCAGGTTCCGCTGAATGCAACAGAGTTCAACTATCTCTGTCCAGCCATCATCAACCAAATTGATGCT<br>AGATCTTGTCTGATTCATACAAGTGAAAGAAGGCTGAAATCCCTCCAAAGACCTATTCATTACAAATAG<br>CCTGGGTTGGTGGTTTTATAGCCATTTCCATCATCAGTTTCCTGTCTCTGCTGGGGGTTATCTTAGTGCC<br>TCTCATGAATCGGGTGTTTTTCAAATTTCTCCTGAGTTTCCTTGTGGCACTGGCCGTTGGGACTTTGAGT<br>GGTGATGCTTTTTTACACCTTCTTCCACATTCTCATGCAAGTCACCACCATAGTCATAGCCATGAAGAAC<br>CAGCAATGGAAATGAAAAGAGGACCACTTTTCAGTCATCTGTCTTCTCAAAACATAGAAGAAAGTGCCTA<br>TTTTGATTCCACGTGGAAGGGTCTAACAGCTCTAGGAGGCCTGTATTTCATGTTTCTTGTTGAACATGTC<br>CTCACATTGATCAAACAATTTAAAGATAAGAACAAGAAAGAAGAATCAGAAGAAACCTGAAAATGATGATGATG<br>TGGAGATTAAGAAGCAGTTGTCCAAGTATGAATCTCAACTTTCAACAAATGAGGAGAAAGTAGATACAGA<br>TGATCGAACTGAAGGCTATTTACGAGCAGACTCACAAGAGCCCTCCCACTTTGATTCTCAGCAGCCTGCA<br>GTCTTGGAAGAAGAAGAGGTCATGATAGCTCATGCTCATCCACAGGAAGTCTACAATGAATATGTACCCA | 136 |

| GENBANK ACCESSION NUMBER | SEQUENCE | SEQ ID NO: |
|---|---|---|
| | GAGGGTGCAAGAATAAATGCCATTCACATTTCCACGATACACTCGGCCAGTCAGACGATCTCATTCACCA CCATCATGACTACCATCATATTCTCCATCATCACCACCACCAAAACCACCATCCTCACAGTCACAGCCAG CGCTACTCTCGGGAGGAGCTGAAAGATGCCGGCGTCGCCACTCTGGCCTGGATGGTGATAATGGGTGATG GCCTGCACAATTTCAGCGATGGCCTAGCAATTGGTGCTGCTTTTACTGAAGGCTTATCAAGTGGTTTAAG TACTTCTGTTGCTGTGTTCTGTCATGAGTTGCCTCATGAATTAGGTGACTTTGCTGTTCTACTAAAGGCT GGCATGACCGTTAAGCAGGCTGTCCTTTATAATGCATTGTCAGCCATGCTGGCGTATCTTGGAATGGCAA CAGGAATTTTCATTGGTCATTATGCTGAAAATGTTTCTATGTGGATATTTGCACTTACTGCTGGCTTATT CATGTATGTTGCTCTGGTTGATATGGTACCTGAAATGCTGCACAATGATGCTAGTGACCATGGATGTAGC CGCTGGGGGTATTTCTTTTTACAGAATGCTGGGATGCCTTTTGGGTTTTGGAATTATGTTACTTATTTCCA TATTTGAACATAAAATCGTGTTTCGTATAAATTTCTAGTTAAGGTTTAAATGCTAGAGTAGCTTAAAAAG TTGTCATAGTTTCAGTAGGTCATAGGGAGATGAGTTTGTATGCTGTACTATGCAGCGTTTAAAGTTAGTG GGTTTTGTGATTTTTGTATTGAATATTGCTGTCTGTTACAAAGTCAGTTAAAGGTACGTTTTAATATTTA AGTTATTCTATCTTGGAGATAAAATCTGTATGTGCAATTCACCGGTATTACCAGTTTATTATGTAAACAA GAGATTTGGCATGACATGTTCTGTATGTTTCAGGGAAAAATGTCTTTAATGCTTTTTCAAGAACTAACAC AGTTATTCCTATACTGGATTTTAGGTCTCTGAAGAACTGCTGGTGTTTAGGAATAAGAATGTGCATGAAG CCTAAAATACCAAGAAAGCTTATACTGAATTTAAGCAAAGAAATAAAGGAGAAAAGAGAAGAATCTGAGA ATTGGGGAGGCATAGATTCTTATAAAAATCACAAAATTTGTTGTAAATTAGAGGGGAGAAATTTAGAATT AAGTATAAAAAGGCAGAATTAGTATAGAGTACATTCATTAAACATTTTTGTCAGGATTATTTCCCGTAAA AACGTAGTGAGCACTTTTCATATACTAATTTAGTTGTACATTTAACTTTGTATAATACAGAAATCTAAAT ATATTTAATGAATTCAAGCAATATATCACTTGACCAAGAAATTGGAATTTCAAAATGTTCGTGCGGGTAT ATACCAGATGAGTACAGTGAGTAGTTTTATGTATCACCAGACTGGGTTATTGCCAAGTTATATATCACCA AAAGCTGTATGACTGGATGTTCTGGTTACCTGGTTTACAAAATTATCAGAGTAGTAAAACTTTGATATAT ATGAGGATATTAAAACTACACTAAGTATCATTTGATTCGATTCAGAAAGTACTTTGATATCTCTCAGTGC TTCAGTGCTATCATTGTGAGCAATTGTCTTTTATATACGGTACTGTAGCCATACTAGGCCTGTCTGTGGC ATTCTCTAGATGTTTCTTTTTTACACAATAAATTCCTTATATCAGCTTGAAAAAAAAAAAAAAAAAA | |
| AK098106 | AACGCACTTGGCGCGCGGCGCGGGCTGCAGACGGCTGCGAGGCGCTGGGCACAGGTGTCCTGATGGCAAA TTTCAAGGGCCACGCGCTTCCAGGGAGTTTCTTCCTGATCATTGGGCTGTGTTGGTCAGTGAAGTACCCG CTGAAGTACTTTAGCCACACGCGGAAGAACAGCCCACTACATTACTATCAGCGTCTCGAGATCGTCGAAG CCGCAATTAGGACTTTGTTTTCCGTCACTGGGATCCTGGCAGAGCAGTTTGTTCCGGATGGGCCCCCACCT GCACCTCTACCATGAGAACCACTGGATAAAGTTAATGAATTGGCAGCACAGCACCATGTACCTATTCTTT GCAGTCTCAGGAATTGTTGACATGCTCACCTATCTGGTCAGCCACGTTCCCTTGGGGGTGGACAGACTGG TTATGGCTGTGGCAGTATTCATGGAAGGTTTCCTCTTCTACTACCACGTCCACAACCGGCCTCCGCTGGA CCAGCACATCCACTCACTCCTGCTGTATGCTCTGTTCGGAGGGTGTGTTAGTATCTCCCTAGAGGTGATC TTCCGGGACCACATTGTGCTGGAACTTTTCCGAACCAGTCTCATCATTCTTCAGGGAACCTGGTTCTGGC AGATTGGGTTTGTGCTGTTCCCACCTTTTGGAACACCCGAATGGGACCAGAAGGATGATGCCAACCTCAT GTTCATCACCATGTGCTTCTGCTGGCACTACCTGGCTGCCCTCAGCATTGTGGCCGTCAACTATTCTCTT GTTTACTGCCTTTTGACTCGGATGAAGAGACACGGAAGGGGAGAAATCATTGGAATTCAGAAGCTGAATT CAGATGACACTTACCAGACCGCCCTCTTGAGTGGCTCAGATGAGGAATGAGCCGAGATGCGGAGGGCGCA GATGTCCCACTGCACAGCTGGAATGAATGGAGTTCATCCCCTCCACCTGAATGCCTGCTGTGGTCTGATC TTAAGGGTCTATATATTTGCACCTCCTCATTCAACACAGGGCTGGAGGTTCTACAACAGGAAATCAGGCC TACAGCATCCTGTGTATCTTGCAGTTGGGATTTTTAAACATACTATAAAGTCTGTGTTGGTATAGTACCC TTCATAAGGAAAAATGAAGTAATGCCTATAAGTAGCAGGCCTTTGTGCCTCAGTGTCAAGAGAAATCAAG AGATGCTAAAAGCTTTACAATGGAAGTGGCCTCATGGATGAATCCGGGGTATGAGCCCAGGAGAACGTGC TGCTTTTGGTAACTTATCCCTTTTTCTCTTAAGAAAGCAGGTACTTTCTTATTAGAAATATGTTAGAATG TGTAAGCAAACGACAGTGCCTTTAGAATTACAATTCTAACTTACATATTTTTTGAAAGTAAAATAATTCA CAAGCTTTGGTATTTTAAAATTATTGTTAAACATATCATAACTAATCATACCAGGGTACTGCAATACCAC TGTTTATAAGTGACAAAATTAGGCCAAAGGTGATTTTTTTTTAAATCAGGAAGCTGGTTACTGGCTCTAC TGAGAGTTGGAGCCCTGATGTTCTGATTCTTCAAAGTCACCCTAAAAGAAGATCTGACAGGAAAGCTGTA TAATGAGATAGAAAAACGTCAGGTATGGAAGGCTTTCAGTTTTAATATGGCTGAAAGCAAAGGATAACGA ATTCAGAATTAGTAATGTAAAATCTTGATACCCTAATCTTGCTTCTGGATCTGTTCTTTTTTAAAAAAAA CTTCCTTCACCGCGCCTATAATCCTAGCACTTTGGGAGGCCGAGGCAGGCAGATCACGGGGTCAGGAGAT CAAGACCATCCTGGCTAACATGGTGAAACCCCGTCTCTACTGAAAATACAAAAAATTAGCCGGGTGTGGT GGCGGGCGCCTGTAGTTCCAGCTACTCGGGAGGCTGAGGCAAGAGAATGGCATGAACCCGGTAGGGGAGC TTGCAGTGAGCCCAGATCATGCCACTGTACTCCAGCCTAGGTGACAGAGCAAGACTCTGTCTCAAAAACA AGCAAACAGACTTCCTTCAACAAATATTTATTAAATATCCACTTTGCAACAGCACTGAAATGGCTGTAAG GACTCCTGAGATATGTGTCCAGCAAGGAGTTTACAGTCAAACAGGAGAGACATGCCTGTAGTTACATCCA GTGTGATGGGTGCTGAGAGGCAAGTACAAACCACGATG | 137 |

(continued)

| GENBANK ACCESSION NUMBER | SEQUENCE | SEQ ID NO: |
|---|---|---|
| BQ056428 | TCCCGCCGCGCCACTTCGCCTGCCTCCGTCCCCCGCCCGCCGCGCCATGCCTGTGGCCGGCTCGGAGCTG CCGCGCCGGCCCTTGCCCCCCGCCGCACAGGAGCGGGACGCCGAGCCGCGTCCGCCGCACGGGGAGCTGC AGTACCTGGGGCAGATCCAACACATCCTCCGCTGCGGCGTCAGGAAGGACGCCCGCCCGGGCACCGGTAC CCTGCCGGTATTCGGCATGCAGGCGCCTACAGCCTGAGAGATGAATTCCCTCTGCTGACAACCAAACGT GTGTTCTGGAACGGTGCTTCGGAGGAGCTGCTGTGGCTTATCAAGGGATCCACAAACGCTATAGACCTGT CTTCCCCGGCAGCGAAAATCTCGGGATGCCACTGGATCCCGACACTCTCTGGACACCCTGGGATTCTCCA CCAGAGAAGAACGCGACTTGGGCCCAGTTTGTGGCTCTCAGCGGAGGCCTCCTGTGGCAGAATACATACA TTTCCAATCAGATCACTTCCCGGACACGGACCNTGACCAGCCTGCCAAAAAGTGGATTTCCCCCCCACCCC AGAACCCANCCCCTGACGCACAGAAACCAACCCATTCGTTGTTGCCGCCTTGCGAACCCCAACCAGAATC TCTCCCCCCTGGCCGGCGCGCCTGCCGCTGCCAATGCCCCTATGGCGGCCTCTTGGCCCGCACCTTCCAA TTGGTCGCCCTGCGCAACCAGCGAGAAAACACTGGCCCGCCCGTCTCCCCCCCCGCTCCGCCTACCCCACT TAATGCGCCTCCGTGGCATGACGCACGCGTTTGGTGTCCGCCGCCGTCTCATGTCCGCGCGGTGTGGACC CCCTTTTCTCTCGCGGCACATCCCCCCTATTCCCTTGCCCTTTGGGGGGCACCCCCTCTAGACCCGCGCT TCTCTTCTCGTCCGGTGGGGGACATTGGTTTGCCTGCCGCGGCGGGGGCGNTAAAAATAAAAACAGCCTG TTAGCCCGGCCCAGTACCCCCCCCCGGCCGGGGCCGCCTTNCGTTTGCATTTATACCCCAACCCATAAAG CCGCGCCCCTTTAGCNCCNTAACTTTTGTGGTGTGGCCTCCCCCCTTTTTCCCGGGGAGCAGCAACGGAC ATCTGTACACTAATGCTGGCCCCGACCTTTCCCAAAAACCCCCCGCCCGTGTCCCGTATAAATTTGGTGC CAANCCTGACGNGTTCTCCCCCGCCCTCGCCCCGTTGGCCGCCCGTTTAAAGCCCCCCCGGTGGTTGCGC CGCCCAACGAGTCCACCTATAGTTAANTCCACCAACACCCCCACCTTTTCCTCCCCGCCGCATCTTCCCC ACGTACCCCCTTTTGTCGCGAGATGGCCACTCCCCCCCCCTGTTTGTTTAAAACAACGAGAATGGTGCT GCCAACGCTGGTCTTTTCCCCCCCCGGACCGCGACCGCCAGGGGGAATACGTACCATAAGCCCCCGCGCC CNCCTTTTTTCCCCCCTCCCCGCCAATCAAGATCCGCCGTCCATTAGACGTATTATTTTTCCCGCGATAC ACGAAAAAACAGGGCCGCCCATTTATAACTAAATTCCCGTCGCCGCCGCGCGGATATGTTTCCCAAAATA CCACCCCCCCCCCCCCCCATTTTCTTTGCCCCCAACTCCTGCGCACCGGTGTTCACCAGCCTCGCGCCGC | 138 |
| BC032677 | GGACGCGTGGGTCGACCCACGCGTCCGGACCCACGCGTCCGGTCGTGTTCTCCGAGTTCCTGTCTCTCTG CCAACGCCGCCCGGATGGCTTCCCAAAACCGCGACCCAGCCGCCACTAGCGTCGCCGCCGCCCGTAAAGG AGCTGAGCCGAGCGGGGGCGCCGCCCGGGGTCCGGTGGGCAAAAGGCTACAGCAGGAGCTGATGACCCTC ATGGTGAGTGATTAAGTGCCCAGAACCCCAGCCTTCCATCCAATTTTCAGTAGCCTCCTTTTTTCCGTCA GCTTTTTTGCTAGACATAGGGGTAATGTAATTTGCTCCCTCCTGGGAAAGAAGTTCATACACCCCACCTA CACCCATTTCTTCCAGCAGTCCCTCCTCCCAATTCCATCCCCCCCACACGAAGTTATCTCGAACACTTCCCT GAAGTCATACAAGACCCTCCCTATCCAGTGTGTCCCTACTTCCTAGCCCCAACCAAGCTTTACCCACACC CAACTCCCCGCCCTTCTTGGTATTTCTAGCCTATGAATTTGGTTGCTTTATTTTGGATCAGAGTGATGAG ATTAAGGGGAGGCTGGGCGCGGTAGCTCACACCTTATAATCCCAAAGTGCTGGGATTACAGGCGTGAGCC ACCGCGCCCGGCCAGCAACTAATATTCTAATTGAACTAAAGCACAGGATGCCAATTTACAATCCTTAGAC CAAAGAGTCACTGATGTCTCCACCAGATAAGAGGAAAGCATCAGGCTAGGCATAGTGGCTCACACCTGTA ATCTCAGCACTTTGGGAGGCTGAGGCAGGCAGATCACATGAGCCCAGGAGTTTGAGACTGGCCTGGGCAA CATGGTGAAACCCTGTCTCTAAAATAAAAACTAAACTAAAAAAACTTTTTAAAAAGGCAGTGGGGAGCAT CAGAACCAGCTCAACAGTTTGTCTACTGTCCGGTCCCAGAGAAACTCAAGATTCTAGCAAGCCCCTTGTG TGGGGCTTGGGTTGGGACATGAGGCTGCTGCTGGAGCTTACTCTGCAACTGTTTCTCCAAATGCCAGGTA TATGAAGACCTGAGGTATAAGCTCTCGCTAGAGTTCCCCAGTGGCTACCCTTACAATGCGCCCACAGTGA AGTTCCTCACGCCCTGCTATCACCCCAACGTGGACACCCAGGGTAACATATGCCTGGACATCCTGAAGGA AAAGTGGTCTGCCCTGTATGATGTCAGGACCATTCTGCTCTCCATCCAGAGCCTTCTAGGAGAACCCAAC ATTGATAGTCCCTTGAACACACATGCTGCCGAGCTCTGGAAAAAACCCCACAGCTTTTAAGAAGTACCTGC AAGAAACCTACTCAAAGCAGGTCACCAGCCAGGAGCCCTGACCCAGGCTGCCCAGCCTGTCCTTGTGTCG TCTTTTTAATTTTTCCTTAGATGGTCTGTCCTTTTTGTGATTTCTGTATAGGACTCTTTATCTTGAGCTG TGGTATTTTTGTTTTGTTTTTGTCTTTTAAATTAAGCCTCGGTTGAGCCCTTGTATATTAAATAAATGCA TTTTTGTCCTTTTTTAAAAAAAAAATAAAAAAAAAAAAAAAAAAAAAAAAAAAAAAAAAAAAAAAAAAAA A | 139 |

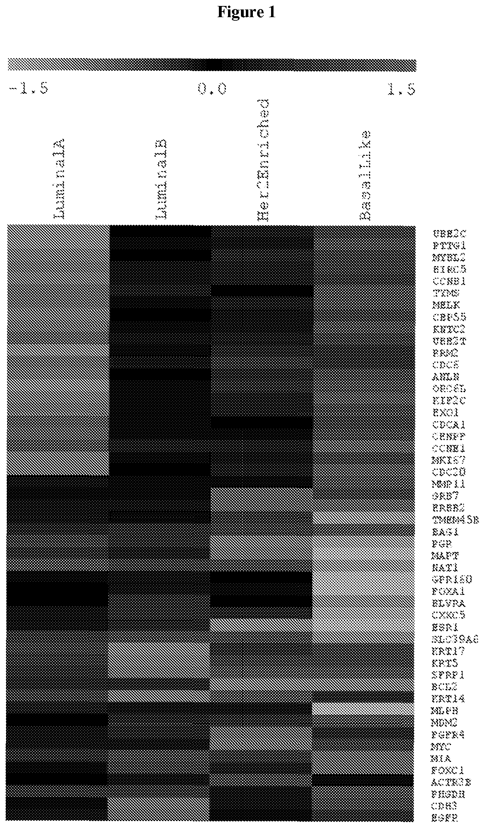

At least 40, at least 41, at least 42, at least 43, at least 44, at least 46 or all 46 of the genes in Table 1 can be utilized in the methods of the disclosure. Preferably, the expression of each of the 46 genes is determined in a biological sample. The prototypical gene expression profiles (*i.e.* centroid) of the four intrinsic subtypes were pre-defined from a training set of FFPE breast tumor samples using hierarchical clustering analysis of gene expression data. A heatmap of the prototypical gene expression profiles (*i.e.* centroids) of these four subtypes is shown in Figure 1, where the level of expression is illustrated by the heatmap. **Table 3** shows the actual values.

**Table 3.**

| Tumor Subtype Centroids for Comparison to a Sample | | | |
|---|---|---|---|
| Target Gene | Basal-like | Her2-enriched | Luminal A | Luminal B |
| ACTR3B | -0.2052 | -0.7965 | -0.2790 | -0.4380 |
| ANLN | 1.0227 | 0.5006 | -0.7289 | 0.1149 |

(continued)

| Tumor Subtype Centroids for Comparison to a Sample | | | |
|---|---|---|---|
| Target Gene | Basal-like | Her2-enriched | Luminal A | Luminal B |
| BAG1 | -0.4676 | -0.3132 | 0.4716 | 0.5879 |
| BCL2 | -0.7365 | -0.7237 | 0.7234 | 0.6363 |
| BLVRA | -0.8761 | 0.2270 | 0.1628 | 0.7138 |
| CCNE1 | 1.3100 | 0.2201 | -0.6231 | -0.2729 |
| CDC20 | 1.0995 | 0.1445 | -1.0518 | -0.1173 |
| CDC6 | 0.5817 | 0.6601 | -0.7032 | 0.3134 |
| CDCA1 | 0.9367 | 0.1623 | -0.4509 | 0.2692 |
| CDH3 | 0.7639 | 0.0144 | -0.0502 | -1.0229 |
| CENPF | 1.0222 | 0.2944 | -0.5657 | 0.2437 |
| CEP55 | 1.0442 | 0.4881 | -0.6365 | 0.2921 |
| CXXC5 | -0.9732 | 0.1866 | 0.5687 | 0.9463 |
| EGFR | 0.3352 | -0.1326 | -0.0011 | -0.9755 |
| ERBB2 | -0.7045 | 1.4182 | 0.2420 | 0.1978 |
| ESR1 | -1.1847 | -0.4926 | 0.7177 | 1.0101 |
| EXO1 | 1.0546 | 0.4317 | -0.7259 | 0.2559 |
| FGFR4 | -0.2073 | 1.4562 | 0.1707 | -0.2223 |
| FOXA1 | -1.3590 | 0.5726 | 0.7131 | 0.7963 |
| FOXC1 | 1.0666 | -0.7362 | -0.4078 | -0.9877 |
| GPR160 | -1.0540 | 0.5524 | 0.6032 | 0.7305 |
| KIF2C | 0.9242 | 0.1104 | -1.1001 | -0.2771 |
| KNTC2 | 1.1373 | 0.2266 | -0.7593 | 0.1656 |
| KRT14 | 0.4759 | -0.5269 | 0.8187 | -0.8879 |
| KRT17 | 0.6863 | -0.3777 | 0.6149 | -1.1415 |
| KRT5 | 0.7136 | -0.4146 | 0.5832 | -0.9462 |
| MAPT | -1.1343 | -0.2711 | 1.0957 | 0.8372 |
| MDM2 | -0.7498 | -0.4855 | -0.1788 | 0.2397 |
| MELK | 1.0209 | 0.2678 | -0.8016 | 0.1012 |
| MIA | 1.2408 | -0.5475 | 0.3289 | -0.6320 |
| MKI67 | 1.0446 | 0.4630 | -0.6717 | 0.3161 |
| MLPH | -1.4150 | 0.4842 | 0.8829 | 0.8194 |
| MMP11 | -0.1295 | 0.5220 | 0.3402 | 0.5653 |
| MYC | 0.5639 | -0.9904 | -0.3015 | -0.2791 |
| NAT1 | -0.9711 | -0.2708 | 1.2256 | 0.9576 |
| ORC6L | 1.0086 | 0.5152 | -1.0385 | -0.0336 |
| PGR | -0.9216 | -0.5755 | 1.2061 | 0.9278 |
| PHGDH | 0.9192 | 0.0322 | -0.5194 | -0.5371 |
| PTTG1 | 0.9541 | 0.2079 | -1.1207 | 0.1052 |
| RRM2 | 0.7895 | 0.6336 | -0.8099 | 0.3228 |
| SFRP1 | 0.7694 | -0.8271 | 0.2617 | -1.0846 |
| SLC39A6 | -0.9992 | -0.4573 | 0.6607 | 0.9222 |
| TMEM45B | -1.0721 | 0.7926 | 0.3190 | 0.2016 |
| TYMS | 0.9823 | -0.0960 | -0.8593 | 0.1827 |
| UBE2C | 0.8294 | 0.3358 | -1.0141 | 0.0608 |
| UBE2T | 0.6258 | 0.0617 | -0.8652 | -0.0487 |

After performing the Breast Cancer Intrinsic Subtyping test with a test breast cancer tumor sample and the reference sample provided as part of the test kit, a computational algorithm based on a Pearson's correlation compares the normalized and scaled gene expression profile of the NANO46 intrinsic gene set of the test sample to the prototypical expression signatures of the four breast cancer intrinsic subtypes. The intrinsic subtype analysis is determined by determining the expression of a NANO50 set of genes (which is determining the expression of the NANO46 set of genes and further includes determining the expression of MYBL2, BIRC5, GRB7 and CCNB1) and the risk of recurrence ("ROR") is determined using the NANO46 set of genes). Specifically, the intrinsic subtype is identified by comparing the expression of the NANO50 set of genes in the biological sample with the expected expression profiles for the four intrinsic subtypes. The subtype with the most similar expression profile is assigned to the biological sample. The ROR score is an integer value on a 0-100 scale that is related to an individual patient's probability of distant recurrence within 10 years for the defined intended use population. The ROR score is calculated by comparing the expression profiles of the NANO46 genes in the biological sample with the expected profiles for the four intrinsic subtypes, as described above, to calculate four different correlation values. These correlation values are then combined with a proliferation score (and optionally one or more clinicopathological variables, such as tumor size) to calculate the ROR score. Preferably, the ROR score is calculated by comparing only the expression profiles of the NANO46 genes.

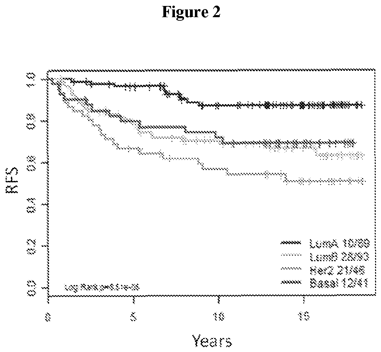

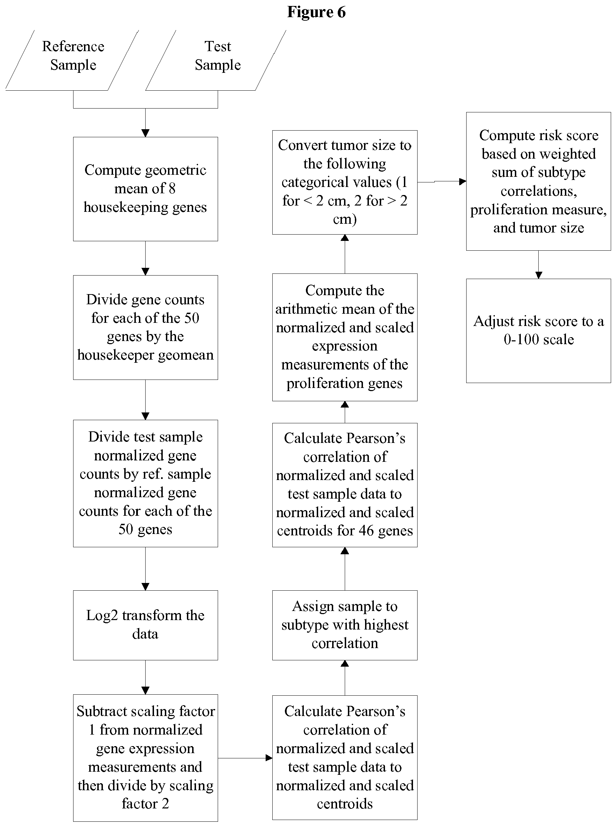

**Figure 6** provides a schematic of the specific algorithm transformations. The tumor sample is assigned the subtype with the largest positive correlation to the sample. Kaplan Meier survival curves generated from a training set of untreated breast cancer patients demonstrate that the intrinsic subtypes are a prognostic indicator of recurrence free survival (RFS) in this test population, which includes both estrogen receptor positive/negative and HER2 positive/negative patients, **Figure 2.**

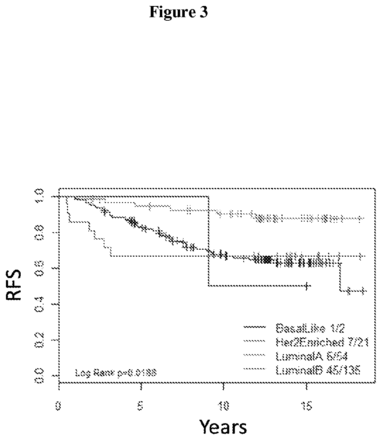

Independent testing on a cohort of node negative, estrogen receptor positive patients treated with tamoxifen shows predominantly Luminal A and B subtype patients with Luminal A patients exhibiting better outcome than Luminal B patients, **Figure 3.** The outcome of Luminal A patients is expected to improve even further using clinical trial specimens that use more modern treatment regimens (*i.e.* aromatase inhibitors) and have better adherence to therapy which will improve outcome

The training set of FFPE breast tumor samples, which had well defined clinical characteristics and clinical outcome data, were used to establish a continuous Risk of Recurrence (ROR) score. The score is calculated using coefficients from a Cox model that includes correlation to each intrinsic subtype, a proliferation score (mean gene expression of a subset of 18 of the 46 genes), and tumor size, **Table 4.**

| Table 4. Coefficients to calculate ROR-PT (equation 1) | |
| --- | --- |
| **Test Variables** | **Coefficient** |
| Basal-like Pearson's correlation (A) | - 0.0067 |
| Her2-enriched Pearson's correlation (B) | 0.4317 |
| Luminal A Pearson's correlation (C) | - 0.3172 |
| Luminal B Pearson's correlation (D) | 0.4894 |
| Proliferation Score (E) | 0.1981 |
| Tumor Size (F) | 0.1133 |

The test variables in **Table 4** are multiplied by the corresponding coefficients and summed to produce a risk score ("ROR-PT").

$$\textbf{ROR-PT equation} = -0.0067*A + 0.4317*B + -0.3172*C + 0.4894*D + 0.1981*E + 0.1133*F$$

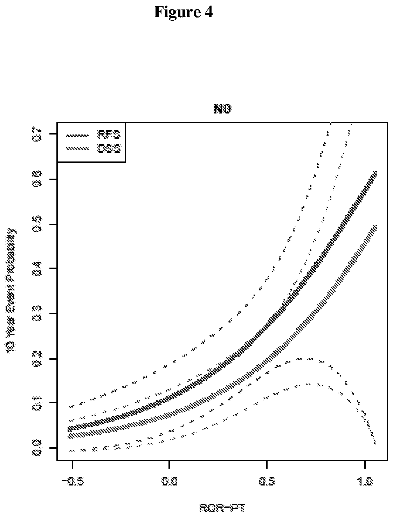

In previous studies, the ROR score provided a continuous estimate of the risk of recurrence for ER-positive, node-negative patients who were treated with tamoxifen for 5 years (Nielsen et al. Clin. Cancer Res., 16(21):5222-5232 (2009)). This result was verified on ER-positive, node-negative patients from the same cohort, **Figure 4.** The ROR score also exhibited a statistically significant improvement over a clinical model based in determining RFS within this test population providing further evidence of the improved accuracy of this decision making tool when compared to traditional clinicopathological measures (Nielsen et al. Clin. Cancer Res., 16(21):5222-5232 (2009)).

The gene set contains many genes that are known markers for proliferation. The methods disclosed herein provide for the determination of subsets of genes that provide a proliferation signature. The methods can include determining the expression of at least one of, a combination of, or each of, a 18-gene subset of the NANO46 intrinsic genes selected from ANLN, CCNE1, CDC20, CDC6, CDCA1, CENPF, CEP55, EXOI, KIF2C, KNTC2, MELK, MKI67, ORC6L, PTTG1, RRM2, TYMS, UBE2C and/or UBE2T. Preferably, the expression of each of the 18-gene subset of the NANO46 gene set is determined to provide a proliferation score. The expression of one or more of these genes may be determined and a proliferation signature index can be generated by averaging the normalized expression estimates of one or more of these genes in a sample. The sample can be assigned a high proliferation signature, a moderate/intermediate proliferation signature, a low proliferation signature or an ultra-low proliferation signature. Methods of determining a proliferation signature from a biological sample are as described in Nielsen et al. Clin. Cancer Res., 16(21):5222-5232 (2009) and supplemental online material.

*Description of Intrinsic Subtype Biology*

Luminal subtypes: The most common subtypes of breast cancer are the luminal subtypes, Luminal A and Luminal B. Prior studies suggest that luminal A comprises approximately 30% to 40% and luminal B approximately 20% of all breast cancers, but they represent over 90 % of hormone receptor positive breast cancers (Nielsen et al. Clin. Cancer Res., 16(21):5222-5232 (2009)). The gene expression pattern of these subtypes resembles the luminal epithelial component of the breast. These tumors are characterized by high expression of estrogen receptor (ER), progesterone receptor (PR), and genes associated with ER activation, such as LIV1, GATA3, and cyclin D1, as well as expression of luminal cytokeratins 8 and 18 (Lisa Carey & Charles Perou (2009). Gene Arrays, Prognosis, and Therapeutic Interventions. Jay R. Harris et al. (4th ed.), Diseases of the breast (pp. 458-472). Philadelphia, PA: Lippincott Williams & Wilkins).

Luminal A: Luminal A (LumA) breast cancers exhibit low expression of genes associated with cell cycle activation and the ERBB2 cluster resulting in a better prognosis than Luminal B. The Luminal A subgroup has the most favorable prognosis of all subtypes and is enriched for endocrine therapy-responsive tumors.
Luminal B: Luminal B (LumB) breast cancers also express ER and ER-associated genes. Genes associated with cell cycle activation are highly expressed and this tumor type can be HER2(+) (~20%) or HER2(-). The prognosis is unfavorable (despite ER expression) and endocrine therapy responsiveness is generally diminished relative to LumA.

HER2-enriched: The HER2-enriched subtype is generally ER-negative and is HER2-positive in the majority of cases with high expression of the ERBB2 cluster, including ERBB2 and GRB7. Genes associated with cell cycle activation are highly expressed and these tumors have a poor outcome.

Basal-like: The Basal-like subtype is generally ER-negative, is almost always clinically HER2-negative and expresses a suite of "basal" biomarkers including the basal epithelial cytokeratins (CK) and epidermal growth factor receptor (EGFR). Genes associated with cell cycle activation are highly expressed.

*Clinical variables*

The NANO46 classification model described herein may be further combined with information on clinical variables to generate a continuous risk of recurrence (ROR) predictor. As described herein, a number of clinical and prognostic breast cancer factors are known in the art and are used to predict treatment outcome and the likelihood of disease recurrence. Such factors include, for example, lymph node involvement, tumor size, histologic grade, estrogen and progesterone hormone receptor status, HER-2 levels, and tumor ploidy. In one embodiment, risk of recurrence (ROR) score is provided for a subject diagnosed with or suspected of having breast cancer. This score uses the NANO46 classification model in combination with clinical factors of lymph node status (N) and tumor size (T). Assessment of clinical variables is based on the American Joint Committee on Cancer (AJCC) standardized system for breast cancer staging. In this system, primary tumor size is categorized on a scale of 0-4 (TO: no evidence of primary tumor; T1 : < 2 cm; T2: > 2 cm - < 5 cm; T3 : > 5 cm; T4: tumor of any size with direct spread to chest wall or skin). Lymph node status is classified as N0-N3 (NO: regional lymph nodes are free of metastasis; N1 : metastasis to movable, same-side axillary lymph node(s); N2: metastasis to same-side lymph node(s) fixed to one another or to other structures; N3: metastasis to same-side lymph nodes beneath the breastbone). Methods of identifying breast cancer patients and staging the disease are well known and may include manual examination, biopsy, review of patient's and/or family history, and imaging techniques, such as mammography, magnetic resonance imaging (MRI), and positron emission tomography (PET).

*Sample Source*

In one embodiment of the present disclosure, breast cancer subtype is assessed through the evaluation of expression patterns, or profiles, of the intrinsic genes listed in Table 1 in one or more subject samples. For the purpose of discussion, the term subject, or subject sample, refers to an individual regardless of health and/or disease status. A subject can be a subject, a study participant, a control subject, a screening subject, or any other class of individual from whom a sample is obtained and assessed in the context of the disclosure. Accordingly, a subject can be diagnosed with breast cancer, can present with one or more symptoms of breast cancer, or a predisposing factor, such as a family (genetic) or medical history (medical) factor, for breast cancer, can be undergoing treatment or therapy for breast cancer, or the like. Alternatively, a subject can be healthy with respect to any of the aforementioned factors or criteria. It will be appreciated that the term "healthy" as used herein, is relative to breast cancer status, as the term "healthy" cannot be defined to correspond to any absolute evaluation or status. Thus, an individual defined as healthy with reference to any specified disease or disease criterion, can in fact be diagnosed with any other one or more diseases, or exhibit any other one or more disease criterion, including one or more cancers other than breast cancer. However, the healthy controls are preferably free of any cancer.

In particular embodiments, the methods for predicting breast cancer intrinsic subtypes include collecting a biological sample comprising a cancer cell or tissue, such as a breast tissue sample or a primary breast tumor tissue sample. By "biological sample" is intended any sampling of cells, tissues, or bodily fluids in which expression of an intrinsic gene can be detected. Examples of such biological samples include, but are not limited to, biopsies and smears. Bodily fluids useful in the present disclosure include blood, lymph, urine, saliva, nipple aspirates, gynecological fluids, or any other bodily secretion or derivative thereof. Blood can include whole blood, plasma, serum, or any derivative of blood. In some embodiments, the biological sample includes breast cells, particularly breast tissue from a biopsy, such as a breast tumor tissue sample. Biological samples may be obtained from a subject by a variety of techniques including, for example, by scraping or swabbing an area, by using a needle to aspirate cells or bodily fluids, or by removing a tissue sample (*i.e.,* biopsy). Methods for collecting various biological samples are well known in the art. In some embodiments, a breast tissue sample is obtained by, for example, fine needle aspiration biopsy, core needle biopsy, or excisional biopsy. Fixative and staining solutions may be applied to the cells or tissues for preserving the specimen and for facilitating examination. Biological samples, particularly breast tissue samples, may be transferred to a glass slide for viewing under magnification. In one embodiment, the biological sample is a formalin-fixed, paraffin-embedded breast tissue sample, particularly a primary breast tumor sample. In various embodiments, the tissue sample is obtained from a pathologist-guided tissue core sample.

*Expression Profiling*

In various embodiments, the present disclosure provides methods for classifying, prognosticating, or monitoring breast cancer in subjects. In this embodiment, data obtained from analysis of intrinsic gene expression is evaluated using one or more pattern recognition algorithms. Such analysis methods may be used to form a predictive model, which can be used to classify test data. For example, one convenient and particularly effective method of classification employs multivariate statistical analysis modeling, first to form a model (a "predictive mathematical model") using data ("modeling data") from samples of known subtype (e.g., from subjects known to have a particular breast cancer intrinsic subtype: LumA, LumB, Basal-like, HER2-enriched, or normal-like), and second to classify an unknown sample (*e.g.,* "test sample") according to subtype. Pattern recognition methods have been used widely to characterize many different types of problems ranging, for example, over linguistics, fingerprinting, chemistry and psychology. In the context of the methods described herein, pattern recognition is the use of multivariate statistics, both parametric and non-parametric, to analyze data, and hence to classify samples and to predict the value of some dependent variable based on a range of observed measurements. There are two main approaches. One set of methods is termed "unsupervised" and these simply reduce data complexity in a rational way and also produce display plots which can be interpreted by the human eye. However, this type of approach may not be suitable for developing a clinical assay that can be used to classify samples derived from subjects independent of the initial sample population used to train the prediction algorithm.

The other approach is termed "supervised" whereby a training set of samples with known class or outcome is used to produce a mathematical model which is then evaluated with independent validation data sets. Here, a "training set" of intrinsic gene expression data is used to construct a statistical model that predicts correctly the "subtype" of each sample. This training set is then tested with independent data (referred to as a test or validation set) to determine the robustness of the computer-based model. These models are sometimes termed "expert systems," but may be based on a range of different mathematical procedures. Supervised methods can use a data set with reduced dimensionality (for example, the first few principal components), but typically use unreduced data, with all dimensionality. In all cases the methods allow the quantitative description of the multivariate boundaries that characterize and separate each subtype in terms of its intrinsic gene expression profile. It is also possible to obtain confidence limits on any predictions, for example, a level of probability to be placed on the goodness of fit. The robustness of the predictive models can also be checked using cross-validation, by leaving out selected samples from the analysis.

The NANO46 classification model described herein is based on the gene expression profile for a plurality of subject samples using the intrinsic genes listed in Table 1. The plurality of samples includes a sufficient number of samples derived from subjects belonging to each subtype class. By "sufficient samples" or "representative number" in this context is intended a quantity of samples derived from each subtype that is sufficient for building a classification model that can reliably distinguish each subtype from all others in the group. A supervised prediction algorithm is developed based on the profiles of objectively-selected prototype samples for "training" the algorithm. The samples are selected and subtyped using an expanded intrinsic gene set according to the methods disclosed in International Patent Publication WO 2007/061876 and US Patent Publication No. 2009/0299640. Alternatively, the samples can be subtyped according to any known assay for classifying breast cancer subtypes. After stratifying the training samples according to subtype, a centroid-based prediction algorithm is used to construct centroids based on the expression profile of the intrinsic gene set described in Table 1.

In one embodiment, the prediction algorithm is the nearest centroid methodology related to that described in Narashiman and Chu (2002) PNAS 99:6567-6572. In the present disclosure, the method computes a standardized centroid for each subtype. This centroid is the average gene expression for each gene in each subtype (or "class") divided by the within-class standard deviation for that gene. Nearest centroid classification takes the gene expression profile of a new sample, and compares it to each of these class centroids. Subtype prediction is done by calculating the Spearman's rank correlation of each test case to the five centroids, and assigning a sample to a subtype based on the nearest centroid.

*Detection of intrinsic gene expression*

Any methods available in the art for detecting expression of the intrinsic genes listed in Table 1 are encompassed herein. By "detecting expression" is intended determining the quantity or presence of an RNA transcript or its expression product of an intrinsic gene. Methods for detecting expression of the intrinsic genes of the disclosure, that is, gene expression profiling, include methods based on hybridization analysis of polynucleotides, methods based on sequencing of polynucleotides, immunohistochemistry methods, and proteomics-based methods. The methods generally detect expression products (*e.g.*, mRNA) of the intrinsic genes listed in Table 1. In preferred embodiments, PCR-based methods, such as reverse transcription PCR (RT-PCR) (Weis et al., TIG 8:263- 64, 1992), and array-based methods such as microarray (Schena et al., Science 270:467- 70, 1995) are used. By "microarray" is intended an ordered arrangement of hybridizable array elements, such as, for example, polynucleotide probes, on a substrate. The term "probe" refers to any molecule that is capable of selectively binding to a specifically intended target biomolecule, for example, a nucleotide transcript or a protein encoded by or corresponding to an intrinsic gene. Probes can be synthesized by one of skill in the art, or derived from appropriate biological preparations. Probes may be specifically designed to be labeled. Examples of molecules that can be utilized as probes include, but are not limited to, RNA, DNA, proteins, antibodies, and organic molecules.

Many expression detection methods use isolated RNA. The starting material is typically total RNA isolated from a biological sample, such as a tumor or tumor cell line, and corresponding normal tissue or cell line, respectively. If the source of RNA is a primary tumor, RNA (*e.g.,* mRNA) can be extracted, for example, from frozen or archived paraffin-embedded and fixed (*e.g.,* formalin-fixed) tissue samples (*e.g.,* pathologist-guided tissue core samples).

General methods for RNA extraction are well known in the art and are disclosed in standard textbooks of molecular biology, including Ausubel et al., ed., Current Protocols in Molecular Biology, John Wiley & Sons, New York 1987-1999. Methods for RNA extraction from paraffin embedded tissues are disclosed, for example, in Rupp and Locker, Lab Invest. 56:A67, (1987); and De Andres et al. Biotechniques 18:42-44, (1995). In particular, RNA isolation can be performed using a purification kit, a buffer set and protease from commercial manufacturers, such as Qiagen (Valencia, CA), according to the manufacturer's instructions. For example, total RNA from cells in culture can be isolated using Qiagen RNeasy mini-columns. Other commercially available RNA isolation kits include MASTERPURE™ Complete DNA and RNA Purification Kit (Epicentre, Madison, Wis.) and Paraffin Block RNA Isolation Kit (Ambion, Austin, TX). Total RNA from tissue samples can be isolated, for example, using RNA Stat-60 (Tel-Test, Friendswood, TX). Total RNA from FFPE can be isolated, for example, using High Pure FFPE RNA Microkit, Cat No. 04823125001 (Roche Applied Science, Indianapolis, IN). RNA prepared from a tumor can be isolated, for example, by cesium chloride density gradient centrifugation. Additionally, large numbers of tissue samples can readily be processed using techniques well known to those of skill in the art, such as, for example, the single-step RNA isolation process of Chomczynski (U.S. Pat. No. 4,843,155).

Isolated RNA can be used in hybridization or amplification assays that include, but are not limited to, PCR analyses and probe arrays. One method for the detection of RNA levels involves contacting the isolated RNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 60, 100, 250, or 500 nucleotides in length and sufficient to specifically hybridize under stringent conditions to an intrinsic gene of the present disclosure, or any derivative DNA or RNA. Hybridization of an mRNA with the probe indicates that the intrinsic gene in question is being expressed.

In one embodiment, the mRNA is immobilized on a solid surface and contacted with a probe, for example by running the isolated mRNA on an agarose gel and transferring the mRNA from the gel to a membrane, such as nitrocellulose. In an alternative embodiment, the probes are immobilized on a solid surface and the mRNA is contacted with the probes, for example, in an Agilent gene chip array. A skilled artisan can readily adapt known mRNA detection methods for use in detecting the level of expression of the intrinsic genes of the present disclosure.

An alternative method for determining the level of intrinsic gene expression product in a sample involves the process of nucleic acid amplification, for example, by RT-PCR (U.S. Pat. No. 4,683,202), ligase chain reaction (Barany, PNAS USA 88: 189-93, (1991)), self sustained sequence replication (Guatelli et al., Proc. Natl. Acad. Sci USA 87: 1874-78, (1990)), transcriptional amplification system (Kwoh et al., Proc. Natl. Acad. ScL USA 86: 1173-77, (1989)), Q-Beta Replicase (Lizardi et al., Bio/Technology 6:1197, (1988)), rolling circle replication (U.S. Pat. No. 5,854,033), or any other nucleic acid amplification method, followed by the detection of the amplified molecules using techniques well known to those of skill in the art. These detection schemes are especially useful for the detection of nucleic acid molecules if such molecules are present in very low numbers.

In particular aspects of the disclosure, intrinsic gene expression is assessed by quantitative RT-PCR. Numerous different PCR or QPCR protocols are known in the art and exemplified herein below and can be directly applied or adapted for use using the presently-described compositions for the detection and/or quantification of the intrinsic genes listed in Table 1. Generally, in PCR, a target polynucleotide sequence is amplified by reaction with at least one oligonucleotide primer or pair of oligonucleotide primers. The primer(s) hybridize to a complementary region of the target nucleic acid and a DNA polymerase extends the primer(s) to amplify the target sequence. Under conditions sufficient to provide polymerase-based nucleic acid amplification products, a nucleic acid fragment of one size dominates the reaction products (the target polynucleotide sequence which is the amplification product). The amplification cycle is repeated to increase the concentration of the single target polynucleotide sequence. The reaction can be performed in any thermocycler commonly used for PCR. However, preferred are cyclers with real time fluorescence measurement capabilities, for example, SMARTCYCLER® (Cepheid, Sunnyvale, CA), ABI PRISM 7700® (Applied Biosystems, Foster City, Calif.), ROTOR- GENE™ (Corbett Research, Sydney, Australia), LIGHTCYCLER® (Roche Diagnostics Corp, Indianapolis, Ind.), ICYCLER® (Biorad Laboratories, Hercules, Calif.) and MX4000® (Stratagene, La Jolla, Calif.).

In another embodiment of the disclosure, microarrays are used for expression profiling. Microarrays are particularly well suited for this purpose because of the reproducibility between different experiments. DNA microarrays provide one method for the simultaneous measurement of the expression levels of large numbers of genes. Each array consists of a reproducible pattern of capture probes attached to a solid support. Labeled RNA or DNA is hybridized to complementary probes on the array and then detected by laser scanning. Hybridization intensities for each probe on the array are determined and converted to a quantitative value representing relative gene expression levels. See, for example, U.S. Pat. Nos. 6,040,138, 5,800,992 and 6,020,135, 6,033,860, and 6,344,316. High-density oligonucleotide arrays are particularly useful for determining the gene expression profile for a large number of RNAs in a sample.

In a preferred embodiment, the nCounter® Analysis system is used to detect intrinsic gene expression. The basis of the nCounter® Analysis system is the unique code assigned to each nucleic acid target to be assayed (International Patent Application Publication No. WO 08/124847, US Patent No. 8,415,102 and Geiss et al. Nature Biotechnology. 2008. 26(3): 317-325). The code is composed of an ordered series of colored fluorescent spots which create a unique barcode for each target to be assayed. A pair of probes is designed for each DNA or RNA target, a biotinylated capture probe and a reporter probe carrying the fluorescent barcode. This system is also referred to, herein, as the nanoreporter code system.

Specific reporter and capture probes are synthesized for each target. Briefly, sequence-specific DNA oligonucleotide probes are attached to code-specific reporter molecules. Preferably, each sequence specific reporter probe comprises a target specifc sequence capable of hybriding to no more than one NANO46 gene of Table 1 and optionally comprises at least two, at least three, or at least four label attachment regions, said attachment regions comprising one or more label monomers that emit light. Capture probes are made by ligating a second sequence-specific DNA oligonucleotide for each target to a universal oligonucleotide containing biotin. Reporter and capture probes are all pooled into a single hybridization mixture, the "probe library". Preferably, the probe library comprises a probe pair (a capture probe and reporter) for each of the NANO46 genes in Table 1.

The relative abundance of each target is measured in a single multiplexed hybridization reaction. The method comprises contacting a biological sample with a probe library, the library comprising a probe pair for the NANO46 genes in Table 1, such that the presence of the target in the sample creates a probe pair - target complex. The complex is then purified. More specifically, the sample is combined with the probe library, and hybridization occurs in solution. After hybridization, the tripartite hybridized complexes (probe pairs and target) are purified in a two-step procedure using magnetic beads linked to oligonucleotides complementary to universal sequences present on the capture and reporter probes. This dual purification process allows the hybridization reaction to be driven to completion with a large excess of target-specific probes, as they are ultimately removed, and, thus, do not interfere with binding and imaging of the sample. All post hybridization steps are handled robotically on a custom liquid-handling robot (Prep Station, NanoString Technologies).

Purified reactions are deposited by the Prep Station into individual flow cells of a sample cartridge, bound to a streptavidin-coated surface via the capture probe, electrophoresed to elongate the reporter probes, and immobilized.

After processing, the sample cartridge is transferred to a fully automated imaging and data collection device (Digital Analyzer, NanoString Technologies). The expression level of a target is measured by imaging each sample and counting the number of times the code for that target is detected. Data is output in simple spreadsheet format listing the number of counts per target, per sample.

This system can be used along with nanoreporters. Additional disclosure regarding nanoreporters can be found in International Publication No. WO 07/076129 and WO 07/076132, and US Patent Publication No. 2010/0015607 and 2010/0261026. Further, the term nucleic acid probes and nanoreporters can include the rationally designed (*e.g.* synthetic sequences) described in International Publication No. WO 2010/019826 and US Patent Publication No. 2010/0047924.

*Data processing*

It is often useful to pre-process gene expression data, for example, by addressing missing data, translation, scaling, normalization, weighting, *etc.* Multivariate projection methods, such as principal component analysis (PCA) and partial least squares analysis (PLS), are so-called scaling sensitive methods. By using prior knowledge and experience about the type of data studied, the quality of the data prior to multivariate modeling can be enhanced by scaling and/or weighting. Adequate scaling and/or weighting can reveal important and interesting variation hidden within the data, and therefore make subsequent multivariate modeling more efficient. Scaling and weighting may be used to place the data in the correct metric, based on knowledge and experience of the studied system, and therefore reveal patterns already inherently present in the data.

If possible, missing data, for example gaps in column values, should be avoided. However, if necessary, such missing data may replaced or "filled" with, for example, the mean value of a column ("mean fill"); a random value ("random fill"); or a value based on a principal component analysis ("principal component fill").

"Translation" of the descriptor coordinate axes can be useful. Examples of such translation include normalization and mean centering. "Normalization" may be used to remove sample-to-sample variation. For microarray data, the process of normalization aims to remove systematic errors by balancing the fluorescence intensities of the two labeling dyes. The dye bias can come from various sources including differences in dye labeling efficiencies, heat and light sensitivities, as well as scanner settings for scanning two channels. Some commonly used methods for calculating normalization factor include: (i) global normalization that uses all genes on the array; (ii) housekeeping genes normalization that uses constantly expressed housekeeping/invariant genes; and (iii) internal controls normalization that uses known amount of exogenous control genes added during hybridization (Quackenbush Nat. Genet. 32 (Suppl.), 496-501 (2002)). In one embodiment, the intrinsic genes disclosed herein can be normalized to control housekeeping genes. For example, the housekeeping genes described in U.S. Patent Publication 2008/0032293 can be used for normalization. Exemplary housekeeping genes include MRPL19, PSMC4, SF3A1, PUM1, ACTB, GAPD, GUSB, RPLPO, and TFRC. It will be understood by one of skill in the art that the methods disclosed herein are not bound by normalization to any particular housekeeping genes, and that any suitable housekeeping gene(s) known in the art can be used.

Many normalization approaches are possible, and they can often be applied at any of several points in the analysis. In one embodiment, microarray data is normalized using the LOWESS method, which is a global locally weighted scatter plot smoothing normalization function. In another embodiment, qPCR data is normalized to the geometric mean of set of multiple housekeeping genes.

"Mean centering" may also be used to simplify interpretation. Usually, for each descriptor, the average value of that descriptor for all samples is subtracted. In this way, the mean of a descriptor coincides with the origin, and all descriptors are "centered" at zero. In "unit variance scaling," data can be scaled to equal variance. Usually, the value of each descriptor is scaled by 1/StDev, where StDev is the standard deviation for that descriptor for all samples. "Pareto scaling" is, in some sense, intermediate between mean centering and unit variance scaling. In pareto scaling, the value of each descriptor is scaled by 1/sqrt(StDev), where StDev is the standard deviation for that descriptor for all samples. In this way, each descriptor has a variance numerically equal to its initial standard deviation. The pareto scaling may be performed, for example, on raw data or mean centered data.

"Logarithmic scaling" may be used to assist interpretation when data have a positive skew and/or when data spans a large range, *e.g.,* several orders of magnitude. Usually, for each descriptor, the value is replaced by the logarithm of that value. In "equal range scaling," each descriptor is divided by the range of that descriptor for all samples. In this way, all descriptors have the same range, that is, 1. However, this method is sensitive to presence of outlier points. In "autoscaling," each data vector is mean centered and unit variance scaled. This technique is a very useful because each descriptor is then weighted equally, and large and small values are treated with equal emphasis. This can be important for genes expressed at very low, but still detectable, levels.

In one embodiment, data is collected for one or more test samples and classified using the NANO46 classification model described herein. When comparing data from multiple analyses (*e.g.,* comparing expression profiles for one or more test samples to the centroids constructed from samples collected and analyzed in an independent study), it will be necessary to normalize data across these data sets. In one embodiment, Distance Weighted Discrimination (DWD) is used to combine these data sets together (Benito et al. (2004) Bioinformatics 20(1): 105-114). DWD is a multivariate analysis tool that is able to identify systematic biases present in separate data sets and then make a global adjustment to compensate for these biases; in essence, each separate data set is a multi-dimensional cloud of data points, and DWD takes two points clouds and shifts one such that it more optimally overlaps the other.

The methods described herein may be implemented and/or the results recorded using any device capable of implementing the methods and/or recording the results. Examples of devices that may be used include but are not limited to electronic computational devices, including computers of all types. When the methods described herein are implemented and/or recorded in a computer, the computer program that may be used to configure the computer to carry out the steps of the methods may be contained in any computer readable medium capable of containing the computer program. Examples of computer readable medium that may be used include but are not limited to diskettes, CD- ROMs, DVDs, ROM, RAM, and other memory and computer storage devices. The computer program that may be used to configure the computer to carry out the steps of the methods and/or record the results may also be provided over an electronic network, for example, over the internet, an intranet, or other network.

*Calculation of risk of recurrence*

Provided herein are methods for predicting breast cancer outcome within the context of the intrinsic subtype and optionally other clinical variables. Outcome may refer to overall or disease-specific survival, event-free survival, or outcome in response to a particular treatment or therapy. In particular, the methods may be used to predict the likelihood of long-term, disease-free survival. "Predicting the likelihood of survival of a breast cancer patient" is intended to assess the risk that a patient will die as a result of the underlying breast cancer. "Long-term, disease-free survival" is intended to mean that the patient does not die from or suffer a recurrence of the underlying breast cancer within a period of at least five years, or at least ten or more years, following initial diagnosis or treatment.

In one embodiment, outcome is predicted based on classification of a subject according to subtype. In addition to providing a subtype assignment, the NANO46 bioinformatics model provides a measurement of the similarity of a test sample to all four subtypes which is translated into a Risk of Recurrence (ROR) score that can be used in any patient population regardless of disease status and treatment options. The intrinsic subtypes and ROR also have value in the prediction of pathological complete response in women treated with, for example, neoadjuvant taxane and anthracycline chemotherapy (Rouzier et al., J Clin Oncol 23:8331-9 (2005)). Thus, in various embodiments of the present disclosure, a risk of recurrence (ROR) model is used to predict outcome. Using these risk models, subjects can be stratified into low, medium, and high risk of recurrence groups. Calculation of ROR can provide prognostic information to guide treatment decisions and/or monitor response to therapy.

In some embodiments described herein, the prognostic performance of the NANO46-defined intrinsic subtypes and/or other clinical parameters is assessed utilizing a Cox Proportional Hazards Model Analysis, which is a regression method for survival data that provides an estimate of the hazard ratio and its confidence interval. The Cox model is a well-recognized statistical technique for exploring the relationship between the survival of a patient and particular variables. This statistical method permits estimation of the hazard (*i.e.*, risk) of individuals given their prognostic variables (*e.g.,* intrinsic gene expression profile with or without additional clinical factors, as described herein). The "hazard ratio" is the risk of death at any given time point for patients displaying particular prognostic variables. See generally Spruance et al., Antimicrob. Agents & Chemo. 48:2787-92 (2004).

The NANO46 classification model described herein can be trained for risk of recurrence using subtype distances (or correlations) alone, or using subtype distances with clinical variables as discussed *supra*. In one embodiment, the risk score for a test sample is calculated using intrinsic subtype distances alone using the following equation:

ROR = 0.05*Basal + 0.11*Her2 + -0.25*LumA + 0.07*LumB + -0.11*Normal, where the variables "Basal," "Her2," "LumA," "LumB," and "Normal" are the distances to the centroid for each respective classifier when the expression profile from a test sample is compared to centroids constructed using the gene expression data deposited with the Gene Expression Omnibus (GEO).

Risk score can also be calculated using a combination of breast cancer subtype and the clinical variables tumor size (T) and lymph nodes status (N) using the following equation: ROR (full) = 0.05*Basal + 0.1*Her2 + -0.19*LumA + 0.05*LumB + - 0.09*Normal + 0.16*T + 0.08*N, again when comparing test expression profiles to centroids constructed using the gene expression data deposited with GEO as accession number GSE2845.

In yet another embodiment, risk score for a test sample is calculated using intrinsic subtype distances alone using the following equation:

ROR-S = 0.05*Basal + 0.12*Her2 + -0.34*LumA + 0.0.23*LumB, where the variables "Basal," "Her2," "LumA," and "LumB" are as described *supra* and the test expression profiles are compared to centroids constructed using the gene expression data deposited with GEO as accession number GSE2845. In yet another embodiment, risk score can also be calculated using a combination of breast cancer subtype and the clinical variable tumor size (T) using the following equation (where the variables are as described *supra*): ROR-C = 0.05*Basal + 0.11*Her2 + -0.23*LumA + 0.09*LumB + 0.17*T.

In yet another embodiment, risk score for a test sample is calculated using intrinsic subtype distances in combination with the proliferation signature ("Prolif") using the following equation:

ROR-P = -0.001*Basal + 0.7*Her2 + -0.95*LumA + 0.49*LumB + 0.34*Prolif, where the variables "Basal," "Her2," "LumA," "LumB" and "Prolif" are as described *supra* and the test expression profiles are compared to centroids constructed using the gene expression data deposited with GEO as accession number GSE2845.

In yet another embodiment, risk score can also be calculated using a combination of breast cancer subtype, proliferation signature and the clinical variable tumor size (T) using the ROR-PT described in conjunction with Table 3 *supra.*

*Detection of Subtypes*

Immunohistochemistry for estrogen (ER), progesterone (PgR), HER2, and Ki67 was performed concurrently on serial sections with the standard streptavidin-biotin complex method with 3,3'-diaminobenzidine as the chromogen. Staining for ER, PgR, and HER2 interpretation can be performed as described previously (Cheang et al., Clin Cancer Res. 2008;14(5): 1368-1376.), however any method known in the art may be used.

For example, a Ki67 antibody (clone SP6; ThermoScientific, Fremont, CA) can be applied at a 1:200 dilution for 32 minutes, by following the Ventana Benchmark automated immunostainer (Ventana, Tucson AZ) standard Cell Conditioner 1 (CC1, a proprietary buffer) protocol at 98°C for 30 minutes. An ER antibody (clone SP1; ThermoFisher Scientific, Fremont CA) can be used at 1:250 dilution with 10-minute incubation, after an 8-minute microwave antigen retrieval in 10 mM sodium citrate (pH 6.0). Ready-to-use PR antibody (clone 1E2; Ventana) can be used by following the CC1 protocol as above. HER2 staining can be done with a SP3 antibody (ThermoFisher Scientific) at a 1:100 dilution after antigen retrieval in 0.05 M Tris buffer (pH 10.0) with heating to 95°C in a steamer for 30 minutes. For HER2 fluorescent *in situ* hybridization (FISH) assay, slides can be hybridized with probes to LSI (locus-specific identifier) HER2/neu and to centromere 17 by use of the PathVysion HER-2 DNA Probe kit (Abbott Molecular, Abbott Park, IL) according to manufacturer's instructions, with modifications to pretreatment and hybridization as previously described (Brown LA, Irving J, Parker R, et al. Amplification of EMSY, a novel oncogene on 11q13, in high grade ovarian surface epithelial carcinomas. Gynecol Oncol. 2006;100(2):264-270). Slides can then be counterstained with 4',6-diamidino-2-phenylindole, stained material was visualized on a Zeiss Axioplan epifluorescent microscope, and signals were analyzed with a Metafer image acquisition system (Metasystems, Altlussheim, Germany). Biomarker expression from immunohistochemistry assays can then be scored by two pathologists, who were blinded to the clinicopathological characteristics and outcome and who used previously established and published criteria for biomarker expression levels that had been developed on other breast cancer cohorts.

Tumors were considered positive for ER or PR if immunostaining was observed in more than 1% of tumor nuclei, as described previously. Tumors were considered positive for HER2 if immunostaining was scored as 3+ according to HercepTest criteria, with an amplification ratio for fluorescent *in situ* hybridization of 2.0 or more being the cut point that was used to segregate immunohistochemistry equivocal tumors (scored as 2+) (Yaziji, et al., JAMA, 291(16):1972-1977 (2004)). Ki67 was visually scored for percentage of tumor cell nuclei with positive immunostaining above the background level by two pathologists.

Other methods can also be used to detect subtypes. These techniques include ELISA, Western blots, Northern blots, or FACS analysis.

*Kits*

The present disclosure also describes kits useful for classifying breast cancer intrinsic subtypes and/or providing prognostic information to identify risk of recurrence These kits comprise a set of capture probes and/or primers specific for the intrinsic genes listed in Table 1. The kit may further comprise a computer readable medium.

In one embodiment of the present disclosure, the capture probes are immobilized on an array. By "array" is intended a solid support or a substrate with peptide or nucleic acid probes attached to the support or substrate. Arrays typically comprise a plurality of different capture probes that are coupled to a surface of a substrate in different, known locations. The arrays of the disclosure comprise a substrate having a plurality of capture probes that can specifically bind an intrinsic gene expression product. The number of capture probes on the substrate varies with the purpose for which the array is intended. The arrays may be low-density arrays or high-density arrays and may contain 4 or more, 8 or more, 12 or more, 16 or more, 32 or more addresses, but will minimally comprise capture probes for the 46 intrinsic genes listed in Table 1.

Techniques for the synthesis of these arrays using mechanical synthesis methods are described in, *e.g.,* U.S. Patent No. 5,384,261. The array may be fabricated on a surface of virtually any shape or even a multiplicity of surfaces. Arrays may be probes (e.g., nucleic-acid binding probes) on beads, gels, polymeric surfaces, fibers such as fiber optics, glass or any other appropriate substrate, see U.S. Pat. Nos. 5,770,358, 5,789,162, 5,708,153, 6,040,193 and 5,800,992. Arrays may be packaged in such a manner as to allow for diagnostics or other manipulation on the device. See, for example, U.S. Pat. Nos. 5,856,174 and 5,922,591.

In another embodiment, the kit comprises a set of oligonucleotide primers sufficient for the detection and/or quantitation of each of the intrinsic genes listed in Table 1. The oligonucleotide primers may be provided in a lyophilized or reconstituted form, or may be provided as a set of nucleotide sequences. In one embodiment, the primers are provided in a microplate format, where each primer set occupies a well (or multiple wells, as in the case of replicates) in the microplate. The microplate may further comprise primers sufficient for the detection of one or more housekeeping genes as discussed *infra.* The kit may further comprise reagents and instructions sufficient for the amplification of expression products from the genes listed in Table 1.

In order to facilitate ready access, *e.g.,* for comparison, review, recovery, and/or modification, the molecular signatures/expression profiles are typically recorded in a database. Most typically, the database is a relational database accessible by a computational device, although other formats, e.g., manually accessible indexed files of expression profiles as photographs, analogue or digital imaging readouts, spreadsheets, *etc.* can be used. Regardless of whether the expression patterns initially recorded are analog or digital in nature, the expression patterns, expression profiles (collective expression patterns), and molecular signatures (correlated expression patterns) are stored digitally and accessed via a database. Typically, the database is compiled and maintained at a central facility, with access being available locally and/or remotely.

*Devices and Tests*

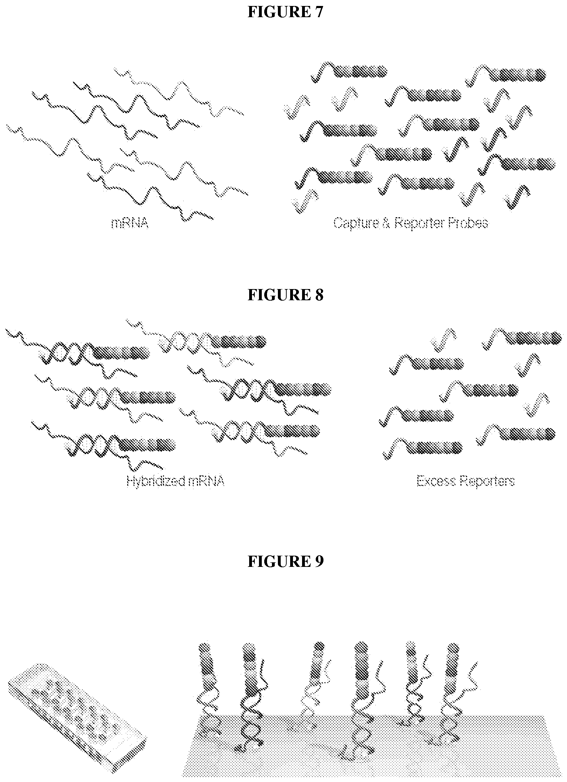

General - The NanoString nCounter Analysis System delivers direct, multiplexed measurements of gene expression through digital readouts of the relative abundance of hundreds of mRNA transcripts. The nCounter Analysis System uses gene-specific probe pairs (**Figure 7**) that are mixed together to form a single reagent called a CodeSet. The probe pairs hybridize directly to the mRNA sample in solution eliminating any enzymatic reactions that might introduce bias in the results.

After hybridization, all of the sample processing steps are automated on the nCounter Prep Station. First, excess capture and reporter probes are removed (**Figure 8**) followed by binding of the probe-target complexes to random locations on the surface of the nCounter cartridge via a streptavidin-biotin linkage (**Figure 9**).

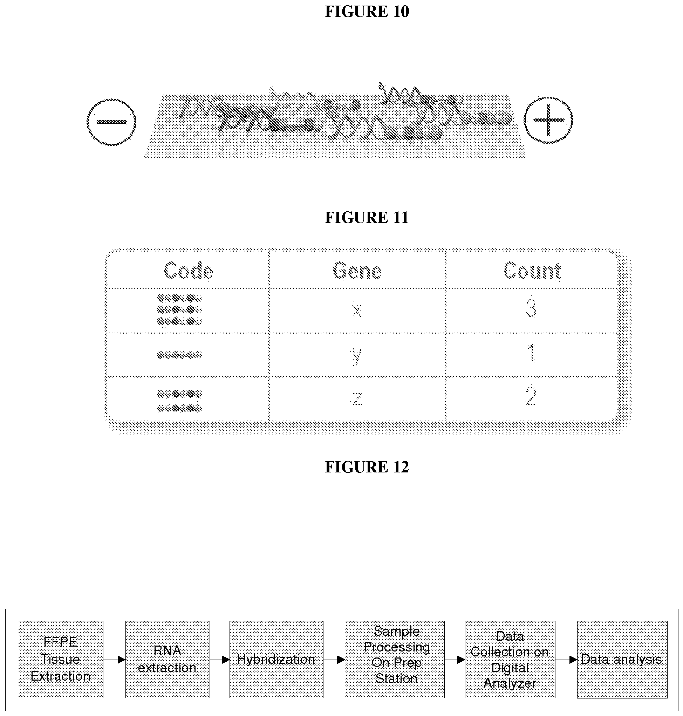

Finally, probe/target complexes are aligned and immobilized (**Figure 10**) in the nCounter Cartridge. The Reporter Probe carries the fluorescent signal; the Capture Probe allows the complex to be immobilized for data collection. Up to 800 pairs of probes, each specific to a particular gene, can be combined with a series of internal controls to form a CodeSet.

After sample processing has completed, cartridges are placed in the nCounter Digital Analyzer for data collection. Each target molecule of interest is identified by the "color code" generated by six ordered fluorescent spots present on the reporter probe. The Reporter Probes on the surface of the cartridge are then counted and tabulated for each target molecule **(Figure 11).**

Reagents and Test Components - The Breast Cancer test will simultaneously measure the expression levels of NANO46 plus eight housekeeping genes in a single hybridization reaction using an nCounter CodeSet designed specifically to those genes. Each assay also includes positive assay controls comprised of a linear titration of *in vitro* transcribed RNA transcripts and corresponding probes, and a set of probes with no sequence homology to element, integer or step, or group of elements, integers or steps.

## EXAMPLES

Example 1. NANO46 Subtyping Test

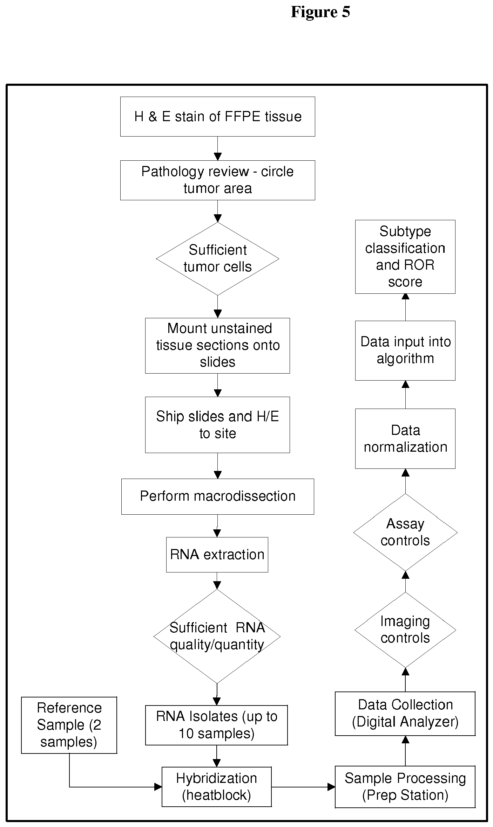

**Figure 5** outlines the assay processes associated with the Breast Cancer Intrinsic Subtyping test. Following RNA isolation, the test will simultaneously measure the expression levels of 46 target genes plus eight housekeeping genes in a single hybridization reaction using an nCounter CodeSet designed specifically to those genes. For example, the housekeeping genes described in U.S. Patent Publication 2008/0032293 can be used for normalization. Exemplary housekeeping genes include MRPL19, PSMC4, SF3A1, PUM1, ACTB, GAPD, GUSB, RPLP0, and TFRC. The housekeeping genes are used to normalize the expression of the tumor sample. Each assay run also includes a reference sample consisting of *in vitro* transcribed RNA's of the 58 targets for normalization purposes.

FFPE Tissue Review/Procurement and RNA Extraction: The Breast Cancer Intrinsic Subtyping Test will use RNA extracted from Formalin-fixed, Paraffin-embedded (FFPE) tissue that has been diagnosed as invasive carcinoma of the breast. A Pathologist reviews an H & E stained slide to identify the tissue area containing sufficient tumor tissue content for the test. Unstained slide mounted tissue sections are processed by macro-dissecting the identified tumor area on each slide to remove any adjacent normal tissue. RNA is then isolated from the tumor tissue, and DNA is removed from the sample.

Assay Setup and Initiation of Hybridization: For each batch of up to 10 RNA samples isolated from a breast tumor, the user will set up a run using the nCounter Analysis x5 system software, which tracks sample processing, reagent lots, and results for each sample. To initiate the assay, the user will pipette the specified amount of RNA into separate tubes within a 12 reaction strip tube and add the CodeSet and hybridization buffer. A reference sample is pipetted into the remaining two tubes with CodeSet and hybridization buffer. The CodeSet consists of probes for each gene that is targeted, additional probes for endogenous "housekeeping" normalization genes and positive and negative controls that are spiked into the assay. The reference sample consists of *in vitro* transcribed RNA for the targeted genes and housekeeping genes. Once the hybridization reagents are added to the respective tubes, the user transfers the strip tube into a heated-lid heatblock for a specified period of time at a set temperature.

Purification and Binding on the Prep Station: Upon completing hybridization, the user will transfer the strip tube containing the set of 10 assays and 2 reference samples onto the measurements in a cuvette-type UV-Vis spectrophotometer; therefore, NanoString's protocol includes a step for quantitating total RNA using a low volume spectrophotometer such as the NanoDrop™ spectrophotometer. NanoString will define performance specifications for the spectrophotometer so that the range of RNA input recommended for the test is above the limit of detection of the low volume spectrophotometer and is reproducibly measurable.

Hybridization - For each set of up to 10 RNA samples, the user will pipette the specified amount of RNA into separate tubes within a 12 reaction strip tube and add the CodeSet and hybridization buffer. A reference sample is pipetted into the remaining two tubes with CodeSet and hybridization buffer. The CodeSet consists of probes for each gene that is targeted, additional probes for endogenous "housekeeping" normalization genes and positive and negative controls. The probes within the CodeSet pertaining to each of these genes within the four groups (target genes, housekeeping genes, and positive and negative controls) are each assigned a unique code and are therefore individually identifiable within each run. The reference sample consists of *in vitro* transcribed RNA for the targeted genes and housekeeping genes. Once the hybridization reagents are added to the respective tubes, the user transfers the strip tube into a heated-lid heatblock for a specified period of time at a set temperature.

Requirement for Heat block with heated lid for hybridization step - The nCounter assay includes an overnight hybridization under isothermal conditions. Because the overnight hybridization is performed in a small volume at elevated temperature, care must be taken to avoid evaporation. Many commercial PCR thermocyclers are equipped with heated lids that will prevent the evaporation of small volumes of liquid. Because the assay does not require any fine control of temperature ramping, any heat block with a programmable heated lid and a block with dimensions that fit the NanoString tubes will work with the NanoString assay. NanoString plans to provide specifications for heat blocks that meet the assay requirements.

Purification and Binding on the Prep Station - Upon completing hybridization, the user will then transfer the strip tube containing the set of 10 assays and 2 reference samples into the nCounter Prep Station along with the required prepackaged reagents and disposables described in **Table 1**. The Prep Plates contain the necessary reagents for purification of excess probes and binding to the cartridge (see section IIIC below for detailed description of purification process). The prep plates are centrifuged in a swinging bucket centrifuge prior to placement on the deck of the Prep Station. An automated purification process then removes excess capture and reporter probe through two successive hybridization-driven magnetic bead capture steps. The nCounter Prep Station then transfers the purified target/probe complexes into an nCounter cartridge for capture to a glass slide. Following completion of the run, the user removes the cartridge from the Prep Station and seals it with an adhesive film.

Imaging and Analysis on the Digital Analyzer - The sealed cartridge is then inserted into the nCounter Digital Analyzer which counts the number of probes captured on the slide for each gene, which corresponds to the amount of target in solution. Automated software then checks thresholds for the housekeeping genes, reference sample, and positive and negative controls to qualify each assay and ensure that the procedure was performed correctly. The housekeeping genes provide a measure of RNA integrity, and the thresholds indicate when a tested RNA sample is too degraded to be analyzed by the test due to improper handling or storage of tissue or RNA (e.g. improper tumor fixation, FFPE block storage, RNA storage, RNA handling introducing RNase). The positive and negative assay controls indicate a failure of the assay process (e.g. error in assay setup such as sample mixing with CodeSet, or sample processing such as temperature). The signals of each sample are next normalized using the housekeeping genes to control for input sample quality. The signals are then normalized to the reference sample within each run to control for run-to-run variations. The resulting normalized data is entered in the Breast Cancer Intrinsic Subtyping algorithm to determine tumor intrinsic subtype, risk of relapse score, and risk classification.

Instrumentation- The nCounter Analysis System is comprised of two instruments, the nCounter Prep Station used for post-hybridization processing, and the Digital Analyzer used for data collection and analysis.

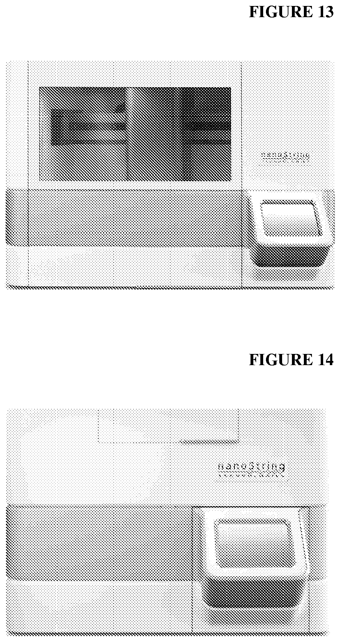

nCounter Prep Station - The nCounter Prep Station (**Figure 13**) is an automated fluid handling robot that processes samples post-hybridization to prepare them for data collection on the nCounter Digital Analyzer. Prior to processing on the Prep Station, total RNA extracted from FFPE (Formalin-Fixed, Paraffin-Embedded) tissue samples is hybridized with the NanoString Reporter Probes and Capture Probes according to the nCounter protocol described above.

Hybridization to the target RNA is driven by excess NanoString probes. To accurately analyze these hybridized molecules they are first purified from the remaining excess probes in the hybridization reaction. The Prep Station isolates the hybridized mRNA molecules from the excess Reporter and Capture probes using two sequential magnetic bead purification steps. These affinity purifications utilize custom oligonucleotide-modified magnetic beads that retain only the tripartite complexes of mRNA molecules that are bound to both a Capture probe and a Reporter probe.

Next, this solution of tripartite complexes is washed through a flow cell in the NanoString sample cartridge. One surface of this flow cell is coated with a polyethylene glycol (PEG) hydrogel that is densely impregnated with covalently bound streptavidin. As the solution passes through the flow cell, the tripartite complexes are bound to the streptavidin in the hydrogel through biotin molecules that are incorporated into each Capture probe. The PEG hydrogel acts not only to provide a streptavidin-dense surface onto which the tripartite complexes can be specifically bound, but also inhibits the non-specific binding of any remaining excess reporter probes.

After the complexes are bound to the flow cell surface, an electric field is applied along the length of each sample cartridge flow cell to facilitate the optical identification and order of the fluorescent spots that make up each reporter probe. Because the reporter probes are charged nucleic acids, the applied voltage imparts a force on them that uniformly stretches and orients them along the electric field. While the voltage is applied, the Prep Station adds an immobilization reagent that locks the reporters in the elongated configuration after the field is removed. Once the reporters are immobilized the cartridge can be transferred to the nCounter Digital Analyzer for data collection. All consumable components and reagents required for sample processing on the Prep Station are provided in the nCounter Master Kit. These reagents are ready to load on the deck of the nCounter Prep Station which can process up to 10 samples and 2 reference samples per run in approximately 2.5 hours.

nCounter Digital Analyzer - The nCounter Digital Analyzer (**Figure 14**) collects data by taking images of the immobilized fluorescent reporters in the sample cartridge with a CCD camera through a microscope objective lens. Because the fluorescent Reporter Probes are small, single molecule barcodes with features of smaller than the wavelength of visible light, the Digital Analyzer uses high magnification, diffraction limited imaging to resolve the sequence of the spots in the fluorescent barcodes.

The Digital Analyzer captures hundreds of consecutive fields-of-view (FOV) that can each contain hundreds or thousands of discrete Reporter Probes. Each FOV is a combination of four monochrome images captured at different wavelengths. The resulting overlay can be thought of as a four-color image in blue, green, yellow, and red. Each 4-color FOV is processed in real time to provide a "count" for each fluorescent barcode in the sample. Because each barcode specifically identifies a single mRNA molecule, the resultant data from the Digital Analyzer is a precise measure of the relative abundance of each mRNA of interest in a biological sample.

Software - The Prep Station and the Digital Analyzer are stand-alone units that do not require connection to an external PC, but must be networked to one another using a Local Area Network (LAN). The nCounter System software securely manages operations through user accounts and permissions. Both instruments use setup and process wizards on an embedded touch screen user interface to guide the user through the sample processing and data collection steps of the assay. The user is led through the procedure by step-by-step instructions on the Prep Station and Digital Analyzer. The instrument touch screen uses a pressure sensitive method for controlling operations and enables the user to interact with the system by touching a selection on the screen. Because the touchscreen provides a limited human interface for data entry, the system also hosts a web-based application for user accounts management, sample batch definition, and sample status tracking.

When samples are processed, the system software tracks the user account and reagent lots for each sample in a centralized data repository. After expression data for a sample is acquired by the Digital Analyzer, it is first analyzed to ensure that all pre-specified quality control metrics are met. The qualified data are then processed through a locked PAM50 algorithm to generate a report containing intrinsic subtype and risk of recurrence (ROR) score. The sample report is transferred to the central repository where it can be securely accessed for download by a user with the correct permissions.

The Breast Cancer Intrinsic Subtyping Algorithm - The nCounter system will be used to identify the intrinsic subtype of an excised invasive carcinoma of the breast using a 50 gene classifier algorithm originally named the PAM50 (Parker J.S., et al. Supervised Risk Predictor of Breast Cancer Based on Intrinsic Subtypes. Journal of Clinical Oncology, 27: 1160-1167 (2009)). The gene expression profile will assign a breast cancer to one of four molecular classes or intrinsic subtypes: Basal-like, Luminal A, Luminal B, and HER2 enriched. A brief description of each subtype is provided below.

Luminal subtypes: The most common subtypes of breast cancer are the luminal subtypes in the hormone-receptor positive population, Luminal A and Luminal B. Prior studies suggest that luminal A comprises approximately 30% to 40% and luminal B approximately 20% of breast cancers[2] and over 90 % of hormone receptor-positive breast cancers. The gene expression pattern of these subtypes resembles the luminal epithelial component of the breast (Nielsen, TO et al. A comparison of PAM50 intrinsic subtyping with immunohistochemistry and clinical prognostic factors in tamoxifen-treated estrogen receptor positive breast cancer. Clinical Cancer Research, 16:5222-5232 (2010)). These tumors are characterized by high expression of estrogen receptor (ER), progesterone receptor (PR), and genes associated with ER activation[2] such as LIV1, GATA3, and cyclin D1, as well as expression of luminal cytokeratins 8 and 18.

Luminal A: Luminal A (LumA) breast cancers exhibit low expression of genes associated with cell cycle activation and the ERBB2 cluster resulting in a better prognosis than luminal B. The Luminal A subgroup has the most favorable prognosis of all subtypes and is enriched for endocrine therapy-responsive tumors.

Luminal B: Luminal B (LumB) breast cancers express ER and ER-associated genes, but to a lower extent than LumA. Genes associated with cell cycle activation are highly expressed and this tumor type can be HER2(+) or HER2(-). The prognosis is unfavorable (despite ER expression) and endocrine therapy responsiveness is generally diminished relative to LumA.

Basal-like: The Basal-like subtype is generally ER-negative, is almost always clinically HER2-negative and expresses a suite of "basal" biomarkers including the basal epithelial cytokeratins (CK) and epidermal growth factor receptor (EGFR). Genes associated with cell cycle activation are highly expressed.

HER2-enriched: The HER2-enriched subtype is generally ER-negative and is HER2-positive in the majority of cases with high expression of the ERBB2 cluster, including ERBB2 and GRB7. Genes associated with cell cycle activation are highly expressed and these tumors have a poor outcome.

Cutoffs for the intrinsic subtyping algorithm are pre-defined from training sets that defined the following: 1) intrinsic subtype centroids (i.e. the prototypical gene expression profile of each subtype), 2) coefficients for Risk of Recurrence (ROR) score, and 3) risk classification (Low/Intermediate/High). The intrinsic subtype centroids (Luminal A, Luminal B, Her2-enriched, Basal-like) were trained using a clinically representative set of archived FFPE breast tumor specimens collected from multiple sites. Hierarchical clustering analysis of gene expression data from the FFPE breast tumor samples was combined with breast tumor biology (i.e. gene expression of previously defined intrinsic subtypes) to define the prototypical expression profile (i.e. centroid) of each subtype. A computational algorithm correlates the normalized 50 gene expression profile of an unknown breast cancer tumor sample to each of the prototypical expression signatures of the four breast cancer intrinsic subtypes. The tumor sample is assigned the subtype with the largest positive correlation to the sample.

304 unique tumor samples with well-defined clinical characteristics and clinical outcome data were used to establish the ROR score. The ROR score is calculated using coefficients from a Cox model that includes the Pearson correlation (R) to each intrinsic subtype, a proliferation score (P), and tumor size (T), as shown in the equation below.

$$ROR = aR_{LumA} + bR_{LumB} + cR_{Her2e} + dR_{basal} + eP + fT$$

To classify tumor samples into specific risk groups (Low Risk/Intermediate Risk/High Risk) based on their calculated ROR score, cutoffs were set based on probability of recurrence free survival in a patient population consisting of hormone receptor positive, post-menopausal patients treated with endocrine therapy alone.

Anticipated Use of NanoString Breast Cancer Test in Clinical Practice - Oncologists currently use a series of tests to develop a treatment protocol for breast cancer patients. Included in these are the IHC / FISH tests such as ER/PR IHC and HER2 IHC / FISH, and the Agendia MammaPrint® assay and the Genomic Health Oncotype Dx® test. These tests offer the oncologist additional information regarding the patient's prognosis and recommended treatment regimens.

These tests, however, have limitations. ER, PgR, and Her2 testing is done locally by pathologists and reference labs, but the challenges with widespread standardization of IHC and FISH testing is well documented (Lester, J et al. Assessment of Tissue Estrogen and Progesterone Receptor Levels: A Survey of Current Practice, Techniques, and Quantitation Methods. The Breast Journal, 6:189-196 (2000); Wolff, A et al. American Society of Clinical Oncology / College of American Pathologists Guideline Recommendations for Human Epidermal Growth Factor Receptor 2 Testing in Breast Cancer. Archives of Pathology and Laboratory Medicine, 131:18-43 (2007)). The MammaPrint test is FDA cleared for use only with frozen or fresh-preserved tissue samples, yet most of the tumor samples collected in the United States are FFPE rather than fresh-frozen. This test is also not distributed and is only available through the Agendia reference labs. The Oncotype Dx test can be used to predict the risk of relapse for stage I/II, node negative, estrogen receptor-positive patients receiving adjuvant Tamoxifen therapy as well as response to cyclophosphamide/methotrexate/5-fluorouracil (CMF) chemotherapy. However this test is only offered as a lab-developed test (LDT) through Genomic Health's CLIA laboratory and is not FDA cleared for prognostic use, or FDA approved for predicting chemotherapy response.

NanoString envisions a model that would have the Breast Cancer test used in conjunction with other sources of clinical data currently available to oncologists for breast cancer prognosis in selected patient segments. The Breast Cancer Test would be an additional source of prognostic information adding significant value to established clinical parameters (i.e tumor size, nodal status) used by oncologists in managing a patient with breast cancer.

*Methods, Assays and Kits*

The methods, assays and kits of the disclosure include a series of quality control metrics that are automatically applied to each sample during analysis. These metrics evaluate the performance of the assay to determine whether the results fall within expected values. Upon successful analysis of these quality control metrics, the Assay gives the following results:

| Result | Output Values |
|---|---|
| The Intrinsic Subtype of the Breast Cancer Specimen | Luminal A<br>Luminal B<br>HER2- Enriched<br>Basal-Like |
| Individual Estimate of the Probability of Distant Recurrence within 10 years | 0-100% |
| Risk of Recurrence (ROR) Score | Integer value on a 0-100 scale |
| Risk Category | Low, Intermediate, High |

Intrinsic Subtypes

The Intrinsic Subtype of a breast cancer tumor has been shown to be related to prognosis in Early Stage Breast Cancer. On average, patients with a Luminal A tumor have significantly better outcomes than patients with Luminal B, HER2-Enriched, or Basal-like tumors.

The Intrinsic Subtype is identified by comparing the gene expression profile of 50 genes in an unknown sample with the expected expression profiles for the four intrinsic subtypes. The subtype with the most similar profile is assigned to the unknown sample.

The most common subtypes of breast cancer are the luminal subtypes, Luminal A (LumA) and Luminal B (LumB). Prior studies suggest that Luminal A comprises approximately 30% to 40% and Luminal B approximately 20% of breast cancers. However, greater than 90% of hormone-receptor positive patients have luminal tumors. The gene expression pattern of these subtypes resembles the luminal epithelial component of the breast tissue. These tumors are characterized by high expression of estrogen receptor (ER), progesterone receptor (PR), and genes associated with ER activation, such as LIV1, GATA3, and cyclin D1, as well as expression of luminal cytokeratins 8 and 18. Luminal A breast cancers exhibit lower expression of genes associated with cell cycle activation when compared to Luminal B breast cancers resulting in a better prognosis.

Prior studies suggest that the HER2-Enriched subtype (Her2E) comprises approximately 20% of breast cancers. However, HER2-Enriched tumors are generally ER-negative, so only 5% of the tested ER-positive patient population was found to have HER2-Enriched breast cancer. Regardless of ER-status, HER2-Enriched tumors are HER2-positive in the majority of cases with high expression of the ERBB2 cluster, including ERBB2 and GRB7. Genes associated with cell cycle activation are also highly expressed.

Published data suggest that the Basal-like subtype comprises approximately 20% of breast cancers. However, Basal-like tumors are generally ER-negative, so only 1% of hormone receptor-positive patients have Basal-like breast cancer. The Basal-like subtype is almost always clinically HER2-negative and expresses a suite of "basal" biomarkers including the basal epithelial cytokeratins (CK) and epidermal growth factor receptor (EGFR). Genes associated with cell cycle activation are highly expressed.

ROR Score

The ROR score is an integer value on a 0-100 scale that is related to an individual patient's probability of distant recurrence within 10 years for the defined intended use population. The ROR score is calculated by comparing the expression profiles of 46 genes in an unknown sample with the expected profiles for the four intrinsic subtypes, as described above, to calculate four different correlation values. These correlation values are then combined with a proliferation score and the tumor size to calculate the ROR score.

Probability of 10-Year Distant Recurrence

The ROR scores for a cohort of post-menopausal women with hormone receptor-positive early stage breast cancer were compared to distant recurrence-free survival following surgery and treatment with 5 years of adjuvant endocrine therapy followed by 5 years of observation. This study resulted in a model relating the ROR score to the probability of distant recurrence in this tested patient population including a 95% confidence interval.

Risk Classification

Risk classification is also provided to allow interpretation of the ROR score by using cutoffs related to clinical outcome in tested patient populations.

**Risk classification by ROR range and nodal status**

| Nodal Status | ROR Range | Risk Classification |
|---|---|---|
| Node- Negative | 0-40 | Low |
| | 41-60 | Intermediate |
| | 61-100 | High |
| Node-Positive (1-3 nodes) | 0-15 | Low |
| | 16-40 | Intermediate |
| | 41-100 | High |

Quality Control

Each lot of the Assay components is tested using predetermined specifications. All kit-level items are lot tracked, and the critical components contained within each kit are tested together and released as a Master Lot.

The assay kit includes a series of internal controls that are used to assess the quality of each run set as a whole and each sample individually. These controls are listed below.

Batch Control Set: *In vitro* transcribed RNA Reference Sample

A synthetic RNA Reference Sample is included as a control within the Assay kit. The reference sample is comprised of in-vitro transcribed RNA targets from the 50 algorithm and 8 housekeeping genes. The Reference Sample is processed in duplicate in each assay run along with a set of up to 10 unknown breast tumor RNA samples in a 12 reaction strip tube. The signal from the Reference Sample is analyzed against pre-defined thresholds to qualify the run.

The signal from each of the 50 algorithm genes of the breast tumor RNA sample is normalized to the corresponding genes of the Reference Sample.

Positive Control Set: *in vitro* transcribed RNA targets and corresponding Capture and Reporter Probes

Synthetic RNA targets are used as positive controls (PCs) for the assay. The PC target sequences are derived from the External RNA Control Consortium (ERCC) DNA sequence library. The RNA targets are in-vitro transcribed from DNA plasmids. Six RNA targets are included within the assay kit in a 4-fold titration series (128 - 0.125 fM final concentration in hybridization reaction) along with the corresponding Capture and Reporter Probes. The PCs are added to each breast tumor RNA sample and Reference RNA Sample tested with the Prosigna Assay. A sample will be disqualified from further analysis if the signal intensities from the PCs do not meet pre-defined thresholds.

Negative control set: exogenous probes without targets

Negative control (NC) target sequences are derived from the ERCC DNA sequence library. The probes designed to detect these target sequences are included as part of the assay kit without the corresponding target sequence. The negative controls (NCs) are added to each breast tumor RNA sample and Reference Sample tested with the Prosigna Assay as a quality control measure. The sample will be disqualified from further analysis if the signal intensities from the NCs do not meet pre-defined thresholds.

RNA Integrity Control Set: Housekeeping genes

Capture and Reporter Probes designed to detect 8 housekeeping genes and 50 algorithm genes are included as part of the kit. The expression levels of the 8 housekeeping genes are analyzed to determine the quality of RNA extracted from the FFPE tissue sample and input into the assay. The sample will be disqualified from further analysis if the expression level of the housekeeping genes falls below pre-defined thresholds.

The housekeeping genes are also used to normalize for any differences in the intact RNA amount in a sample prior to Reference Sample normalization.

*Definitions*

For the purposes of the present disclosure, "breast cancer" includes, for example, those conditions classified by biopsy or histology as malignant pathology. The clinical delineation of breast cancer diagnoses is well known in the medical arts. One of skill in the art will appreciate that breast cancer refers to any malignancy of the breast tissue, including, for example, carcinomas and sarcomas. Particular embodiments of breast cancer include ductal carcinoma *in situ* (DCIS), lobular carcinoma *in situ* (LCIS), or mucinous carcinoma. Breast cancer also refers to infiltrating ductal (IDC) or infiltrating lobular carcinoma (ILC). In most embodiments of the disclosure, the subject of interest is a human patient suspected of or actually diagnosed with breast cancer.

The article "a" and "an" are used herein to refer to one or more than one (*i.e.,* to at least one) of the grammatical object of the article. By way of example, "an element" means one or more element.

Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps.

## EXAMPLES

Example 1. NAN046 Subtyping Test

**Figure** 5 outlines the assay processes associated with the Breast Cancer Intrinsic Subtyping test. Following RNA isolation, the test will simultaneously measure the expression levels of 46 target genes plus eight housekeeping genes in a single hybridization reaction using an nCounter CodeSet designed specifically to those genes. For example, the housekeeping genes described in U.S. Patent Publication 2008/0032293 can be used for normalization. Exemplary housekeeping genes include MRPL19, PSMC4, SF3A1, PUM1, ACTB, GAPD, GUSB, RPLP0, and TFRC. The housekeeping genes are used to normalize the expression of the tumor sample. Each assay run also includes a reference sample consisting of *in vitro* transcribed RNA's of the 58 targets for normalization purposes.

FFPE Tissue Review/Procurement and RNA Extraction: The Breast Cancer Intrinsic Subtyping Test will use RNA extracted from Formalin-fixed, Paraffin-embedded (FFPE) tissue that has been diagnosed as invasive carcinoma of the breast. A Pathologist reviews an H & E stained slide to identify the tissue area containing sufficient tumor tissue content for the test. Unstained slide mounted tissue sections are processed by macro-dissecting the identified tumor area on each slide to remove any adjacent normal tissue. RNA is then isolated from the tumor tissue, and DNA is removed from the sample.

Assay Setup and Initiation of Hybridization: For each batch of up to 10 RNA samples isolated from a breast tumor, the user will set up a run using the nCounter Analysis x5 system software, which tracks sample processing, reagent lots, and results for each sample. To initiate the assay, the user will pipette the specified amount of RNA into separate tubes within a 12 reaction strip tube and add the CodeSet and hybridization buffer. A reference sample is pipetted into the remaining two tubes with CodeSet and hybridization buffer. The CodeSet consists of probes for each gene that is targeted, additional probes for endogenous "housekeeping" normalization genes and positive and negative controls that are spiked into the assay. The reference sample consists of *in vitro* transcribed RNA for the targeted genes and housekeeping genes. Once the hybridization reagents are added to the respective tubes, the user transfers the strip tube into a heated-lid heatblock for a specified period of time at a set temperature.

Purification and Binding on the Prep Station: Upon completing hybridization, the user will transfer the strip tube containing the set of 10 assays and 2 reference samples onto the nCounter Prep Station along with the required prepackaged reagents and disposables. An automated purification process then removes excess capture and reporter probe through two successive hybridization-driven magnetic bead capture steps. The nCounter Prep Station then transfers the purified target/probe complexes into an nCounter cartridge for capture to a glass slide. Following completion of the run, the user removes the cartridge from the Prep Station and seals it with an adhesive film.

Imaging and Analysis on the Digital Analyzer: The cartridge is then sealed and inserted into the nCounter Digital Analyzer which counts the number of probes captured on the slide for each gene, which corresponds to the amount of target in solution. Automated software will then check thresholds for the housekeeping genes, reference sample, and positive and negative controls to qualify each assay and ensure that the procedure was performed correctly. The signals of each sample are next normalized using the housekeeping genes to control for input sample quality. The signals are then normalized to the reference sample within each run to control for run-to-run variations. The resulting normalized data is entered in the Breast Cancer Intrinsic Subtyping algorithm to determine tumor intrinsic subtype and risk of recurrence score.

Example 2: Clinical Validation of the NAN046 risk of recurrence (ROR) score for predicting residual risk of distant-recurrence (DR) after endocrine therapy in postmenopausal women with HR+ early breast cancer (EBC): An ABSCSG Study.

The aim of the study is to assess the performance of the ROR score in predicting distal recurrence for post-menopausal patients with hormone receptor positive early breast cancer (HR+ EBC) treated with tamoxifen or tamoxifen followed by anastrozole when the NANO46 test is performed in a routine hospital pathology lab. Does the ROR score add prognostic information (Distant RFS) beyond the Clinical Treatment Score in all patients (CTS includes: nodes, grade, tumor size, age, treatment)? Do the ROR-based risk groups at prognostic information (Distant RFS) beyond the Clinical Treatment Score in all patients?

Study Overview: 3,714 patients were enrolled in a ABCSG8. Patients were postmenopausal women with HR+ EBC (node negative and note positive), grade one or two, with no prior treatment. 1,671 patients re-consented for long-term follow-up or are deceased. The median follow-up was 11 years. 1,620 FFPE blocks were collected. 25 had insufficient cancer in the block on path review, 73 had insufficient RNA included, 44 failed QC specs for the NanoString device. 1,478 patients (91.2%) passed the NANO46 analysis.

Methods: Three unstained 10 micron sections and 1 H&E slide for each patient was sent to an independent academic pathology laboratory at BCCA where tissue review, manual micro-dissection and RNA extraction were performed. NANO46 analysis was then conducted on 250 ng of the extracted RNA using the NanoString nCounter Analysis System; both intrinsic subtype and ROR score were calculated.

Results: The ROR Score adds statistically significant prognostic information (Distant RFS) beyond CTS in all patients (Likelihood ratio test $\Delta LR\chi^2 = 53.5$, p < 0.0001). The ROR-based risk groups add statistically significant prognostic information (Distant RFS) beyond CTS in all patients (Likelihood ratio test $\Delta LR\chi^2 = 34.1$, p < 0.0001). Differentiation between Luminal A and Luminal B adds statistically significant prognostic information (Distant RFS) beyond CTS in all patients (Luminal B vs. A: HR=2.38, 95% CI; 1.69-3.35, p < 0.0001). Results in the node-negative and node-positive subgroups are similar to the results for all patients that are reported in the study.

Conclusions: The results show that both the ROR score and the ROR-based risk groups add statistically significant prognostic information beyond the Clinical Treatment Score. The results demonstrate that a complex, multi-gene-expression test can be performed in a hospital pathology laboratory and meet the same quality metrics as a central reference laboratory. The results of the TransATAC and ABCSG8 studies together provide Level 1 evidence for the clinical validity of the NANO46 test for predicting the risk of distant recurrence in postmenopausal women with HR+ EBC treated with endocrine therapy alone. The results also show that Luminal A subtypes have better outcomes than Luminal B subtypes in postmenopausal women with HR+ EBC treated with endocrine therapy alone.

**Claims**

1. A method of determining low or high risk of recurrence (ROR) in a subject having breast cancer comprising:

determining the size of the breast cancer tumor from the subject;
determining a correlation value of a breast cancer tumor for each of at least four intrinsic subtypes including a Basal-like, Luminal A, Luminal B, or HER-2 enriched, by measuring the RNA expression of at least 40 of the genes in the NANO46 intrinsic gene list of Table 1;
determining a proliferation score by measuring the RNA expression of each of the 18-gene subset of the NANO46 intrinsic genes selected from ANLN, CCNE1, CDC20, CDC6, CDCA1, CENPF, CEP55, EXO1, KIF2C, KNTC2, MELK, MKI67, ORC6L, PTTG1, RRM2, TYMS, UBE2C, and UBE2T;
calculating a risk of recurrence (ROR) score using the following equation: ROR-PT = - 0.0067*Basal + 0.4317*Her2 + -0.3172*LumA + 0.4894*LumB + 0.1981*ProliferationScore + 0.1133*TumorSize; wherein the risk of recurrence score is an integer value on a 0-100 scale, wherein the level of RNA expression for at least 40 of the genes in the NANO46 intrinsic gene list of Table 1 is set forth in Table 3;
thereby determining whether the subject has a low or high risk of recurrence based on the risk of recurrence (ROR) score and interpretation of the ROR score using the risk classification by risk of recurrence (ROR) range and nodal status.

2. The method of claim 1, further comprising determining at least one of the following: tumor grade, tumor ploidy, estrogen receptor expression, progesterone receptor expression, and HER2/ERBB2 expression.

3. The method of claim 1, further comprising determining each of the following: tumor grade, tumor ploidy, estrogen receptor expression, progesterone receptor expression, and HER2/ERBB2 expression.

4. The method of claim 1, wherein the risk is breast cancer specific survival, event-free survival, or response to therapy.

5. The method of claim 1, wherein the RNA expression of the members of the NANO46 intrinsic gene list is determined using the nanoreporter code system (nCounter® Analysis system).

**Patentansprüche**

1. Verfahren zur Bestimmung eines niedrigen oder hohen Risikos für das Wiederauftreten (Risk of Recurrence; ROR) bei einer Patientin mit Brustkrebs, das Folgendes umfasst:

Bestimmen der Größe des Brustkrebstumors bei der Patientin;
Bestimmen eines Korrelationswerts von einem Brustkrebstumor für jeden von mindestens vier intrinsischen Subtypen, einschließlich eines basal-ähnlichen, Luminal A, Luminal B oder HER-2 angereicherten, durch Messen der RNA-Expression von mindestens 40 der Gene in der intrinsischen NANO46-Genliste in Tabelle 1;
Bestimmen eines Proliferationsscores durch Messen der RNA-Expression von jedem des 18-Gen-Subsets von den intrinsischen NANO46-Genen, ausgewählt aus ANLN, CCNE1, CDC20, CDC6, CDCA1, CENPF, CEP55, EXO1, KIF2C, KNTC2, MELK, MKI67, ORC6L, PTTG1, RRM2, TYMS, UBE2C und UBE2T;
Berechnen eines Risk-of-Recurrence-Scores (ROR-Scores) mittels der folgenden Gleichung: ROR-PT = -0,0067*Basal + 0,4317*Her2 + -0,3172*LumA + 0,4894*LumB + 0,1981*Proliferationsscore + 0,1133*Tumorgröße; wobei der Risk-of-Recurrence-Score ein Ganzzahlenwert auf einer Skala von 0 bis 100 ist, wobei der RNA-Expressionsspiegel für mindestens 40 der Gene in der intrinsischen NANO46-Genliste von Tabelle 1 in Tabelle 3 dargelegt ist;
damit Bestimmen, ob die Patientin ein niedriges oder hohes Risiko für das Wiederauftreten bezogen auf den Risk-of-Recurrence-Score (ROR-Score) aufweist und Interpretation des ROR-Scores mithilfe der Risiko-Klassifikation mittels des Risk-of-Recurrence-Bereichs (ROR-Bereichs) und des Nodalstatus.

2. Verfahren nach Anspruch 1, weiter umfassend das Bestimmen mindestens eines des Folgenden: Tumorgrad, Tumorploidie, Expression von Östrogenrezeptoren, Expression von Progesteronrezeptoren und Expression von HER2/ERBB2.

3. Verfahren nach Anspruch 1, weiter umfassend das Bestimmen jedes des Folgenden: Tumorgrad, Tumorploidie, Expression von Östrogenrezeptoren, Expression von Progesteronrezeptoren und Expression von HER2/ERBB2.

4. Verfahren nach Anspruch 1, wobei das Risiko das brustkrebsspezifische Überleben, das ereignisfreie Überleben oder das Ansprechen auf eine Therapie beinhaltet.

5. Verfahren nach Anspruch 1, wobei die RNA-Expression der Mitglieder der intrinsischen NANO46-Genliste mittels des Nanoreporter-Codesystems (nCounter® Analysensystems) bestimmt wird.

**Revendications**

1. Procédé permettant déterminer un risque de récidive (RDR) faible ou élevé chez un sujet atteint d'un cancer du sein, comprenant les étapes qui consistent à :

déterminer la taille de la tumeur cancéreuse du sein du sujet ;
déterminer une valeur de corrélation d'une tumeur cancéreuse du sein pour chacun de quatre au moins soustypes intrinsèques, y compris Basal-like, Luminal A, Luminal B ou HER-2 enrichi, en mesurant l'expression de l'ARN de 40 au moins des gènes répertoriés dans la liste des gènes intrinsèques NANO46 du Tableau 1 ;

déterminer un score de prolifération en mesurant l'expression de l'ARN de chacun des 18 gènes d'un sous-groupe de gènes intrinsèques NANO46 sélectionnés parmi les suivants : ANLN, CCNE1, CDC20, CDC6, CDCA1, CENPF, CEP55, EXO1, KIF2C, KNTC2, MELK, MKI67, ORC6L, PTTG1, RRM2, TYMS, UBE2C et UBE2T ;

calculer un score du risque de récidive (RDR) en utilisant l'équation suivante : RDR-PT = -0,0067*Basal + 0,4317*Her2 + -0,3172*LumA + 0,4894* LumB + 0,1981*Score de prolifération + 0,1133*Taille de la tumeur ; où le score du risque de récidive est un nombre entier sur une échelle 0-100, où le niveau d'expression de l'ARN pour 40 au moins des gènes de la liste de gènes intrinsèques NANO46 du Tableau 1 est indiqué au Tableau 3 ;

déterminer ainsi si le sujet a un risque de récidive faible ou élevé sur la base du score du risque de récidive (RDR) et l'interprétation du score du RDR en utilisant la classification du risque par plage de risque de récidive (RDR) et état ganglionnaire.

2. Procédé de la revendication 1, comprenant en outre une détermination de un au moins des suivants : grade de la tumeur, ploïdie de la tumeur, expression du récepteur aux œstrogènes, expression du récepteur à la progestérone et expression de HER2/ERBB2.

3. Procédé de la revendication 1, comprenant en outre une détermination de chacun des suivants : grade de la tumeur, ploïdie de la tumeur, expression du récepteur aux œstrogènes, expression du récepteur à la progestérone et expression de HER2/ERBB2.

4. Procédé de la revendication 1, où le risque est la survie spécifique au cancer du sein, la survie sans événement ou la réponse à un traitement.

5. Procédé de la revendication 1, où l'expression de l'ARN des membres de la liste de gènes intrinsèques NANO46 est déterminée en utilisant le système de code associé à des nanorapporteurs (système d'analyse nCounter®).

## Figure 1

**Figure 2**

**Figure 3**

**Figure 4**

**Figure 5**

**Figure 6**

Reference Sample

Test Sample

Compute geometric mean of 8 housekeeping genes

Divide gene counts for each of the 50 genes by the housekeeper geomean

Divide test sample normalized gene counts by ref. sample normalized gene counts for each of the 50 genes

Log2 transform the data

Subtract scaling factor 1 from normalized gene expression measurements and then divide by scaling factor 2

Calculate Pearson's correlation of normalized and scaled test sample data to normalized and scaled centroids

Assign sample to subtype with highest correlation

Calculate Pearson's correlation of normalized and scaled test sample data to normalized and scaled centroids for 46 genes

Compute the arithmetic mean of the normalized and scaled expression measurements of the proliferation genes

Convert tumor size to the following categorical values (1 for < 2 cm, 2 for > 2 cm)

Compute risk score based on weighted sum of subtype correlations, proliferation measure, and tumor size

Adjust risk score to a 0-100 scale

## FIGURE 7

mRNA

Capture & Reporter Probes

## FIGURE 8

Hybridized mRNA

Excess Reporters

## FIGURE 9

## FIGURE 10

## FIGURE 11

| Code | Gene | Count |
|------|------|-------|
|  | x | 3 |
|  | y | 1 |
|  | z | 2 |

## FIGURE 12

**FIGURE 13**

**FIGURE 14**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009158143 A
- EP 1641810 A2
- WO 2007061876 A
- US 20090299640 A
- US 4843155 A, Chomczynski
- US 4683202 A
- US 5854033 A
- US 6040138 A
- US 5800992 A
- US 6020135 A
- US 6033860 A
- US 6344316 B
- WO 08124847 A
- US 8415102 B
- WO 07076129 A
- WO 07076132 A
- US 20100015607 A
- US 20100261026 A
- WO 2010019826 A
- US 20100047924 A
- US 20080032293 A
- US 5384261 A
- US 5770358 A
- US 5789162 A
- US 5708153 A
- US 6040193 A
- US 5856174 A
- US 5922591 A

**Non-patent literature cited in the description**

- Supervised risk predictor of breast cancer based on intrinsic subtypes. **PARKER JOEL S et al.** J. of Clinical Oncology. Americal Soc. of Clinical Oncology, 10 March 2019, vol. 27, 1160-1167
- **PEROU et al.** *Nature,* 2000, vol. 406 (6797), 747-52
- **SORLIE et al.** *PNAS,* 2001, vol. 98 (19), 10869-74
- **NIELSEN et al.** *Clin. Cancer Res.,* 2009, vol. 16 (21), 5222-5232
- Diseases of the breast. **LISA CAREY ; CHARLES PEROU et al.** Gene Arrays, Prognosis, and Therapeutic Interventions. Lippincott Williams & Wilkins, 2009, 458-472
- **NARASHIMAN ; CHU.** *PNAS,* 2002, vol. 99, 6567-6572
- **WEIS et al.** *TIG,* 1992, vol. 8, 263-64
- **SCHENA et al.** *Science,* 1995, vol. 270, 467-70
- Current Protocols in Molecular Biology. John Wiley & Sons, 1987
- **RUPP ; LOCKER.** *Lab Invest.,* 1987, vol. 56, A67
- **DE ANDRES et al.** *Biotechniques,* 1995, vol. 18, 42-44
- **BARANY.** *PNAS USA,* 1991, vol. 88, 189-93
- **GUATELLI et al.** *Proc. Natl. Acad. Sci USA,* 1990, vol. 87, 1874-78
- **KWOH et al.** *Proc. Natl. Acad. ScL USA,* 1989, vol. 86, 1173-77
- **LIZARDI et al.** *Bio/Technology,* 1988, vol. 6, 1197
- **GEISS et al.** *Nature Biotechnology,* 2008, vol. 26 (3), 317-325
- **QUACKENBUSH.** *Nat. Genet.,* 2002, vol. 32, 496-501
- **BENITO et al.** *Bioinformatics,* 2004, vol. 20 (1), 105-114
- **ROUZIER et al.** *J Clin Oncol,* 2005, vol. 23, 8331-9
- **SPRUANCE et al.** *Antimicrob. Agents & Chemo.,* 2004, vol. 48, 2787-92
- **CHEANG et al.** *Clin Cancer Res.,* 2008, vol. 14 (5), 1368-1376
- **BROWN LA ; IRVING J ; PARKER R et al.** Amplification of EMSY, a novel oncogene on 11q13, in high grade ovarian surface epithelial carcinomas. *Gynecol Oncol.,* 2006, vol. 100 (2), 264-270
- **YAZIJI et al.** *JAMA,* 2004, vol. 291 (16), 1972-1977
- **PARKER J.S. et al.** Supervised Risk Predictor of Breast Cancer Based on Intrinsic Subtypes. *Journal of Clinical Oncology,* 2009, vol. 27, 1160-1167
- **NIELSEN, TO et al.** A comparison of PAM50 intrinsic subtyping with immunohistochemistry and clinical prognostic factors in tamoxifen-treated estrogen receptor positive breast cancer. *Clinical Cancer Research,* 2010, vol. 16, 5222-5232

- **LESTER, J et al.** Assessment of Tissue Estrogen and Progesterone Receptor Levels: A Survey of Current Practice, Techniques, and Quantitation Methods. *The Breast Journal,* 2000, vol. 6, 189-196

- **WOLFF, A et al.** American Society of Clinical Oncology / College of American Pathologists Guideline Recommendations for Human Epidermal Growth Factor Receptor 2 Testing in Breast Cancer. *Archives of Pathology and Laboratory Medicine,* 2007, vol. 131, 18-43